# EUROPEAN PATENT SPECIFICATION

(11) **EP 4 076 466 B1**
(45) Date of publication and mention of the grant of the patent: **11.03.2026**
(21) Application number: 20903959.3
(22) Date of filing: 18.12.2020
(51) Int. Cl.: A61K 31/519, A61P 35/00

(54) **MODULATORS OF CULLIN 3 ADAPTOR KBTBD4 AS ANTI-CANCER COMPOUNDS**
MODULATOREN VON CULLIN-3-ADAPTER KBTBD4 ALS VERBINDUNGEN GEGEN KREBS
MODULATEURS DE L'ADAPTATEUR DE CULLIN 3 KBTBD4 EN TANT QUE COMPOSÉS ANTICANCÉREUX

(30) Priority: 18.12.2019 US 201962949678 P
(43) Date of publication of application: 26.10.2022
(73) Proprietor: UNIVERSITE DE MONTREAL, Montréal, Québec H3T 1J4 (CA)
(72) Inventor: SAUVAGEAU, Guy, Montréal, Québec H3Y 2B9 (CA); CHAGRAOUI, Jalila, Montréal, Québec H3C 3J7 (CA); FORTIER, Simon, Québec J0L 2H0 (CA); GIRARD, Simon, Longueuil, Québec J4L 3A6 (CA); MARINIER, Anne, Kirkland, Québec H9J 3E9 (CA)
(74) Representative: Regimbeau
(86) International application number: PCT/CA2020/051755
(87) International publication number: WO 2021/119834

(56) References cited:
- WO-A1-2004/058764
- WO-A1-2017/066530
- WO-A1-2017/161001
- WO-A1-2019/161494
- CA-A1- 2 604 284
- CA-A1- 2 673 038
- CA-A1- 2 673 384
- CA-A1- 2 940 554
- CA-A1- 2 946 446
- FR-A1- 2 876 377
- US-A1- 2014 343 051
- US-A1- 2019 093 081
- US-B2- 7 547 706
- US-B2- 9 409 906

## Description

### TECHNICAL FIELD

It is provided the use of Pyrimido[4,5-B]indole derivatives as anti-cancer compounds.

### BACKGROUND

Humans suffer from various types of cancer, including lymphoma and leukemia, which are very aggressive tumors and result in high mortality rates. In the majority of the cases, the currently existing treatment modalities (chemotherapy, radiotherapy, surgery, certain additional anticancer drugs and bone marrow transplantation) are far from satisfactory, and only a relatively small proportion of for example lymphoma and leukemia patients can survive for many years.

The ubiquitin-proteasome system (UPS) promotes the timely degradation of short-lived proteins with key regulatory roles in cell cycle progression, oncogenesis and genome integrity. Abnormal regulation of UPS disrupts the protein homeostasis and causes many human diseases, particularly cancer. Bortezomib is a FDA therapeutic proteasome inhibitor approved drug for the treatment of relapsed multiple myeloma and mantle cell lymphoma. Accordingly, modulators of the ubiquitin-proteasome system are anti-cancer targets. The normal cell toxicity associated with bortezomib, resulting from global inhibition of protein degradation, promotes the focus of drug discovery efforts on targeting enzymes upstream of the proteasome for better specificity. E3 ubiquitin ligases, particularly those known to be activated in human cancer, become an attractive choice. Cullin-RING Ligases (CRLs) with multiple components are the largest family of E3 ubiquitin ligases and are responsible for ubiquitination of ~20% of cellular proteins degraded through the ubiquitin-proteasome system (Zhao and Sun, 2013, Curr Pharm Des, 19: 3215-3225).

Pevonedistat (MLN4924), currently ongoing clinical trials, is a selective inhibitor of NEDD8. The inhibition of NEDD8-activating enzyme (NAE) prevents activation of cullin-RING ligases (CRLs), which are critical for proteasome-mediated protein degradation CRLs.

Indisulam is an anti-cancer agent mediating the interaction between RBM39 (a splicing factor) and the E3 ligase DCAF15 leading to RBM39 poly-ubiquitination and proteasomal degradation which acts as a molecular glue degrader that binds to DCAF15, creating a novel ligase surface that enhances RBM39 binding (Bussière et al., 2019, bioRxiv, 737510).

Thus, there is still a need to be provided with novel drugs and/or treatment alternatives that can kill selectively cancer cells.

### SUMMARY

The invention is set out in the appended set of claims. Any embodiment not falling under the scope of the appended claims does not form part of the invention.

Any references in the description to methods of treatment by therapy refer to the compounds, pharmaceutical compositions and medicaments of the present invention for use in those methods.

It is provided a method of treating cancer in a patient comprising the step of administering to said patient at least one compound of formula I: 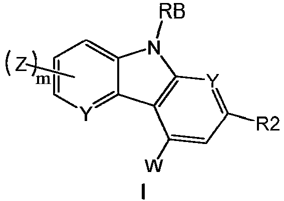
or a salt or a prodrug thereof,
wherein:
   each Y is independently selected from N and CH;
   m is an integer from 0 to 3 (or 0 to 4 when Y is CH in the ring comprising substituent Z);
   Z is each time independently selected from:

      -CN

      -C(O)OR1,

      -C(O)N(R1)R3,

      -C(O)R1,

      or
      -heteroaryl optionally substituted with one or more RA or R4 substituents,
      -aryl optionally substituted with one or more RA or R4 substituents,
   wherein, when (R1) and R3 are attached to a nitrogen atom, optionally they join together with the nitrogen atom to form a 3 to 7-membered ring which optionally includes one or more other heteroatom selected from N, O and S, optionally the ring is substituted with one or more RA or R4;
   W is

      -CN,

      -N(R1)R3,

      -C(O)OR1,

      -C(O)N(R1)R3,

      -NR1C(O)R1,

      -NR1C(O)OR1,

      -OC(O)N(R1)R3,

      -OC(O)R1,

      -C(O)R1,

      -NR1C(O)N(R1)R3,

      -NR1S(O)₂R1,

      -benzyl optionally substituted with 1, 2 or 3 RA or R1 substituents,

         -X-L-(X-L)n - N(R1)R3,
      -X-L-(X-L)n - heteroaryl optionally substituted with one or more RA or R4 substituents attached on either or both the L and heteroaryl groups,
      -X-L-(X-L)n - heterocyclyl optionally substituted with one or more RA or R4 substituents attached on either or both the L and heterocyclyl groups,
      -X-L-(X-L)n- aryl optionally substituted with one or more RA or R4 substituents,

         -X-L-(X-L)ₙ-NR1RA,

         -(N(R1)-L)ₙ - N⁺R1R3R5 R6⁻

         or
      - halogen;
   wherein n is an integer equal to either 0, 1, 2, 3, 4, or 5,
   and wherein, when R1 and R3 are attached to a nitrogen atom, optionally they join together with the nitrogen atom to form a 3 to 7-membered ring which optionally includes one or more other heteroatom selected from N, O and S, optionally the ring is substituted with one or more RA or R4;
   each X is independently selected from CH₂, O, S and NR1;
   each L is independently

      -C₁₋₆ alkylene,

      -C₂₋₆ alkenylene,

      -C₂₋₆ alkynylene,

      -C₃₋₇cycloalkylene, which optionally includes one or more other heteroatom selected from N, O and S or
      -C₃₋₇ cycloalkenylene, which optionally includes one or more other heteroatom selected from N, O and S
   wherein the alkylene, the alkenylene, the alkynylene the cycloalkylene and the cycloalkenylene groups are each independently optionally substituted with one or two R4 or RA substituent;
   R1 is each independently

      -H,

      -C₁₋₆ alkyl,

      -C₂₋₆ alkenyl,

      -C₂₋₆ alkynyl,

      -C₃₋₇ cycloalkyl,

      -C₃₋₇ cycloalkenyl,

      -C₁₋₅ perfluorinated alkyl,

      -heterocyclyl,

      -aryl,

      -heteroaryl,or

      -benzyl,
   wherein the alkyl, the alkenyl, the alkynyl, the cycloalkenyl, the perfluorinated alkyl, the heterocyclyl, the aryl, the heteroaryl and the benzyl groups are each independently optionally substituted with 1, 2 or 3 RA or Rd substituents;
   R2 is
      -H,
      -C₁₋₆ alkyl, optionally substituted with one more RA substituents,
      -C(O)R4,
      -L-heteroaryl optionally substituted with one or more RA or R4 substituents,
      -L-heterocyclyl optionally substituted with one or more RA or R4, or
      -L-aryl optionally substituted with one or more RA or R4 substituents,
      -N(R1)aryl optionally substituted with one or more RA or R4 substituents
   R3 is each independently

      -H,

      -C₁₋₆ alkyl,

      -C₂₋₆ alkenyl,

      -C₂₋₆ alkynyl,

      -C₃₋₇ cycloalkyl,

      -C₃₋₇ cycloalkenyl,

      -C₁₋₅ perfluorinated alkyl,

      -heterocyclyl,

      -aryl,

      -heteroaryl,

      -benzyl,

      or

      methyl 2-benzyl-9H-pyrido[2',3':4,5]pyrrolo[2,3-d]pyrimidine-7-carboxylate-4- yl,
   wherein the alkyl, the alkenyl, the alkynyl, the cycloalkyl, the cycloalkenyl, the perfluorinated alkyl, the heterocyclyl, the aryl, the heteroaryl and the benzyl groups are each independently optionally substituted with 1, 2 or 3 RA or Rd substituents;
   R4 is each independently

      -H,

      -C₁₋₆ alkyl,

      -C₁₋₆ haloalkyl,

      -C₂₋₆ alkenyl,

      -C₂₋₆ alkynyl,

      -C₃₋₇ cycloalkyl,

      -C₃₋₇ cycloalkenyl,

      -C₁₋₅ perfluorinated alkyl,

      -heterocyclyl,

      -aryl,

      -heteroaryl, or

      -benzyl,
   wherein the alkyl, the alkenyl, the alkynyl, the cycloalkyl, the cycloalkenyl, the perfluorinated alkyl, the heterocyclyl, the aryl, the heteroaryl and the benzyl groups are each independently optionally substituted with 1, 2 or 3 RA or Rd substituents;
   R5 is each independently
      -C₁₋₆ alkyl,
      -C₁₋₆ alkylene-C₂₋₆ alkenyl which optionally includes one or more other heteroatom selected from N, O and S
      -C₁₋₆ alkylene-C₂₋₆ alkynyl which optionally includes one or more other heteroatom selected from N, O and S
      -L-aryl which optionally includes one or more RA or R4 substituents
      -L-heteroaryl which optionally includes one or more RA or R4 substituents
      -C₁₋₆ alkylene-C(O)O-
      -C₁₋₆ alkylene-C(O)OR1
      -C₁₋₆ alkylene-CN
      -C₁₋₆ alkylene-C(O)NR1R3, wherein R1 and R3 optionally they join together with the nitrogen atom to form a 3 to 7-membered ring which optionally includes one or more other heteroatom selected from N, O and S; or
      -C₁₋₆ alkylene-OH;
   R6 is

      -Halogen

      -OC(O)CF₃

      or

      -OC(O)R1;
   RA is each independently

      -halogen,

      -CF₃,

      -OR1,

      -L-OR1,

      -OCF₃,

      -SR1,

      -CN,

      -NO₂,

      -NR1R3,

      -L-NR1R1,

      -C(O)OR1,

      -S(O)₂R4

      -C(O)N(R1)R3,

      -NR1C(O)R1,

      -NR1C(O)OR1,

      -OC(O)N(R1)R3,

      -OC(O)R1,

      -C(O)R4,

      -NHC(O)N(R1)R3,

      -NR1C(O)N(R1)R3,

      -N₃;

      or

      -(CH₂CH₂O)₂-CH₂CH₂OH;

      wherein R1 and R3 optionally they join together with the nitrogen atom to form a 3 to 7-membered ring which optionally includes one or more other heteroatom selected from N, O and S; and
   Rd is each independently

      -H,

      -C₁₋₆ alkyl,

      -C₂₋₆ alkenyl,

      -C₂₋₆ alkynyl,

      -C₃₋₇ cycloalkyl,

      -C₃₋₇ cycloalkenyl,

      -C₁₋₅ perfluorinated alkyl

      -benzyl or

      -heterocyclyl;
   RB is

      -H,

      or

      -C₁₋₆ alkyl;
   optionally together with at least one cell expanding factor.

In an embodiment, the compound of formula I is 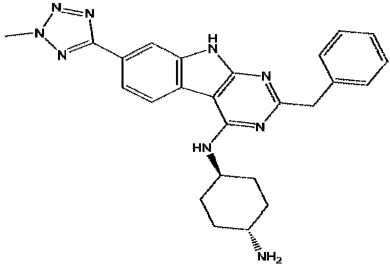 or a pharmaceutically acceptable salt thereof.

In another embodiment, the compound of formula I is a hydrobromide salt of

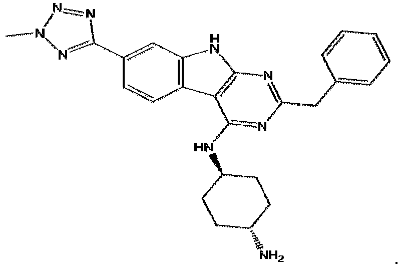

In a further embodiment, the compound of formula I is 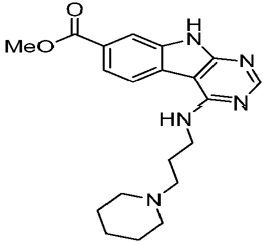 or a pharmaceutically acceptable salt thereof.

In an embodiment, the compound of formula I is as defined herein, especially as defined in Table 3.

In an additional embodiment, the patient is a human or an animal.

In a further embodiment, the animal is a mouse.

In an embodiment, the cancer is a cancer based on K27 mutation, EZH2 or PRC2 mutation.

In an embodiment, the compound is formulated for an administration orally, intramuscularly, intravenously or subcutaneously.

In a further embodiment, the compound degrades at least one of LSD1, RCOR1, HDAC2 and CoREST.

In another embodiment it is provided a method of inhibiting proliferation of cancerous cells *ex vivo* comprising administering the compound described herein into the medium containing the cells in proliferation, wherein the compound has anantineoplastic on the proliferation of the cancerous cells.

The present invention is directed to a compound of formula I: 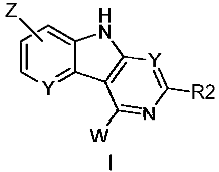
or a salt thereof,
wherein:
   each Y is independently selected from N and CH;
   Z is

      -CN

      -C(O)OR1,

      -C(O)N(R1)R3,

      -C(O)R1,

      or -heteroaryl optionally substituted with one or more RA or R4 substituents,
   wherein, when (R1) and R3 are attached to a nitrogen atom, optionally they join together with the nitrogen atom to form a 3 to 7-membered ring which optionally includes one or more other heteroatom selected from N, O and S, optionally the ring is substituted with one or more RA or R4;
   W is

      -CN,

      -N(R1)R3,

      -C(O)OR1,

      -C(O)N(R1)R3,

      -NR1C(O)R1,

      -NR1C(O)OR1,

      -OC(O)N(R1)R3,

      -OC(O)R1,

      -C(O)R1,

      -NR1C(O)N(R1)R3,

      -NR1S(O)₂R1,

      -benzyl optionally substituted with 1, 2 or 3 RA or R1 substituents,

         -X-L-(X-L)n - N(R1)R3,
      -X-L-(X-L)n - heteroaryl optionally substituted with one or more RA or R4 substituents attached on either or both the L and heteroaryl groups,
      -X-L-(X-L)n - heterocyclyl optionally substituted with one or more RA or R4 substituents attached on either or both the L and heterocyclyl groups,
      -X-L-(X-L)n- aryl optionally substituted with one or more RA or R4 substituents,

         -X-L-(X-L)ₙ-NR1RA

         or

         -(N(R1)-L)ₙ-N⁺R1R3R5 R6⁻
   wherein n is an integer equal to either 0, 1, 2, 3, 4, or 5,
   and wherein, when R1 and R3 are attached to a nitrogen atom, optionally they join together with the nitrogen atom to form a 3 to 7-membered ring which optionally includes one or more other heteroatom selected from N, O and S, optionally the ring is substituted with one or more RA or R4;
   each X is independently selected from C, O, S, and NR1;
   each L is independently

      -C₁₋₆ alkylene,

      -C₂₋₆ alkenylene,

      -C₂₋₆ alkynylene,

      -C₃₋₇cycloalkylene, which optionally includes one or more other heteroatom selected from N, O and S or
      -C₃₋₇ cycloalkenylene, which optionally includes one or more other heteroatom selected from N, O and S
   wherein the alkylene, the alkenylene, the alkynylene the cycloalkylene and the cycloalkenylene groups are each independently optionally substituted with one or two R4 or RA substituent;
   R1 is each independently

      -H,

      -C₁₋₆ alkyl,

      -C₂₋₆ alkenyl,

      -C₂₋₆ alkynyl,

      -C₃₋₇ cycloalkyl,

      -C₃₋₇ cycloalkenyl,

      -C₁₋₅ perfluorinated alkyl,

      -heterocyclyl,

      -aryl,

      -heteroaryl,

      or

      -benzyl,
   wherein the alkyl, the alkenyl, the alkynyl, the cycloalkenyl, the perfluorinated alkyl, the heterocyclyl, the aryl, the heteroaryl and the benzyl groups are each independently optionally substituted with 1, 2 or 3 RA or Rd substituents;
   R2 is
      -H,
      -C₁₋₆ alkyl, optionally substituted with one more RA substituents
      -C(O)R4,
      -L-heteroaryl optionally substituted with one or more RA or R4 substituents
      -L-heterocyclyl optionally substituted with one or more RA or R4, or
      -L-aryl optionally substituted with one or more RA or R4 substituents;
   R3 is each independently

      -H,

      -C₁₋₆ alkyl,

      -C₂₋₆ alkenyl,

      -C₂₋₆ alkynyl,

      -C₃₋₇ cycloalkyl,

      -C₃₋₇ cycloalkenyl,

      -C₁₋₅ perfluorinated alkyl,

      -heterocyclyl,

      -aryl,

      -heteroaryl

      ,or

      -benzyl,
   wherein the alkyl, the alkenyl, the alkynyl, the cycloalkyl, the cycloalkenyl, the perfluorinated alkyl, the heterocyclyl, the aryl, the heteroaryl and the benzyl groups are each independently optionally substituted with 1, 2 or 3 RA or Rd substituents;
   R4 is each independently

      -H,

      -C₁₋₆ alkyl,

      -C₂₋₆ alkenyl,

      -C₂₋₆ alkynyl,

      -C₃₋₇ cycloalkyl,

      -C₃₋₇ cycloalkenyl,

      -C₁₋₅ perfluorinated alkyl,

      -heterocyclyl,

      -aryl,

      -heteroaryl,

      or

      -benzyl,
   wherein the alkyl, the alkenyl, the alkynyl, the cycloalkyl, the cycloalkenyl, the perfluorinated alkyl, the heterocyclyl, the aryl, the heteroaryl and the benzyl groups are each independently optionally substituted with 1, 2 or 3 RA or Rd substituents;
   R5 is each independently
      -C₁₋₆ alkyl,
      -C₁₋₆ alkylene-C₂₋₆ alkenyl which optionally includes one or more other heteroatom selected from N, O and S
      -C₁₋₆ alkylene-C₂₋₆ alkynyl which optionally includes one or more other heteroatom selected from N, O and S
      -L-aryl which optionally includes one or more RA or R4 substituents
      -L-heteroaryl which optionally includes one or more RA or R4 substituents
      -C₁₋₆ alkylene-C(O)O-
      -C₁₋₆ alkylene-C(O)OR1
      -C₁₋₆ alkylene-CN
      -C₁₋₆ alkylene-C(O)NR1R3, wherein R1 and R3 optionally they join together with the nitrogen atom to form a 3 to 7-membered ring which optionally includes one or more other heteroatom selected from N, O and S; or
      -C₁₋₆ alkylene-OH;
   R6 is

      -Halogen

      -OC(O)CF₃ or

      -OC(O)R1;
   RA is each independently

      -halogen,

      -CF₃,

      -OR1,

      -L-OR1,

      -OCF₃,

      -SR1,

      -CN,

      -NO₂,

      -NR1R3,

      -L-NR1R1,

      -C(O)OR1,

      -S(O)₂R4

      -C(O)N(R1)R3,

      -NR1C(O)R1,

      -NR1C(O)OR1,

      -OC(O)N(R1)R3,

      -OC(O)R1,

      -C(O)R4,

      -NHC(O)N(R1)R3,

      -NR1C(O)N(R1)R3,

      or

      -N₃;

      and
   Rd is each independently

      -H,

      -C₁₋₆ alkyl,

      -C₂₋₆ alkenyl,

      -C₂₋₆ alkynyl,

      -C₃₋₇ cycloalkyl,

      -C₃₋₇ cycloalkenyl,

      -C₁₋₅ perfluorinated alkyl,

      -benzyl

      or

      -heterocyclyl,
   for use in treating cancer.

### BRIEF DESCRIPTION OF THE DRAWINGS

Reference will now be made to the accompanying drawings.
Fig. 1A illustrates the experimental outline of whole genome CRISPR/Cas9 screening of OCI-AML5 or OCI-AML1 cell lines exposed or not to UM171 (250nM and 800nM) for 8 and 14 days.
Fig. 1B illustrates the total cell population doubling (upper panel) and pairwise sample correlations (lower panel) in OCI-AML5 CRISPR/Cas9 screen.
Fig. 1C illustrates total cell population doubling (upper panel) and pairwise sample correlations (lower panel) in OCI-AML1 CRISPR/Cas9 screen.
Fig. 1D illustrates the scatter plot showing MAGeCK outputs beta scores of CRISPR knockout OCI-AML5 (displayed on the y-axis) and OCI-AML1 (x-axis) exposed to UM171 (250nM) for 8 days (left panel) or 14 days (right panel), wherein synthetic rescue and synthetic lethal interactions are identified, and KBTBD4 (synthetic rescue) and RCOR1 (synthetic lethal) are identified independently of UM171 dose or time of exposure.
Fig. 2A illustrates a heatmap of the abundance of protein candidates identified in BiolD pulldown/MS experiment using BirA or BirA-KBTBD4 fusion protein as baits.
Fig. 2(B) illustrates proteins interaction in the targeted complex.
Fig. 2(C) illustrates a flow cytometry-based analysis of H3K27 acetylation (upper panel) and H3K4 di-methylation (lower panel) in OCI-AML1 cells expressing shLuc (control Luciferase) or shKBTBD4 and exposed to 0.1% DMSO or UM171 (250nM) for 24hrs. Data show relative mean fluorescent intensity (MFI) of modified histones in GFP negative (uninfected, black dots) and GFP positive (infected, lighter dots) subsets. Total H3 levels were used for normalization. Representative of 3 independent experiments.
Fig. 2(D) illustrates a flow cytometry-based analysis of CD201 (left) and CD86 (right) surface expression in OCI-AML1 cells expressing shKBTBD4 (upper panels) or shRCOR1 (lower panels) and exposed to DMSO, UM171 (250nM), HDAC inhibitor (panobinostat, 5nM) or LSD1 inhibitor (TCP, 10µM) for 24hrs. Representative of 3 independent experiments.
Fig. 2(E) illustrates a flow cytometry-based analysis of CD86 and CD201 surface expression in sgAAVS1 (left panels) or sgKBTBD4 (right panels) knockout OCI-AML1 cells exposed or not to UM171 (250nM, 24hrs) and engineered to expressed a control, a KBTBD4 full-length (wt), a BTB deleted (ΔBTB) or a Kelch domain deleted (ΔKelch) mutant vector.
Fig. 3(A) illustrates the validation of CRISPR screen candidates KBTBD4 and RCOR1. Fold-change in absolute cell count of cells expressing shLuc, shKBTBD4 (upper panel) or shRCOR1 (lower panel) cultured for 4 days in presence of DMSO or increasing concentration of UM171.
Fig. 3(B) illustrates a Western Blot (upper panel) and Flow cytometry-based analysis (lower panel) of H3K27 acetylation in response to UM171 (250nM or more) or HDAC inhibitor (Panobinostat, 5nM).
Fig. 3(C) illustrates representative FACS profiles of CD201/GFP (upper panel) or CD86/GFP (lower panel) expression in OCI-AML1 cells expressing shLuc or sh KBTBD4 (3 shRNA are shown) and exposed to DMSO or UM171 (250nM) for 48hrs. Note that all 3 shRNA against KBTBD4 abolished UM171 activity (compare GFP+ to GFP- subsets).
Fig. 3(D) illustrates a Western Blot analysis of total proteins extracted from shLuc or shKBTBD4 (sh1-3) expressing OCI-AML1 cells exposed to DMSO or UM171 (250nM) for 24hrs. Representative blots showing KBTBD4 and Nucleolin (NCL, loading control) levels confirmed KBTBD4 knockdown.
Fig. 3(E) illustrates representative FACS profile of CD201 and CD86 expression in OCI-AML1 cells expressing shLuc, shKBTBD4 (sh2) and shRCOR1 and their distribution in untransduced GFP negative cells vs transduced GFP positive cells. While shKBTBD4 abolishes UM171-mediated CD201/CD86 surface expression, shRCOR1 enhances their expression, consistent with CRISPR screen results.
Fig. 3(F) illustrates representative FACS profile of CD201 and CD86 surface expression in OCI-AML1 cells expressing shLuc, shKBTBD4 (sh2) exposed to increasing concentration of UM171.
Fig. 3(G) illustrates relative mean fluorescent intensity (MFI) of CD201 (upper panel) and CD86 (lower panel) upon UM171 (250nM) exposure (24hrs) of OCI-AML1 expressing shLuc, shKBTBD4 or shRCOR1. CD201/CD86 levels are measured in GFP negative (uninfected) and GFP positive (infected) cells. Representative of 3 independent experiments.
Fig. 3(H) illustrates representative FACS profile of CD86/GFP expression in OCI-AML1 expressing shLuc, shKBTBD4 or shRCOR1 and treated with DMSO, UM171 (250nM), HDAC inhibitor (Panobinostat, 5nM) or LSD1 inhibitor (TCP, 5µM). Note that HDACi and LSDi induces CD86 expression in shKBTBD4 expressing cells (middle panel), suggesting that KBTBD4 acts upstream of HDAC/LSD1 corepressor complex.
Fig. 4(A) illustrates representative FACS analysis showing CD201 and CD86 upregulation in OCI-AML5 exposed to UM171 (250nM), HDACi (Panobinostat, 5nM) or LSD1i (TCP, 10µM) for 24hrs when compared to DMSO.
Fig. 4(B) illustrates relative mean fluorescent intensity (MFI) of H3K27Ac, H3K4me2 and H3 in OCI-AML5 exposed to DMSO, UM134 (inactive analog of UM171 (250nM)), UM171 (250nM), HDACi (Panobinostat, 5nM) or LSD1i (TCP, 10µM) for 24hrs, wherein UM171 as HDACi and LSDi induces H3K27Ac and H3K4me2 marks in OCI-AML5 cell lines.
Fig. 5 illustrates representative FACS analysis of CD86/GFP expression in sgAAVS1 (upper panels) or sgKBTBD4 (lower panels) knockout OCI-AML1 cells exposed or not to UM171 (250nM, 24hrs) and engineered to expressed a control (empty vector), a KBTBD4 full-length (wt), a BTB deleted (ΔBTB) or a Kelch domain deleted (Kelch) mutant vector. Note that only full-length KBTBD4 enhances (in sgAAVS1) or rescue (sgKBTBD4) UM171-mediated CD86 upregulation.
Fig. 6(A) illustrates a flow cytometry-based analysis of histone 3 (H3), H3K27ac and H3K4me2 epigenetic marks in purified CD34+ cord blood cells expanded for 4 days in presence of DMSO or UM171 (35nM). Results show relative levels of histone marks in HSPC-enriched CD34+ gated subsets (see upper panel). Middle panel show histogram overlays and lower panel show relative MFI normalized on total H3 Levels.
Fig. 6(B) illustrate representative FACS profiles of CD34+ cord blood cells cultured for 7 days in presence of DMSO, UM171 (35nM), HDACi (panobinostat, 5nM) or LSD1i (TCP, 5µM). CD34+, CD34+CD45RA-, CD34+CD201+, CD34+CD90+CD201+ and CD34+CD86+ subsets are represented.
Fig. 6(C) illustrate a bar graph showing absolute counts of HSC-enriched CD34+CD201+ cell subset after 7 days in cultures supplemented with indicated compounds (mean±SD).
Fig. 6(D) illustrate representative FACS profiles of CD34+ cord blood cells infected with shLuc (left panel), shRCOR1 (2 shRNA) or shLSD1 (2shRNA) (right panel) and cultured for 7 days in presence or absence of UM171 (35nM) as indicated. CD34+ and CD34+CD201+ subsets are shown. Also shown are representative FACS profiles of CD34+ cord blood cells infected with shLuc or shKBTBD4 (2 shRNA) and cultured for 7 days in presence of UM171 (35nM).
Fig. 6(E) illustrate representative FACS profiles of CD34+ cord blood cells infected with control vector (CT), full-length KBTBD4 (wt), ΔBTB or ΔKelch mutants and cultured for 7 days in presence of DMSO or UM171 (35nM). Data are presented after gating on GFP-transduced cells.
Fig. 6(F) illustrate a bar graph showing absolute counts (mean±SD) of HSC-enriched CD34+CD201+ cell subset in cultures initiated with indicated infected cells (shRNA (upper panel), ectopic expression (lower panel)) after 7 days in presence of DMSO, UM171 (5nM) or UM171 (35nM).
Fig. 7(A) illustrate % CD34+ and CD34+CD201+ HSPCs obtained after a 7 day culture of CD34+ cord blood cells exposed to indicated compound (median ± SD).
Fig. 7(B) illustrate fresh CD34+ and day 7 cultures exposed to DMSO, UM171 (35nM) or indicated HDACi were transplanted in immunocompromised NSG mice (outcome of 3000 day 0 cells). Human CD45 engraftment was assessed at 3, 8 and 26 wks post-transplantation. Human CD34 engraftment was assessed at 26 wks post-transplantation.
Fig. 8(A) illustrate a bar graph showing total cell counts (upper panel) and absolute count of CD34+ cell (lower panel) subset after 7 days in cultures supplemented with indicated compounds (mean±SD).
Fig. 8(B) illustrate a bar graph showing total cell counts (left panel) and absolute count of CD34+ cell subset (right panel) after 7 days cultures of cord blood cells transduced with indicated shRNA (upper panel) or KBTBD4 vectors (lower panel).
Fig. 8(C) illustrate representative FACS profiles of CD34+ (left panel), CD34+CD201+CD90+ (middle panel) and CD34+CD86+ (right panel) subsets after 7 days culture of CD34+ cord blood cells with indicated concentration of UM171 and/or LSD1 inhibitor (TCP), showing a synergistic effect of LSD1i with UM171 at 5nM.
Fig. 8(D) illustrate representative FACS profiles of CD34+ and CD34+CD201+ subsets after 7 days culture of CD34+ cord blood cells infected with shLuc or shKBTBD4 (2 shRNA) in presence of DMSO.
Fig. 9 illustrate representative FACS profiles of CD34+CD201+ and CD34+CD86+ subsets (UM171 signature) obtained after 7 days culture of CD34+ cord blood cells exposed to UM171 (35nM) in absence (0) or presence of iBET bromodomain inhibitors (50nM and 100nM). Note that iBET completely abolishes UM171 activity in cord blood cells.
Fig. 10 illustrate RCOR1 protein levels measured by western blot analysis of cytoplasmic and chromatin bound proteins extracted from sgAAVS1 or sgKBTBD4 knockout OCI-AML1 cell lines transduced with shLuc or shRCOR1 (sh1).
Fig. 11(A) illustrate a Western Blot analysis of total proteins extracted from sgAAVS1 (OCI-AML1 cells engineered to express control vector (CT), full-length KBTBD4 (wt), ΔBTB or ΔKelch mutants. Representative blots showing KBTBD4, RCOR1 and CUL1 (loading control) levels.
Fig. 11(B) illustrate a Western Blot analysis of nucleoplasmic proteins extracted from shLuc or shKBTBD4- transduced OCI-AML1 cells exposed to DMSO or UM171 (250nM) for 24hrs and treated or not with MG132 (10µM) for the last 4 hrs. Representative blots (upper panel) showing KBTBD4, RCOR1, LSD1, HDAC2 and Nucleolin (NCL, loading control) protein levels. Quantification of RCOR1 protein levels were evaluated in 3 independent experiments (lower panel).
Fig. 11(C) illustrate Western Blot analysis of total proteins extracted from shLuc or shKBTBD4 transduced OCI-AML1 cells exposed to DMSO, UM171 (250nM), MLN2494 (100nM), MLN2494/UM171 or MG132/UM171 for 4 hrs. Representative blots (upper panel) showing CUL3, RCOR1 and TUBA (loading control) protein levels. Quantification of RCOR1 protein levels were evaluated in 2 independent experiments (lower panel).
Fig. 11(D) illustrate Western Blot analysis of total proteins extracted from shLuc, shKBTBD4, shNUDCD3 or shCAND1 exposed to DMSO or UM171 (250nM) for 4hrs. Representative blots (upper panel) showing NUDCD3, CAND1, RCOR1 and TUBA (loading control) protein levels. Quantification of RCOR1 protein levels were evaluated in 2 independent experiments (lower panel).
Fig. 11(E) illustrate representative FACS profile of CD201 and CD86 surface expression in OCI-AML1 cells expressing shLuc, shNUDCD3, shCUL3 or shCAND1 and exposed to UM171 (250nM) for 24hrs. Results show only transduced cells (GFP+).
Fig. 11(F) illustrate relative mean fluorescent intensity (MFI) of CD201 (upper panel) and CD86 (lower panel) upon UM171 (250nM) exposure of GFP negative (uninfected) and GFP positive (infected) subsets for each indicated condition.
Fig. 12(A) illustrate a Western Blot analysis of total proteins extracted from HEK293 cell lines exposed to DMSO, UM134 (inactive UM171 analog) or UM171 (1µM) for 4 hrs and treated or not with MG132 (10µM) for the last 3hrs.
Fig. 12(B) illustrate a Western Blot analysis of total proteins extracted from HEK293 cell line expressing shLuc or shKBTBD4 and exposed to DMSO or UM171 (1µM) for 1 hr.
Fig. 12(C) illustrate a Western Blot analysis of total proteins extracted from U2OS cell lines exposed to DMSO or UM171 (1µM) for 1, 6 or 24 hrs. KBTBD4, RCOR1 and TUBA protein levels are shown.
Fig. 12(D) illustrate a Western Blot analysis of total proteins extracted from sgKBTBD4 knockout OCI-AML1 cells engineered to express control vector (CT), full-length KBTBD4 (wt), ΔBTB or ΔKelch mutants.
Fig. 13(A) illustrate KBTBD4 and RCOR1 protein levels are measured by Western Blot analysis of total protein extracted from sgAAVS1 or sgNUDCD3 knockout OCI-AML1 cells engineered to express control or KBTBD4 (wt) vector. CUL1 is used as a loading control. Note that sgRNA mediated NUDCD3 deletion dramatically reduces endogenous KBTBD4 levels (first lane, right panel) and re-introduction of KBTBD4 (wt) in NUDCD3 deleted cells reduces RCOR1 protein levels (second lane, right panel).
Fig. 13(B) illustrate KBTBD4 and RCOR1 protein levels are measured by Western Blot analysis of total protein extracted from OCI-AML1 expressing shLuc or shNUDCD3. TUBA is used as a loading control.
Fig. 13(C) illustrate representative FACS profile of CD86/GFP expression in sgAAVS1 (left panels) or sgNUDCD3 (lower panels) knockout OCI-AML1 cells exposed or not to UM171 (250nM, 24hrs) and engineered to expressed a control (empty vector), a KBTBD4 full-length (wt), a BTB deleted (ΔBTB) or a Kelch domain deleted (ΔKelch) mutant vector.
Fig. 13(D) illustrate results of CD201 (left panel) and CD86 (right panel) surface expression in sgAAVS1 or sgNUDCD3 knockout OCI-AML1 cells exposed or not to UM171 (250nM, 24hrs) and engineered to expressed control, KBTBD4 full-length (wt), ΔBTB or ΔKelch mutant vector.
Fig. 14 illustrate flow cytometry-based analysis of CD201 surface expression in OCI-AML1 cells expressing shLuc, shNUDCD3 or shCUL3 and exposed to HDAC inhibitor (panobinostat, 5nM) (left panel) or LSD1 inhibitor (TCP, 10µM) (right panel) for 24hrs. Graph show median ± SD of relative CD201 MFI in GFP negative or GFP positive subsets. Note that both HDACi and LSD1i induce CD201 upregulation in shNUDCD3 and shCUL3 expressing cells suggesting that CUL3 and NUDCD3, as shown for KBTBD4, act upstream of UM171 mediated transcriptional activation.
Fig. 15A illustrate a Western Blot analysis of total proteins extracted from d3 expanded CD34+ derived cord blood cells exposed to DMSO, UM171 (500nM) or UM171/MG132 (5µM) for 4 hrs. Representative blot showing KBTBD4, RCOR1, LSD1, HDAC2, CUL3 and TUBA as loading control.
Fig. 15B illustrate a Western Blot analysis of nucleoplasmic proteins extracted from shLuc or shKBTBD4- transduced OCI-AML1 cells exposed to DMSO or UM171 (250nM) for 24hrs and treated or not with MG132 (10µM) for the last 4 hrs. Representative blots showing KBTBD4, RCOR1, LSD1, HDAC2 and Nucleolin (NCL, loading control) protein levels. Quantification of RCOR1 protein levels were evaluated in 3 independent experiments.
Fig. 16 illustrates the bioavailability of the compound illustrated therein administered intravenously to mice.
Fig. 17A illustrates the bioavailability of compound UM681 administered intravenously to mice.
Fig. 17B illustrates the bioavailability of compound UM681 administered orally to mice.

### DETAILED DESCRIPTION

It is provided pyrimido[4,5-B]indole derivatives, as well as 5H-pyrido[4,3-b]indole derivatives and pyrido[2',3':4,5]pyrrolo[2,3-d]pyrimidine derivatives as anti-cancer compounds.

One example of such pyrimido[4,5-B]indole derivative is UM171 (see U.S. patent nos. 9,409,906 and 10,336,747), which has an activity on primitive cells which is rapidly reversible if the compound is washed out from culture. UM171 does not independently trigger cell proliferation in the absence of growth factors; it is not mitogenic but rather prevents cell differentiation. The molecule was reported to preferentially expand long-term hematopoietic stem cells (HSC) in ex *vivo* cultures, and this expansion was maximal by day 7. Results initially described in NSG mice were confirmed in a clinical trial in which 22 patients were transplanted with a single UM171-expanded cord blood graft. These patients showed rapid neutrophil recovery and less fever than control transplants. Most interestingly, the UM171 patients never developed extended chronic graft versus host disease and presented a very low rate of transplantation-related mortality and disease relapse. It was found that UM171 induced an important expansion of CD86+ dendritic progenitors and mast cells in addition to CD201+ (EPCR) primitive HSCs (see WO 2019/161494). In addition, the molecule was also shown to rapidly induce the expression of numerous pro- and antiinflammatory genes and of several essential stem cell marker genes including CD201 and CD86. Other related compounds that are similarly contemplated herein are referred to as 5H-pyrido[4,3-b]indole derivatives and pyrido[2',3':4,5]pyrrolo[2,3-d]pyrimidine derivatives. Those compounds are disclosed for example in US 10,647,718 B.

These results prompted the investigation to determine if UM171 can have a broad anti-cancer use.

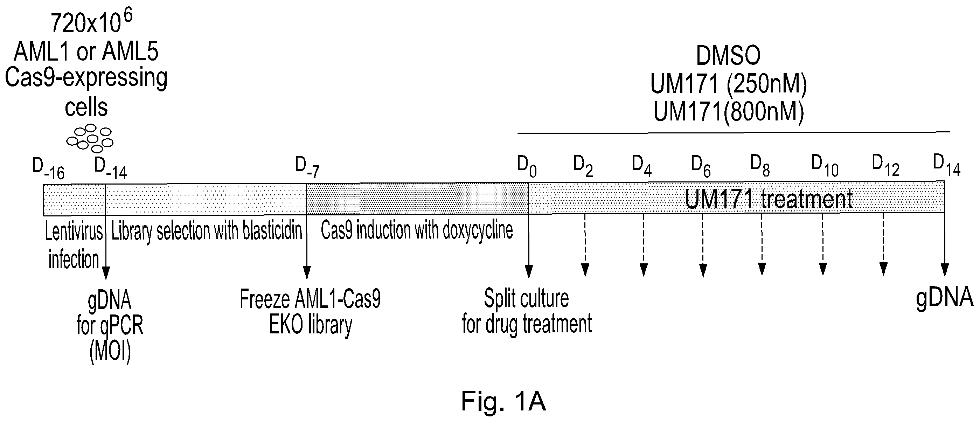

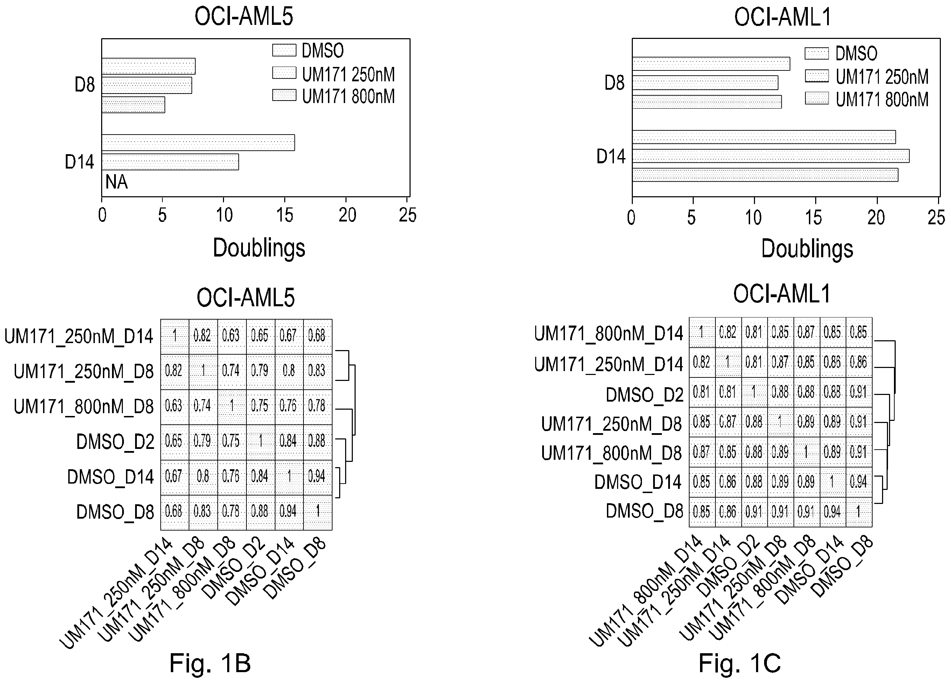

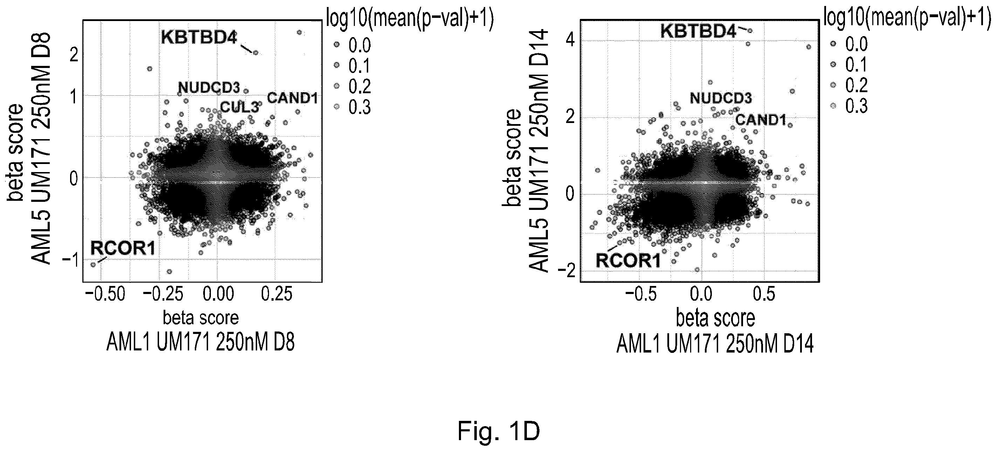

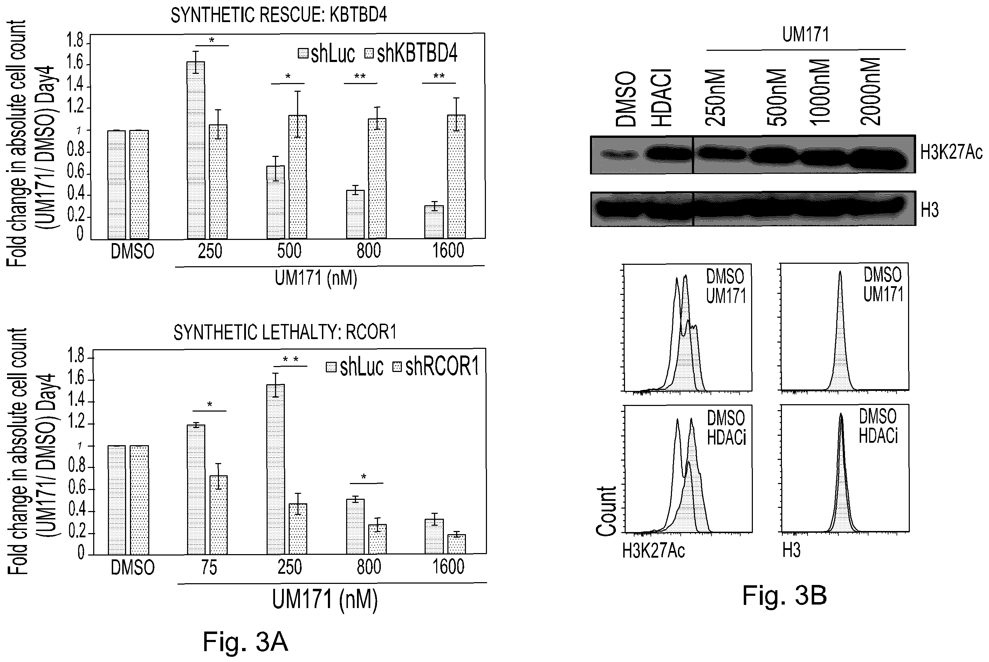

Hematopoietic cell lines such as OCI-AML5 exposed to UM171 upregulate more than 400 genes within 6 hours with less than a dozen genes repressed. This nearly exclusive effect on gene upregulation pointed to a potential transcriptional repressor being targeted by the molecule. CRISPR screens in 2 different cells lines over a range of UM171 dose (Fig. 1) revealed a poorly characterized Kelch-BTB domain protein KBTBD4 as a suppressor of UM171 phenotype and of the transcriptional corepressor RCOR1 or CoREST as a strong enhancer (Fig. 1 and 3A).

While the function of KBTBD4 is unknown to date, Kelch BTB domain proteins are known to bridge CULLIN3-RING ubiquitin ligases (CRL3 complex) to their substrate (Genschik et al., 2013, EMBO J, 32: 2307-2320), thereby dictating specificity in targeting proteins for proteolysis. In line with this observation, several core and key components of the CRL3 complex were strong suppressors of UM171 in the different CRISPR screens, namely CULLIN3 itself and its essential regulator Cullin-associated NEDD8-dissociated protein 1 (CAND1) (Fig. 1D).

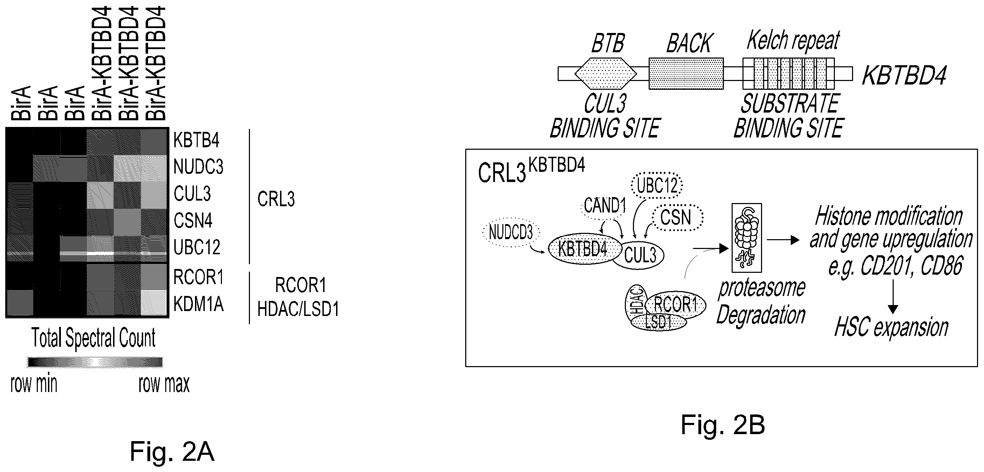

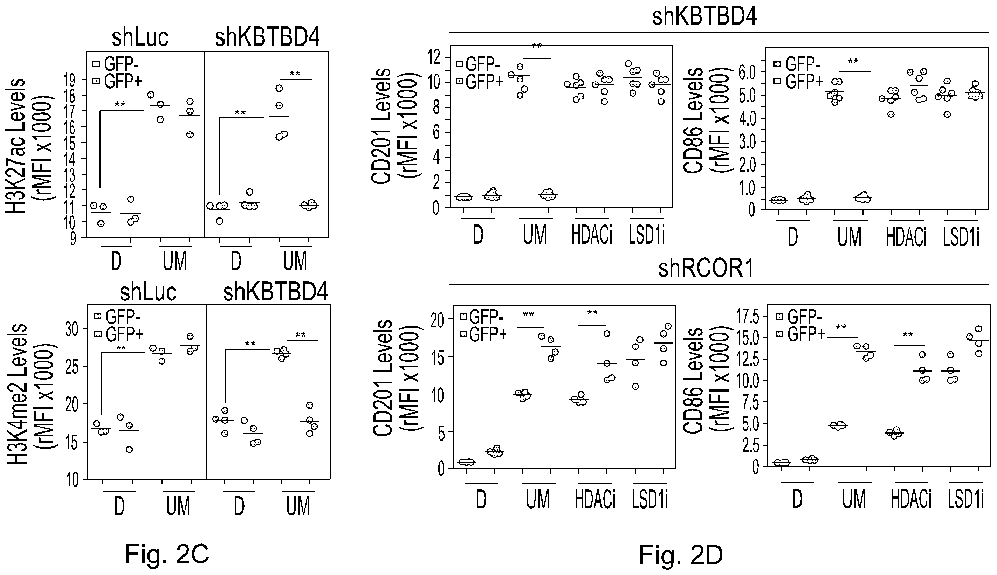

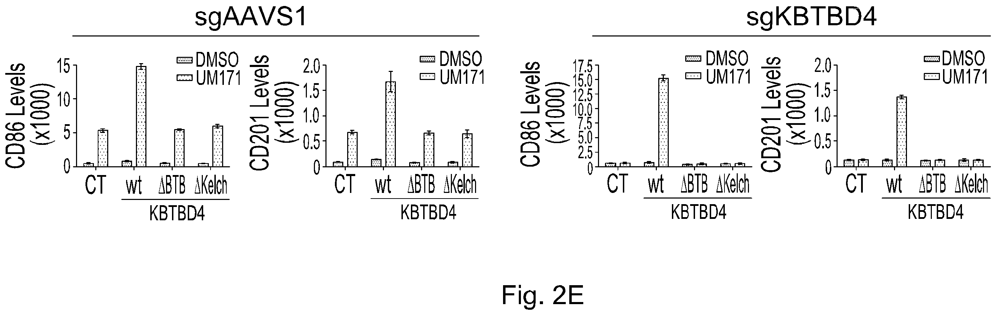

Directed mass spectrometry analysis of BirA-KBTBD4 proximity labeling also readily identified several components of CRL3 or associated proteins such as CULLIN3, CSN4, UBC12 and the KELCH-domain-directed HSP90 adaptor NUDCD3 (Fig. 2D). Most interestingly, RCOR1 and its associated lysine demethylase 1A (KDM1A or LSD1) were also identified in these experiments (Fig. 2D). Based on these results, it is provided that UM171 activates a novel CLR3^{KBTBD4} complex that targets the LSD1-RCOR1 CoREST repressor complex for proteosomal degradation, leading to histone modification and upregulation of key stem cell genes (Fig. 2E).

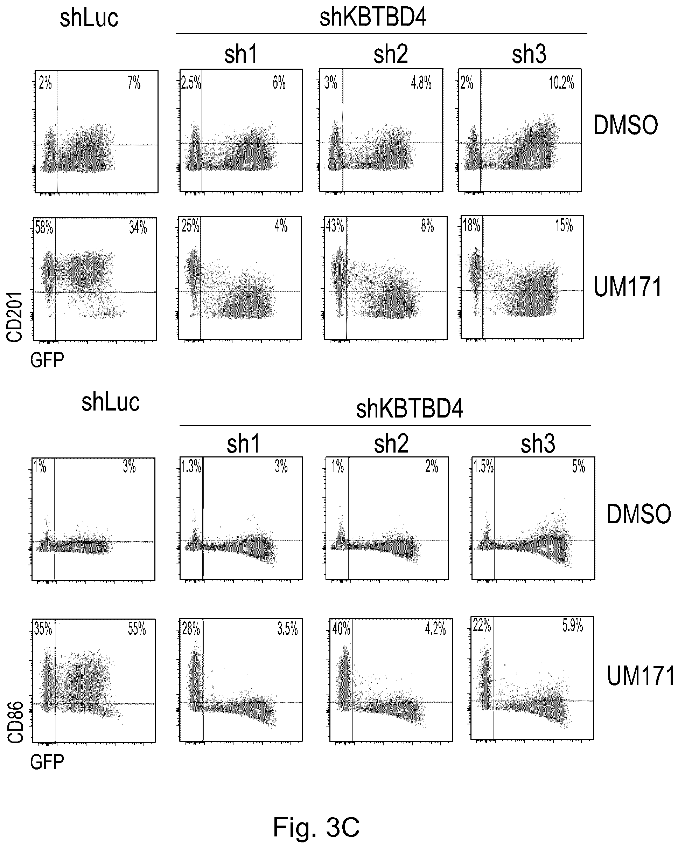

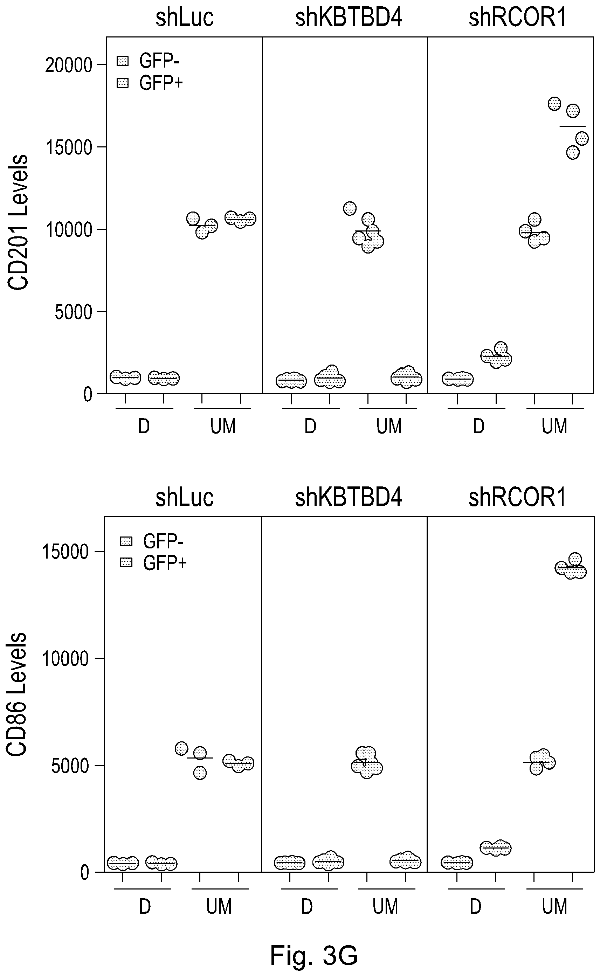

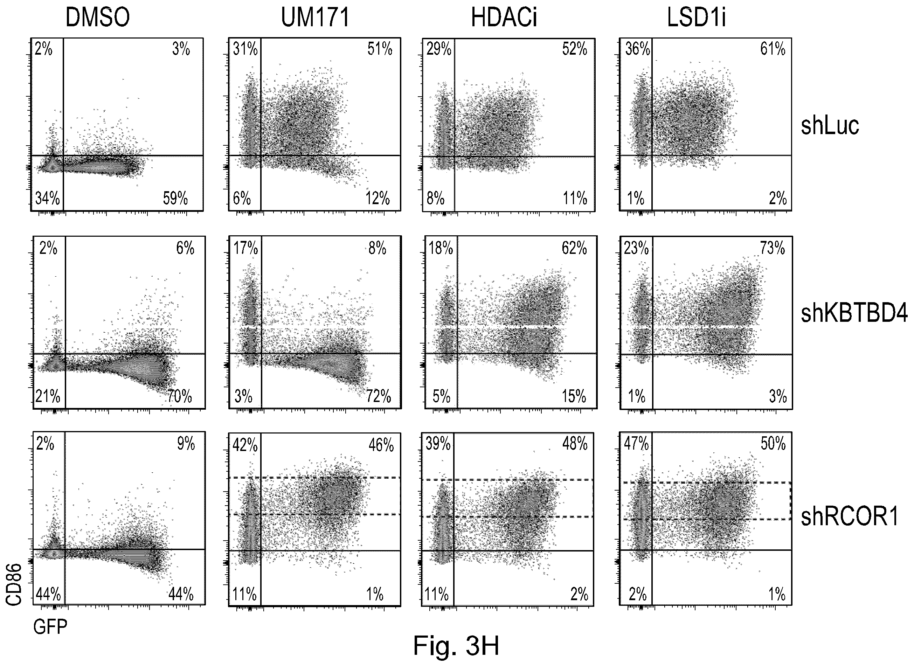

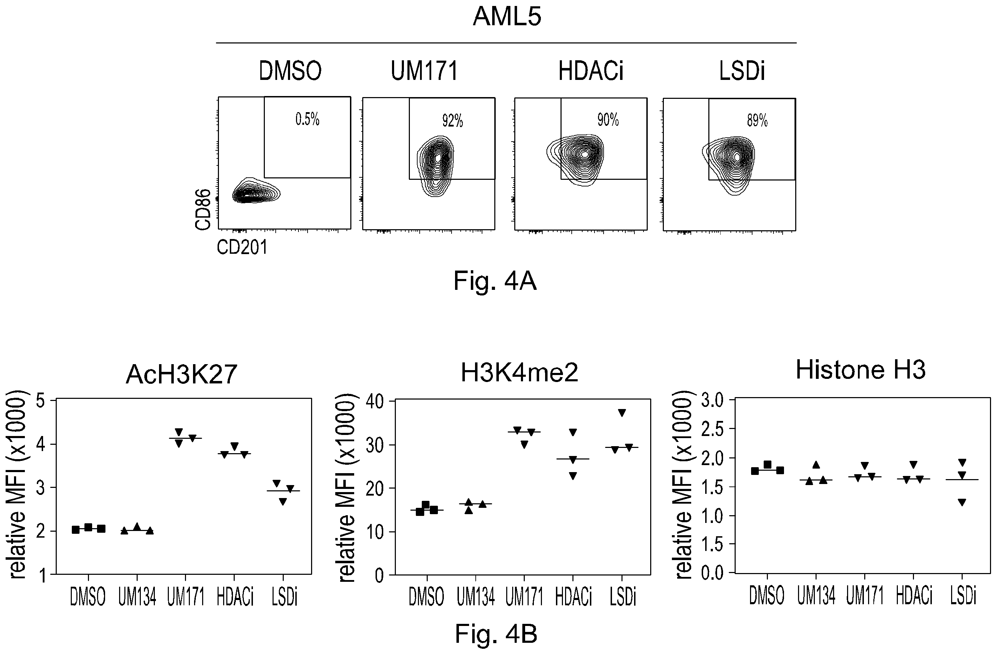

It is provided that histone 3 lysine 27 acetylation (H3K27ac) mark is rapidly enhanced upon exposure to UM171 and of the essential role of KBTBD4 for the rapid impact of UM171 on this epigenetic mark (Fig. 2C, upper panel and Fig. 3B and 4). Likewise, histone 3 lysine 4 dimethylation (H3K4me2) was enhanced by UM171 treatment and shown to be dependent on KBTBD4 (Fig. 2C, lower panel). Similarly, CD201 and CD86 expression induced by UM171 is dependent on KBTBD4 (Fig. 2D upper left and right panels, compare first 4 columns and Fig. 3C-G). It is proposed that inhibitors of HDAC and LSD1, two core members of the RCOR1 complex, induced the expression of CD201 and CD86 independently of KBTBD4 (Fig. 2D upper left and right panels, last 4 columns and Fig. 3H). In line with RCOR1 being downstream to KBTBD4, it is demonstrated that both CD201 and CD86 are strongly induced by UM171 when RCOR1 levels are experimentally decreased (Fig. 2D lower panels, first 4 columns and Fig. 3G-H).

Moreover, reduction in RCOR1 levels further enhance the impact of HDAC or LSD inhibitors on CD201 and CD86 expression (Fig. 2D lower panels, last 4 columns). A small increase, much more prominent in primary cord blood cells, in the expression of these two markers is spontaneously observed with shRCOR1 (Fig. 2D lower panels, first 2 columns). This supports the epistatic interaction between UM171, KBTBD4 and RCOR1 that ultimately leads to the upregulation of CD201 and CD86 in treated cells (Fig. 2B).

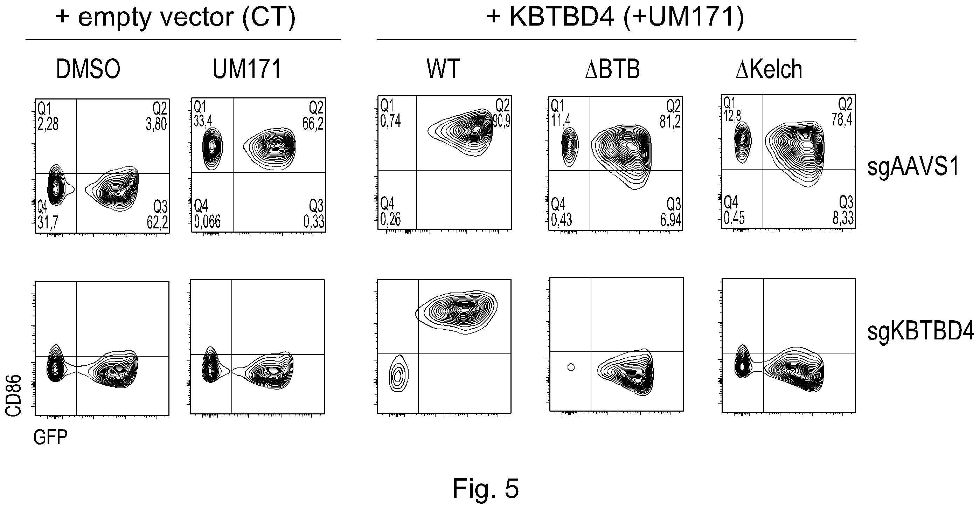

In further support to this model, it is shown that the induction of CD201 and CD86 by UM171 is strictly dependent on KBTBD4 in OCI-AML1 cells engineered by CRISPR/Cas9 to lack this gene (see sgKBTBD4 panels in Fig. 2E and compare to control sgAAVS1 panels and Fig. 5) and that neither BTB nor KELCH deletion domain mutants can rescue these phenotypes (Fig. 2E and Fig. 5).

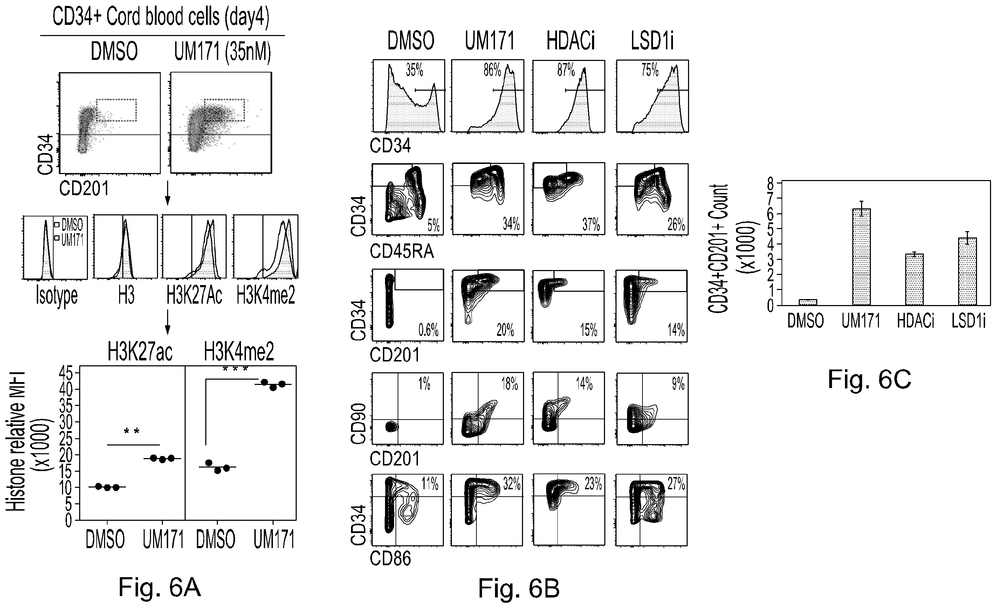

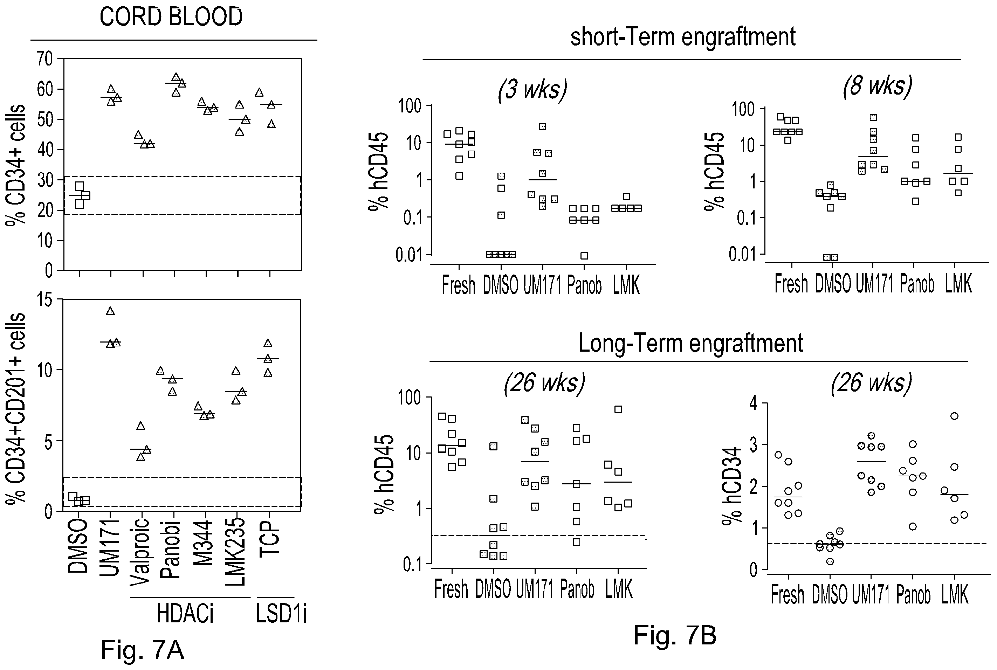

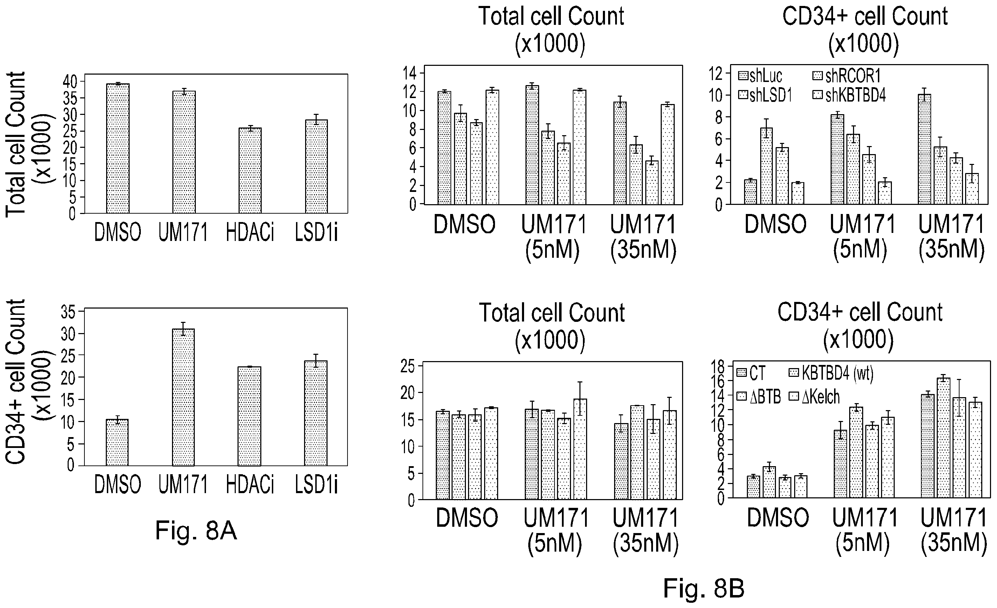

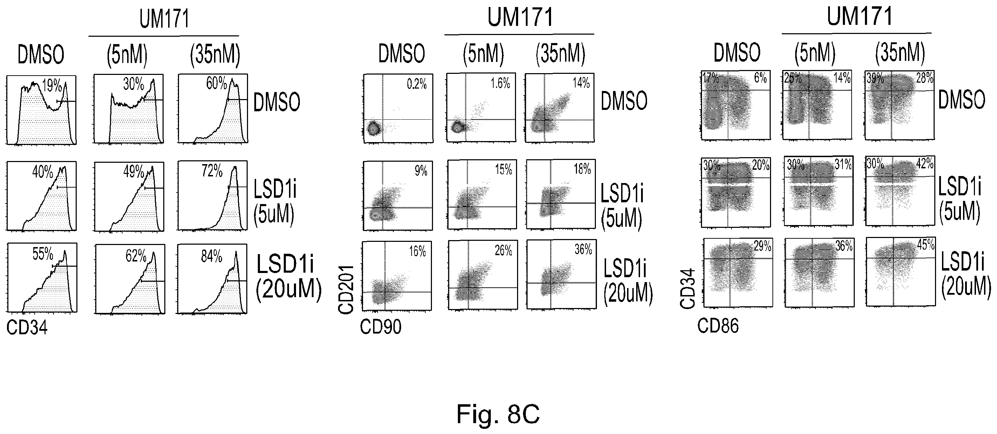

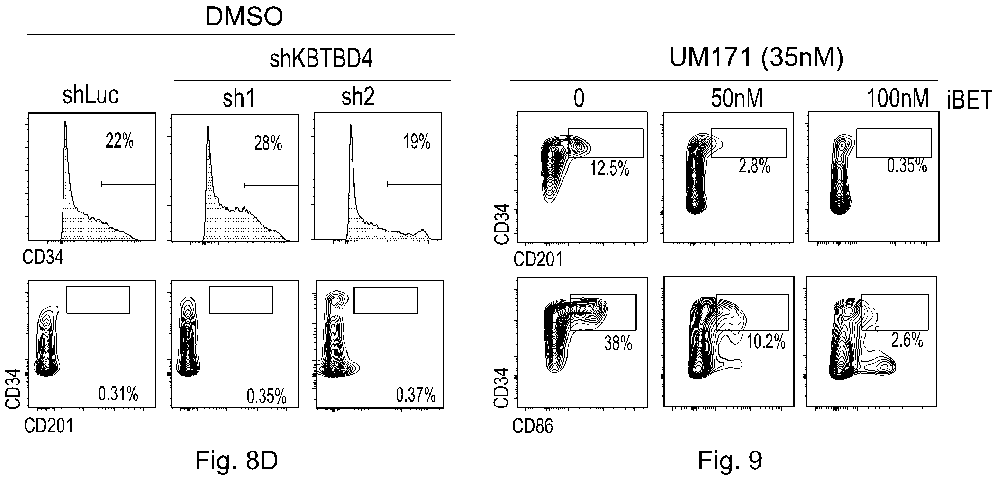

These results were validated in primary CD34+ human cord blood cells to show that CD34+CD201+ hematopoietic stem/progenitor cells (HSPC) exposed to UM171 show higher levels of both H3K27ac and H3K4me2 activation marks (Fig. 6A). As reported by others (Broxmeyer, 2014, J Clin Invest, 124: 2365-2368), treatment with HDAC or LSD1 inhibitors lead to HSPC amplification *in vitro* and improved engraftment *in vivo,* a phenotype shared with UM171 on several primitive subpopulations including CD34+CD201+ (Fig. 6B-C, Fig. 7A, Fig. 8A). It is thus provided the sensitisation to UM171 in LSD1i treated cells (Fig. 8C). Different HDAC inhibitors were then tested, both in short- (3 weeks) and long-term (26 weeks) *in vivo* experiments, and found them to be equivalent to UM171 in providing robust human engraftment in immunocompromised mice (Fig. 7B). Consistently, UM171-mediated CD34+CD201+ HSPC phenotype is abolished by iBET, a compound that prevents interactions between BRD domain proteins and acetylated histones, further emphasizing the importance of this mark in HSC expansion and UM171 activity (Fig. 9).

LSD1 is a member of a corepressor complex composed of LSD1, CoREST and HDAC1/2. This complex represses hematopoietic stem and progenitor cell transcriptional gene signatures likely by coupling LSD1 (H3K4me1/2 demethylase) and HDAC1/2 (e.g. H3K27ac deacetylase) activities. Recent studies showed that CoREST (or RCOR1) has a bi-lobed structure with the two enzymes located at opposite ends, a conformation which allows extensive crosstalk for cis activation or inhibition. As such, LSD1 inhibition is associated with some level of HDAC1/2 inhibition and vice-versa. Notably, HDAC inhibitors, in particular those inhibiting class I HDACs (including HDAC1/2) such as valproic acid, are capable of reversing the *ex vivo* dysfunction of human HSCs to some extent.

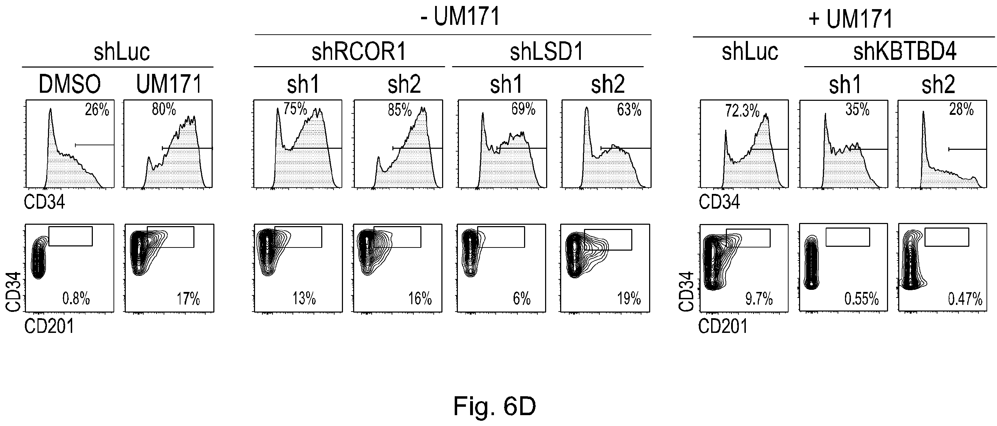

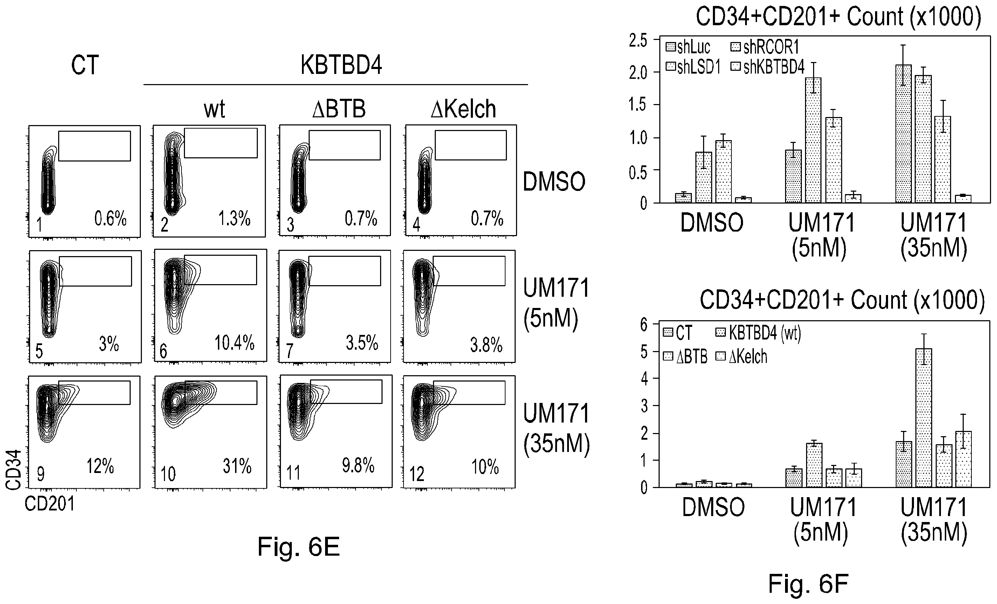

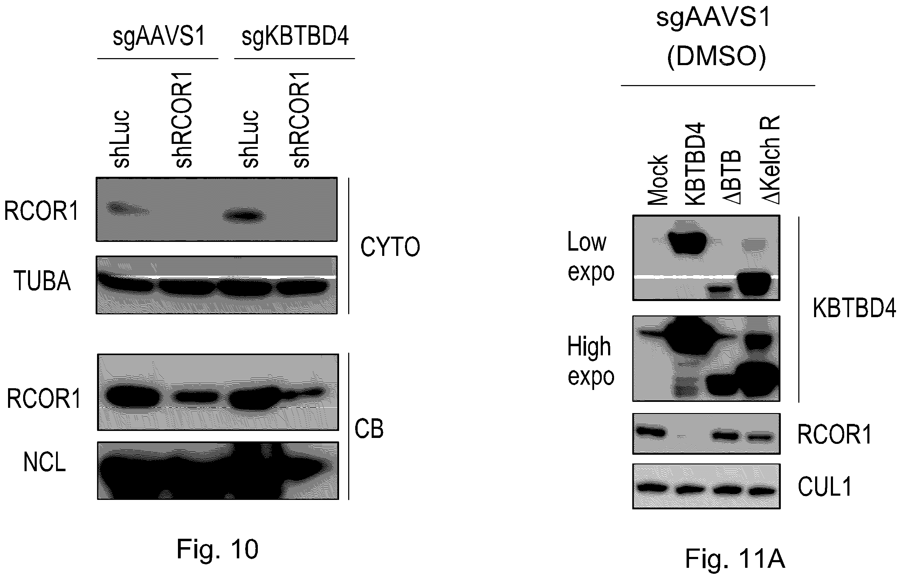

Consistent with the hypothesis presented in Fig. 2B, it is further shown that reduction in RCOR1 or LSD1 levels (Fig. 10) mimics the UM171 phenotype on primitive CD34+CD201+ HSPC (Fig. 6D middle panels labeled as "-UM171" and Fig. 8B) and that KBTBD4 is essential for UM171-induced HSPC phenotype (Fig. 6D, right panels labeled as "+UM171 and Fig. 8D). Most interestingly, overexpression of KBTBD4 strongly enhances the CD34+CD201+ phenotype found with UM171 treatment, both in relative and absolute cell numbers (Fig. 6E and Fig. 8B). However, on its own, KBTBD4 overexpression could only partially mimic exposure to low dose UM171 (compare panel 2 (high KBTBD4, no UM171) with panel 5 (5nM UM171) and panel 1 (no UM171) in Fig. 6E), potentially indicating that full activation of KBTBD4 may occur as a result of UM171 treatment and that levels of this protein are limiting for a given dose of UM171. This is best observed when comparing cells exposed to optimized levels of UM171 (35nM) but varying range of KBTBD4 levels (compare panel 10 (high KBTBD4) versus panel 9 (normal KBTBD4) in Fig. 6E) and is also reflected in absolute CD34+CD201+ counts of UM171-treated HSPCs (Fig. 6F, bottom panel).

In summary, and focusing on absolute CD34+CD201+ cell counts, it is disclosed that shRCOR1 or shLSD1 could at least partly mimic the impact of UM171 and that shKBTBD4 completely abrogates the UM171-induced HSPC phenotype (see Fig. 6F, upper panel and Fig. 8B upper panel). However, only higher levels of KBTBD4 were able to further improve absolute CD34+CD201+ cell counts indicating that it is a limiting factor for improving HSC expansion induced by UM171.

It is provided that chemical inhibition of LSD1 (partner to RCOR1 in CoREST complex) induced the surface expression of CD201 and CD86 independently of KBTBD4.

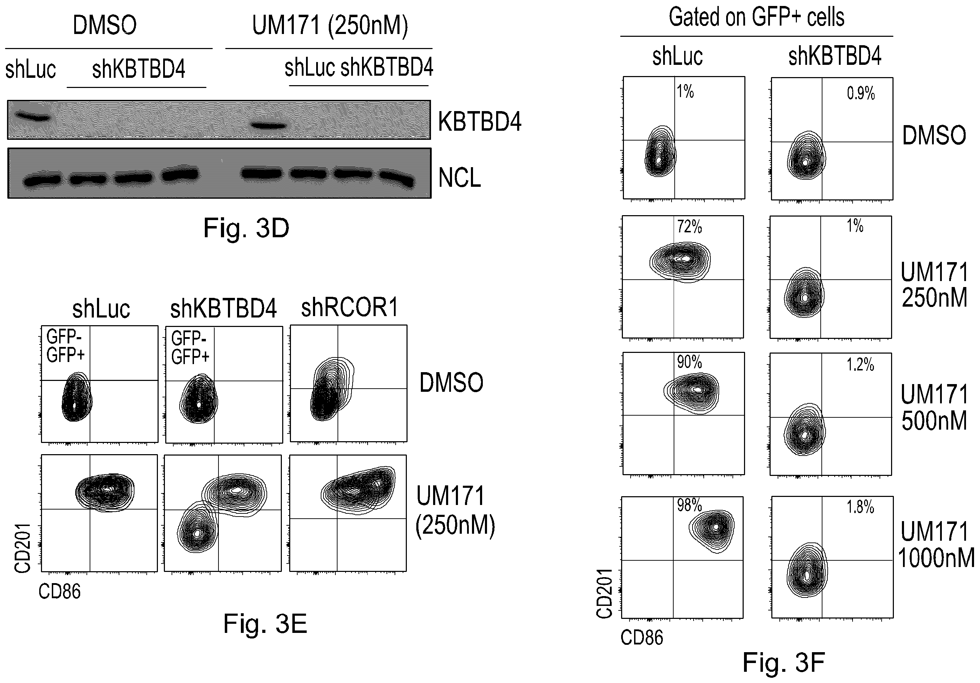

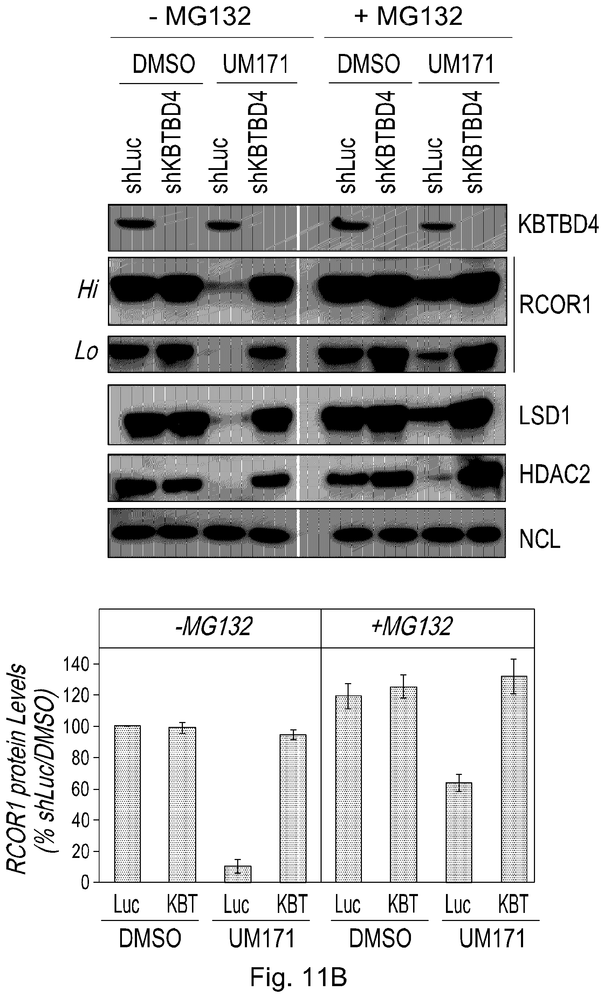

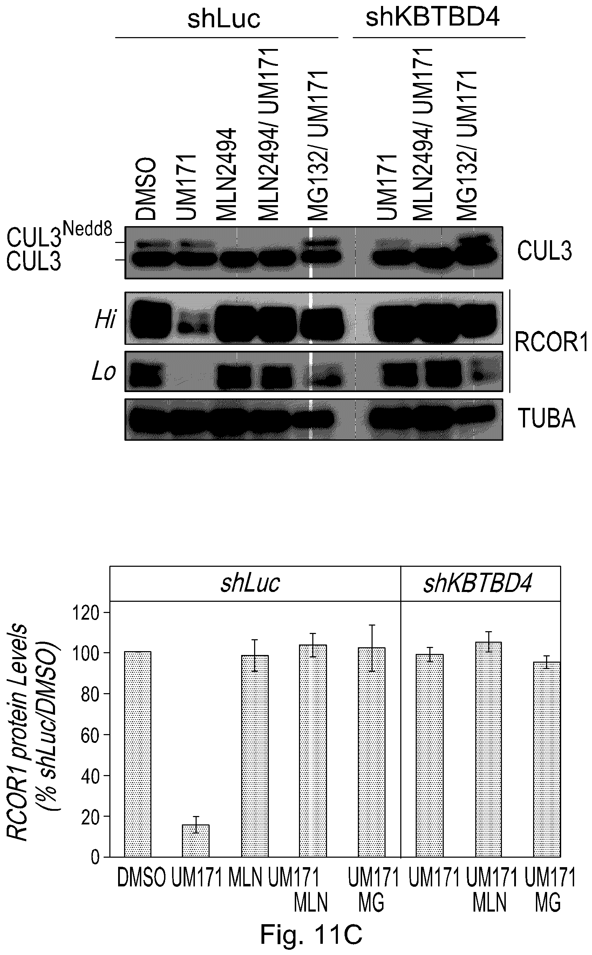

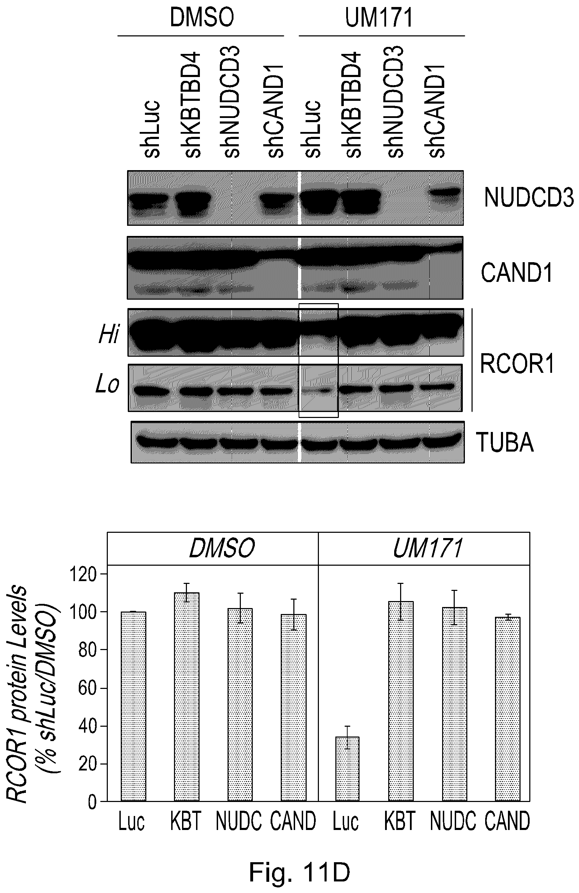

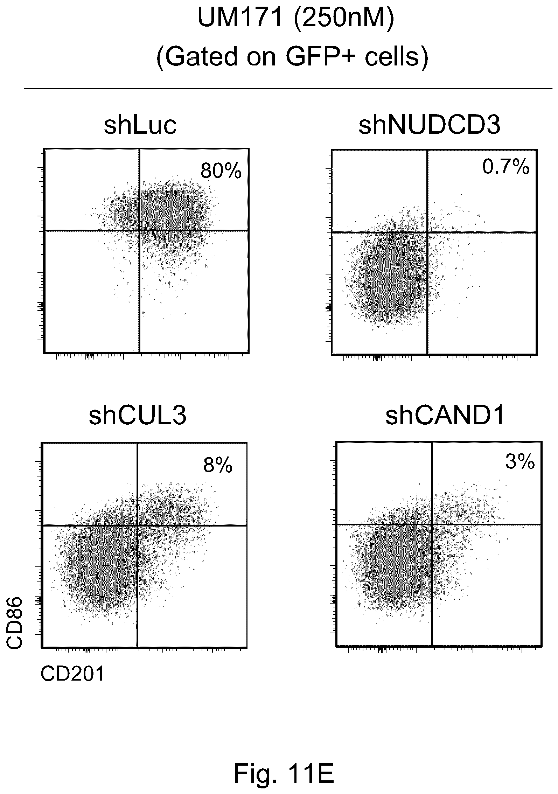

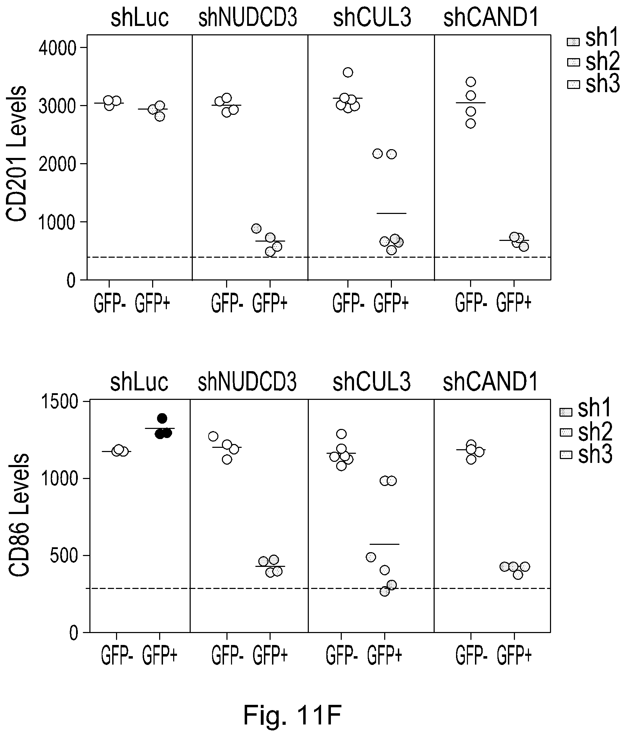

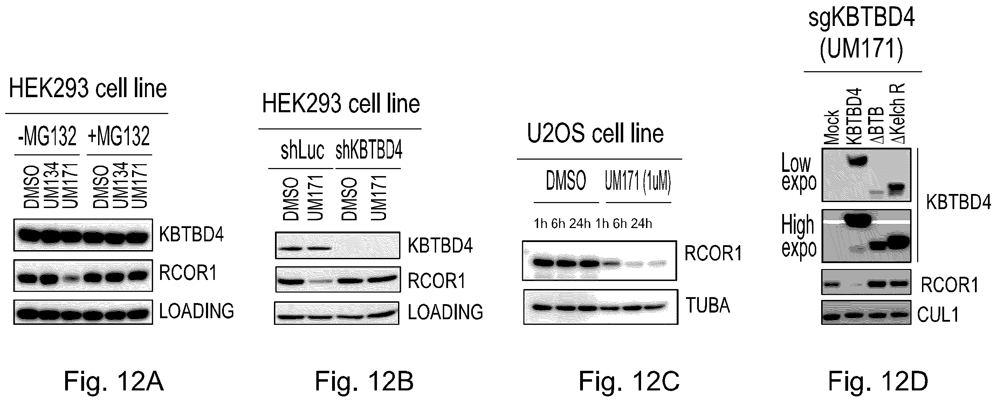

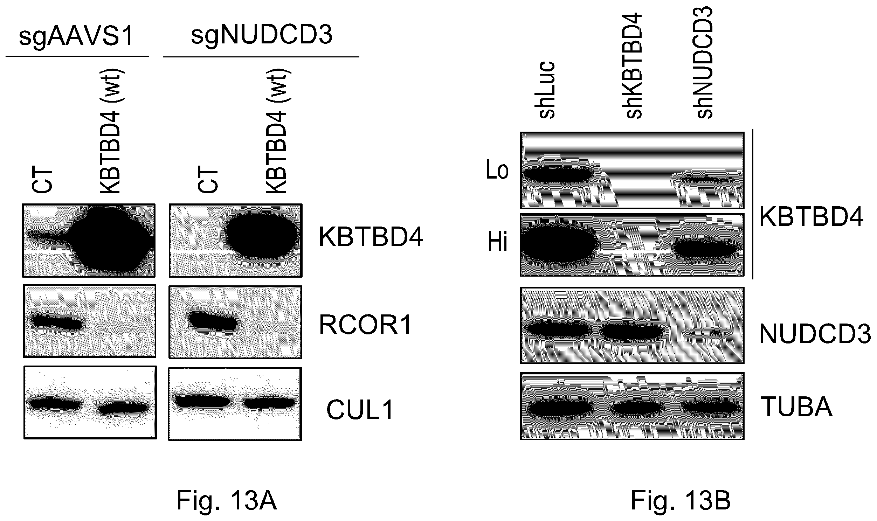

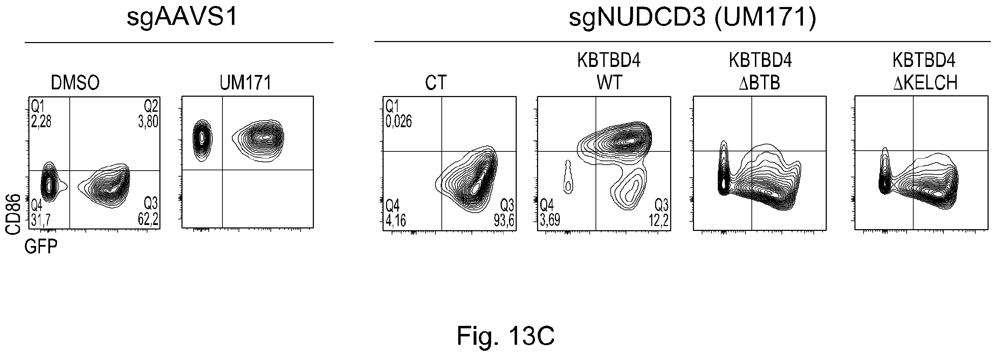

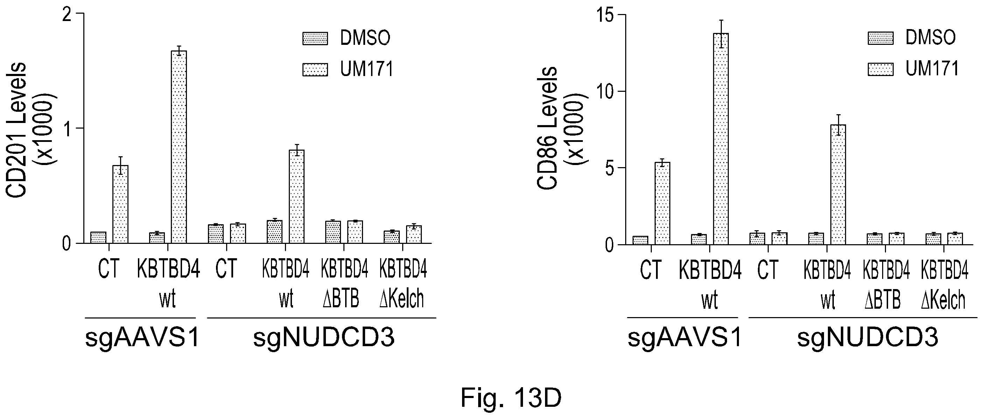

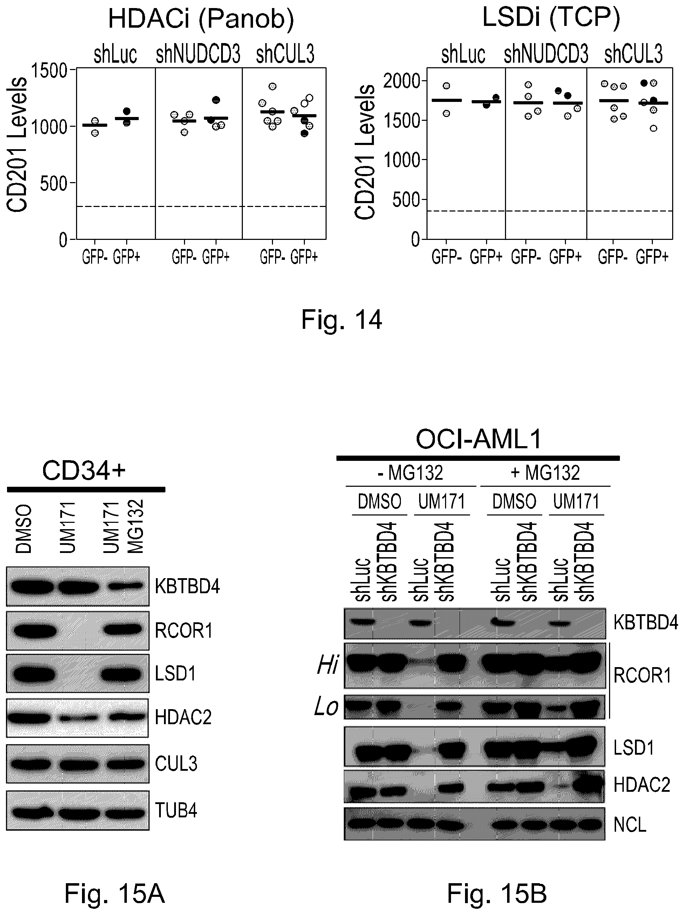

To further understand the epistatic relationship between KBTBD4 and the reported RCOR1-HDAC1/2-LSD1 complex, it is first demonstrated that RCOR1 levels are reduced in AML1 cells engineered to express high levels of KBTBD4 and that both Kelch (substrate-binding) and BTB (CUL3-binding) domains are essential (Fig. 11A). Furthermore, UM171 treatment leads to important reduction in RCOR1 (Fig. 11B and Fig. 12), LSD1 and HDAC2 protein levels in a KBTBD4-dependent manner (Fig. 11B, 3^{rd} and 4^{th} lanes and graph below). This effect is reduced in the presence of the proteasome inhibitor MG132 (Fig. 11B, lanes 5-8 and Fig. 12A). Although CULLIN3 neddylation (activation) appears normal in UM171 treated cells (Fig. 11C, see band labelled as CUL3^{NEDD8} in 2^{nd} lane), it is noteworthy that RCOR1 levels are normal in UM171 treated cells if exposed to the neddylation inhibitor MLN2494 indicating that CUL3 activation is required for RCOR1 loss (Fig. 11C, lane 4). It is further demonstrated that the neddylation-dependent exchanger CAND1 and the kelch co-chaperone NUDCD3 are essential for UM171-induced loss of RCOR1 (compare in Fig. 11D RCOR1 levels in lane 5 (shLuc) versus lane 6 (shKBTBD4), lane 7 (shNUDCD3) and lane 8 (shCAND1) and quantification in the graph below). CUL3, CAND1 and NUDCD3, found in the CRISPR screen are indeed necessary for UM171-induced loss of RCOR1 protein (Fig. 11D) and for induction of CD201 and CD86 expression (Fig. 11E-F). A link between the NUDCD3 co-chaperone and Kelch domain proteins was previously established (Taipale et al., 2014, Cell, 158: 434-448). It is shown that loss of NUDCD3, either through sgRNA or shRNA, leads to reduction in KBTBD4 protein levels (Fig. 13A-B) and that KBTBD4 gain of function partially rescues the UM171-induced CD201 and CD86 expression in the absence of NUDCD3 (Fig. 13C-D). Consistently, both HDAC and LSD1 inhibition restore CD201 expression in cells engineered to express low levels of NUDCD3 or CULLIN 3 (Fig. 14), similarly to what was observed in KBTBD4 null context.

In summary, using UM171 CRISPR-based screens and BiolD-directed proteomics, it is provided a novel CRL3 complex that uses the hitherto uncharacterized KBTBD4 adaptor leading to RCOR1 protein loss. This, in turns, promotes the upregulation of several HSC essential genes including CD201 and CD86 through the downstream modulation of chromatin-bound LSD1-RCOR1-HDAC epigenetic modifiers (Fig. 11B) possibly explaining the correlation between UM171 treatment and exposure to HDAC or LSD1 inhibitors.

Using purified human CD34+ cells, UM171 treatment reduces RCOR1 and LSD1 levels within 4 hours. HDAC2 levels were less affected in these cells at this time point (Fig. 15A). Importantly, MG132 co-treatment abolishes the loss of LSD1 and RCOR1 proteins (Fig. 15A). Furthermore, it is demonstrated that consistent with the results obtained with CD34+ cells, levels of LSD1, RCOR1 and this time HDAC2 were rapidly reduced upon UM171 exposure (lane 3 Fig. 15B).

Together these results document that UM171 operates through KBTBD4 to rapidly (within 4 hours) induce the degradation of CoREST members.

As seen in Table 1 below, UM171 and a variant (UM681) were tested to evaluate their ability in inhibiting cell proliferation in various cancerous cell line.

**Table 1**

| Antineoplastic effect of UM171 and UM681 on cell proliferation of cancerous cell lines | | |
|---|---|---|
| Cell lines | UM171 | UM681 |
| SKNO | A | C |
| KBM7 | C | D |
| MUTZ-8 | C | D |
| UT-7 | C | D |
| EOL1 | A | B |
| MOLM-13 | A | B |
| NB-4 | B | C |
| MUTZ-2 | B | C |
| AML3 | B | C |
| AML4 | B | B |
| KG1 | C | C |
| KG1a | C | C |
| HL-60 | C | C |
| MonoMac | B | C |
| AML5 | B | C |
| KU-812 | C | C |
| Z-138 | C | C |
| LY-1 | C | D |

| | | |
|---|---|---|
| A : IC50 less than 250 nM B : IC50 between 250 nM and 1 uM C: IC50 between 1 uM and 10 uM D: IC50 greater than 10 uM | | |

UM134 and UM681 have the following structure:

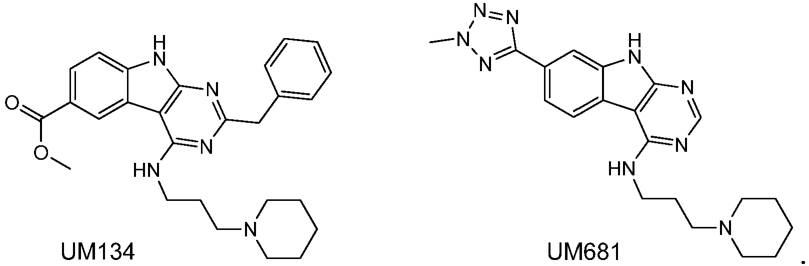

Further, as seen in Table 2, the antineoplastic effect of UM171, and two variants (UM681 and UM134) is also demonstrated in cancerous samples (acute myelogenous leukemia or "AML" samples) from patients.

**Table 2**

| .Antineoplastic effect of UM171 on cell proliferation of cancerous samples from patients | | | |
|---|---|---|---|
| AML samples | UM681 | UM171 | UM134 |
| 13H150 | C | A | C |
| 04H063 | C | B | C |
| 05H094 | C | B | C |
| 05H149 | C | B | C |
| 06H045 | C | A | C |
| 06H088 | D | B | C |
| 06H117 | C | B | C |
| 07H005 | C | A | C |
| 07H038 | C | B | C |
| 07H045 | D | B | C |
| 07H125 | C | B | C |
| 07H135 | C | B | C |
| 07H151 | D | C | C |
| 09H010 | C | B | C |
| 09H079 | C | B | C |
| 09H115 | C | A | C |
| 10H127 | C | B | C |
| 11H002 | C | B | C |
| 11H046 | B | A | C |
| 11H103 | C | B | C |

| AML samples | UM681 | UM171 | UM134 |
|---|---|---|---|
| 12H045 | C | B | C |
| 12H058 | C | B | C |
| 12H096 | D | B | C |
| 12H106 | D | C | C |
| 12H151 | C | A | C |
| 13H100 | C | B | C |
| 08H018 | D | C | C |
| 09H046 | D | C | C |
| 10H022 | B | A | C |
| CB | C | B | C |

| | | | |
|---|---|---|---|
| A : IC50 less than 250 nM B : IC50 between 250 nM and 1 uM C: IC50 between 1 uM and 10 uM D: IC50 greater than 10 uM | | | |

As demonstrated herein, UM171 and its derivatives activate the CULLIN3-RING ubiquitin ligase complex (CRL3 complex described herein). The CRL3/KBTBD4 complex degrades RCOR1 which normally acts as the scaffolding protein for the RCOR1/LSD1 and HDAC2 complex, itself being dissociated in the presence of UM171.

Accordingly, it is encompassed a method of treating cancer in a subject following administration of the compound of general formula I as defined herein: 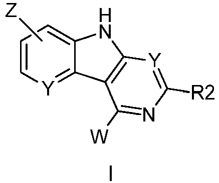
or a salt or a prodrug thereof,
wherein:
   each Y is independently selected from N and CH;
   Z is -CN; -C(O)OR1; -C(O)N(R1)R3; -C(O)R1; or -heteroaryl optionally substituted with one or more RA or R4 substituents, wherein, when (R1) and R3 are attached to a nitrogen atom, optionally they join together with the nitrogen atom to form a 3 to 7-membered ring which optionally includes one or more other heteroatom selected from N, O and S, optionally the ring is substituted with one or more RA or R4;
   W is -CN; -N(R1)R3; -C(O)OR1; -C(O)N(R1)R3; -NR1C(O)R1; -NR1C(O)OR1; - OC(O)N(R1)R3; -OC(O)R1; -C(O)R1; -NR1C(O)N(R1)R3; -NR1S(O)₂R1; -benzyl optionally substituted with 1, 2 or 3 RA or R1 substituents; -X-L-(X-L)n; -N(R1)R3; -X-L-(X-L)n - heteroaryl optionally substituted with one or more RA or R4 substituents **attached** on either or both the L and heteroaryl groups; -X-L-(X-L)n - heterocyclyl optionally substituted with one or more RA or R4 substituents attached on either or both the L and heterocyclyl groups; -X-L-(X-L)n- aryl optionally substituted with one or more RA or R4 substituents; -X-L-(X-L)ₙ-NR1RA or -(N(R1)-L)ₙ - N⁺R1R3R5 R6⁻, wherein n is an integer equal to either 0, 1, 2, 3, 4, or 5,
   and wherein, when R1 and R3 are attached to a nitrogen atom, optionally they join together with the nitrogen atom to form a 3 to 7-membered ring which optionally includes one or more other heteroatom selected from N, O and S, optionally the ring is substituted with one or more RA or R4;
   each X is independently selected from C, O, S, and NR1;
   L is each independently -C₁₋₆ alkylene; -C₂₋₆ alkenylene; -C₂₋₆ alkynylene; -C₃₋₇ cycloalkylene, which optionally includes one or more other heteroatom selected from N, O and S; or -C₃₋₇ cycloalkenylene, which optionally includes one or more other heteroatom selected from N, O and S, wherein the alkylene, the alkenylene, the alkynylene, the cycloalkylene and the cycloalkenylene groups are each independently optionally substituted with one or two R4 or RA substituent;
   R1 is each independently -H; -C₁₋₆ alkyl; -C₂₋₆ alkenyl; -C₂₋₆ alkynyl; -C₃₋₇ cycloalkyl; -C₃₋₇ cycloalkenyl; -C₁₋₅ perfluorinated; -heterocyclyl; -aryl; -heteroaryl; or -benzyl, wherein the alkyl, the alkenyl, the alkynyl, the cycloalkenyl, the perfluorinated alkyl, the heterocyclyl, the aryl, the heteroaryl and the benzyl groups are each independently optionally substituted with 1, 2 or 3 RA or Rd substituents;
   R2 is -H; -C₁₋₆ alkyl, optionally substituted with one more RA substituents; -C(O)R4; -L-heteroaryl optionally substituted with one or more RA or R4 substituents; -L-heterocyclyl optionally substituted with one or more RA or R4; or -L-aryl optionally substituted with one or more RA or R4 substituents;
   R3 is each independently -H; -C₁₋₆ alkyl; -C₂₋₆ alkenyl; -C₂₋₆ alkynyl; -C₃₋₇ cycloalkyl; -C₃₋₇ cycloalkenyl; -C₁₋₅ perfluorinated; -heterocyclyl; -aryl; -heteroaryl; or -benzyl, wherein the alkyl, the alkenyl, the alkynyl, the cycloalkyl, the cycloalkenyl, the perfluorinated alkyl, the heterocyclyl, the aryl, the heteroaryl and the benzyl groups are each independently optionally substituted with 1, 2 or 3 RA or Rd substituents;
   R4 is each independently -H; -C₁₋₆ alkyl; -C₂₋₆ alkenyl; -C₂₋₆ alkynyl; -C₃₋₇ cycloalkyl; -C₃₋₇ cycloalkenyl; -C₁₋₅ perfluorinated; -heterocyclyl; -aryl; -heteroaryl, or -benzyl; wherein the alkyl, the alkenyl, the alkynyl, the cycloalkyl, the cycloalkenyl, the perfluorinated alkyl, the heterocyclyl, the aryl, the heteroaryl and the benzyl groups are each independently optionally substituted with 1, 2 or 3 RA or Rd substituents;
   R5 is each independently -C₁₋₆ alkyl; -C₁₋₆ alkylene-C₂₋₆ alkenyl which optionally includes one or more other heteroatom selected from N, O and S; -C₁₋₆ alkylene-C₂₋₆ alkynyl which optionally includes one or more other heteroatom selected from N, O and S; -L-aryl which optionally includes one or more RA or R4 substituents; -L-heteroaryl which optionally includes one or more RA or R4 substituents; -C₁₋₆ alkylene-C(O)O-; -C₁₋₆ alkylene-C(O)OR1; -C₁₋₆ alkylene-CN; -C₁₋₆ alkylene-C(O)NR1R3, wherein R1 and R3 optionally they join together with the nitrogen atom to form a 3 to 7-membered ring which optionally includes one or more other heteroatom selected from N, O and S; or - C₁₋₆ alkylene-OH;
   R6 is halogen; -OC(O)CF₃; or -OC(O)R1;
   RA is each independently -halogen; -CF₃; -OR1; -L-OR1; -OCF₃; -SR1; -CN; -NO₂; - NR1R3; -L-NR1R1; -C(O)OR1; -S(O)₂R4; -C(O)N(R1)R3; -NR1C(O)R1; - NR1C(O)OR1; -OC(O)N(R1)R3; -OC(O)R1; -C(O)R4; -NHC(O)N(R1)R3; - NR1C(O)N(R1)R3; or -N₃; and
   Rd is each independently -H; -C₁₋₆ alkyl; -C₂₋₆ alkenyl; -C₂₋₆ alkynyl; -C₃₋₇ cycloalkyl; -C₃₋₇ cycloalkenyl; -C₁₋₅ perfluorinated; -benzyl; or -heterocyclyl.

In one embodiment, the compound has the formula:

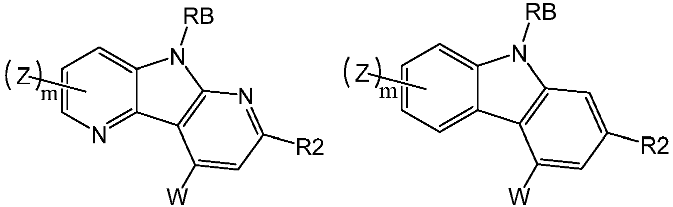

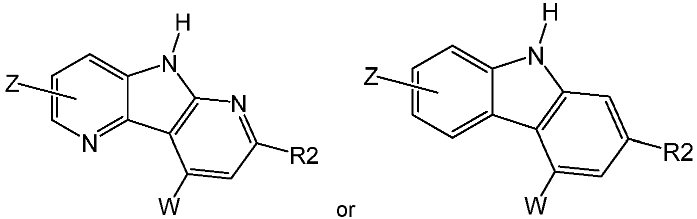

In one embodiment, in any formula herein comprising m substituents Z, m is an integer of 1 or 2.

In one embodiment, Z is -C(O)OR1, or -heteroaryl optionally substituted with one or more RA or R1 substituents, R2 is H, -C₁₋₆ alkyl optionally substituted with one or more RA substituents or -L-aryl optionally substituted with one or more RA or R4 substituents, W is -N(R1)R3 wherein R1 is C₃₋₇ cycloalkyl substituted by RA and R3 is H.

In one embodiment, Z is -C(O)O-C₁₋₄ alkyl or 5-membered ring heteroaryl, the heteroaryl comprising 2-4 heteroatoms (N or O), R2 is H, or -L-aryl optionally substituted by halogen, OR1, C₁₋₆ alkyl optionally substituted by RA, C(O)R4, - heterocyclyl, C(O)OR4 OR C₂₋₆ alkynyl, W is -N(R1)R3 wherein R1 is cyclohexyl substituted by RA, and R3 is H.

In accordance with another embodiment,
Z is CO₂Me or 2-methyl-2H-tetrazol-5-yl;
R2 is benzyl, or H; and
W is NH-L-N(R1)R3 wherein L is C₂₋₄ alkylene or C₃₋₇ cycloalkylene and R1 and R3 is C₁₋₄ alkyl or H; or R1 and R3 join together with the nitrogen atom to which they are attached to form a 3 to 7-membered ring, which optionally includes one or more other heteroatom selected from N, O and S, optionally the ring is substituted with one or more RA or R4.

In accordance with another embodiment,
Z is -C(O)O-C₁₋₄ alkyl or 5-membered ring heteroaryl, the heteroaryl comprising 2-4 heteroatoms (N or O) or an aryl comprising 6 carbon atoms;
R2 is = H, or -CH₂-aryl optionally substituted by substituted by halogen, OR1, C₁₋₆ alkyl optionally substituted by RA, C(O)R4, -heterocyclyl, C(O)OR4 OR C₂₋₆ alkynyl, wherein the aryl is phenyl; or R2 is -C₁₋₆ alkylene-heteroaryl or -C₁₋₆ alkylene-aryl, optionally substituted with one or more RA or R4 substituents; and
W is -X-L-(X-L)n-N(R1)R3, X= NR1 wherein R1 is H or C₁₋₆ alkyl (such as a methyl), n=0, (R1) and R3 are attached to the nitrogen atom to form a 3 to 7-membered ring (such as a pyperidinyl or pyrrolidinyl ring) and L is -C₁₋₆ alkylene (such as a propyl or ethyl chain), or W= -X-L-(X-L)n-NR1RA, X=NR1, wherein R1 is H or C₁₋₆ alkyl (such as a methyl), n=0, R1 and RA = respectively -H, or -C₁₋₆ alkyl (for R1) and RA is as described herein, and for example as illustrated in table 3 herein.

In accordance with another embodiment,
Z is COOMe, COOEt, methyl-tetrazole, ethyl-tetrazole or methyl-oxadiazole, thiophenyl, or phenyl;
R2 is H, -CH₃, -CH₂N(CH₃)₂, -CH₂-phenyl, -CH₂CH₂-phenyl, -CH₂-thiophenyl, -CH₂-pyridyl, CH₂-cyclohexyl, -NH-phenyl, -CH(Obenzyl)-phenyl, -CH(OH)-phenyl, - CH(CH₃)-phenyl,-C(O)-phenyl, -CH₂naphthyl, optionally substituted with one or more RA or R4 substituents; and
W is -X-L-(X-L)n-N(R1)R3, X= NR1 wherein R1 is H or C₁₋₆ alkyl (such as a methyl), n=0, (R1) and R3 are attached to the nitrogen atom to form a 3 to 7-membered ring (such as a pyperidinyl or pyrrolidinyl ring) and L is -C₁₋₆ alkylene (such as a propyl or ethyl chain), or W= -X-L-(X-L)n-NR1RA, X=NR1, wherein R1 is H or C₁₋₆ alkyl (such as a methyl), n=0, R1 and RA = respectively -H, or -C₁₋₆ alkyl (for R1) and RA is as described herein, and for example as illustrated in table 3 herein, and in particular W =

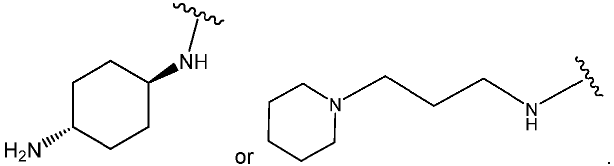

In one embodiment, Z is -C(O)O-C₁₋₄ alkyl or 5-membered ring heteroaryl, the heteroaryl comprising 2-4 heteroatoms (N or O) or an aryl comprising 6 carbon atoms.

In one embodiment, Z is COOMe, COOEt, methyl-tetrazole, ethyl-tetrazole or methyl-oxadiazole, thiophenyl, or phenyl.

In one embodiment, Z is COOMe, COOEt, tetrazole or oxadiazole.

In one embodiment, R2 is = H, or -CH₂-aryl optionally substituted by substituted by halogen, OR1, C₁₋₆ alkyl optionally substituted by RA, C(O)R4, - heterocyclyl, C(O)OR4 OR C₂₋₆ alkynyl, wherein the aryl is phenyl.

In one embodiment, R2 is H, -C₁₋₆ alkylene-heteroaryl or -C₁₋₆ alkylene-aryl, optionally substituted with one or more RA or R4 substituents.

In one embodiment, R2 is H, -CH₃, -CH₂N(CH₃)₂, -CH₂-phenyl, -CH₂CH₂-phenyl, -CH₂-thiophenyl, -CH₂-pyridyl, CH₂-cyclohexyl, -NH-phenyl, -CH(Obenzyl)-phenyl, -CH(OH)-phenyl, -CH(CH₃)-phenyl,-C(O)-phenyl, -CH₂naphthyl, optionally substituted with one or more RA or R4 substituents.

In accordance with another embodiment, W= -X-L-(X-L)n-N(R1)R3, X= NR1 wherein R1 is H or C₁₋₆ alkyl (such as a methyl), n=0, (R1) and R3 are attached to the nitrogen atom to form a 3 to 7-membered ring (such as a pyperidinyl or pyrrolidinyl ring) and L is -C₁₋₆ alkylene (such as a propyl or ethyl chain),

In one embodiment, W= -X-L-(X-L)n-NR1RA, X=NR1, wherein R1 is H or C₁₋₆ alkyl (such as a methyl), n=0, R1 and RA = respectively -H, or -C₁₋₆ alkyl (for R1) and RA is as described herein, and for example as illustrated in table 3 herein.

In accordance with another embodiment, the compound is of Formula I wherein W is

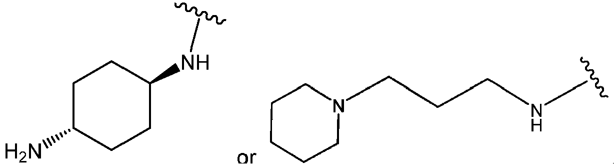

The compounds of formula I (including the representative compounds set forth below) disclosed herein, including the preparation and characterization thereof, are described in PCT publication No. WO 2013/110198.

As used herein, the term "alkyl" is intended to include both branched and straight chain saturated aliphatic hydrocarbon groups having the specified number of carbon atoms, for example, C1-C6 in C1-C6 alkyl is defined as including groups having 1, 2, 3, 4, 5 or 6 carbons in a linear or branched saturated arrangement. Examples of C1-C6 alkyl as defined above include, but are not limited to, methyl, ethyl, n-propyl, i-propyl, n-butyl, t-butyl, i-butyl, pentyl, and hexyl.

As used herein, the term "cycloalkyl" is intended to mean a monocyclic saturated aliphatic hydrocarbon group having the specified number of carbon atoms therein, for example, C3-C7 in C3-C7 cycloalkyl is defined as including groups having 3, 4, 5, 6 or 7 carbons in a monocyclic saturated arrangement. Examples of C3-C7 cycloalkyl as defined above include, but are not limited to, cyclopropyl, cyclobutyl, cyclopentyl, cyclohexyl and cycloheptyl.

As used herein, the term, "alkenyl" is intended to mean unsaturated straight or branched chain hydrocarbon groups having the specified number of carbon atoms therein, and in which at least two of the carbon atoms are bonded to each other by a double bond, and having either E or Z regiochemistry and combinations thereof. For example, C2-C6 in C2-C6 alkenyl is defined as including groups having 2, 3, 4, 5 or 6 carbons in a linear or branched arrangement, at least two of the carbon atoms being bonded together by a double bond. Examples of C2-C6 alkenyl include, but are not limited to, ethenyl (vinyl), 1-propenyl, 2-propenyl, 1-butenyl and the like.

As used herein, the term "alkynyl" is intended to mean unsaturated, straight chain hydrocarbon groups having the specified number of carbon atoms therein and in which at least two carbon atoms are bonded together by a triple bond. For example C2-C4 alkynyl is defined as including groups having 2, 3 or 4 carbon atoms in a chain, at least two of the carbon atoms being bonded together by a triple bond. Examples of such alkynyl include, but are not limited to, ethynyl, 1-propynyl, 2-propynyl and the like.

As used herein, the term "cycloalkenyl" is intended to mean a monocyclic saturated aliphatic hydrocarbon group having the specified number of carbon atoms therein, for example, C3-C7 in C3-C7 cycloalkenyl is defined as including groups having 3, 4, 5, 6 or 7 carbons in a monocyclic arrangement. Examples of C3-C7 cycloalkenyl as defined above include, but are not limited to, cyclopentenyl, cyclohexenyl and the like.

As used herein, the term "halo" or "halogen" is intended to mean fluorine, chlorine, bromine or iodine.

As used herein, the term "haloalkyl" is intended to mean an alkyl as defined above, in which each hydrogen atom may be successively replaced by a halogen atom. Examples of haloalkyl include, but are not limited to, CH₂F, CHF₂ and CF₃.

As used herein, the term "aryl," either alone or in combination with another radical, means a carbocyclic aromatic monocyclic group containing 6 carbon atoms which may be further fused to a second 5- or 6-membered carbocyclic group which may be aromatic, saturated or unsaturated. Examples of aryl include, but are not limited to, phenyl, indanyl, 1-naphthyl, 2-naphthyl, tetrahydronaphthyl and the like. The aryl may be connected to another group either at a suitable position on the cycloalkyl ring or the aromatic ring.

As used herein, the term "heteroaryl" is intended to mean a monocyclic or bicyclic ring system of up to 10 atoms, wherein at least one ring is aromatic, and contains from 1 to 4 hetero atoms selected from the group consisting of O, N, and S. The heteroaryl may be attached either via a ring carbon atom or one of the heteroatoms. Examples of heteroaryl include, but are not limited to, thienyl, benzimidazolyl, benzo[b]thienyl, furyl, benzofuranyl, pyranyl, isobenzofuranyl, chromenyl, xanthenyl, 2H-pyrrolyl, pyrrolyl, imidazolyl, pyrazolyl, pyridyl, pyrazinyl, pyrimidinyl, pyridazinyl, indolizinyl, isoindolyl, 3H-indolyl, indolyl, indazolyl, purinyl, 4H-quinolizinyl, isoquinolyl, quinolyl, phthalazinyl, napthyridinyl, quinoxalinyl, quinazolinyl, cinnolinyl, pteridinyl, isothiazolyl, isochromanyl, chromanyl, isoxazolyl, furazanyl, indolinyl, isoindolinyl, thiazolo[4,5-b]-pyridine, tetrazolyl, oxadiazolyl, thiadiazolyl, thienyl, pyrimido-indolyl, pyrido-indolyl, pyrido-pyrrolo-pyrimidinyl, pyrrolo-dipyridinyl and fluoroscein derivatives.

As used herein, the term "heterocycle," "heterocyclic" or "heterocyclyl" is intended to mean a 3, 4, 5, 6, or 7 membered non-aromatic ring system containing from 1 to 4 heteroatoms selected from the group consisting of O, N and S. Examples of heterocycles include, but are not limited to, pyrrolidinyl, tetrahydrofuranyl, piperidyl, 3,5-dimethylpiperidyl, pyrrolinyl, piperazinyl, imidazolidinyl, morpholinyl, imidazolinyl, pyrazolidinyl, pyrazolinyl, tetrahydro-1H-thieno[3,4-d]imidazole-2(3H)-one, diazirinyl, and the like, where the attachment to the ring can be on either the nitrogen atom or a carbon atom of the ring such as described hereafter:

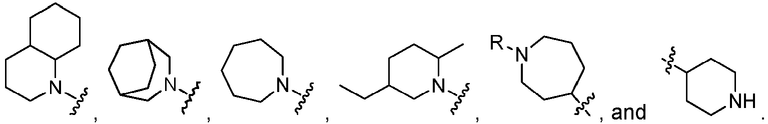

As used herein, the term "optionally substituted with one or more substituents" or its equivalent term "optionally substituted with at least one substituent" is intended to mean that the subsequently described event of circumstances may or may not occur, and that the description includes instances where the event or circumstance occurs and instances in which it does not. The definition is intended to mean from zero to five substituents.

As used herein, the term "subject" or "patient" is intended to mean humans and non-human mammals such as primates, cats, dogs, swine, cattle, sheep, goats, horses, rabbits, rats, mice and the like.

If the substituents themselves are incompatible with the synthetic methods described herein, the substituent may be protected with a suitable protecting group (PG) that is stable to the reaction conditions used in these methods. The protecting group may be removed at a suitable point in the reaction sequence of the method to provide a desired intermediate or target compound. Suitable protecting groups and the methods for protecting and de-protecting different substituents using such suitable protecting groups are well known to those skilled in the art; examples of which may be found in T. Greene and P. Wuts, "Protecting Groups in Chemical Synthesis" (4th ed.), John Wiley & Sons, NY (2007). Examples of protecting groups used throughout include, but are not limited to, Fmoc, Bn, Boc, CBz and COCF₃. In some instances, a substituent may be specifically selected to be reactive under the reaction conditions used in the methods described herein. Under these circumstances, the reaction conditions convert the selected substituent into another substituent that is either useful in an intermediate compound in the methods described herein or is a desired substituent in a target compound.

As used herein, the term "pharmaceutically acceptable salt" is intended to mean both acid and base addition salts.

As used herein, the term "pharmaceutically acceptable acid addition salt" is intended to mean those salts which retain the biological effectiveness and properties of the free bases, which are not biologically or otherwise undesirable, and which are formed with inorganic acids such as hydrochloric acid, hydrobromic acid, sulfuric acid, nitric acid, phosphoric acid and the like, and organic acids such as acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like.

As used herein, the term "pharmaceutically acceptable base addition salt" is intended to mean those salts which retain the biological effectiveness and properties of the free acids, which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Salts derived from organic bases include, but are not limited to, salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, dicyclohexylamine, lysine, arginine, histidine, caffeine, procaine, hydrabamine, choline, betaine, ethylenediamine, glucosamine, methylglucamine, theobromine, purines, piperazine, piperidine, N-ethylpiperidine, polyamine resins and the like.

The compounds encompassed herein or their pharmaceutically acceptable salts may contain one or more asymmetric centers, chiral axes and chiral planes and may thus give rise to enantiomers, diastereomers, and other stereoisomeric forms and may be defined in terms of absolute stereochemistry, such as (R)- or (S)- or, as (D)- or (L)- for amino acids. The present is intended to include all such possible isomers, as well as, their racemic and optically pure forms. Optically active (+) and (-), (R)- and (S)- or (D)- and (L)-isomers may be prepared using chiral synthons or chiral reagents, or resolved using conventional techniques, such as reverse phase HPLC. The racemic mixtures may be prepared and thereafter separated into individual optical isomers or these optical isomers may be prepared by chiral synthesis. The enantiomers may be resolved by methods known to those skilled in the art, for example by formation of diastereoisomeric salts which may then be separated by crystallization, gas-liquid or liquid chromatography, selective reaction of one enantiomer with an enantiomer specific reagent. It will also be appreciated by those skilled in the art that where the desired enantiomer is converted into another chemical entity by a separation technique, an additional step is then required to form the desired enantiomeric form. Alternatively specific enantiomers may be synthesized by asymmetric synthesis using optically active reagents, substrates, catalysts, or solvents or by converting one enantiomer to another by asymmetric transformation.

Certain compounds encompassed herein may exist as a mix of epimers. Epimers means diastereoisomers that have the opposite configuration at only one of two or more stereogenic centers present in the respective compound.

Compounds encompassed herein may exist in Zwitterionic form and the present includes Zwitterionic forms of these compounds and mixtures thereof.

In addition, the compounds encompassed herein also may exist in hydrated and anhydrous forms. Hydrates of the compound of any of the formulas described herein are included. In a further embodiment, the compound according to any of the formulas described herein is a monohydrate. In embodiments, the compounds described herein comprise about 10% or less, about 9 % or less, about 8% or less, about 7% or less, about 6% or less, about 5% or less, about 4% or less, about 3% or less, about 2% or less, about 1% or less, about 0.5% or less, about 0.1% or less by weight of water. In other embodiments, the compounds described herein comprise, about 0.1% or more, about 0.5% or more, about 1% or more, about 2% or more, about 3% or more, about 4% or more, about 5% or more, or about 6% or more by weight of water.

It may be convenient or desirable to prepare, purify, and/or handle the compound in the form of a prodrug. Thus, the term "prodrug", as used herein, pertains to a compound which, when metabolized (e.g., *in vivo*), yields the desired active compound. Typically, the prodrug is inactive, or less active than the desired active compound, but may provide advantageous handling, administration, or metabolic properties. Unless otherwise specified, a reference to a particular compound also includes prodrugs thereof.

In one embodiment, the compounds as defined herein or a pharmaceutically acceptable salt thereof, are provided in association with one or more pharmaceutically acceptable carrier.

Many pharmaceutically acceptable carriers are known in the art. It will be understood by those in the art that a pharmaceutically acceptable carrier must be compatible with the other ingredients of the formulation and tolerated by a subject in need thereof. The pharmaceutical compositions can be formulated according to known methods for preparing pharmaceutically useful compositions. Carriers are described in a number of sources which are well known and readily available to those skilled in the art.

The proportion of each carrier is determined by the solubility and chemical nature of the agent(s), the route of administration, and standard pharmaceutical practice.

In order to ensure consistency of administration, in an embodiment, the pharmaceutical composition may be in the form of a unit dose.

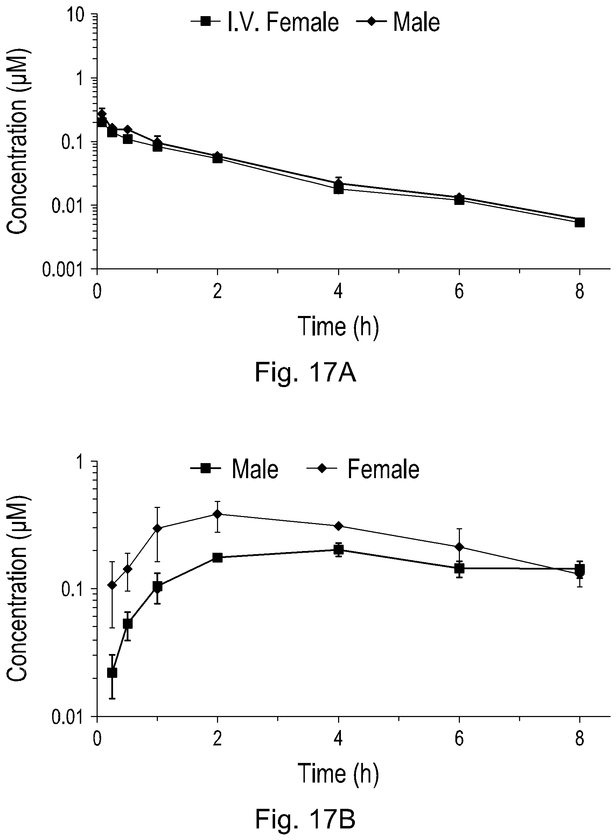

The compounds or composition may be for oral administration as exemplified in Fig. 17B in the form of solid oral composition/ dosage form such as tablets, capsules, or granules, containing pharmaceutically acceptable carriers. The compounds or composition may also be for sublingual administration.

The solid oral compositions/dosage form may be prepared by conventional methods of blending, filling, tabletting, or the like. Repeated blending operations may be used to distribute the active agent(s) throughout those compositions employing carriers. Such operations are, of course, conventional in the art.

The solid oral compositions/dosage forms may be coated according to methods well known in normal pharmaceutical practice, in particular with an enteric coating.

Oral liquid preparations may be in the form of emulsions, syrups, or elixirs, or may be presented as a dry product for reconstitution with water or other suitable vehicles before use. Such liquid preparations may or may not contain conventional additives.

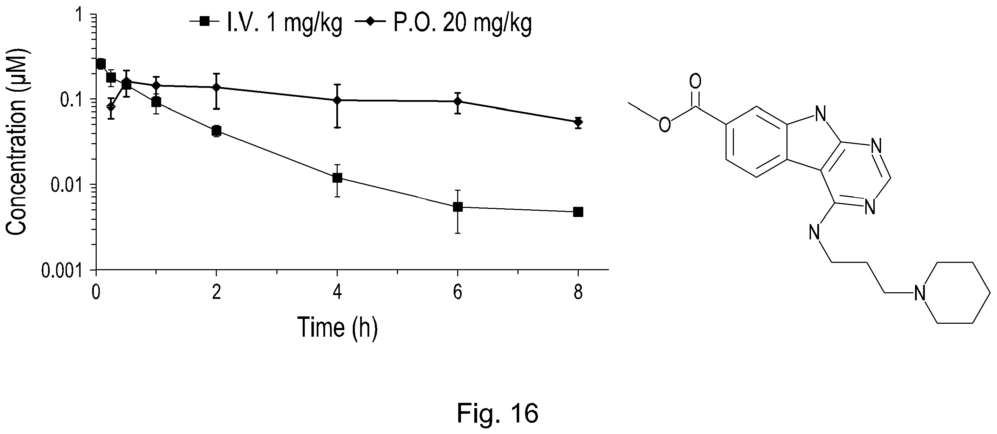

The compounds or composition may be for parenteral injection; this being intramuscularly, intravenously (as exemplified in Figs. 16 and 17A), or subcutaneously.

For parenteral administration, the compounds or composition may be used in the form of sterile solutions, optionally containing solutes, for example sufficient saline or glucose to make the solution isotonic. The compounds or composition for parenteral injection may be prepared by utilizing the compounds and a sterile vehicle, and, depending on the concentration employed, the compounds may be either suspended or dissolved in the vehicle. Once in solution, the compounds may be injected and filter sterilized before filling a suitable vial or ampoule followed by subsequently sealing the carrier or storage package.

In one embodiment, the compound or pharmaceutical composition comprising said compounds can be further used in combination with at least one additional active ingredient.

It is encompassed that the compound encompassed herein be administered orally, intravenously, or subcutaneously.

In a particular embodiment, the compound of formula I is UM171 which as the following structure:

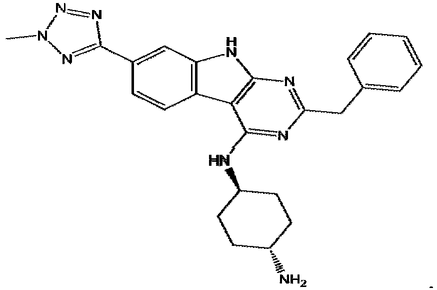

In an embodiment, the compound of formula I is 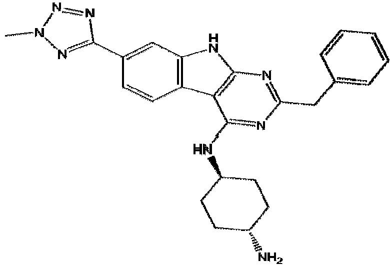 or a pharmaceutically acceptable salt thereof.

In another embodiment, the compound of formula I is a hydrobromide salt of

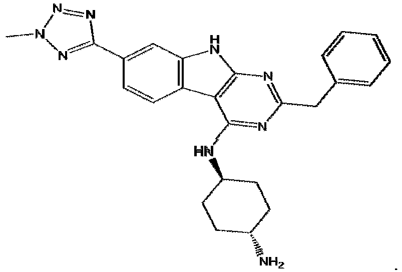

In a supplemental embodiment, the compound of formula I is 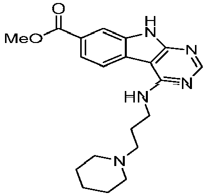 or a pharmaceutically acceptable salt thereof.

The compounds pyrimido[4,5-B]indole derivatives disclosed herein may for example be prepared according to U.S. patent nos. 9,409,906 and 10,336,747. The 5H-pyrido[4,3-b]indole derivatives and pyrido[2',3':4,5]pyrrolo[2,3-d]pyrimidine derivatives may for example be prepared according to US 10,647,718 B.

Alternatively, representative compounds disclosed herein can be prepared according to the following chemistry examples. It will be apparent to the skilled person that the other compounds may be prepard using similar conditions, using different starting materials.

### CHEMISTRY EXAMPLES

### Example 1

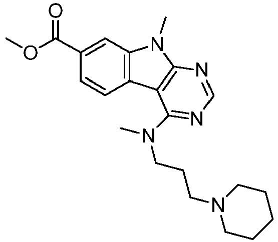

### Methyl 9-methyl-4-(methyl(3-(piperidin-1-yl)propyl)amino)-9H-pyrimido[4,5-b]indole-7-carboxylate

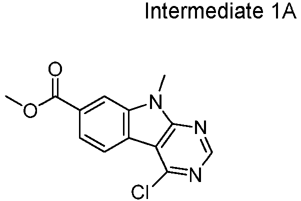

### Methyl 4-chloro-9-methyl-9H-pyrimido[4,5-b]indole-7-carboxylate

In a 100 mL round-bottomed flask were added methyl 4-chloro-9H-pyrimido[4,5-b]indole-7-carboxylate (0.1 g, 0.382 mmol) and N,N-Dimethylformamide dimethyl acetal (0.297 ml, 2.217 mmol) in toluene (35 ml) to give a tan suspension. Heated to 110 °C for 17 hours. The reaction mixture was cooled to 20 °C and concentrated to dryness to give 94 mg as a tan solid. The residue was purified on ISCO (RediSep 12 g column eluting with CH2Cl₂/EtOAc) to give methyl 4-chloro-9-methyl-9H-pyrimido[4,5-b]indole-7-carboxylate (53 mg, 0.192 mmol, 50.3 % yield) as a white solid. 1H NMR (400 MHz, DMSO-d6) δ ppm 3.96 (s, 3 H) 4.03 (s, 3 H) 8.08 (dd, J=8.2, 1.6 Hz, 1 H) 8.39 (dd, J=1.6, 0.8 Hz, 1 H) 8.46 (dd, J=8.2, 0.8 Hz, 1 H) 8.93 (s, 1 H). LCMS m/z 276.0 (M + H)+.

### Example 1

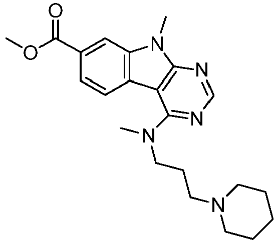

### Methyl 9-methyl-4-(methyl(3-(piperidin-1-yl)propyl)amino)-9H-pyrimido[4,5-b]indole-7-carboxylate

In a microwave vial was added methyl 4-chloro-9-methyl-9H-pyrimido[4,5-b]indole-7-carboxylate (0.02 g, 0.073 mmol), N-methyl-3-(piperidin-1-yl)propan-1-amine (0.024 g, 0.109 mmol) and triethylamine (0.020 ml, 0.145 mmol) in methanol (0.76 ml) to give a tan suspension. The vial was sealed and heated in the microwave to 140 °C for 15 minutes. The reaction mixture was concentrated to dryness to give a red oil. The residue was purified on ISCO (RediSep 4 g column eluting with CH₂Cl₂/MeOH/NH₄OH) to give methyl 9-methyl-4-(methyl(3-(piperidin-1-yl)propyl)amino)-9H-pyrimido[4,5-b]indole-7-carboxylate (21 mg, 0.053 mmol, 73.2 % yield) as a colorless oil. 1H NMR (400 MHz, DMSO-d6) δ ppm 1.13 - 1.33 (m, 6 H) 1.74 - 1.90 (m, 2 H) 2.00 - 2.25 (m, 6 H) 3.29 (s, 3 H) 3.82 (t, J=6.8 Hz, 2 H) 3.91 (s, 3 H) 3.92 (s, 3 H) 7.91 (dd, J=8.6, 1.6 Hz, 1 H) 8.02 (d, J=8.6 Hz, 1 H) 8.19 (d, J=1.6 Hz, 1 H) 8.45 (s, 1 H). HRMS m/z 396.2517 (M+H)+.

### Example 2

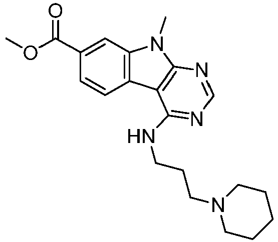

### methyl 9-methyl-4-((3-(piperidin-1-yl)propyl)amino)-9H-pyrimido[4,5-b]indole-7-carboxylate

The title compound was obtained according to the procedure described in example 1. 1H NMR (400 MHz, DMSO-d6) δ ppm 1.32 - 1.44 (m, 2 H) 1.45 - 1.57 (m, 4 H) 1.76 - 1.91 (m, 2 H) 2.25 - 2.45 (m, 6 H) 3.61 - 3.70 (m, 2 H) 3.89 (s, 3 H) 3.92 (s, 3 H) 7.49 (t, J=5.3 Hz, 1 H) 7.90 (dd, J=8.2, 1.6 Hz, 1 H) 8.19 (d, J=1.6 Hz, 1 H) 8.44 (s, 1 H) 8.46 (d, J=8.2 Hz, 1 H). HRMS m/z 382.2374 (M+H)+.

### Example 3

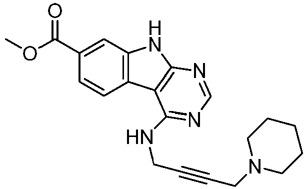

### Methyl 4-((4-(piperidin-1-yl)but-2-yn-1-yl)amino)-9H-pyrimido[4,5-b]indole-7-carboxylate

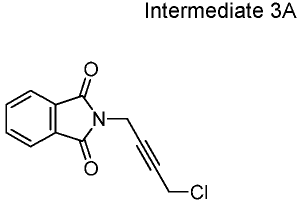

### 2-(4-Chlorobut-2-yn-1-yl)isoindoline-1,3-dione

In a 200 mL round-bottomed flask was added potassium 1,3-dioxoisoindolin-2-ide (4.75 g, 25.6 mmol) in DMF (30.0 ml) to give a white suspension. 1,4-dichlorobut-2-yne (20.06 ml, 205 mmol) was added. Heated the brown suspension to 100 °C. After 2 hrs, cooled to 20 °C. Water (50.0 ml) was added and concentrated to dryness to give a brown solid. The solid was partitioned between water (75 mL) and CH₂Cl₂ (50 mL). Separated layers and extracted aqueous layer with CH₂Cl₂ (50 mL). The combined organic layer were washed with water (50 mL). The organic layer was dried over MgSO₄, filtered and concentrated to give 6.8 g as a brown solid. The residue was purified on ISCO (RediSep 120 g column eluting with hexane/CH₂Cl₂) to give 2-(4-chlorobut-2-yn-1-yl)isoindoline-1,3-dione (3.90 g, 16.69 mmol, 65.1 % yield) as a white solid. 1H NMR (400 MHz, DMSO-d6) δ ppm 4.44 (t, J=2.0 Hz, 2 H) 4.47 (t, J=2.0 Hz, 2 H) 7.81 - 7.96 (m, 4 H). LCMS m/z 234.0 (M + H)+.

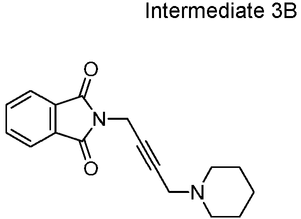

### 2-(4-(Piperidin-1-yl)but-2-yn-1-yl)isoindoline-1,3-dione

In a 100 mL round-bottomed flask were added 2-(4-chlorobut-2-yn-1-yl)isoindoline-1,3-dione (1 g, 4.28 mmol) and piperidine (0.932 ml, 9.42 mmol) in acetonitrile (15 ml) to give a yellow solution. Heated to 80 °C and stirred the resulting suspension for 16.5 hours. Concentrated to dryness to give an orange solid. Partitioned the mixture between CH₂Cl₂ (30 mL) and water (20 mL). Separated layers. Extracted aqueous with CH₂Cl₂ (10 mL). The combined organic layers were dried over MgSO4, filtered and concentrated to give 1.65 g as an orange oil. The residue was purified on ISCO (RediSep 40 g column eluting with CH₂Cl₂/EtOAc) to give 2-(4-(piperidin-1-yl)but-2-yn-1-yl)isoindoline-1,3-dione (752 mg, 2.66 mmol, 62.2 % yield) as a white solid. 1H NMR (400 MHz, DMSO-d6) δ ppm 1.31 (s, 2 H) 1.46 (quin, J=5.7 Hz, 4 H) 2.33 (s, 3 H) 3.18 (s, 2 H) 4.40 (t, J=2.2 Hz, 2 H) 7.84 - 7.89 (m, 2 H) 7.89 - 7.94 (m, 2 H). LCMS m/z 283.2 (M + H)+.

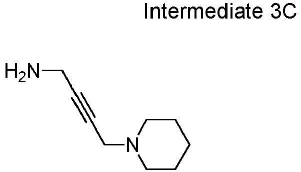

### 4-(Piperidin-1-yl)but-2-yn-1-amine

In a 50 mL round-bottomed flask were added 2-(4-(piperidin-1-yl)but-2-yn-1-yl)isoindoline-1,3-dione (0.752 g, 2.66 mmol) and hydrazine hydrate (0.152 ml, 2.80 mmol) in ethanol (13 ml) to give a yellow solution. Heated to reflux (ca. 80 °C) for 3 hours then added hydrazine hydrate (0.043 ml, 0.799 mmol) and continued heating to reflux for 1 hour. Cooled to 20 °C and stirred for 16 hours. Cooled to 0 °C and stirred the suspension for 1 hour. Filtered the solids over a Buchner funnel and rinsed solids with cold ethanol (2 x 1 mL). Concentrated the filtrate to dryness to give 615 mg as a yellow solid. Suspended the solid in Et₂O (10 mL) and filtered over a Buchner funnel. Rinsed solids with Et₂O (5 mL). Concentrated to dryness on rotovap to give 4-(piperidin-1-yl)but-2-yn-1-amine (315 mg, 2.069 mmol, 78 % yield) as a yellow oil. 1H NMR (400 MHz, DMSO-d6) δ ppm 1.29 - 1.41 (m, 2 H) 1.49 (quin, J=5.6 Hz, 4 H) 2.25 - 2.43 (m, 4 H) 3.16 (t, J=2.1 Hz, 2 H) 3.28 (t, J=2.1 Hz, 2 H). LCMS m/z 153.2 (M + H)+.

### Example 3

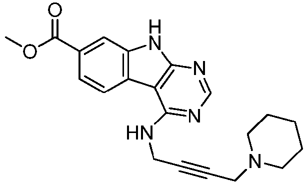

### Methyl 4-((4-(piperidin-1-yl)but-2-yn-1-yl)amino)-9H-pyrimido[4,5-b]indole-7-carboxylate

The title compound was obtained according to the procedure described in example 1 using methyl 4-chloro-9H-pyrimido[4,5-b]indole-7-carboxylate and 4-(piperidin-1-yl)but-2-yn-1-amine. 1H NMR (400 MHz, DMSO-d6) δ ppm 1.22 - 1.35 (m, 2 H) 1.44 (quin, J=5.4 Hz, 4 H) 2.28 - 2.40 (m, 4 H) 3.17 (br. s., 2 H) 3.90 (s, 3 H) 4.43 (d, J=5.9 Hz, 2 H) 7.81 - 7.89 (m, 2 H) 8.06 (d, J=1.6 Hz, 1 H) 8.43 - 8.48 (m, 2 H) 12.24 (s, 1 H). LCMS m/z 378.2 (M + H)+.

### Example 4

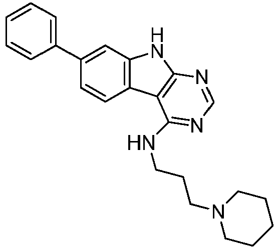

### 7-Phenyl-N-(3-(piperidin-1-yl)propyl)-9H-pyrimido[4,5-b]indol-4-amine

In a vial was added 7-bromo-N-(3-(piperidin-1-yl)propyl)-9H-pyrimido[4,5-b]indol-4-amine (0.04 g, 0.103 mmol), phenylboronic acid (0.025 g, 0.206 mmol) and tetrakis(triphenylphosphine)palladium(0) (0.018 g, 0.015 mmol). The vial was sealed and evacuated with nitrogen (3 cycles vacuum + nitrogen refill). 1,4-dioxane (0.52 ml) and sodium carbonate 2M in water (0.309 ml, 0.618 mmol) were added. The vial was evacuated with nitrogen (3 cycles vacuum + nitrogen refill). Heated the biphasic mixture to 85 °C and stirred for 18 hrs. Cooled to 20 °C and diluted with methanol (2 mL) and CH₂Cl₂ (2 mL). Filtered the mixture over a 0.45 µm filter. Rinsed the vial and filter with methanol (2 x 2 mL) and CH₂Cl₂ (2 x 2 mL). Concentrated to dryness to give an orange solid. The residue was purified on ISCO (RediSep 12 g column eluting with CH₂Cl₂/MeOH/NH₄OH) to give 7-phenyl-N-(3-(piperidin-1-yl)propyl)-9H-pyrimido[4,5-b]indol-4-amine (33 mg, 0.086 mmol, 83 % yield) as a white solid. 1H NMR (400 MHz, DMSO-d6) δ ppm 1.39 (br. s., 2 H) 1.46 - 1.59 (m, 4 H) 1.77 - 1.91 (m, 2 H) 2.38 (br. s., 6 H) 3.64 (q, J=6.5 Hz, 2 H) 7.24 (t, J=4.9 Hz, 1 H) 7.34 - 7.41 (m, 1 H) 7.50 (t, J=7.6 Hz, 2 H) 7.54 (dd, J=8.2, 1.2 Hz, 1 H) 7.66 (d, J=1.2 Hz, 1 H) 7.74 (d, J=7.4 Hz, 2 H) 8.34 (s, 1 H) 8.38 (d, J=8.2 Hz, 1 H) 11.93 (s, 1 H). LCMS m/z 386.2 (M + H)+.

### Example 5

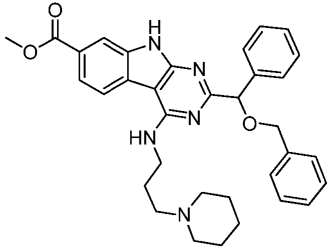

### Methyl 2-((benzyloxy)(phenyl)methyl)-4-((3-(piperidin-1-yl)propyl)amino)-9H-pyrimido[4,5-b]indole-7-carboxylate

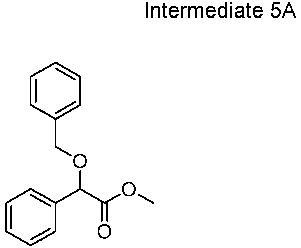

### Methyl 2-(benzyloxy)-2-phenylacetate

In a 50 mL round-bottomed flask was added NaH 60%wt. in mineral oil (0.265 g, 6.62 mmol) in DMF (10 ml) to give a grey suspension. Cooled to 0 °C and added methyl 2-hydroxy-2-phenylacetate (1 g, 6.02 mmol) was added. The resulting orange suspension was stirred for 45 minutes. Benzyl bromide (0.787 ml, 6.62 mmol) was added. Warmed to 20 °C and stirred for 30 minutes. Quenched the reaction by adding sat. NH₄Cl (40 mL) + water (10 mL). Extracted aqueous layer with EtOAc (2 x 50 mL). Washed the combined organic layer with water (2 x 50 mL) then with brine (35 mL). The organic layer was dried over MgSO₄, filtered and concentrated to give 1.72 g as a light yellow oil. The residue was purified on ISCO (RediSep Gold 40 g column eluting with Hexane/Et₂O). The ISCO was repeated two more times to give methyl 2-(benzyloxy)-2-phenylacetate (508 mg, 1.982 mmol, 32.9 % yield) as a colorless oil. 1H NMR (400 MHz, DMSO-d6) δ ppm 3.64 (s, 3 H) 4.49 (d, J=11.3 Hz, 1 H) 4.59 (d, J=11.3 Hz, 1 H) 5.11 (s, 1 H) 7.04 - 7.19 (m, 1 H) 7.23 - 7.45 (m, 9 H).

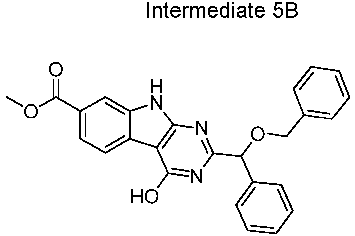

### Methyl 2-((benzyloxy)(phenyl)methyl)-4-hydroxy-9H-pyrimido[4,5-b]indole-7-carboxylate

In a microwave vial was added methyl 2-amino-3-carbamoyl-1H-indole-6-carboxylate (0.185 g, 0.793 mmol), methyl 2-(benzyloxy)-2-phenylacetate (0.508 g, 1.983 mmol) and sodium methoxide 30%wt. in methanol (0.372 ml, 1.983 mmol) in methanol (2 ml). The vial was sealed and heated in the microwave to 140 °C for 1 h. Cooled to 20 °C and added AcOH (0.118 ml, 2.062 mmol). The resulting suspension was stirred at 20 °C for 1 hour. The solids were collected on Buchner. Cake was washed with methanol (3 x 0.5 mL). Dried the product at 20 °C under high vacuum until constant weight to give methyl 2-((benzyloxy)(phenyl)methyl)-4-hydroxy-9H-pyrimido[4,5-b]indole-7-carboxylate (238 mg, 0.542 mmol, 68.3 % yield) as a tan solid. 1H NMR (400 MHz, DMSO-d6) δ ppm 3.87 (s, 3 H) 4.58 (d, J=11.7 Hz, 1 H) 4.69 (d, J=11.7 Hz, 1 H) 5.56 (s, 1 H) 7.27 - 7.46 (m, 8 H) 7.55 - 7.64 (m, 2 H) 7.84 (dd, J=8.2, 1.2 Hz, 1 H) 8.00 - 8.06 (m, 2 H) 12.49 (br. s., 1 H) 12.60 (br. s., 1 H). LCMS m/z 440.2 (M + H)+.

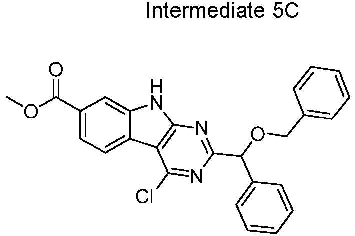

### Methyl 2-((benzyloxy)(phenyl)methyl)-4-chloro-9H-pyrimido[4,5-b]indole-7-carboxylate

In a 15 mL round-bottomed flask was added methyl 2-((benzyloxy)(phenyl)methyl)-4-hydroxy-9H-pyrimido[4,5-b]indole-7-carboxylate (0.234 g, 0.532 mmol) in phosphorus oxychloride (3.47 ml, 37.3 mmol) and heated to 85°C for 16.5 hrs. Concentrated to dryness. The residue was suspended in sat. NaHCO₃ (10 mL) and stirred for 1 hour. The solids were collected on Buchner. Cake was washed with water (3 x 2 mL). Dried the product at 40 °C under high vacuum until constant weight to give methyl 2-((benzyloxy)(phenyl)methyl)-4-chloro-9H-pyrimido[4,5-b]indole-7-carboxylate (228 mg, 0.498 mmol, 94 % yield) as a tan solid. LCMS m/z 458.2 (M + H)+.

### Example 5

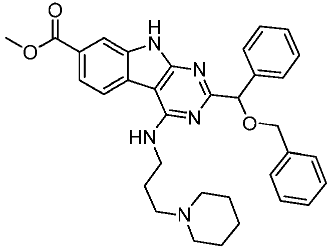

### Methyl 2-((benzyloxy)(phenyl)methyl)-4-((3-(piperidin-1-yl)propyl)amino)-9H-pyrimido[4,5-b]indole-7-carboxylate

In a microwave vial was added methyl 2-((benzyloxy)(phenyl)methyl)-4-chloro-9H-pyrimido[4,5-b]indole-7-carboxylate (0.228 g, 0.498 mmol), 3-(piperidin-1-yl)propan-1-amine (0.158 ml, 0.996 mmol) and triethylamine (0.173 ml, 1.245 mmol) in methanol (3.8 ml). The vial was sealed and heated in the microwave to 140 °C for 30 minutes. Concentrated to dryness. The residue was purified on ISCO (RediSep Gold 40 g eluting with CH₂Cl₂/MeOH/NH₄OH). The ISCO purification was repeated two times to give methyl 2-((benzyloxy)(phenyl)methyl)-4-((3-(piperidin-1-yl)propyl)amino)-9H-pyrimido[4,5-b]indole-7-carboxylate (172 mg, 0.305 mmol, 61.3 % yield) as a light yellow solid. 1H NMR (400 MHz, DMSO-d6) δ ppm 1.29 - 1.42 (m, 2 H) 1.42 - 1.56 (m, 4 H) 1.72 - 1.91 (m, 2 H) 2.18 - 2.47 (m, 6 H) 3.66 (tt, J=13.2, 6.6 Hz, 2 H) 3.88 (s, 3 H) 4.54 (d, J=11.7 Hz, 1 H) 4.64 (d, J=12.1 Hz, 1 H) 5.50 (s, 1 H) 7.21 - 7.43 (m, 8 H) 7.51 (t, J=5.9 Hz, 1 H) 7.57 (d, J=7.0 Hz, 2 H) 7.82 (dd, J=8.2, 1.2 Hz, 1 H) 8.00 (d, J=1.2 Hz, 1 H) 8.38 (d, J=8.2 Hz, 1 H) 12.22 (s, 1 H). HRMS m/z 564.2979 (M+H)+.

### Example 6

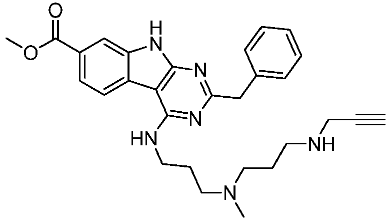

### Methyl 2-benzyl-4-((3-(methyl(3-(prop-2-yn-1-ylamino)propyl)amino)propyl)amino)-9H-pyrimido[4,5-b]indole-7-carboxylate

In a 10 mL round-bottomed flask were added methyl 4-((3-((3-aminopropyl)(methyl)amino)propyl)-amino)-2-benzyl-9H-pyrimido[4,5-b]indole-7-carboxylate (25 mg, 0.054 mmol) and tert-butylamine (8.63 µl, 0.081 mmol) in THF (2.7 ml). Propargyl bromide (7.26 µl, 0.065 mmol) was added and stirred at 20 °C for 69.5 hrs. Concentrated to dryness. The residue was purified on ISCO (RediSep 12 g column eluting with CH₂Cl₂/MeOH/NH₄OH) to give methyl 2-benzyl-4-((3-(methyl(3-(prop-2-yn-1-ylamino)propyl)amino)propyl)amino)-9H-pyrimido[4,5-b]indole-7-carboxylate (3.2 mg, 6.42 µmol, 11.82 % yield) as a white solid. 1H NMR (400 MHz, DMSO-d6) δ ppm 1.23 (s, 1 H) 1.56 (dt, J=14.1, 7.0 Hz, 2 H) 1.80 (dt, J=13.5, 6.6 Hz, 2 H) 2.20 (s, 3 H) 2.34 - 2.46 (m, 4 H) 2.56 (t, J=7.0 Hz, 2 H) 2.99 (t, J=2.2 Hz, 1 H) 3.25 (d, J=2.2 Hz, 2 H) 3.58 - 3.70 (m, 2 H) 3.88 (s, 3 H) 4.04 (s, 2 H) 7.15 - 7.23 (m, 1 H) 7.28 (t, J=7.6 Hz, 2 H) 7.37 (m, J=7.4 Hz, 2 H) 7.54 (t, J=5.5 Hz, 1 H) 7.83 (dd, J=8.2, 1.2 Hz, 1 H) 7.99 (d, J=1.2 Hz, 1 H) 8.27 (d, J=8.2 Hz, 1 H) 12.05 (s, 1 H). HRMS m/z 499.2823 (M+H)+.

### Example 7

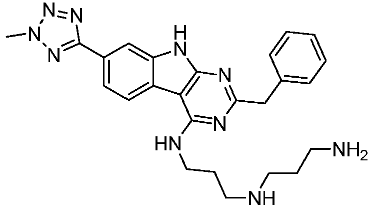

### N1-(3-aminopropyl)-N3-(2-benzyl-7-(2-methyl-2H-tetrazol-5-yl)-9H-pyrimido[4,5-b]indol-4-yl)propane-1,3-diamine

In a microwave vial was added 2-benzyl-4-chloro-7-(2-methyl-2H-tetrazol-5-yl)-9H-pyrimido[4,5-b]indole (0.100 g, 0.266 mmol) and N1-(3-aminopropyl)propane-1,3-diamine (0.375 ml, 2.66 mmol) in methanol (2 ml). The vial was sealed and heated in the microwave to 140 °C for 30 minutes. Concentrated to dryness. The residue was purified on ISCO (RediSep 12 g column eluting with CH₂Cl₂/MeOH/NH₄OH) to give N1-(3-aminopropyl)-N3-(2-benzyl-7-(2-methyl-2H-tetrazol-5-yl)-9H-pyrimido[4,5-b]indol-4-yl)propane-1,3-diamine (112 mg, 0.238 mmol, 89 % yield) as a light yellow solid. 1H NMR (400 MHz, DMSO-d6) δ ppm 1.54 (quin, J=6.85 Hz, 2 H) 1.79 (quin, J=6.26 Hz, 2 H) 2.53 - 2.69 (m, 6 H) 3.68 (q, J=6.26 Hz, 2 H) 4.04 (s, 2 H) 4.43 (s, 3 H) 7.15 - 7.22 (m, 1 H) 7.28 (t, J=7.43 Hz, 2 H) 7.38 (d, J=7.43 Hz, 2 H) 7.57 (t, J=4.89 Hz, 1 H) 7.90 (dd, J=8.22, 1.17 Hz, 1 H) 8.08 (s, 1 H) 8.36 (d, J=8.22 Hz, 1 H). HRMS m/z 471.2737 (M+H)+.

### Example 8

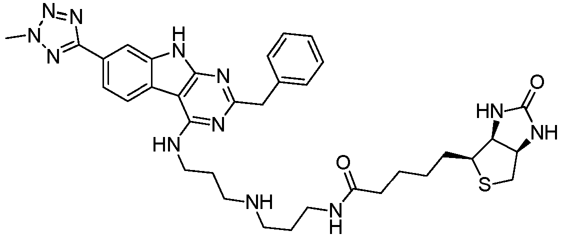

### N-(3-((3-((2-benzyl-7-(2-methyl-2H-tetrazol-5-yl)-9H-pyrimido[4,5-b]indol-4-yl)amino)propyl)amino)propyl)-5-((3aS,4S,6aR)-2-oxohexahydro-1H-thieno[3,4-d]imidazol-4-yl)pentanamide

In a 15 mL round-bottomed flask were added N1-(3-aminopropyl)-N3-(2-benzyl-7-(2-methyl-2H-tetrazol-5-yl)-9H-pyrimido[4,5-b]indol-4-yl)propane-1,3-diamine (57 mg, 0.121 mmol) and triethylamine (25.3 µl, 0.182 mmol) in DMF (2.8 ml). 2,5-dioxopyrrolidin-1-yl 5-((3aS,4S,6aR)-2-oxohexahydro-1H-thieno[3,4-d]imidazol-4-yl)pentanoate (44.7 mg, 0.131 mmol) was added and stirred for 1 hr. Concentrated the reaction mixture to dryness. The residue was suspended in methanol (3 mL), water (2 mL) and Na₂CO₃ 2M in water (5 drops). Stirred for 30 minutes. The solids were collected on Buchner. Cake was washed with water (2 x 1 mL) then with methanol (1 x 1 mL). Dried the product at 40 °C under high vacuum until constant weight to give N-(3-((3-((2-benzyl-7-(2-methyl-2H-tetrazol-5-yl)-9H-pyrimido[4,5-b]indol-4-yl)amino)propyl)amino)propyl)-5-((3aS,4S,6aR)-2-oxohexahydro-1H-thieno[3,4-d]imidazol-4-yl)pentanamide (78 mg, 0.112 mmol, 92 % yield) as a light yellow solid. 1H NMR (400 MHz, DMSO-d6) δ ppm 1.28 (m, J=13.79, 13.79, 6.85 Hz, 2 H) 1.36 - 1.52 (m, 3 H) 1.52 - 1.63 (m, 3 H) 1.79 (dt, J=12.23, 5.82 Hz, 2 H) 2.04 (t, J=7.24 Hz, 2 H) 2.52 - 2.58 (m, 3 H) 2.62 (t, J=6.26 Hz, 2 H) 2.78 (dd, J=12.13, 5.09 Hz, 1 H) 2.99 - 3.06 (m, 1 H) 3.06 - 3.13 (m, 2 H) 3.68 (q, J=6.26 Hz, 2 H) 4.04 (s, 2 H) 4.06 - 4.11 (m, 1 H) 4.22 - 4.31 (m, 1 H) 4.43 (s, 3 H) 6.34 (s, 1 H) 6.40 (s, 1 H) 7.14 - 7.23 (m, 1 H) 7.28 (t, J=7.43 Hz, 2 H) 7.34 - 7.41 (m, 2 H) 7.56 (t, J=5.28 Hz, 1 H) 7.77 (t, J=5.48 Hz, 1 H) 7.89 (dd, J=8.20, 1.20 Hz, 1 H) 8.07 (d, J=1.20 Hz, 1 H) 8.36 (d, J=8.22 Hz, 1 H) 12.00 (br. s., 2 H). HRMS m/z 697.3498 (M+H)+.

### Example 9

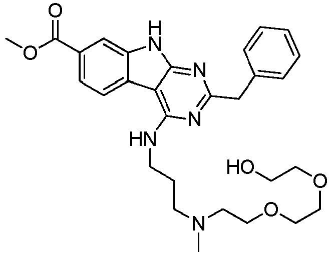

### methyl 2-benzyl-4-((3-(methylamino)propyl)amino)-9H-pyrimido[4,5-b]indole-7-carboxylate

### Intermediate 9A

A mixture of methyl 2-benzyl-4-chloro-9H-pyrimido[4,5-b]indole-7-carboxylate (0.100 g, 0.284 mmol), Et3N (0.079 ml, 0.569 mmol) and tert-butyl (3-aminopropyl)(methyl)carbamate (0.080 g, 0.426 mmol) in MeOH (1.0 ml) was heated in the microwave at 140 °C for 20 min. The reaction was not completed and more reagent were added and again heated at 140 °C for 20 min. The solvent was removed and the crude residue purified on a short pad of SiO₂ with Hx-EA (65-35) to give 0.092g of methyl 2-benzyl-4-((3-((tert-butoxycarbonyl)(methyl)amino)propyl)amino)-9H-pyrimido[4,5-b]indole-7-carboxylate. LRMS + H+: 504.2.

### Intermediate 9B

TFA (1.0 ml, 12.9 mmol) was added dropwise to a cold (0-5 °C) suspension of the previous intermediate (0.092 g, 0.18 mmol) and then brought to rt. After 30 min it was diluted with some toluene and the solvent was removed. The residue was taken in more toluene and the solvent was removed. Finally it was taken in EA ant the solvent was removed to give 0.085g of the trifluoroacetate salt of methyl 2-benzyl-4-((3-(methylamino)propyl)amino)-9H-pyrimido[4,5-b]indole-7-carboxylate. LRMS + H+: 404.2.

### Example 9

The previous TFA salt (0.020g), sodium carbonate (8.81 mg, 0.083 mmol), sodium iodide (1.4 mg, 9.6 µmol) and 2-(2-(2-chloroethoxy)ethoxy)ethanol (6.5 µl, 0.04 mmol) was heated at 70 °C in acetone (0.2 ml) overnight. More 2-(2-(2-chloroethoxy)ethoxy)ethanol (6.4 µl, 0.04 mmol) were added and again stirred overnight at 70 °C. It was then diluted with EA-water and the organic phase separated, dried over Na₂SO₄, filtered. The crude adduct was purified on combi-flash using DCM-MeOH (0-25%) to give 0.009 g of the title compound. 1H NMR (400 MHz, DMSO-d6) δ ppm 1.74 - 1.85 (m, 2 H) 2.25 (br. s., 3 H) 2.55 (br. s., 2 H) 3.32 - 3.36 (m, 4 H) 3.39 - 3.46 (m, 6 H) 3.51 (t,J=5.87 Hz, 2 H) 3.64 (q, J=6.52 Hz, 2 H) 3.88 (s, 3 H) 4.04 (s, 2 H) 4.53 (br. s., 1 H) 7.18 (t, J=7.40 Hz, 1 H) 7.28 (t, J=7.63 Hz, 2 H) 7.37 (d, J=7.04 Hz, 2 H) 7.55 (t, J=5.28 Hz, 1 H) 7.82 (dd, J=8.22, 1.17 Hz, 1 H) 7.99 (s, 1 H) 8.27 (d, J=8.22 Hz, 1 H) 12.05 (s, 1 H). LRMS + H+: 536.3.

### Example 10

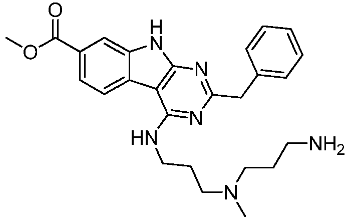

### Methyl 4-((3-((3-aminopropyl)(methyl)amino)propyl)amino)-2-benzyl-9H-pyrimido[4,5-b]indole-7-carboxylate

In a microwave vial was added methyl 2-benzyl-4-chloro-9H-pyrimido[4,5-b]indole-7-carboxylate (0.050 g, 0.142 mmol) and N1-(3-aminopropyl)-N1-methylpropane-1,3-diamine (0.12 ml, 0.71 mmol) in MeOH (2.0 ml, 49.4 mmol). The vial was placed in the microwave and heated to 140 °C for 30 min. After 30 minutes it was concentrated to dryness. The residue was purified on ISCO RediSep Gold column using DCM-MeOH-NH₄OH to give 0.044g of the title compound. 1H NMR (400 MHz, DMSO-d6) δ ppm 1.44 (dt, J=13.8, 6.6 Hz, 2 H) 1.72 (dt, J=13.7, 6.8 Hz, 2 H) 2.11 (s, 3 H) 2.24 - 2.30 (m, 2 H) 2.33 (t, J=6.7 Hz, 2 H) 2.47 (br. s., 2 H) 3.51 - 3.61 (m, 2 H) 3.76 - 3.85 (m, 3 H) 3.97 (s, 2 H) 7.08 - 7.15 (m, 1 H) 7.17 - 7.24 (m, 2 H) 7.27 - 7.34 (m, 2 H) 7.50 (t, J=5.3 Hz, 1 H) 7.76 (dd, J=8.2, 1.6 Hz, 1 H) 7.92 (d, J=1.6 Hz, 1 H) 8.20 (d, J=8.2 Hz, 1 H). LRMS + H+: 461.2.

### Example 11

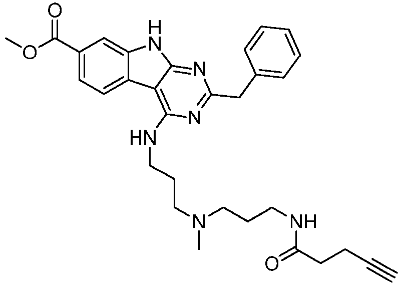

### Methyl 2-benzyl-4-((3-(methyl (3-(pent-4-ynamido)propyl)amino)propyl)amino)-9H-pyrimido[4,5- b]indole-7-carboxylate

To a mixture of pent-4-ynoic acid (2.56 mg, 0.026 mmol), EDC (6.24 mg, 0.033 mmol) and triethylamine (4.58 µl, 0.033 mmol) in DMF (0.12 ml) at 5 °C was added methyl 4-((3-((3-aminopropyl)(methyl)amino)propyl)amino)-2-benzyl-9H-pyrimido[4,5-b]indole-7-carboxylate (0.010 g, 0.022 mmol). After 5 min, bring to rt and stirred overnight. The mixture was diluted with DMSO and purified directly on the preparative HPLC (Zorbax SB-C18 PrepHT 5um; 21.2x100mm) with a gradient of 20% MeOH (0.065% TFA) to 100 MeOH (0.05% TFA) to give after lyophilization from ethanol 0.006g of the title compound as the TFA salt. 1H NMR (400 MHz, DMSO-d6) δ ppm 1.74 (quin, J=7.20 Hz, 2 H) 2.02 (br. s., 2 H) 2.20 - 2.29 (m, 2 H) 2.31 - 2.38 (m, 2 H) 2.68 - 2.74 (m, 3 H) 2.76 (t, J=2.54 Hz, 1 H) 3.07 - 3.13 (m, 3 H) 3.69 (q, J=6.30 Hz, 2 H) 3.88 (s, 3 H) 4.08 (s, 2 H) 7.21 (t, J=7.04 Hz, 1 H) 7.30 (t, J=7.63 Hz, 2 H) 7.34 - 7.40 (m, 2 H) 7.51 (t, J=5.48 Hz, 1 H) 7.84 (dd, J=8.22, 1.17 Hz, 1 H) 7.98 - 8.02 (m, 1 H) 8.05 (t, J=5.48 Hz, 1 H) 8.37 (d, J=8.22 Hz, 1 H) 9.18 (br. s., 1 H) 12.15 (s, 1 H). LRMS + H+: 541.3.

### Example 12

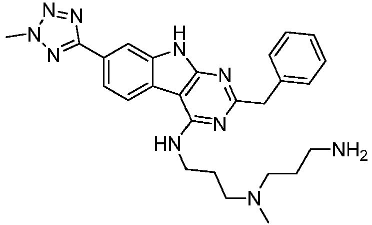

### N1-(3-aminopropyl)-N3-(2-benzyl-7-(2-methyl-2H-tetrazol-5-yl)-9H-pyrimido[4,5-b]indol-4-yl)-N1-methylpropane-1,3-diamine

Following a similar procedure as previously described with 2-benzyl-4-chloro-7-(2-methyl-2H-tetrazol-5-yl)-9H-pyrimido[4,5-b]indole and N1-(3-aminopropyl)-N1-methylpropane-1,3-diamine as the amine, the title compound was obtained. 1H NMR (400 MHz, DMSO-d6) δ ppm 1.52 (quin, J=6.85 Hz, 2 H) 1.80 (quin, J=6.85 Hz, 2 H) 2.18 (s, 3 H) 2.36 (t, J=7.24 Hz, 2 H) 2.41 (t, J=6.65 Hz, 2 H) 2.53 - 2.61 (m, 2 H) 3.64 (q, J=6.52 Hz, 2 H) 4.04 (s, 2 H) 4.43 (s, 3 H) 7.14 - 7.23 (m, 1 H) 7.28 (t, J=7.43 Hz, 2 H) 7.38 (d, J=7.43 Hz, 2 H) 7.49 (t, J=5.09 Hz, 1 H) 7.91 (d, J=8.22 Hz, 1 H) 8.08 (s, 1 H) 8.32 (d, J=8.22 Hz, 1 H). LRMS + H+: 485.4.

### Example 13

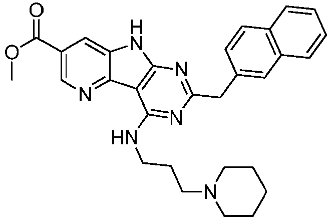

### Methyl 2-(naphthalen-2-ylmethyl)-4-((3-(piperidin-1-yl)propyl)amino)-9H-pyrido[2',3':4,5]pyrrolo[2,3-d]pyrimidine-7-carboxylate

### Intermediate 13A

A mixture of methyl 2-amino-3-carbamoyl-1H-pyrrolo[3,2-b]pyridine-6-carboxylate (0.090 g, 0.384 mmol), methyl 2-(naphthalen-2-yl)acetate (0.269 g, 1.345 mmol) and sodium methoxide 5.4M (0.28 ml, 1.53 mmol) in MeOH (1.601 ml) was heated in the microwave for 75 min at 140 °C. The mixture was diluted with MeOH-NH4Cl saturated and filtered. The solid was rinsed with MeOH-water and dried under high vacuum to give crude 0.120g of methyl 4-hydroxy-2-(naphthalen-2-ylmethyl)-9H-pyrido[2',3':4,5]pyrrolo[2,3-d]pyrimidine-7-carboxylate.

### Intermediate 13B

To this intermediate (0.060g) in dioxane (0.41 ml)-DCE (0.62 ml) was added POCl₃ (0.13 ml, 1.4 mmol) and the mixture was heated in the microwave for 10 min at 160 °C. The reaction was not completed and more POCl₃ (0.131 ml, 1.405 mmol) were added. Ok. Solvent removed. The solvent was removed and the residue was suspended in sat. NaHCO₃ (10 mL) and stirred for 1 hour. The solids were collected on uchner and the cake was washed with water (3 x 2 mL) and dried at 40 °C under high vacuum to give 0.040g of methyl 4-chloro-2-(naphthalen-2-ylmethyl)-9H-pyrido[2',3':4,5]pyrrolo[2,3-d]pyrimidine-7-carboxylate. LRMS + H+: 403.1.

### Example 13

Following a similar procedure as before with the previous intermediate and 3-(piperidin-1-yl)propan-1-amine as the amine, the title compound was obtained after purification on the preparative HPLC (Zorbax SB-C18 PrepHT 5um; 21.2x100mm) with a gradient of 20% MeOH (0.065% TFA) to 100 MeOH (0.05% TFA) to give 0.012g of the title compound as the TFA salt. 1H NMR (400 MHz, DMSO-d6) δ ppm 1.15 - 1.31 (m, 1 H) 1.43 - 1.72 (m, 5 H) 1.91 - 2.04 (m, 2 H) 2.58 (q, J=11.00 Hz, 2 H) 2.95 (br. s., 2 H) 3.26 (d, J=11.35 Hz, 2 H) 3.72 (q, J=5.50 Hz, 2 H) 3.92 (s, 3 H) 4.27 (s, 2 H) 7.48 (quin, J=6.26 Hz, 2 H) 7.58 (d, J=8.61 Hz, 1 H) 7.64 (t, J=5.09 Hz, 1 H) 7.78 - 7.93 (m, 4 H) 8.22 (s, 1 H) 8.94 (br. s., 1 H) 9.01 (s, 1 H) 12.32 (s, 1 H). LRMS + H+: 509.3.

The compounds encompassed herein have been further tested for their ability of inhibiting cell proliferation in AML3, showing high potency (IC₅₀ = <500 nM) to less potency (IC₅₀ = 2000-5000 nM). Accordingly, also encompassed are the following compounds with level of activity in AML3 cells as described above in parenthesis (wherein A: IC₅₀ = <500 nM; B: IC₅₀ = 500-1000 nM; C: IC₅₀ = 1000-2000 nM; and D: IC₅₀ = 2000-5000 nM):

**Table 3**

| Antineoplastic effect of tested compounds on cell proliferation of cancerous AML3 cells | |
|---|---|
| 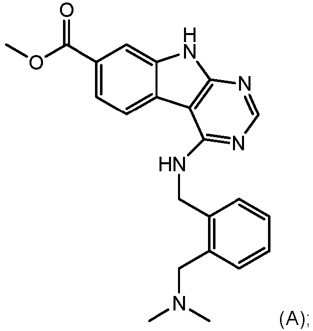 | 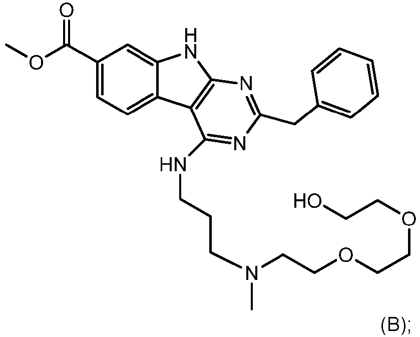 |
| 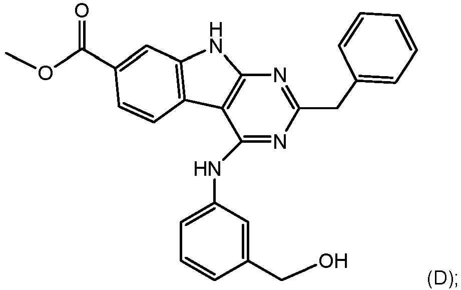 | 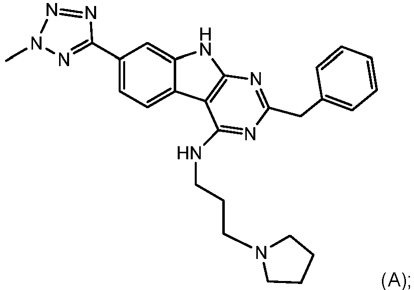 |
| 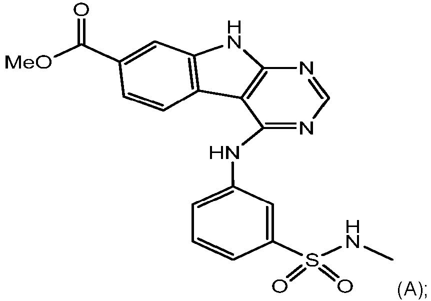 | 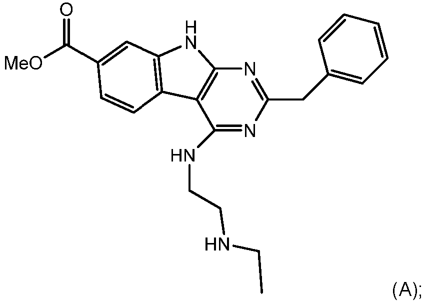 |
| 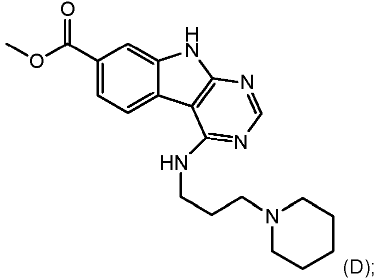 | 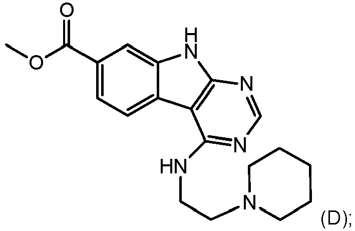 |
| 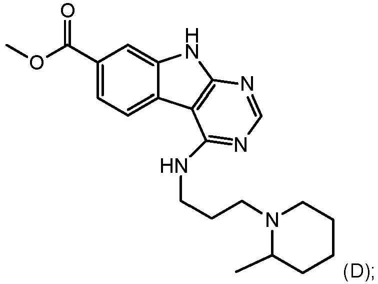 | 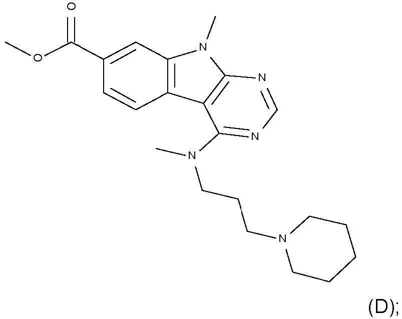 |
| 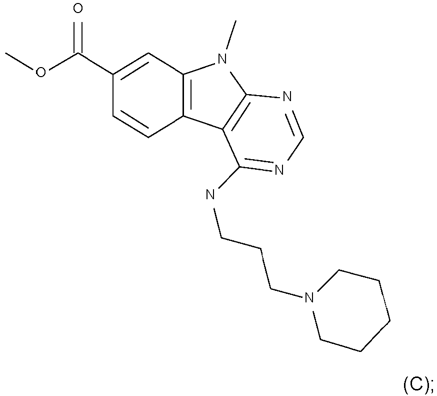 | 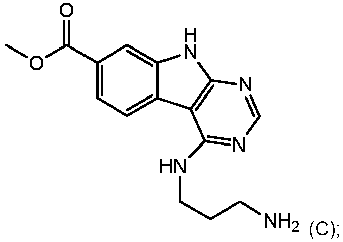 |
| 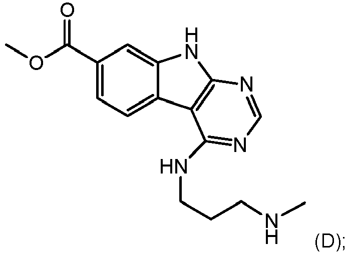 | 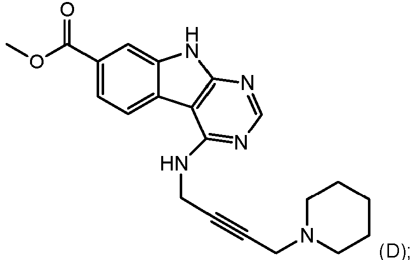 |
| 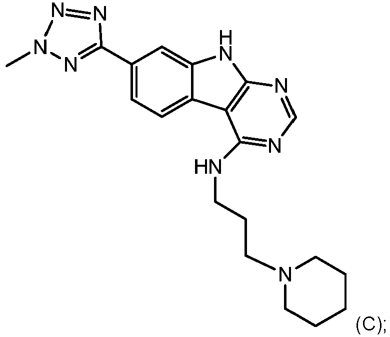 | 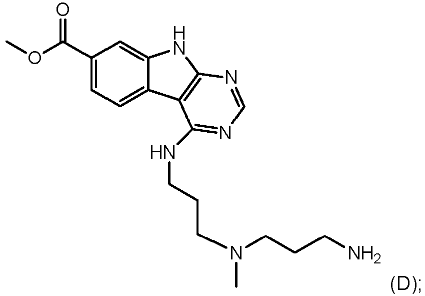 |
| 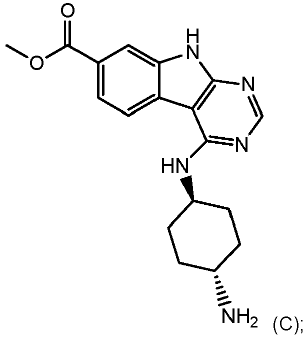 | 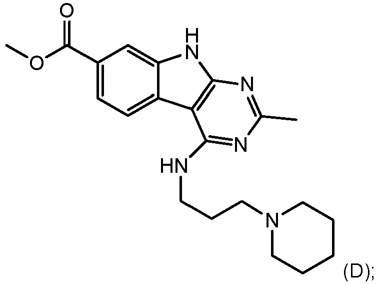 |
| 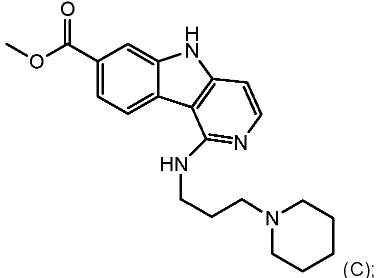 | 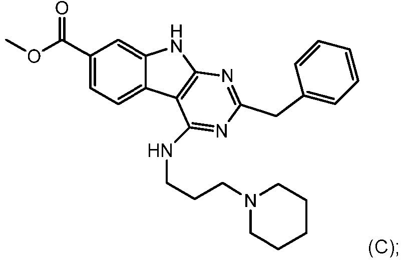 |
| 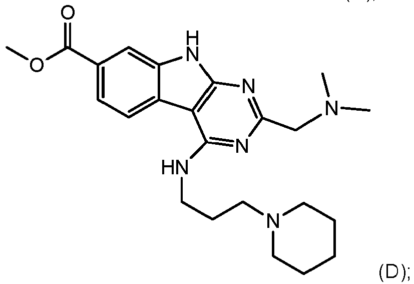 | 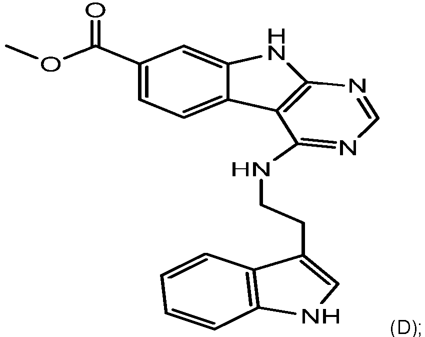 |
| 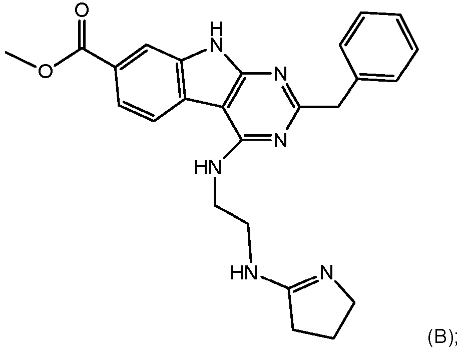 | 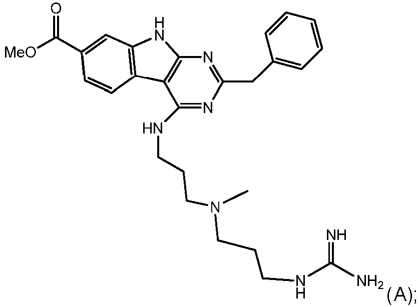 |
| 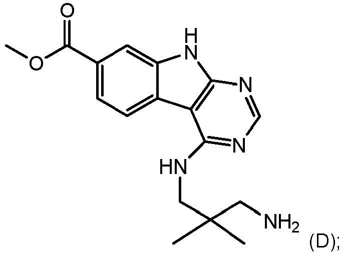 | 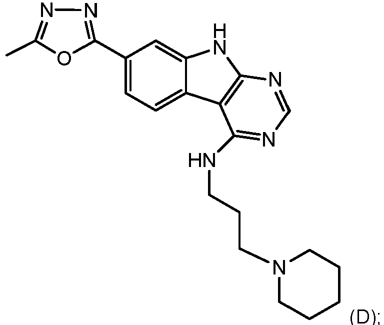 |
| 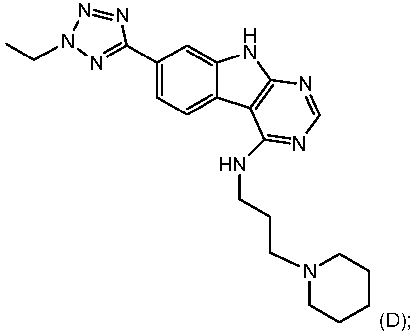 | 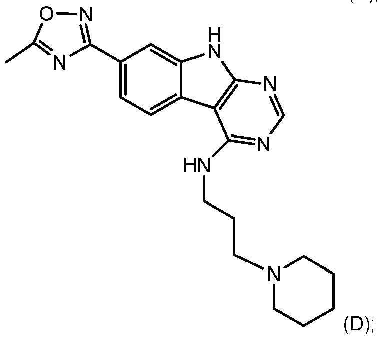 |
| 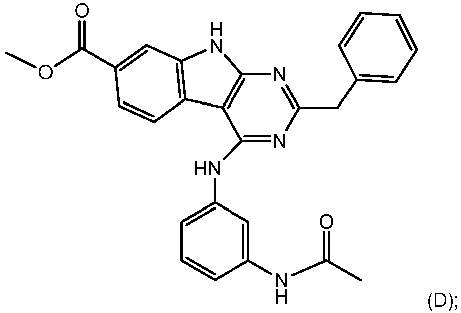 | 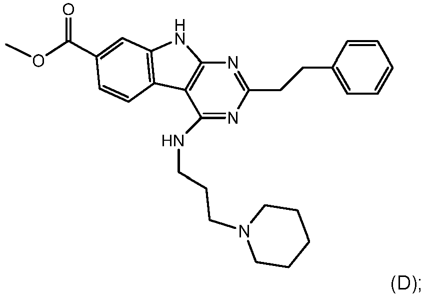 |
| 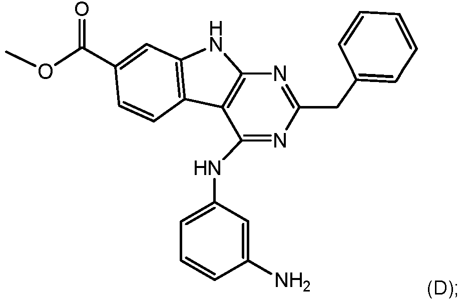 | 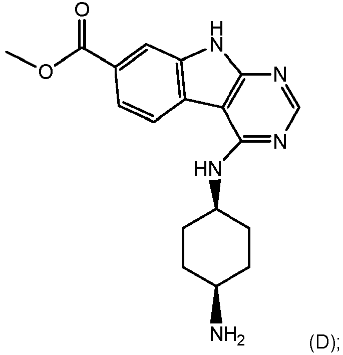 |
| 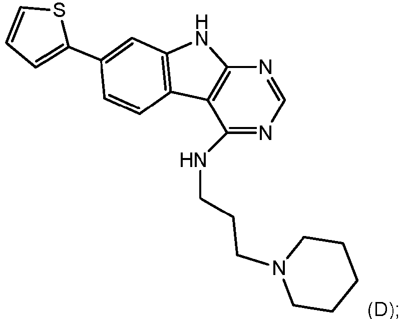 | 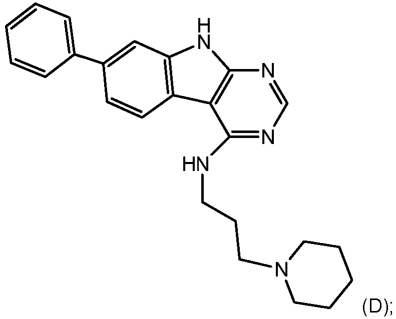 |
| 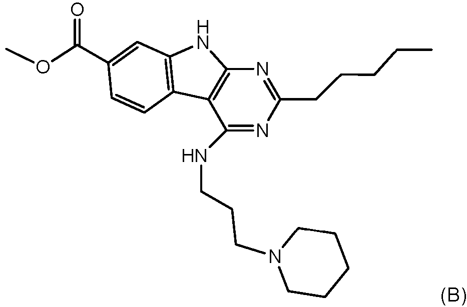 | 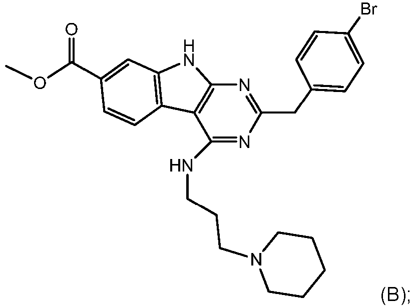 |
| 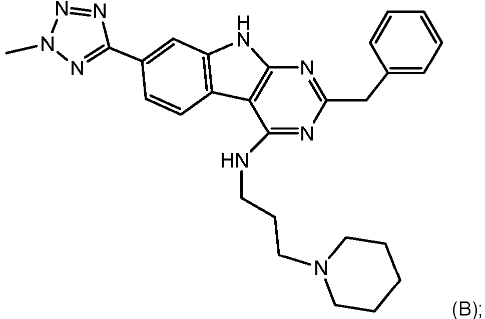 | 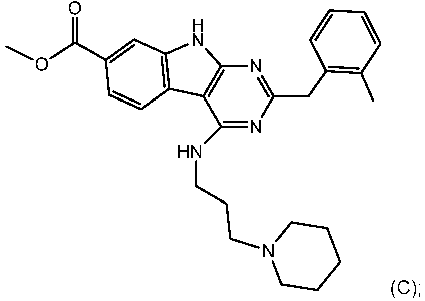 |
| 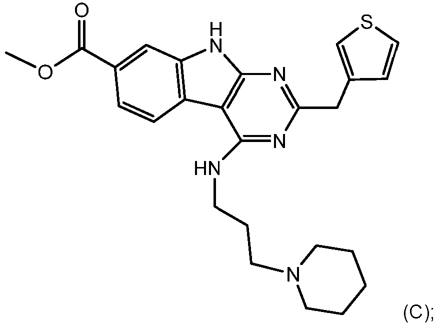 | 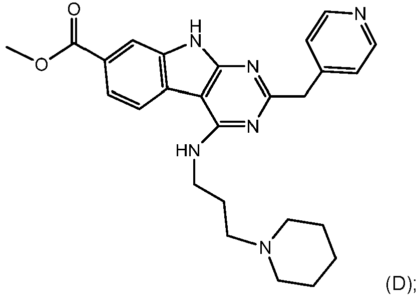 |
| 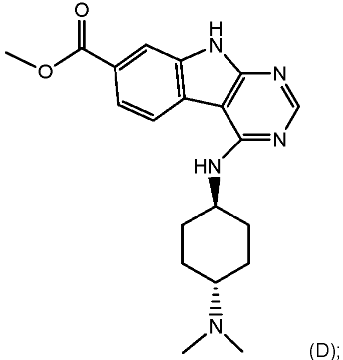 | 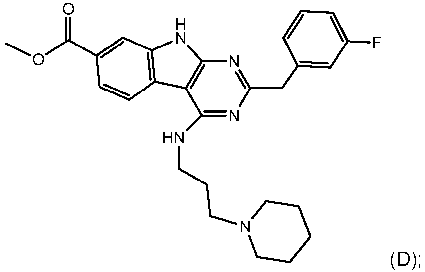 |
| 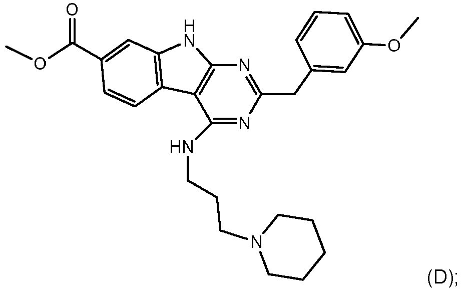 | 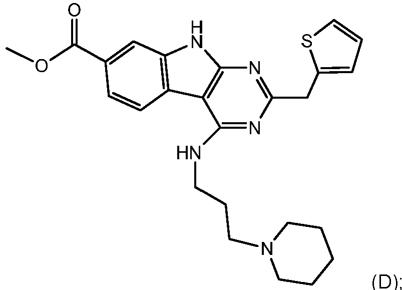 |
| 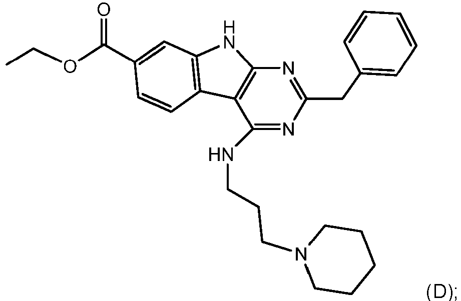 | 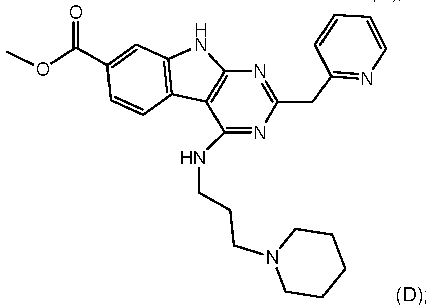 |
| 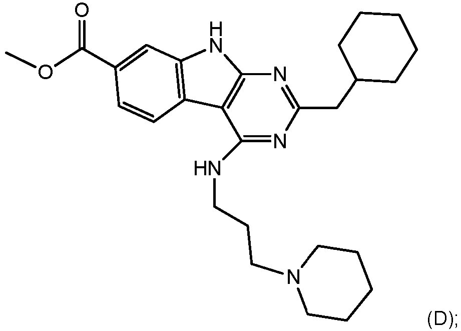 | 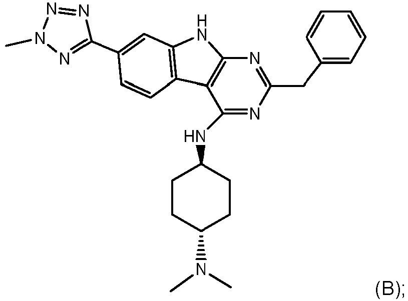 |
| 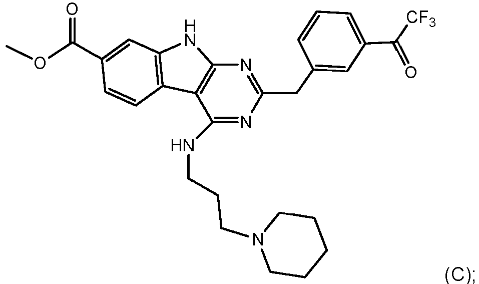 | 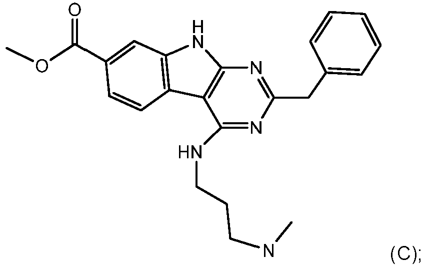 |
| 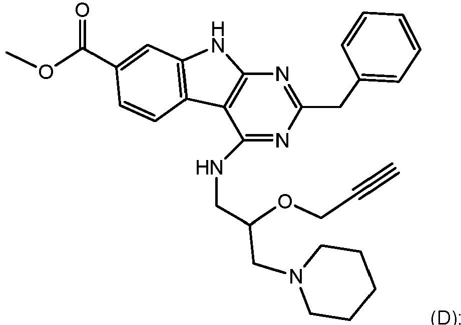 | 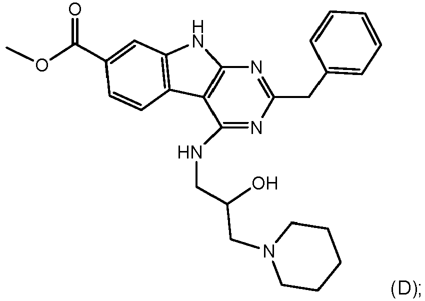 |
| 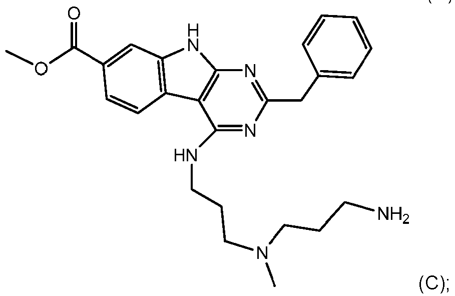 | 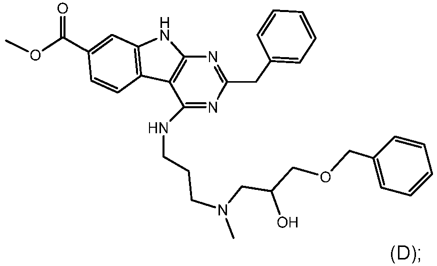 |
| 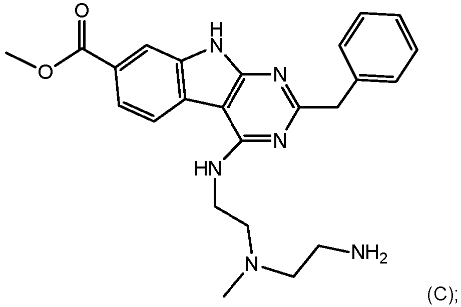 | 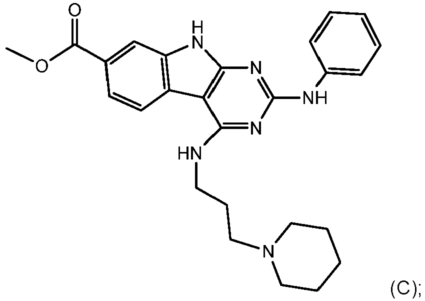 |
| 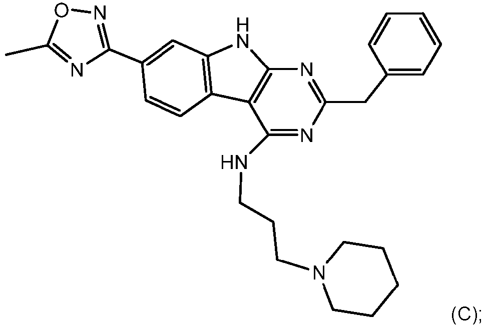 | 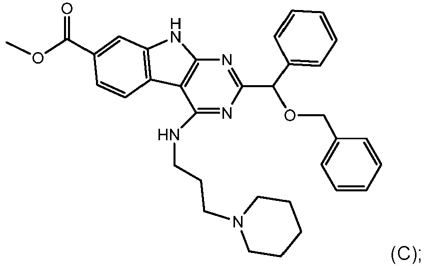 |
| 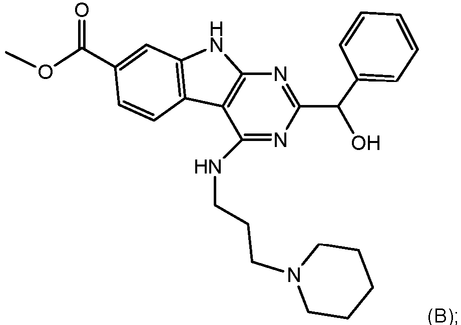 | 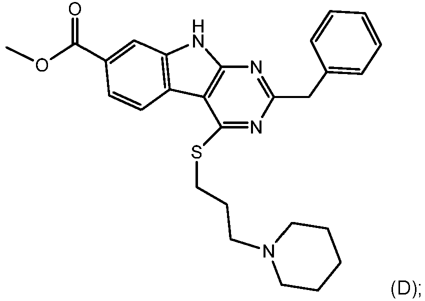 |
| 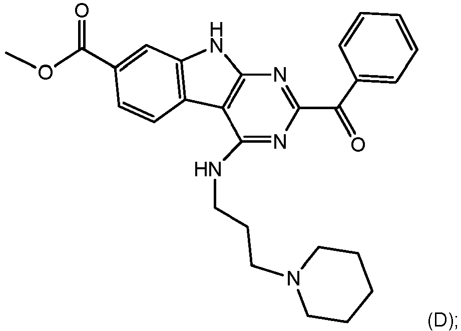 | 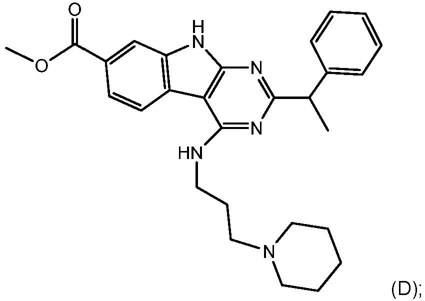 |
| 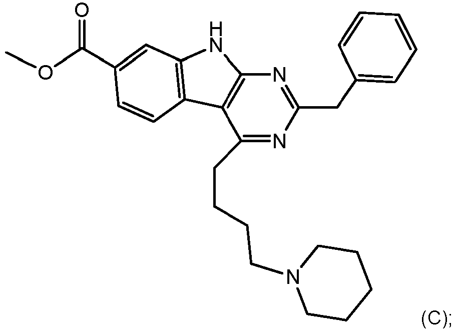 | 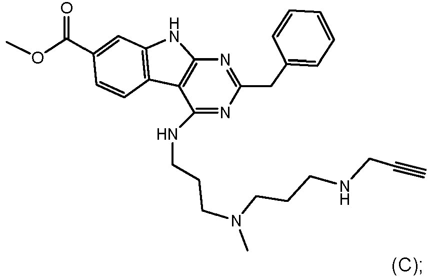 |
| 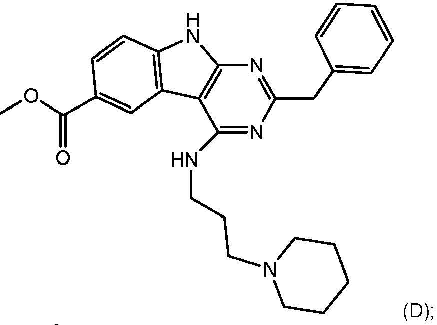 | 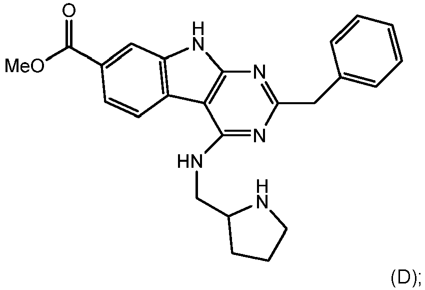 |
| 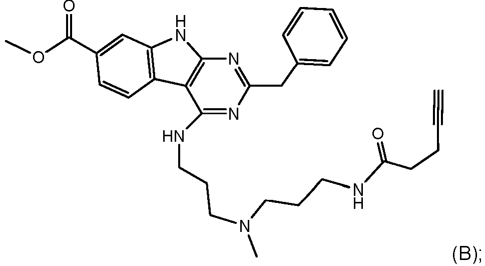 | 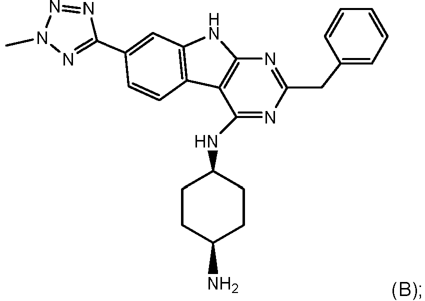 |
| 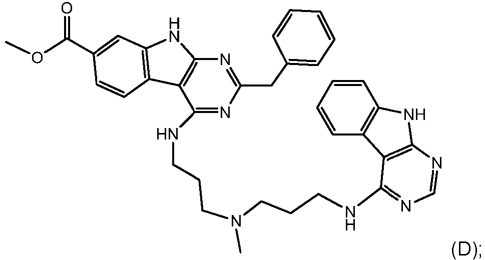 | 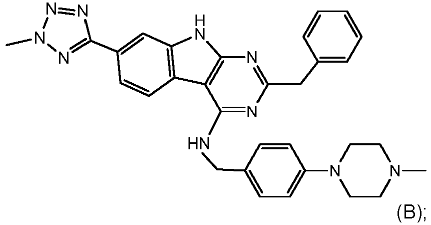 |
| 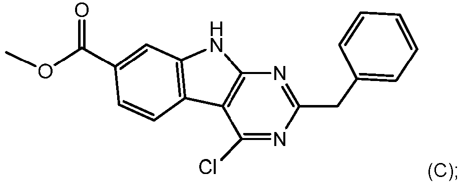 | 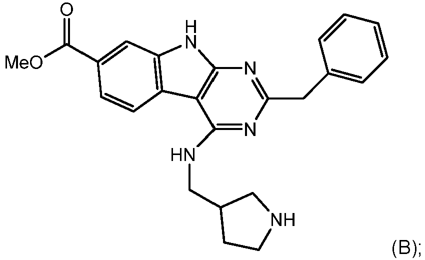 |
| 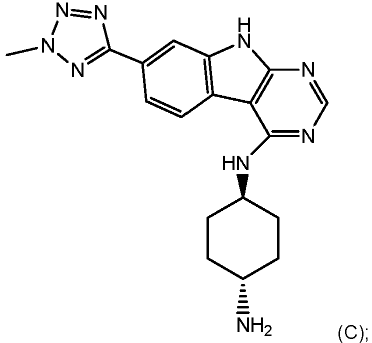 | 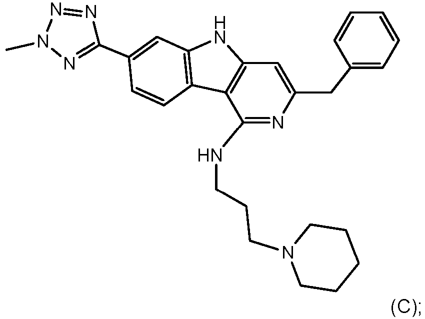 |
| 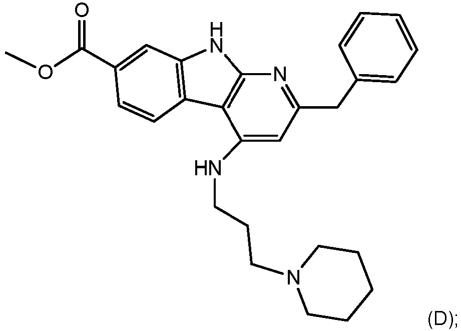 | 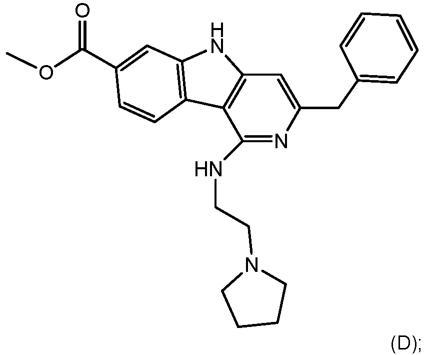 |
| 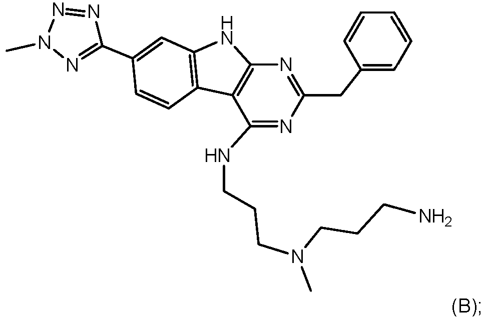 | 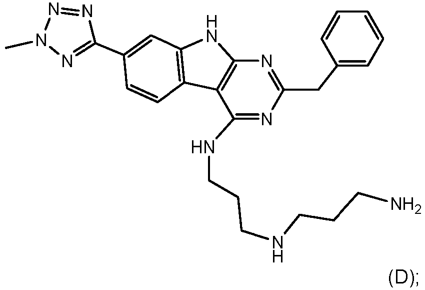 |
| 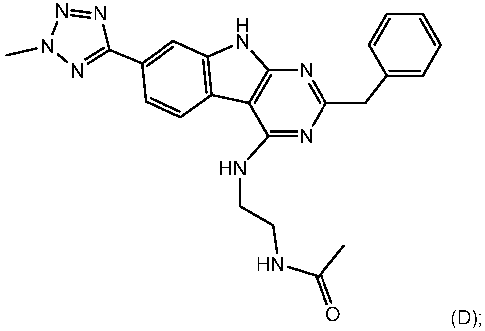 | 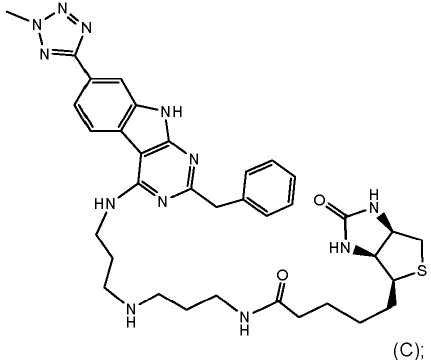 |
| 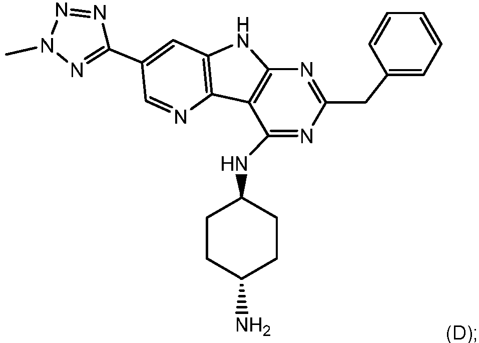 | 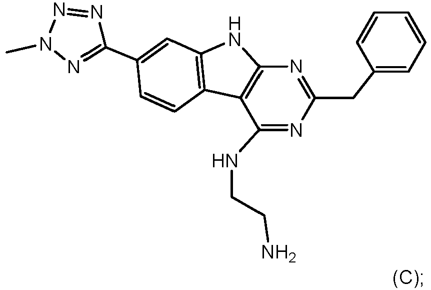 |
| 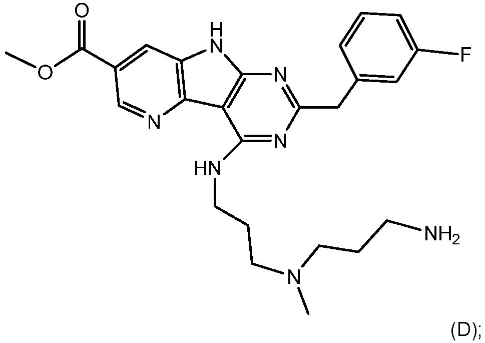 | 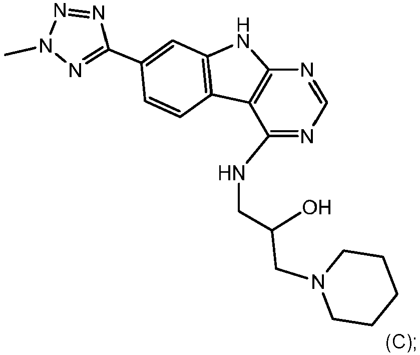 |
| 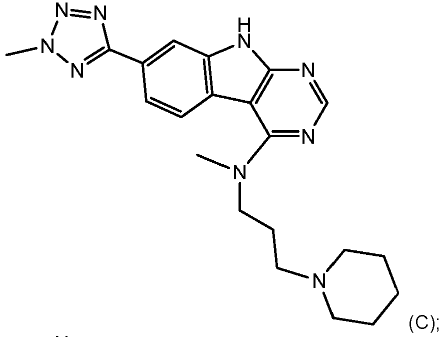 | 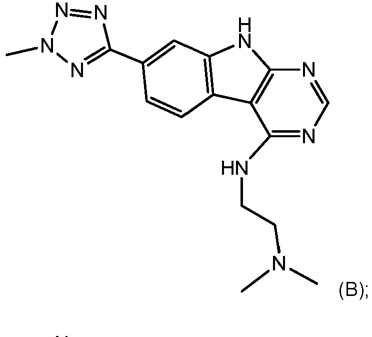 |
| 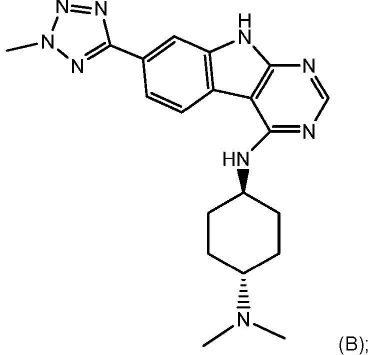 | 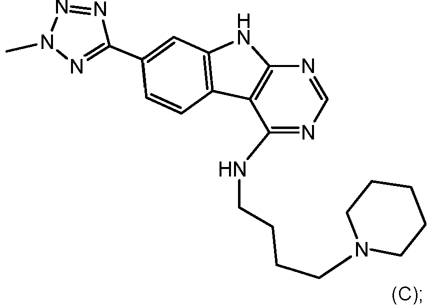 |
| 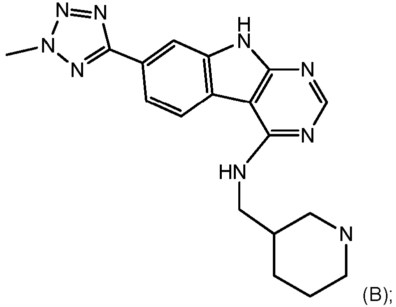 | 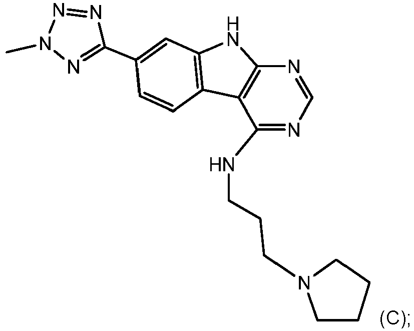 |
| 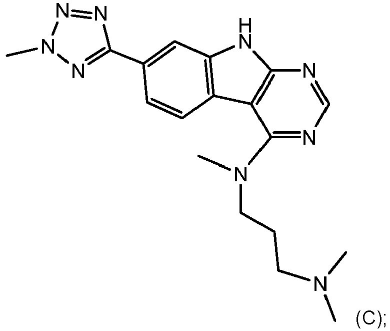 | 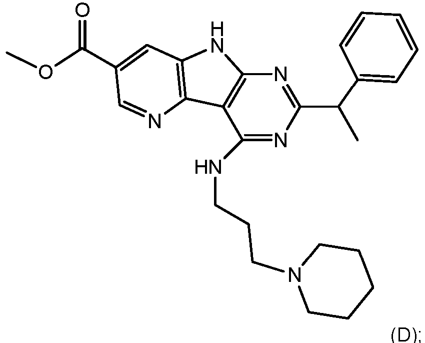 |
| 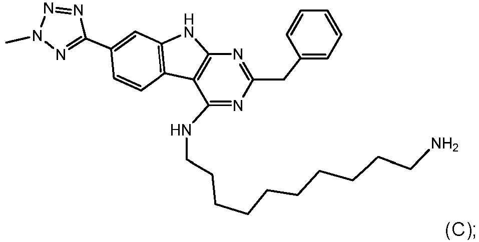 | 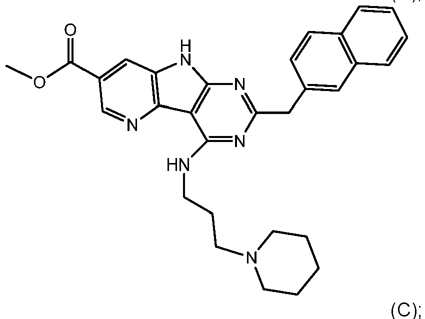 |
| 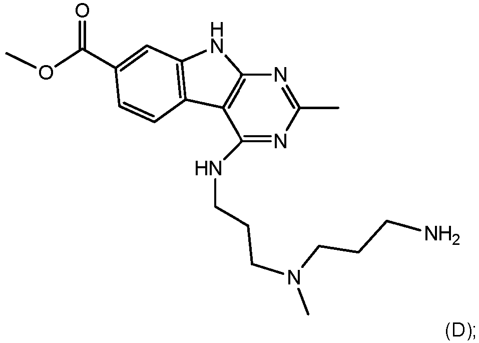 | 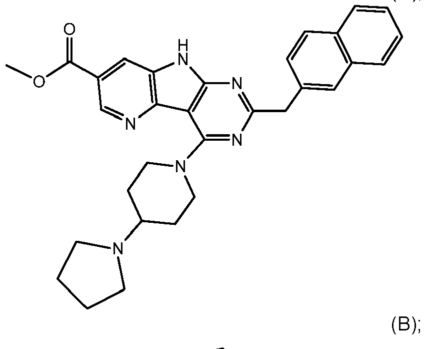 |
| 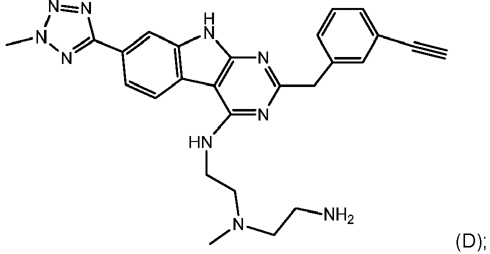 | 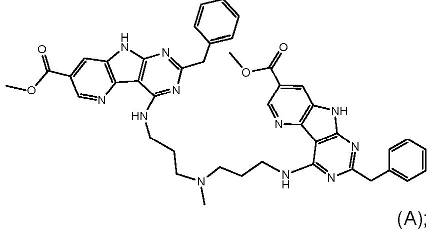 |
| 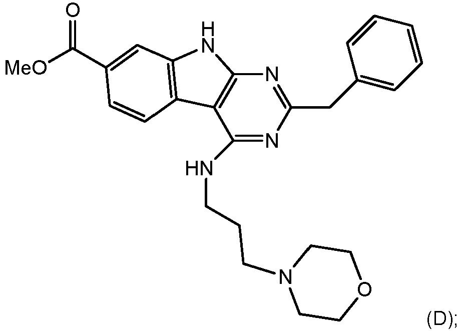 | 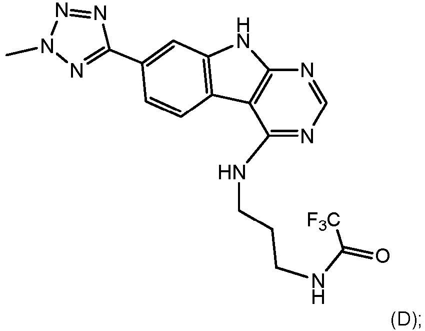 |
| 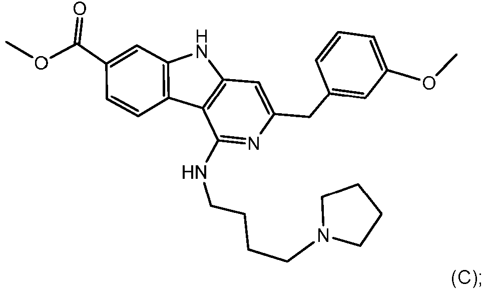 | 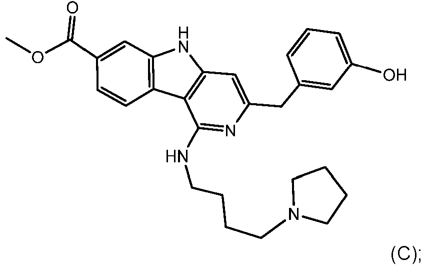 |
| 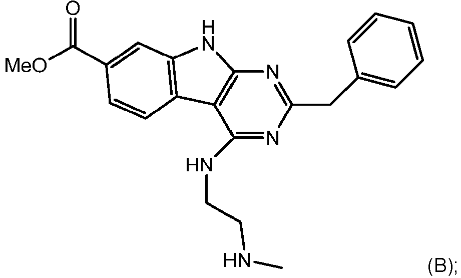 | 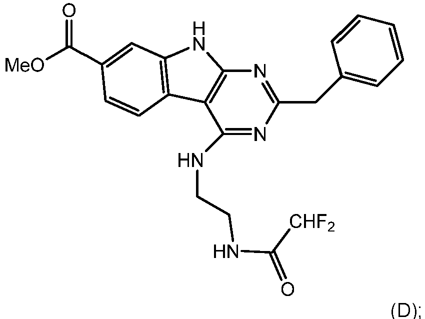 |
| 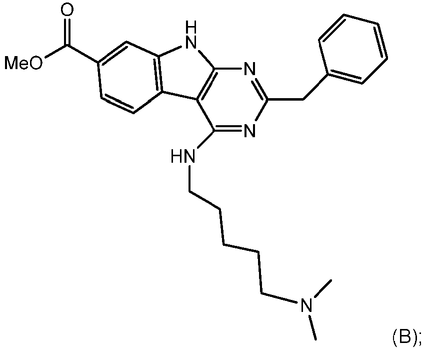 | 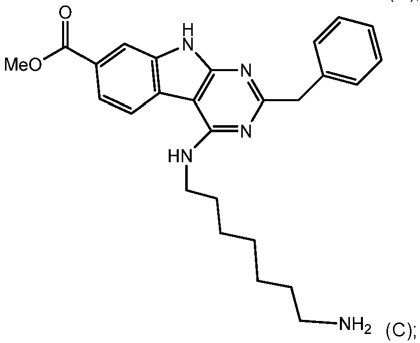 |
| 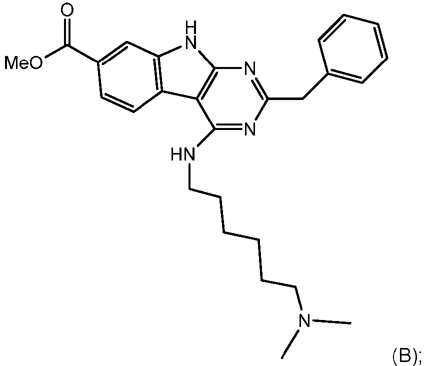 | 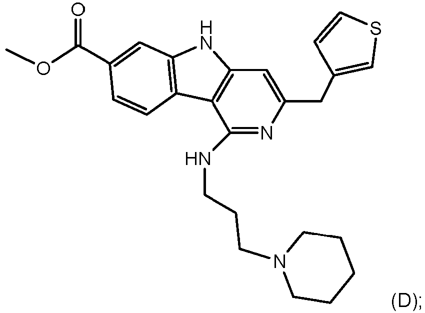 |
| 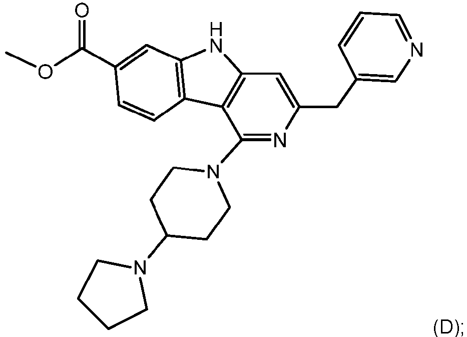 | 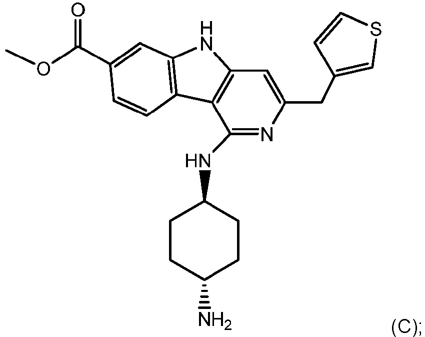 |
| 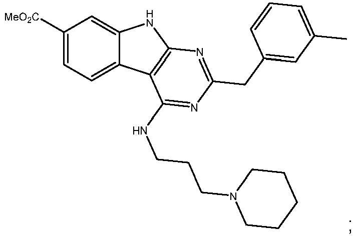 | 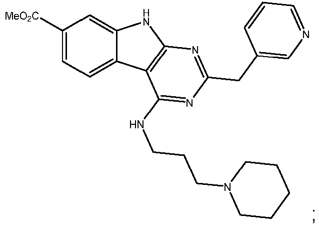 |
| 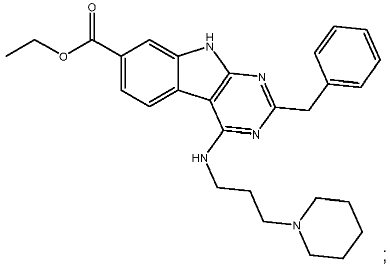 | 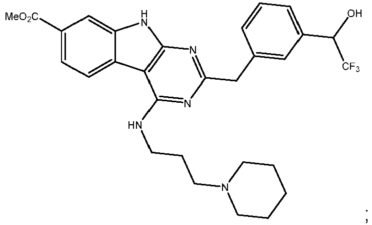 |
| 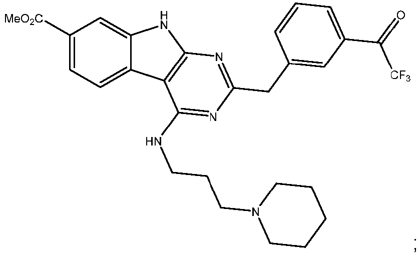 | 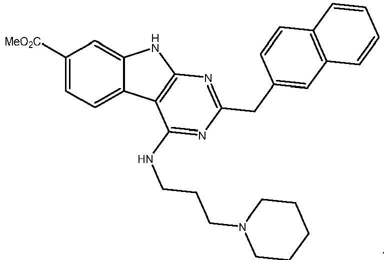 |
| 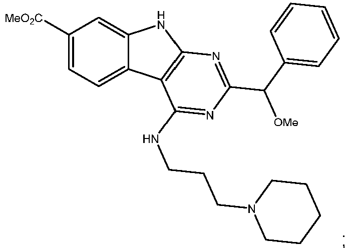 | 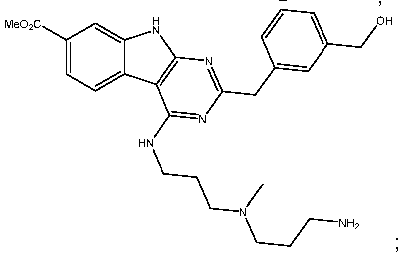 |
| 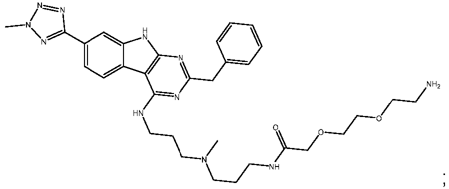 | 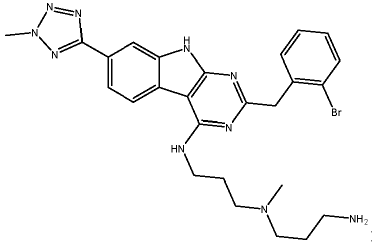 |
| 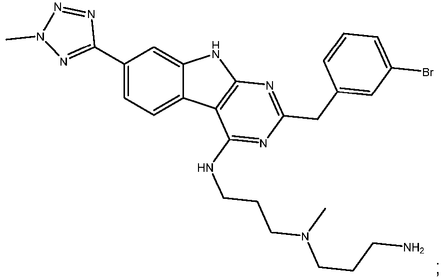 | 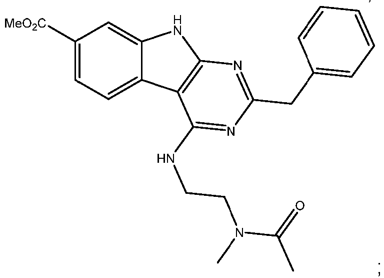 or |
| 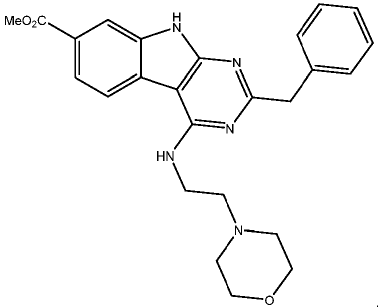 | |

In an embodiment, the Pyrimido[4,5-B]indole derivatives described herein allow to treat cancer. Encompassed herein are any other cancer, based on similar mechanism of action (e.g. K27 mutation, EZH2 or PRC2 mutation).

UM171 by activating the CRL3 complex as described herein degrades RCOR1 which normally acts as the scaffolding protein for the RCOR1/LSD1 and HDAC2 complex, itself being dissociated in the presence of UM171. Thus UM171 acts like a molecular glue degrader, as an anti-cancer agent resulting in inhibition of HDACs and LSD1.

It is demonstrated herein that KBTB4 complex targets CoREST for degradation under UM171 treatment. The UM171 molecule leads to a clear target of CoREST for proteasome degradation, by increasing the interaction between the adaptor/receptor KBTBD4 and CoREST.

### BIOLOGICAL EXAMPLE I

### Whole Genome CRISPR/Cas9 Screen and methodology

The Extended Knockout (EKO) pooled lentiviral library of 278,754 sgRNAs targeting 19,084 RefSeq genes, 3,872 hypothetical ORFs and 20,852 alternatively spliced isoforms was introduced within a clone of OCI-AML5 and OCI-AML1-cell lines engineered to express a doxycycline-inducible Cas9 as previously describe by Bertomeu et al. (2018, Mol Cell Biol, 38(1): e00302-1) The EKO library (kept at a minimum of 500 cells per sgRNA) was cultured in 10 % FBS DMEM supplemented with 2 µg/mL doxycycline for a period of 7 days to induce knockouts. At day 7, 140 million cells were spun at 1,200 rpm for 5 min, washed with 1X PBS, pelleted and frozen. The library was left to expand 8 or 14 more days without doxycycline with 250nM and 800nM UM171 or DMSO only (250 cells per sgRNA on average). Cell concentration was assessed every 2 days. Genomic DNA was extracted from all samples using the QIAamp DNA blood maxi kit (Qiagen). SgRNA sequences were recovered and fitted with Illumina adaptors by PCR and NGS performed on an Illumina HiSeq 2000 device (IRIC) as previously described Bertomeu et al. (2018, Mol Cell Biol, 38(1): e00302-1). Synthetic rescue/positive selection and synthetic lethality/negative selection beta scores were determined using MAGeCK-VISPR (Li et al., 2015, Genome Biol, 16: 281).

Mouse anti-human antibodies were used to detect CD34 (APC or BV421-BD Biosciences), CD45RA (PE- BD Biosciences), CD86 (PerCP-eFluor710-eBioscience), CD90 (PECY7- BioLegend), and CD201 (APC-BioLegend). Flow cytometry acquisitions were performed on a Canto II cytometer (BD Biosciences) and data analysis was performed using FowJo software (Tree Star, Ashland, OR, USA) and GraphPad Prism software. Dead cells were excluded using 7AAD staining.

For intracellular staining, cells were washed in phosphate-buffered saline, pelleted, and fixed using True Nuclear fixation kit (BioLegend, Cat # 424401). Cells were then stained with mouse monoclonal anti-H3K27Ac (Cell Signaling Technology, Catalog #15562), rabbit anti-H3K4me2 (Abcam, Catalog #7766) and rabbit anti-H3 (Cell Signaling Technology, #12167S) and FACS analysis was performed on a BD Biosciences Canto II cytometer.

Umbilical cord blood units were collected from consenting mothers according to ethically approved protocol at Charles-Lemoyne Hospital, Montreal, QC, Canada. Human CD34⁺cord blood (CB) cells were isolated using The EasySep^{™} positive selection kit (StemCell Technologies Cat # 18056). Sorting for more primitive phenotypes was done in additional step using BD Aria II sorter.

Human CD34⁺ cells were cultured in HSC expansion media consisting of StemSpan SFEM (StemCell Technologies) supplemented with human 100 ng/ml stem cell factor (SCF, R&D Systems), 100 ng/ml FMS-like trysine kinase 3 ligand (FLT3, R&D Systems), 50 ng/ml thrombopoietin (TPO, R&D Systems), and 10 µg/ml low-density lipoproteins (StemCell Technologies).

All experiments with animals were conducted under protocols approved by the University of Montreal Animal Care Committee. Fresh CD34+ CB cells or progeny were transplanted by tail vein injection into sub-lethally irradiated (250 cGy, <24 hr before transplantation) 8 to 10-week-old female NSG (NOD-Scid IL2Rynull, Jackson Laboratory) mice. Human cells NSG-BM cells were collected by femoral aspiration or by flushing the two femurs, tibias and hips when animals were sacrificed at week 26.

HDAC inhibitors (panobinostat (Adooq Bioscience, #A10518), Valproic acid (Sigma, #P4543), M344 (Sigma, #M5820), LMK235 (Sigma, #SML1053) were used in CD34+ cord blood cells at 5,1000, 500 and 500nM respectively. LSD1 inhibitor (tranylcypromine (TCP)) was used at 5 and 20 µM. iBET (Selleckchem, #S7189) was used at 50 and 100nM in CD34+ cord blood cells. NEDD8 E1 Activating Enzyme (NAE) Inhibitor MLN4924 (Adooq Bioscience, #A11260-5) was used at 100nM. Proteasome inhibitor MG132 (Adooq Bioscience, #A11043) was used at 10µM.

Lentiviral vectors carrying shRNAs (KBTBD4, CUL3, NUDCD3, CAND1, RCOR1, LSD1) were generated by cloning appropriate shRNA sequences as described in Fellmann et al. (2013, Cell Rep, 5: 1704-1713) into MNDU vectors comprising miR-E sequences as well as GFP. Control vectors contained shRNA-targeting Renilla luciferase (shLuc).

Lentiviral vectors carrying sgRNA (KBTBD4, NUDCD3) were generated by cloning appropriate sequences (KBTBD4: GGCTAGCATGGAATCACCAG; SEQ ID NO: 1; NUDCD3: GGAGCGCTCCATGGCCACCG; SEQ ID NO: 2) into pLKO5.sg.EFS.tRFP647 lentiviral vector. Control vector contained sgRNA-targeting AAVS1 locus (sgAAVS1).

KBTBD4 cDNA (Dharmacon, # MHS6278-202829492) was subcloned into MNDU-eGFP lentiviral vector. BTB and Kelch mutants were generated by deleting the BTB domain and the first 3 kelch domains, respectively.

Lentiviruses were produced in HEK-293 cells and primary CD34+ cells or AML cell lines were infected with lentiviruses in media supplemented with 10 ng/mL polybrene for 24 hours. Infection efficiency, as determined by the percentage of GFP positive cells, was monitored by flow cytometry using a BD FACSCantoll flow cytometer. When needed, infected cells were sorted using a BD Aria II cell sorter and knockdown efficiency was determined by Western blotting using standard methods.

Total protein extraction was performed in lysis buffer (25mM tris ph7.5, 150mM NaCl, 1% NP-40, protease inhibitors). Chromatin-bound (CB) and cytoplasmic/nucleoplasmic (CN) fractionation were performed as described by Mladenov and colleagues (2007, J Cell Physiol, 211: 468-476). Cytoplasmic fractionation was performed in lysis buffer (10mM pipes ph6.8, 0.3M sucrose, 0.1M NaCl, 3mM MgCl2, 5mM EGTA, 0.015% digitonin and protease inhibitors).

Anti-human antibodies were used to detect KBTBD4 (Novus Biologicals, # NBP1-88587), NUDCD3 (Novus Biologicals, #NBP1-82940), CUL3 (Novus Biologicals, #NB100-58788), RCOR1 (Novus Biologicals, #NB600-240), LSD1 (Cell Signaling Technology, #2139S), HDAC2 (Bethyl Laboratories, #A300-705A), H3K27Ac (Cell Signaling Technology, #8173S), CUL1 (Santa Cruz Biotechnology, #sc-17775), NCL (Cell Signaling Technology, #14574S), TUBA (Cell Signaling Technology, #2144) and H3 (Millipore, #06-755).

BiolD pulldown experiments were performed as described previously (Comartin et al., 2013, Curr Biol, 23: 1360-1366). In brief, full-length human KBTBD4 coding sequences were amplified by PCR and cloned into pcDNA-FRT/TO-FLAGBirA expression vector. 293T cells stably expressing FLAGBirA or FLAGBirA*-KBTBD4 were generated and incubated for 24 hrs in complete media supplemented with 1 µg/ml tetracycline (Sigma), 50µM biotin (BioShop, Burlington, ON, Canada) and 10µM MG132. Cells were collected and lysed in lysis buffer. Protein extracts were incubated with streptavidin-Sepharose beads.

Peptides were analyzed by LC-MS/MS using a Proxeon nanoflow HPLC system coupled to a tribrid Fusion mass spectrometer (Thermo Fisher Scientific). Each sample was loaded and separated on a reverse-phase analytical column (18 cm length, 150mmi.d.) (Jupiter C18,3mm, 300 A°, Phenomenex) packed manually. LC separations were performed at a flow rate of 0.6mL/min using alinear gradient of 5-30 % aqueous ACN (0.2 % FA) in 106 min. MS spectra were acquired with a resolution of 60,000. "TopSpeed"(maximum number of sequencing events within 5 s window) method was used for data dependent scans on the most intense ions using high energy dissociation (HCD). AGC target values for MS and MS/MS scans were set to 5e5 (max fill time 200 ms) and 5e4 (maxfill time 200 ms), respectively. The precursor isolation window was set to m/z 1.6 with a HCD normalized collision energy of 25. The dynamic exclusion window was set to 30 s. MS data were analyzed using MaxQuant software version 1.3.0.3 and searched against the SwissProt subset of the H.Sapiens uniprot database.

5 x 10⁵ OCI-AML5 cells were exposed or not to UM171 (250nM) for 6 to 72h and preserved at -80C in TRIzol Reagent (Thermo Fisher Scientific cat # 15596026). cDNA libraries were constructed according to TruSeq Protocols (Illumina) and sequencing was performed using an Illumina HiSeq 2000 instrument.

Gene expression statistics were obtained using the kallisto/sleuth analysis pipeline and the GRCh38 version 84 annotation. TPM values were loaded into R and differential expression was tested using Wilcoxon rank sum statistics. Differentially expressed genes were selected based on significance (p ≤ 0.01, Mann-Whitney test).

### BIOLOGICAL EXAMPLE II

### PK assays demonstrating bioavailability of compounds administered orally and/or intravenously

PK assays were performed in mice (n=3). The compounds were intravenously administered at 1 mg/kg (coumpound in Fig. 16: 5% dextrose and UM681 in Fig. 17A: 25 mM citric acid:5% Dextrose (1:3)) and plasma samples were collected at 5, 15, 30 minutes and 1, 2, 4, 6 and 8 hours after administration. The following pharmacokinetic parameters were calculated. The half-life of compound in Fig. 16 (T_{1/2}) was 1.7 hours, the volume of distribution (Vss) was determined to be 13.9 L/kg and the clearance (CL) was 141.6 mL/min/kg. For UM681, T_{1/2} was 1.9 hours, *Vss* was determined to be 14.3 L/kg and CL was 109 mL/min/kg.

The compounds were orally administered at 20 mg/kg and plasma samples were collected at 15, 30 minutes and 1, 2, 4, 6 and 8 hours after administration. The following pharmacokinetic parameters were calculated. The maximal concentration (Cₘₐₓ) of UM729 was 0.2 uM, the maximal time (tₘₐₓ) was determined to be 0.8 hours, the area under the curve (AUC) was 1.2 uM*h and the % bioavailability (%F) was 19% (see Fig. 17B). For UM681, Cₘₐₓ was also 0.2 uM, tₘₐₓ was determined to be 4 hours, the AUC was 2.45 uM*h and the % bioavailability (%F) was 31% (male) and 39% (female).

While the present disclosure has been described in connection with specific embodiments thereof, it will be understood that it is capable of further modifications and this application is intended to cover any variations, uses, or adaptations, including such departures from the present disclosure as come within known or customary practice within the art and as may be applied to the essential features hereinbefore set forth, and as follows in the scope of the appended claims.

## Claims

1. A compound of formula I for use in the treatment of cancer in a patient, wherein said at least one compound of formula I is: 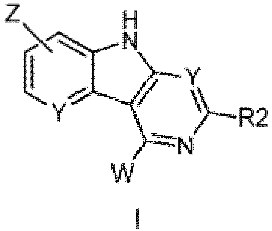
or a salt thereof,
wherein:
each Y is independently selected from N and CH;
Z is each time independently selected from:
-CN
-C(O)OR1,
-C(O)N(R1)R3,
-C(O)R1,
or -heteroaryl optionally substituted with one or more RA or R4 substituents,
wherein, when (R1) and R3 are attached to a nitrogen atom, optionally they join together with the nitrogen atom to form a 3 to 7-membered ring which optionally includes one or more other heteroatom selected from N, O and S, optionally the ring is substituted with one or more RA or R4;
W is
-CN,
-N(R1)R3,
-C(O)OR1,
-C(O)N(R1)R3,
-NR1C(O)R1,
-NR1C(O)OR1,
-OC(O)N(R1)R3,
-OC(O)R1,
-C(O)R1,
-NR1C(O)N(R1)R3,
-NR1S(O)₂R1,
-benzyl optionally substituted with 1, 2 or 3 RA or R1 substituents,
-X-L-(X-L)n - N(R1)R3,
-X-L-(X-L)n - heteroaryl optionally substituted with one or more RA or R4 substituents attached on either or both the L and heteroaryl groups,
-X-L-(X-L)n - heterocyclyl optionally substituted with one or more RA or R4 substituents attached on either or both the L and heterocyclyl groups,
-X-L-(X-L)n- aryl optionally substituted with one or more RA or R4 substituents,
-X-L-(X-L)ₙ-NR1RA,
or
-(N(R1)-L)ₙ - N⁺R1R3R5 R6⁻;
wherein n is an integer equal to either 0, 1, 2, 3, 4, or 5,
and wherein, when R1 and R3 are attached to a nitrogen atom, optionally they join together with the nitrogen atom to form a 3 to 7-membered ring which optionally includes one or more other heteroatom selected from N, O and S, optionally the ring is substituted with one or more RA or R4;
each X is independently selected from CH₂, O, S and NR1;
each L is independently
-C₁₋₆ alkylene,
-C₂₋₆ alkenylene,
-C₂₋₆ alkynylene,
-C₃₋₇cycloalkylene, which optionally includes one or more other heteroatom selected from N, O and S or
-C₃₋₇ cycloalkenylene, which optionally includes one or more other heteroatom selected from N, O and S
wherein the alkylene, the alkenylene, the alkynylene the cycloalkylene and the cycloalkenylene groups are each independently optionally substituted with one or two R4 or RA substituent;
R1 is each independently
-H,
-C₁₋₆ alkyl,
-C₂₋₆ alkenyl,
-C₂₋₆ alkynyl,
-C₃₋₇ cycloalkyl,
-C₃₋₇ cycloalkenyl,
-C₁₋₅ perfluorinated alkyl,
-heterocyclyl,
-aryl,
-heteroaryl,
or
-benzyl,
wherein the alkyl, the alkenyl, the alkynyl, the cycloalkenyl, the perfluorinated alkyl, the heterocyclyl, the aryl, the heteroaryl and the benzyl groups are each independently optionally substituted with 1, 2 or 3 RA or Rd substituents;
R2 is
-H,
-C₁₋₆ alkyl, optionally substituted with one more RA substituents,
-C(O)R4,
-L-heteroaryl optionally substituted with one or more RA or R4 substituents,
-L-heterocyclyl optionally substituted with one or more RA or R4, or
-L-aryl optionally substituted with one or more RA or R4 substituents,
R3 is each independently
-H,
-C₁₋₆ alkyl,
-C₂₋₆ alkenyl,
-C₂₋₆ alkynyl,
-C₃₋₇ cycloalkyl,
-C₃₋₇ cycloalkenyl,
-C₁₋₅ perfluorinated alkyl,
-heterocyclyl,
-aryl,
-heteroaryl,
or
-benzyl,
wherein the alkyl, the alkenyl, the alkynyl, the cycloalkyl, the cycloalkenyl, the perfluorinated alkyl, the heterocyclyl, the aryl, the heteroaryl and the benzyl groups are each independently optionally substituted with 1, 2 or 3 RA or Rd substituents;
R4 is each independently
-H,
-C₁₋₆ alkyl,
-C₁₋₆ haloalkyl,
-C₂₋₆ alkenyl,
-C₂₋₆ alkynyl,
-C₃₋₇ cycloalkyl,
-C₃₋₇ cycloalkenyl,
-C₁₋₅ perfluorinated alkyl,
-heterocyclyl,
-aryl,
-heteroaryl,
or
-benzyl,
wherein the alkyl, the alkenyl, the alkynyl, the cycloalkyl, the cycloalkenyl, the perfluorinated alkyl, the heterocyclyl, the aryl, the heteroaryl and the benzyl groups are each independently optionally substituted with 1, 2 or 3 RA or Rd substituents;
R5 is each independently
-C₁₋₆ alkyl,
-C₁₋₆ alkylene-C₂₋₆ alkenyl which optionally includes one or more other heteroatom selected from N, O and S
-C₁₋₆ alkylene-C₂₋₆ alkynyl which optionally includes one or more other heteroatom selected from N, O and S
-L-aryl which optionally includes one or more RA or R4 substituents
-L-heteroaryl which optionally includes one or more RA or R4 substituents
-C₁₋₆ alkylene-C(O)O-
-C₁₋₆ alkylene-C(O)OR1
-C₁₋₆ alkylene-CN
-C₁₋₆ alkylene-C(O)NR1R3, wherein R1 and R3 optionally they join together with the nitrogen atom to form a 3 to 7-membered ring which optionally includes one or more other heteroatom selected from N, O and S; or
-C₁₋₆ alkylene-OH;
R6 is
-Halogen
-OC(O)CF₃
or
-OC(O)R1;
RA is each independently
-halogen,
-CF₃,
-OR1,
-L-OR1,
-OCF₃,
-SR1,
-CN,
-NO₂,
-NR1R3,
-L-NR1R1,
-C(O)OR1,
-S(O)₂R4
-C(O)N(R1)R3,
-NR1C(O)R1,
-NR1C(O)OR1,
-OC(O)N(R1)R3,
-OC(O)R1,
-C(O)R4,
-NHC(O)N(R1)R3,
-NR1C(O)N(R1)R3,
or
-N₃;
and
Rd is each independently
-H,
-C₁₋₆ alkyl,
-C₂₋₆ alkenyl,
-C₂₋₆ alkynyl,
-C₃₋₇ cycloalkyl,
-C₃₋₇ cycloalkenyl,
-C₁₋₅ perfluorinated alkyl
-benzyl
or
-heterocyclyl.

2. The compound for use according to claim 1, wherein the compound of formula I is 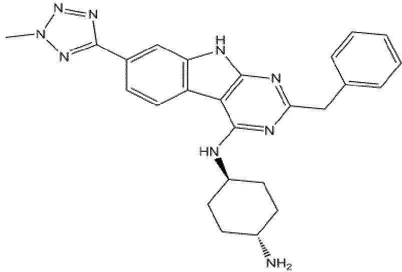 or a pharmaceutically acceptable salt thereof.

3. The compound for use according to claim 1, wherein the compound of formula I is a hydrobromide salt of

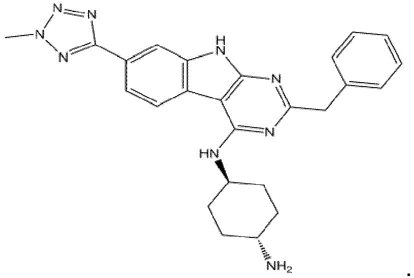

4. The compound for use according to claim 1, wherein the compound of formula I is 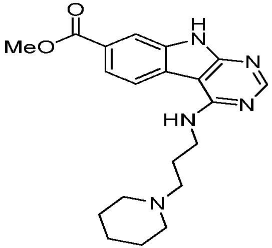 or a pharmaceutically acceptable salt thereof.

5. The compound for use according to claim 1, wherein the compound of formula I is 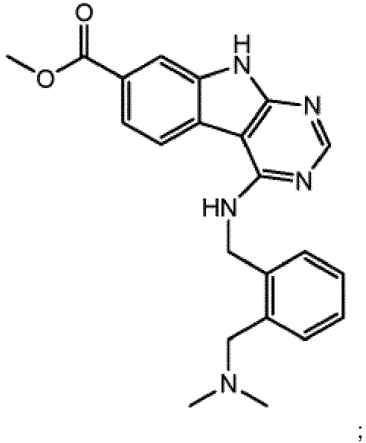 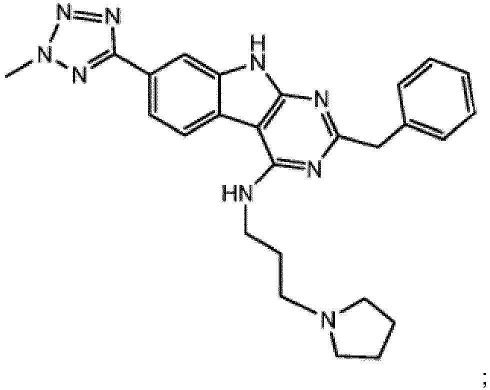 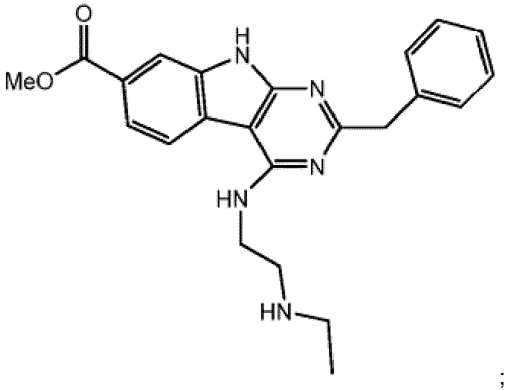 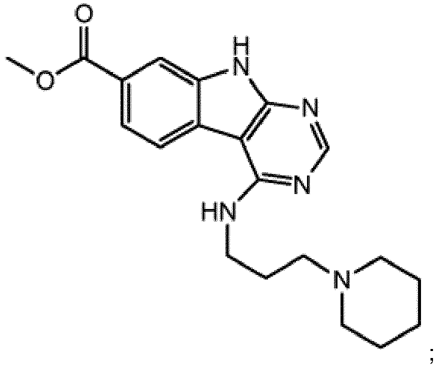 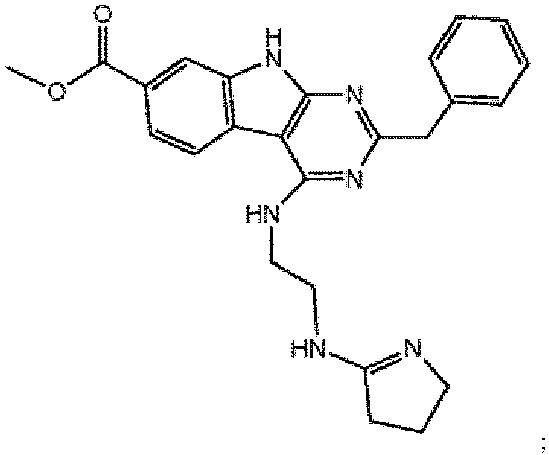 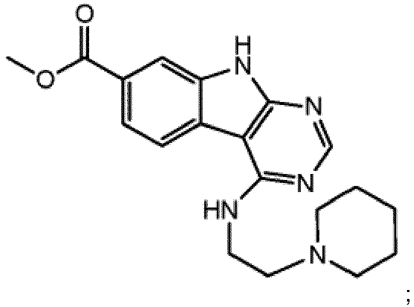 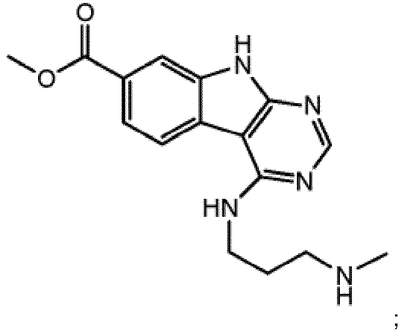 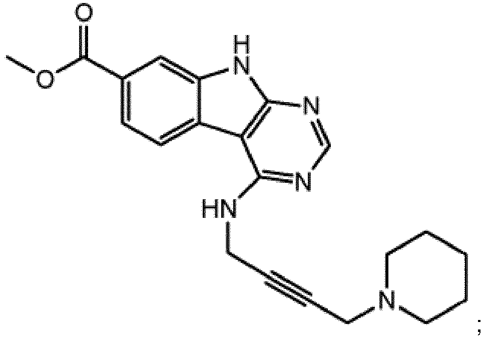 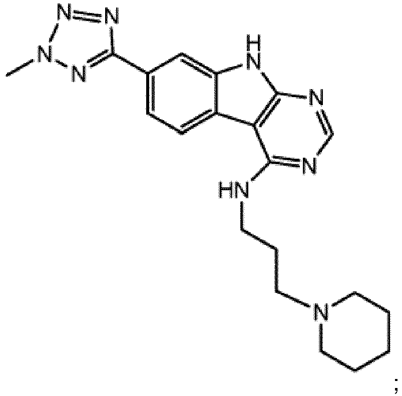 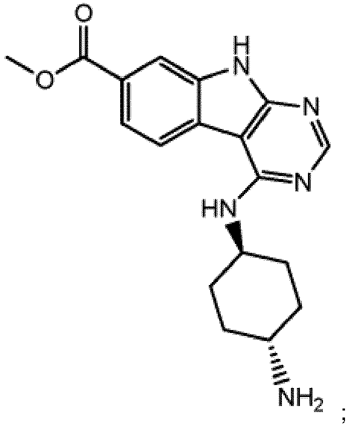 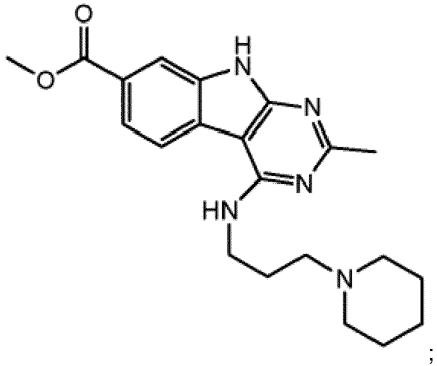 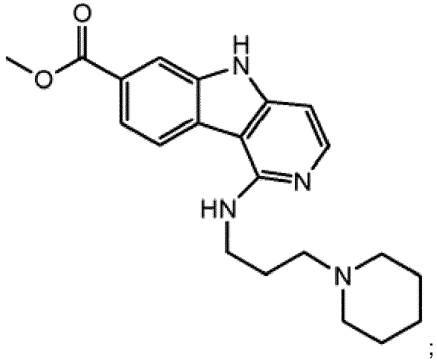 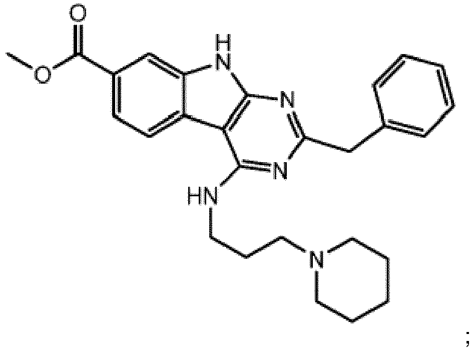 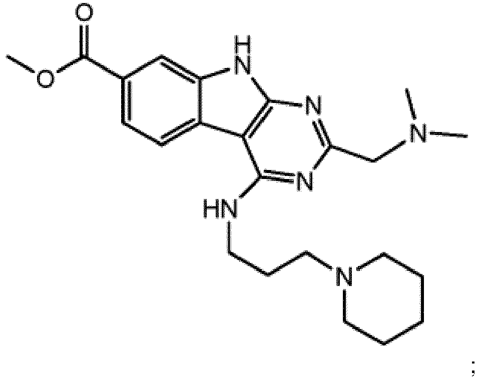 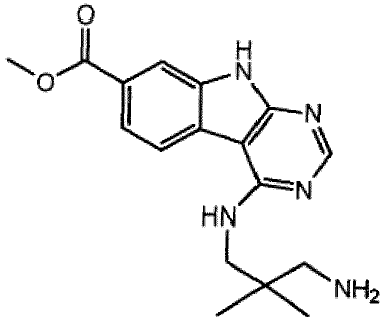 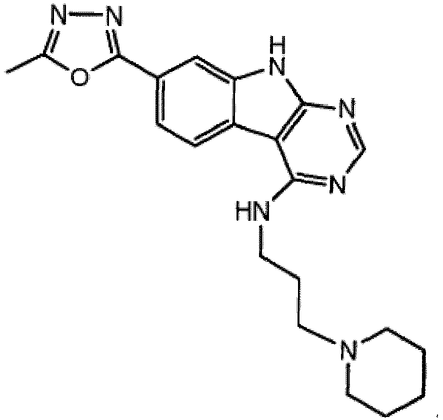 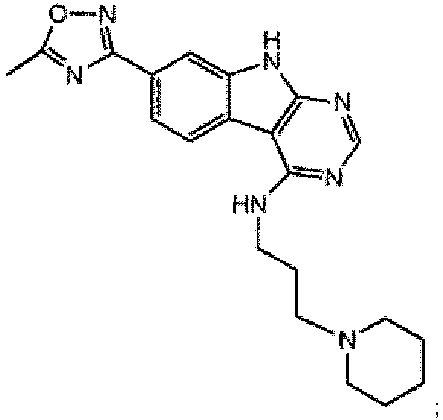 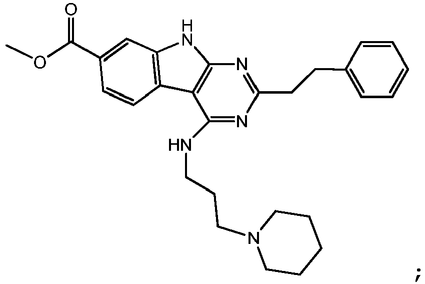 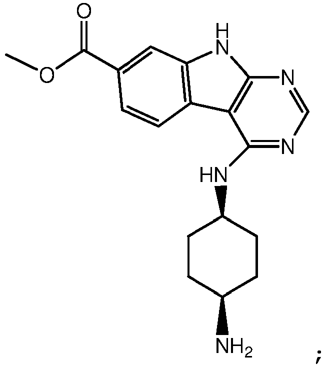 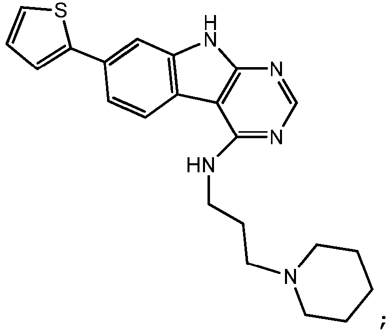 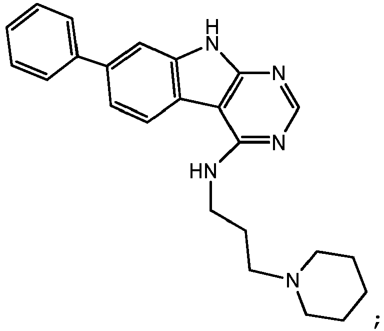 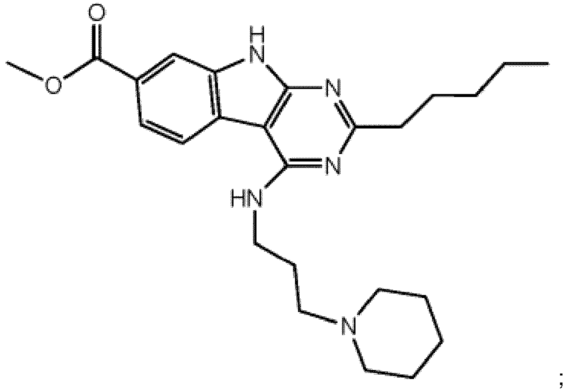 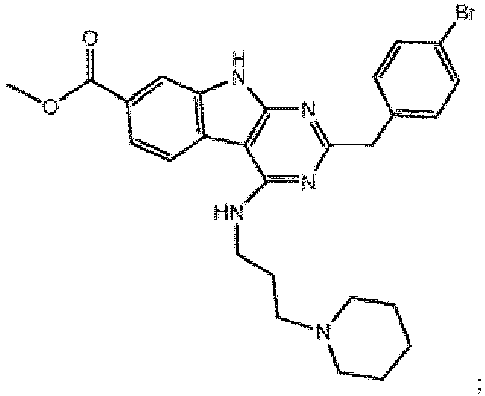 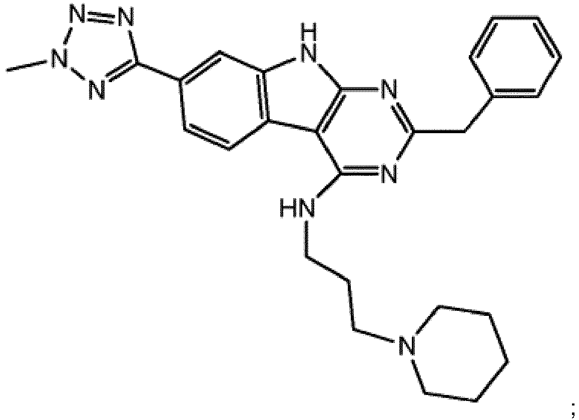 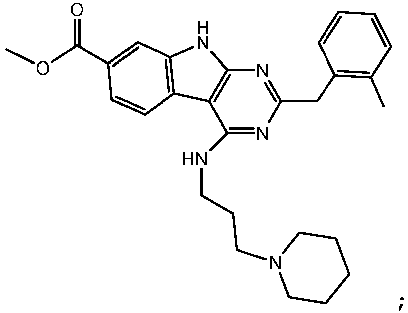 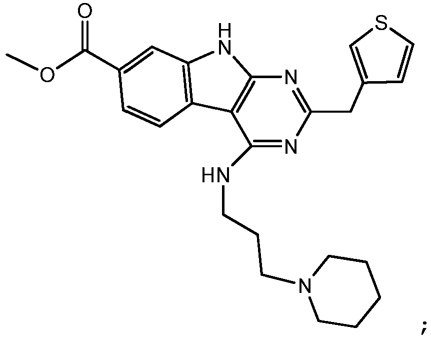 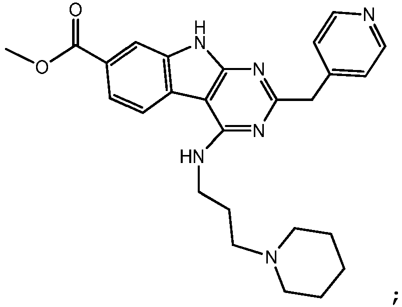 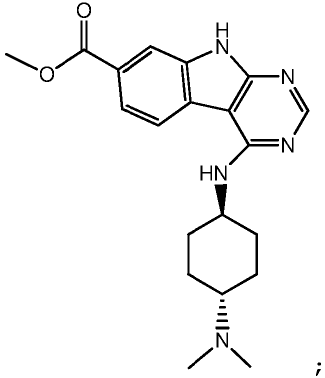 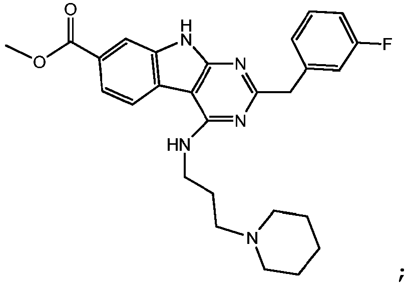 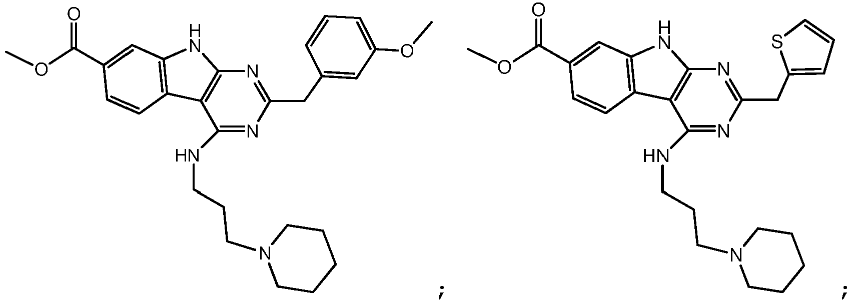 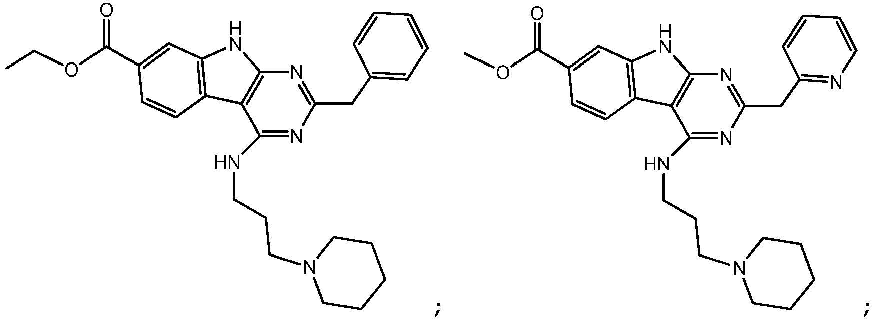 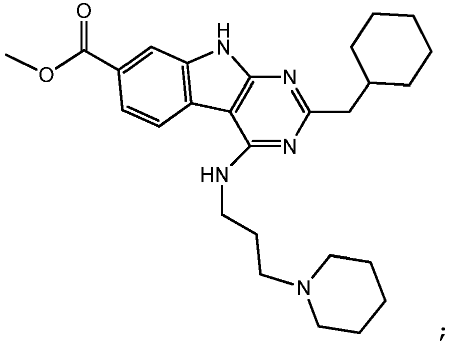 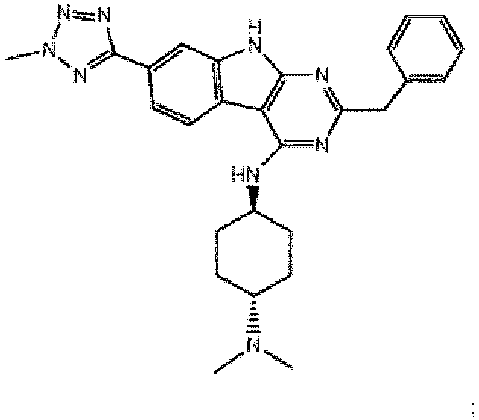 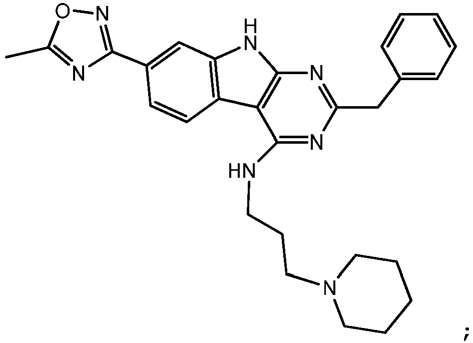 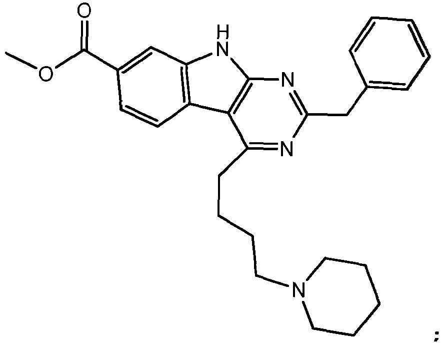 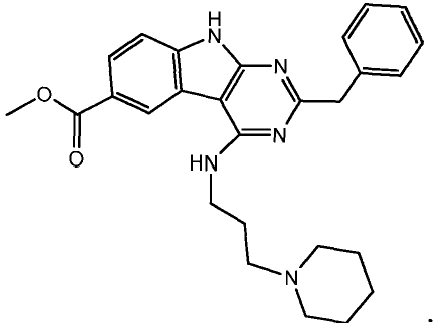 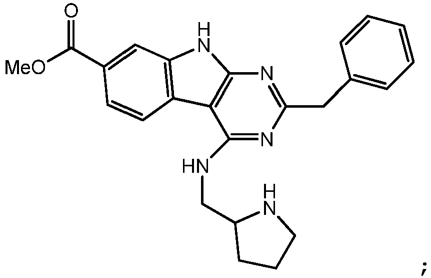 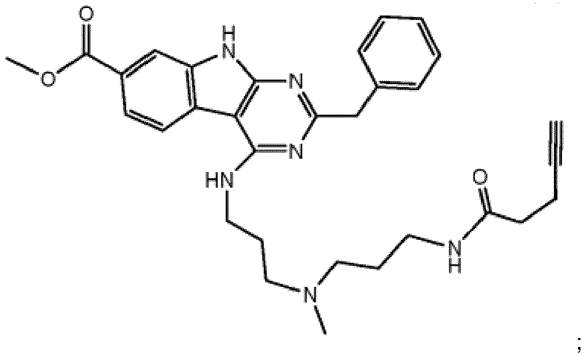 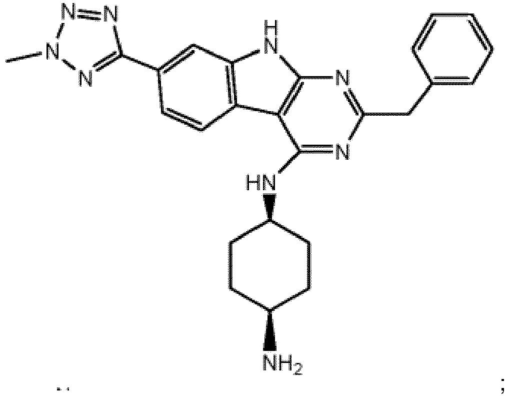 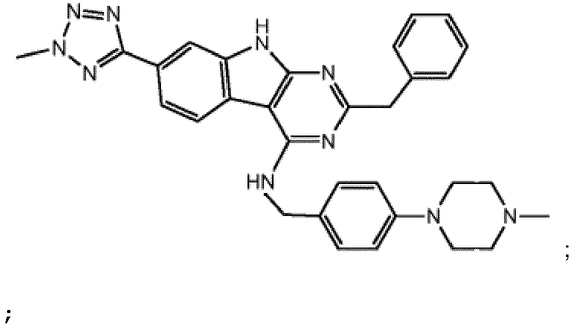 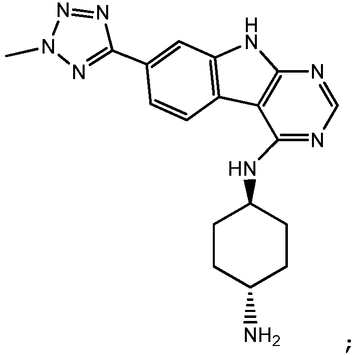 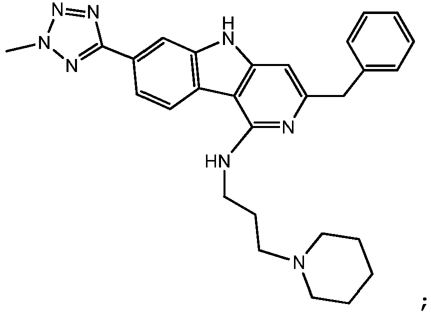 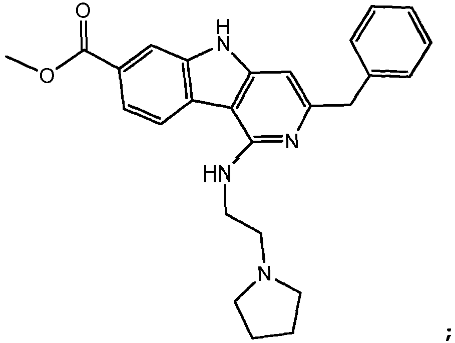 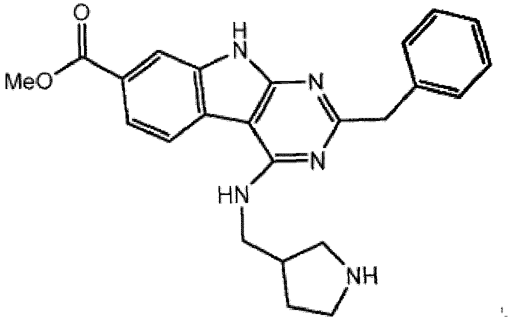 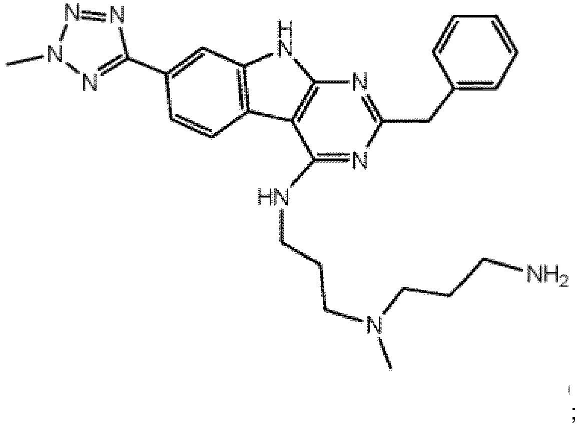 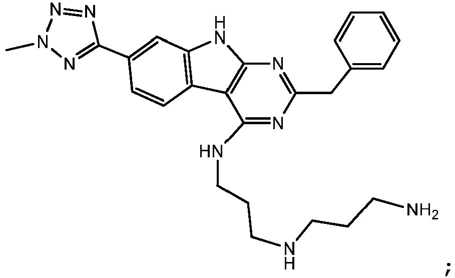 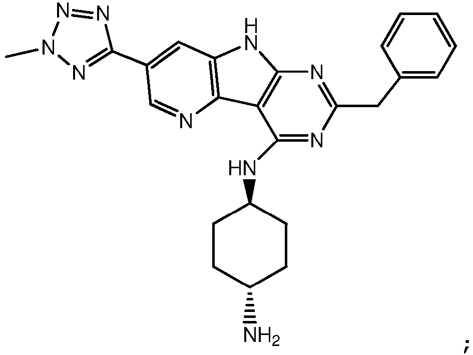 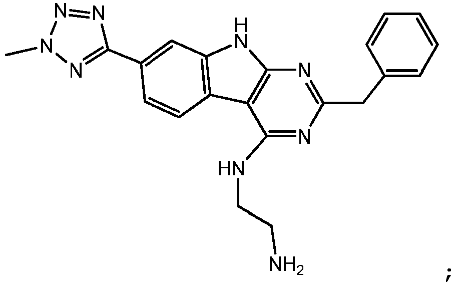 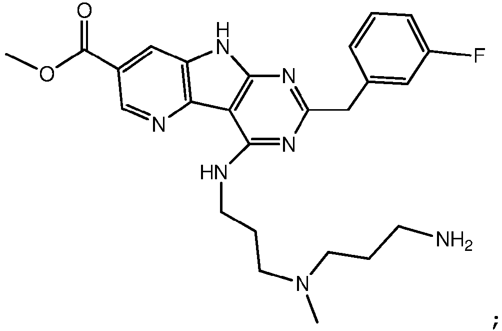 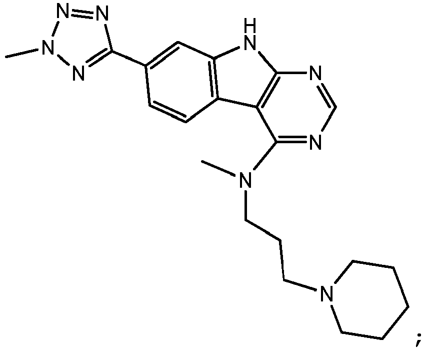 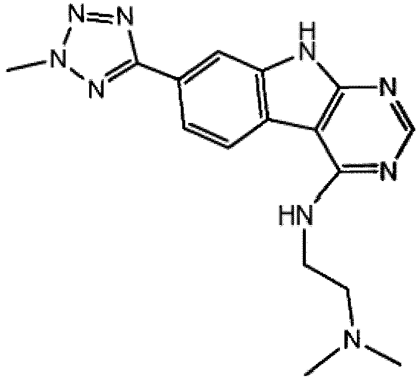 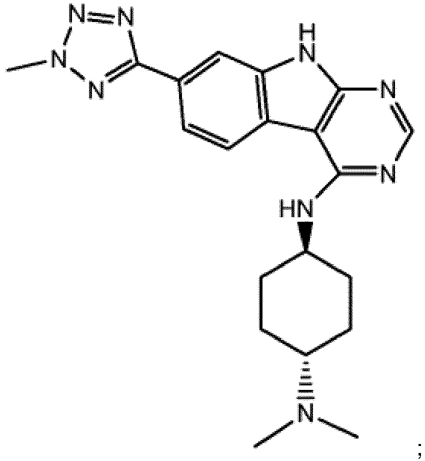 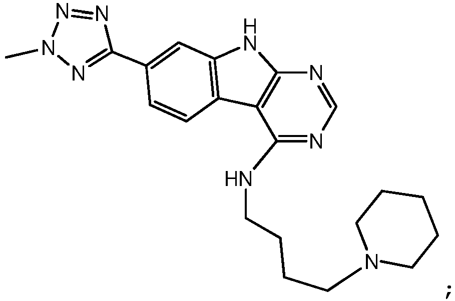 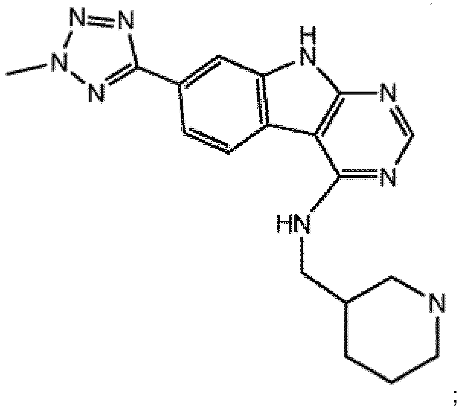 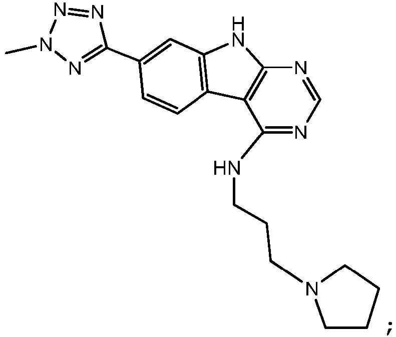 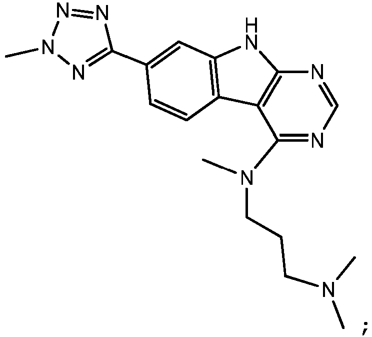 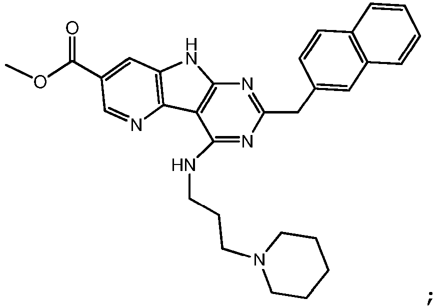 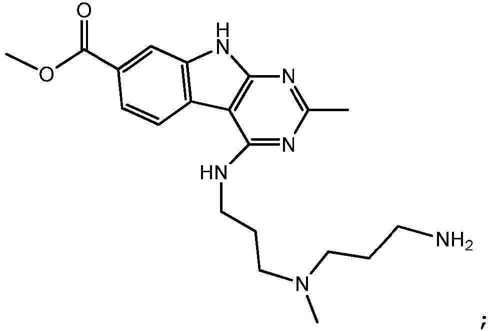 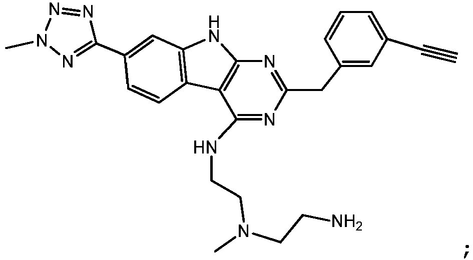 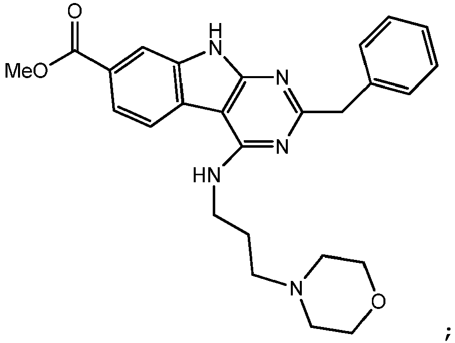 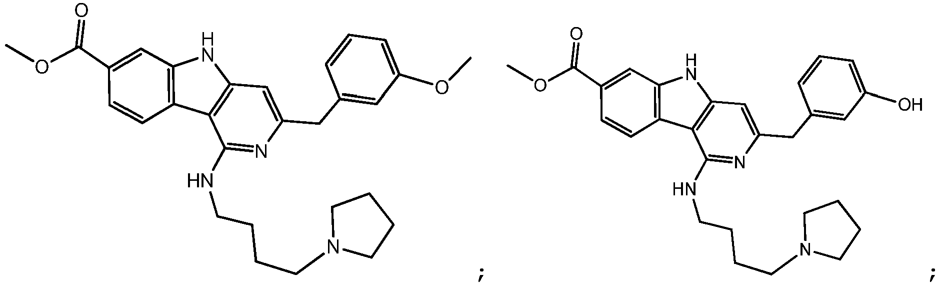 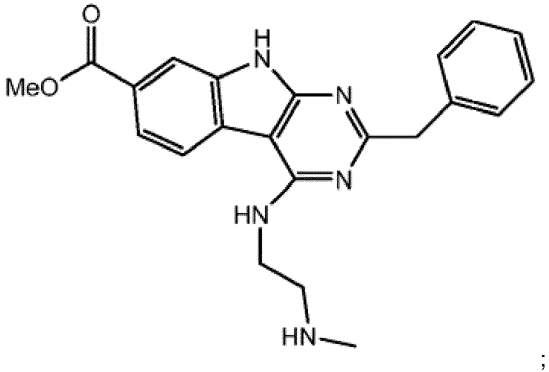 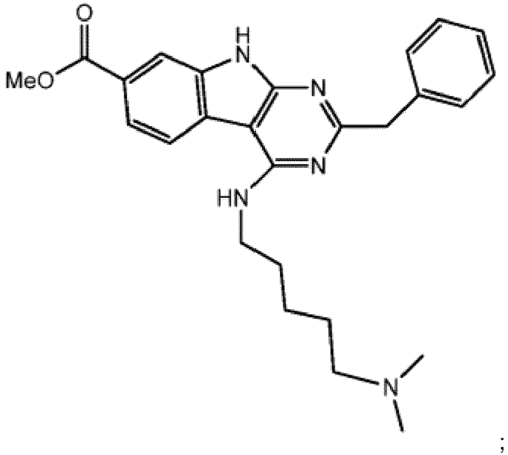 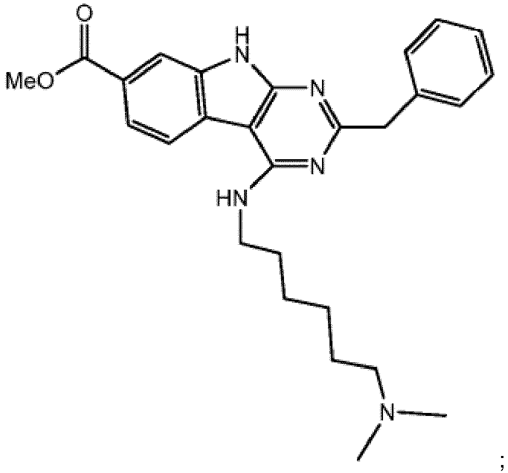 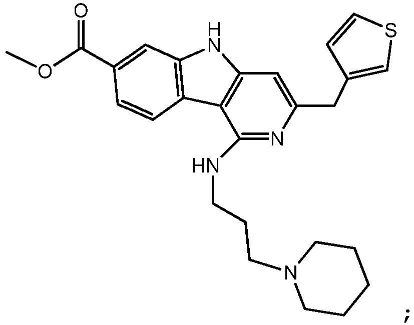 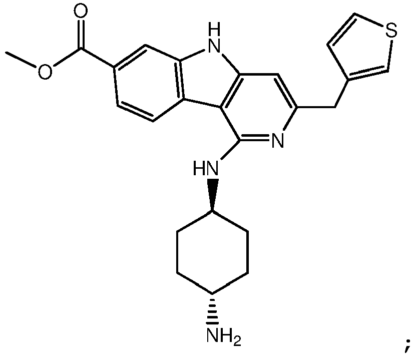 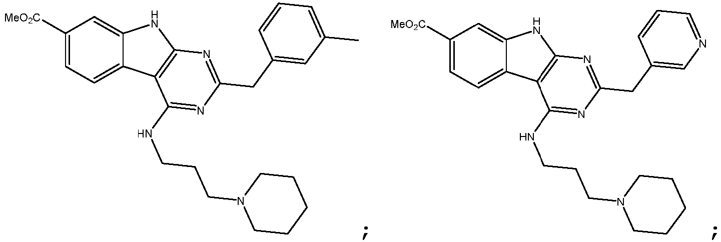 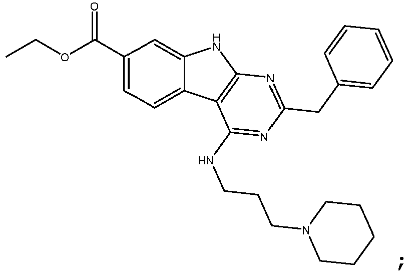 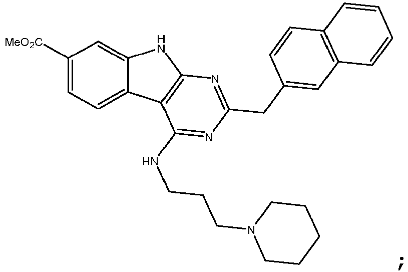 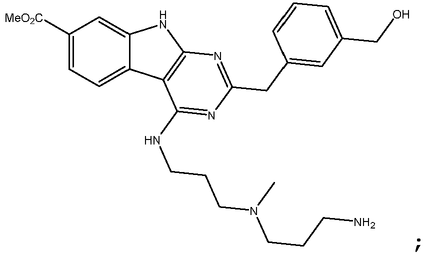 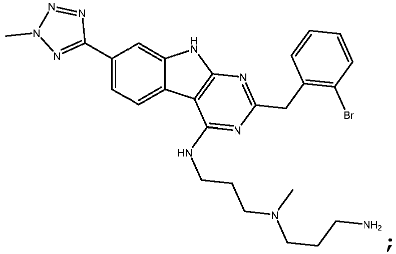 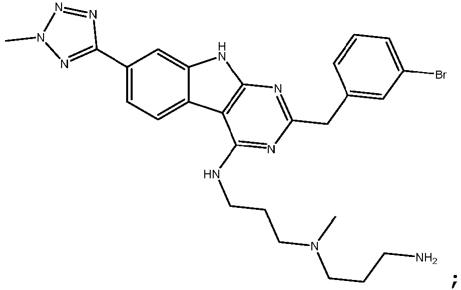 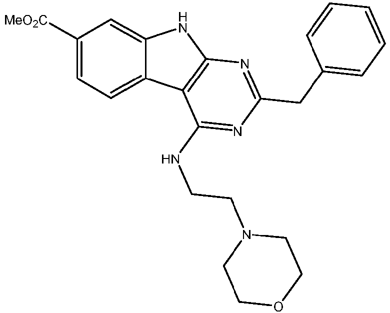 or a salt thereof.

6. The compound for use according to any one of claims 1-5, wherein said patient is a human or an animal.

7. The compound for use according to claim 6, wherein said animal is a mouse.

8. The compound for use according to any one of claims 1-7, wherein said compound is formulated for an administration orally, intramuscularly, intravenously or subcutaneously.

9. The compound for use according to any one of claims 1-8, wherein the compound degrades at least one of LSD1, RCOR1, HDAC2 and CoREST.

10. The compound for use according to any of claims 1-9, wherein said cancer is a cancer based on a K27 mutation, EZH2 mutation or PRC2 mutation.

11. A compound selected from 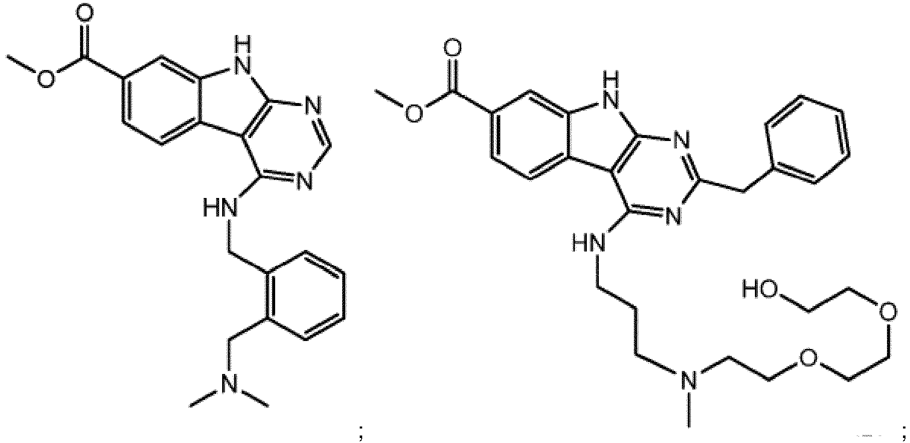 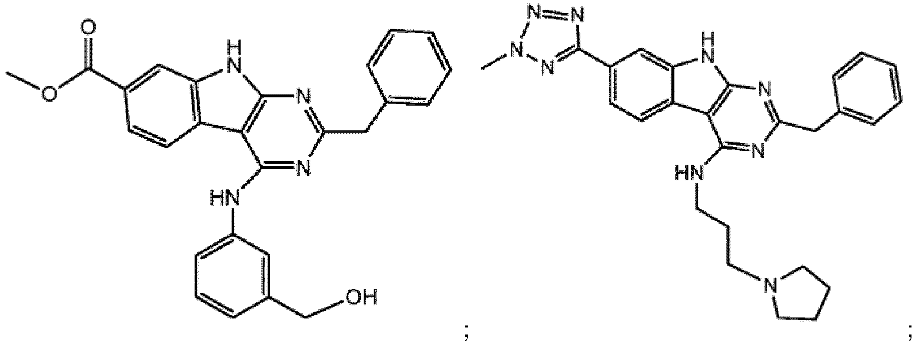 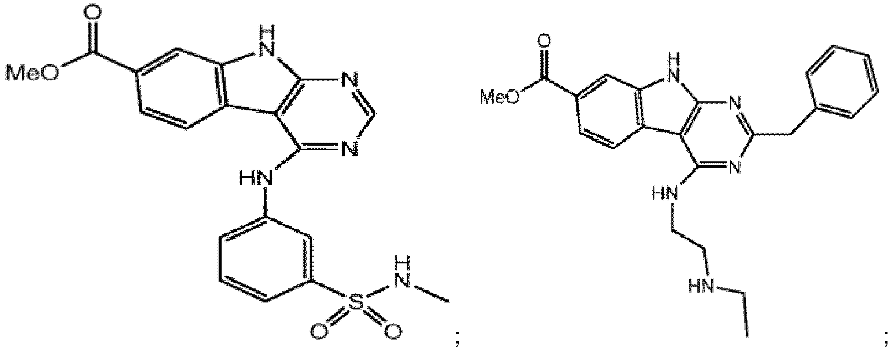 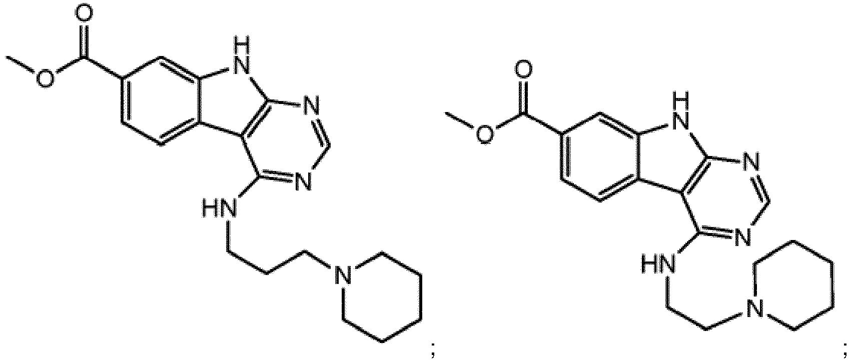 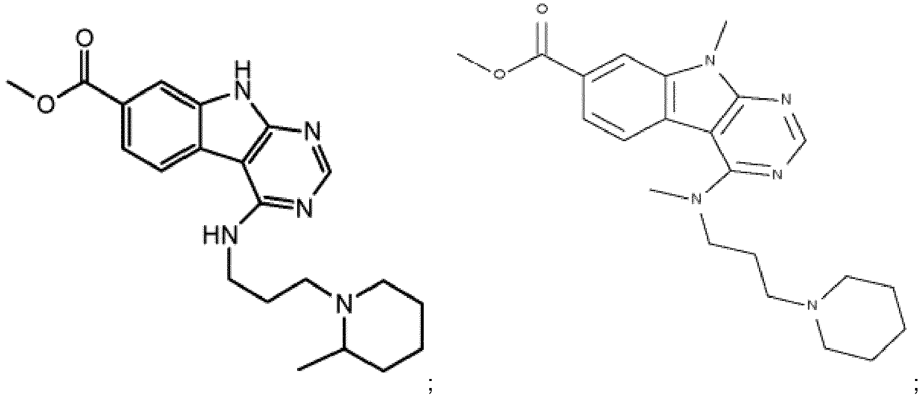 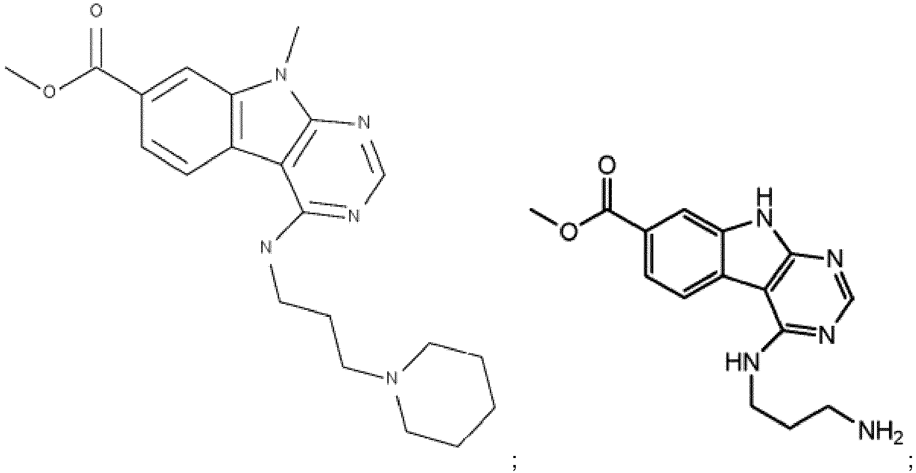 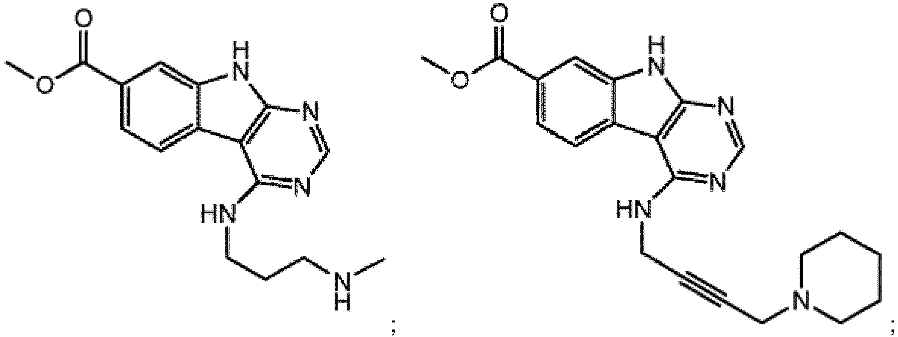 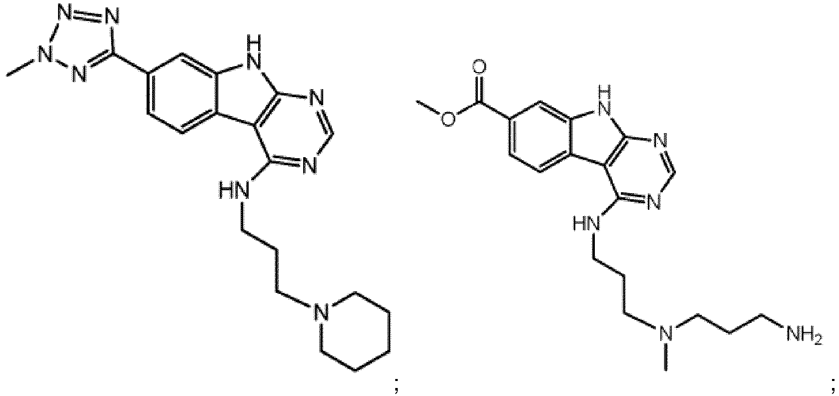 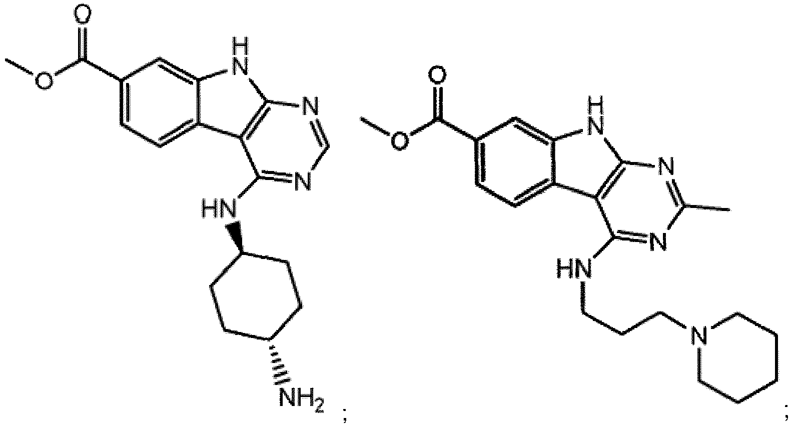 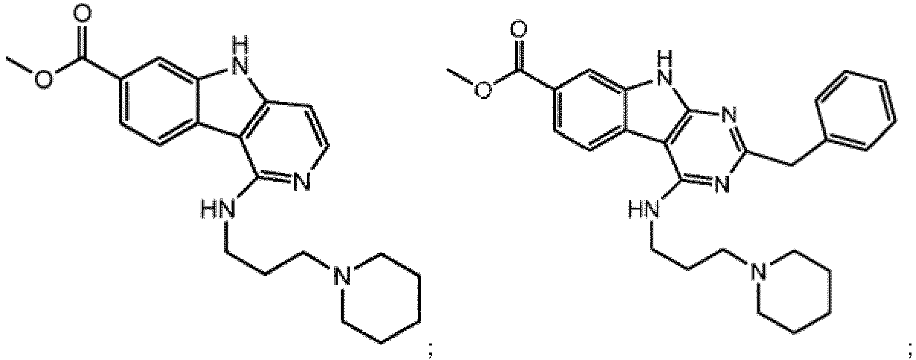 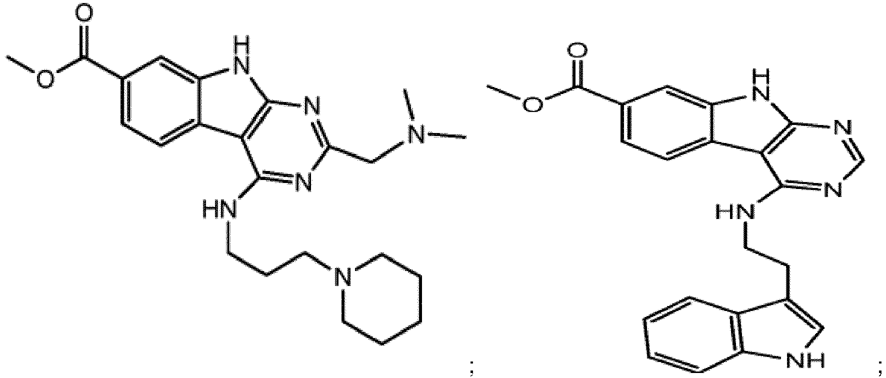 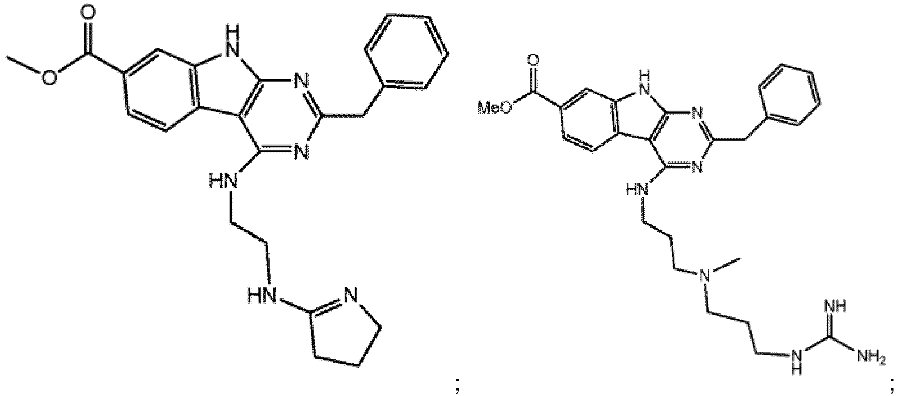 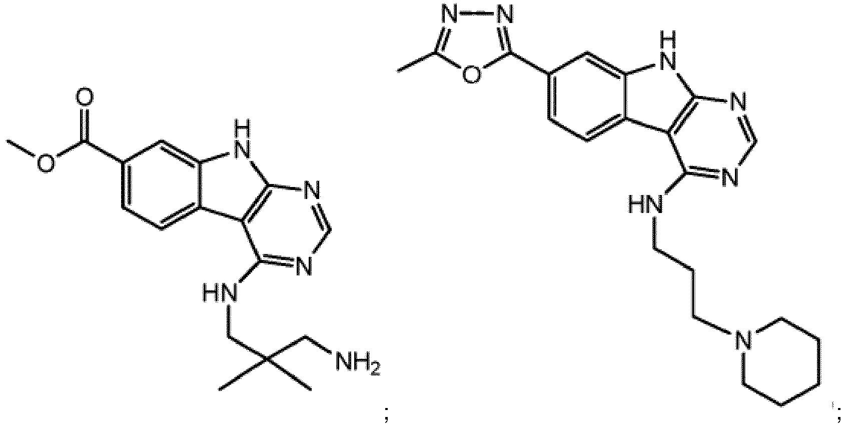 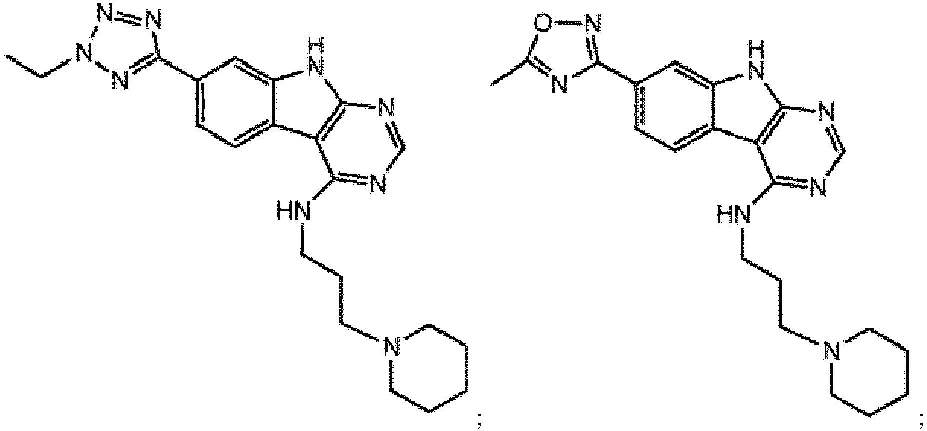 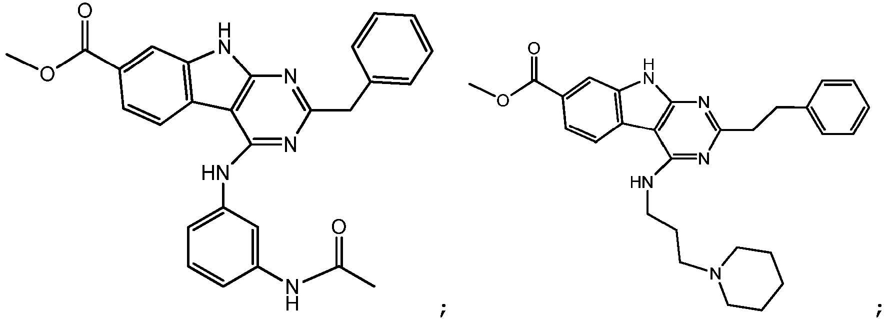 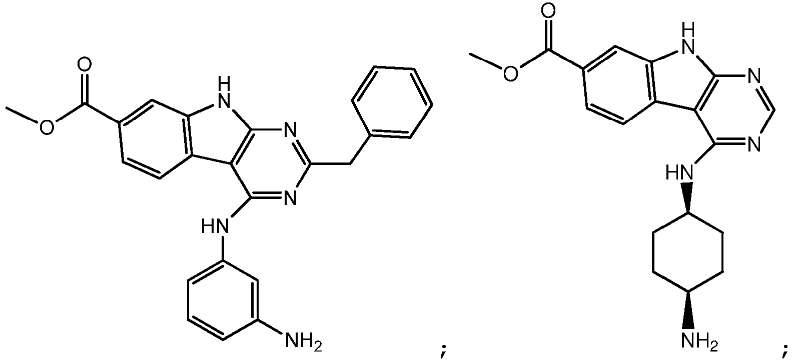 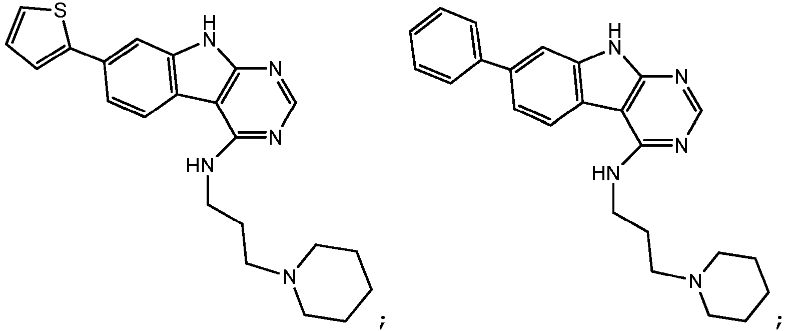 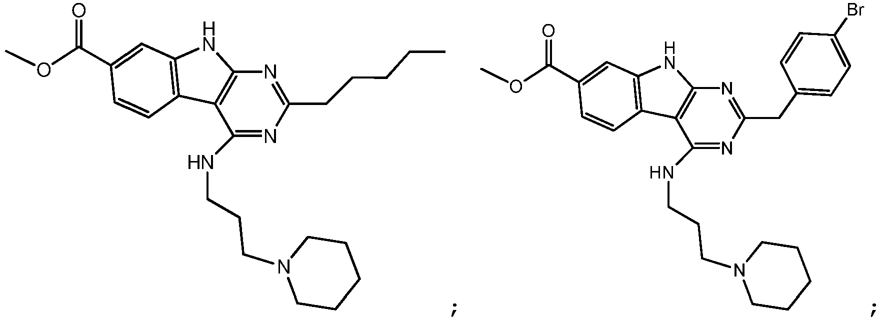 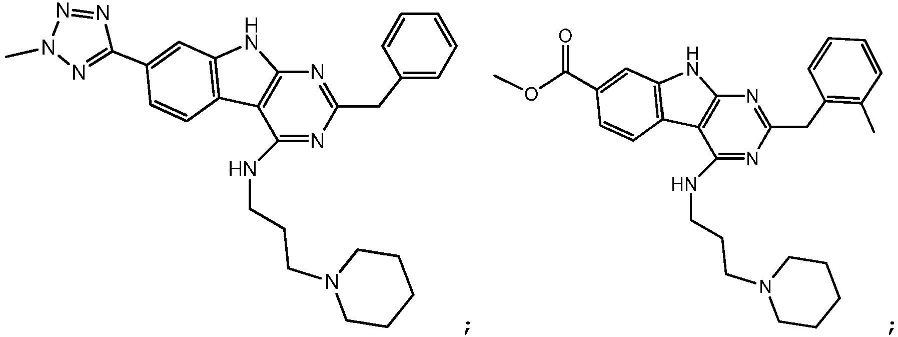 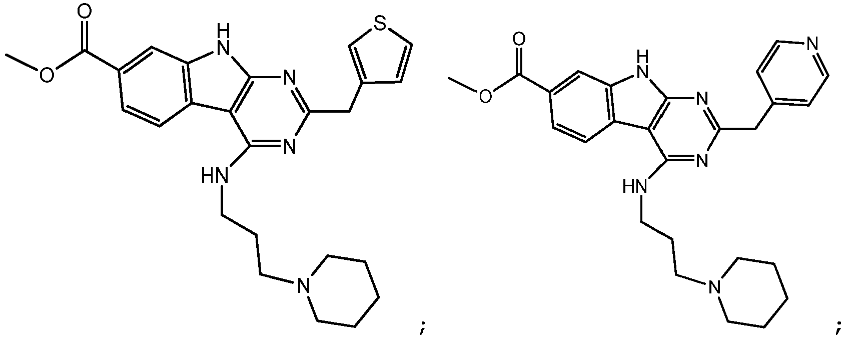 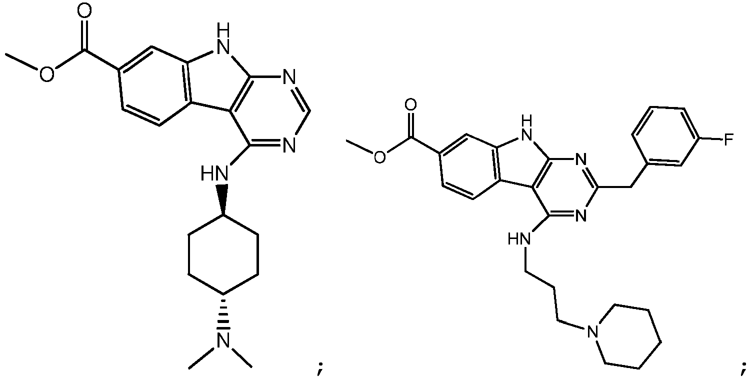 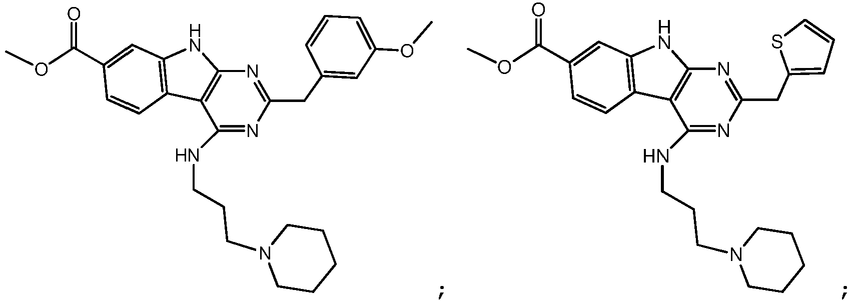 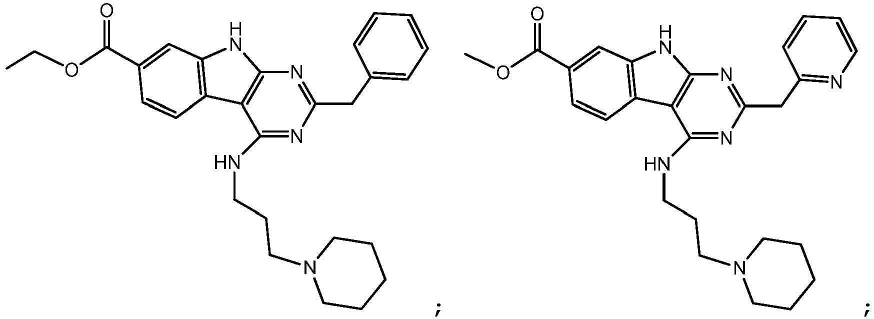 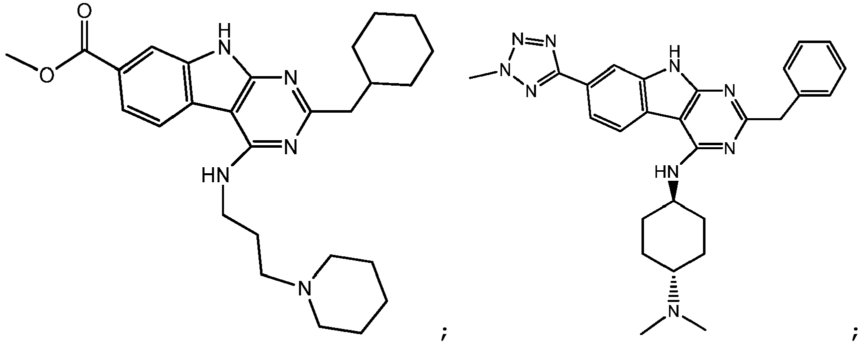 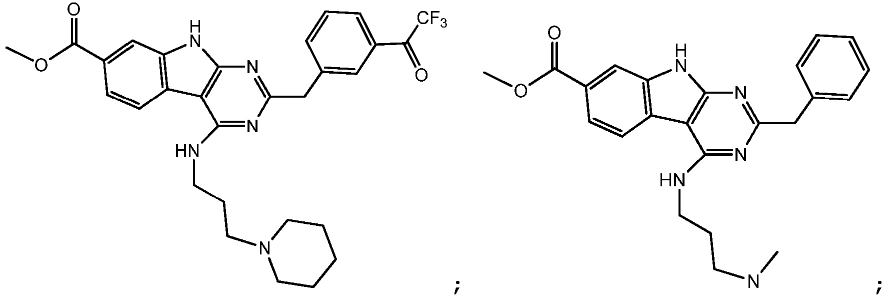 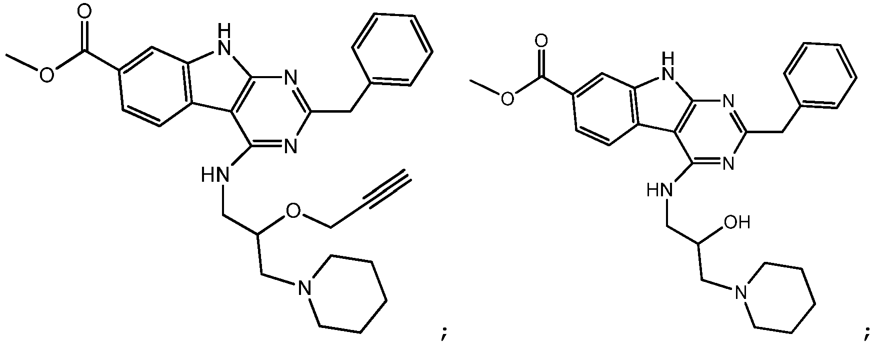 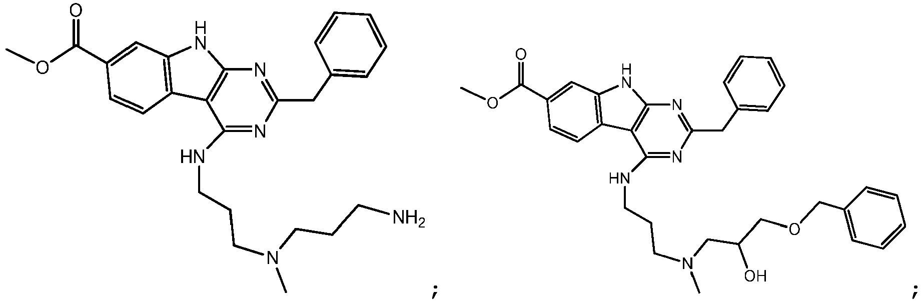 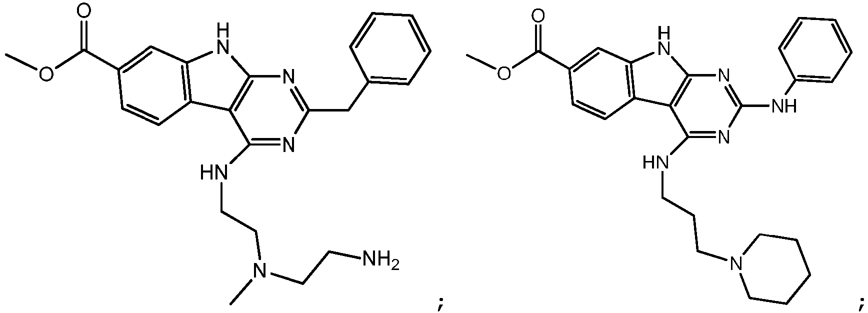 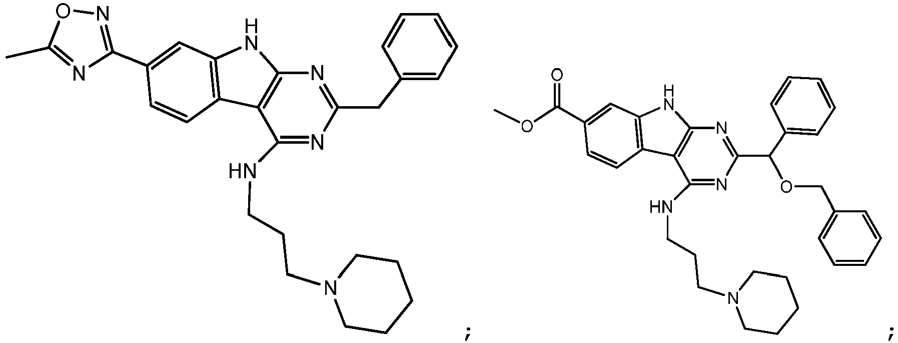 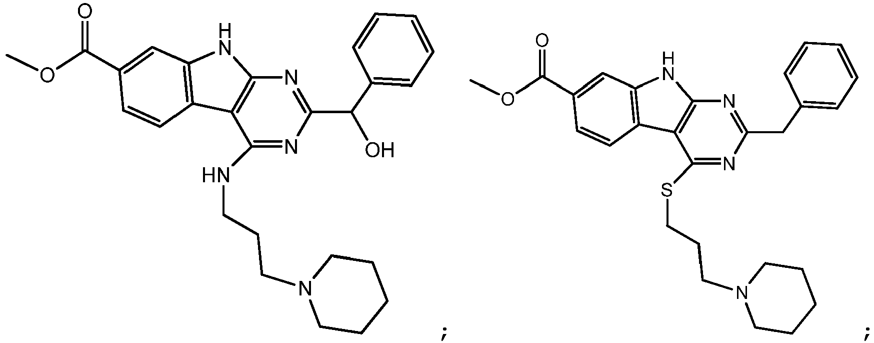 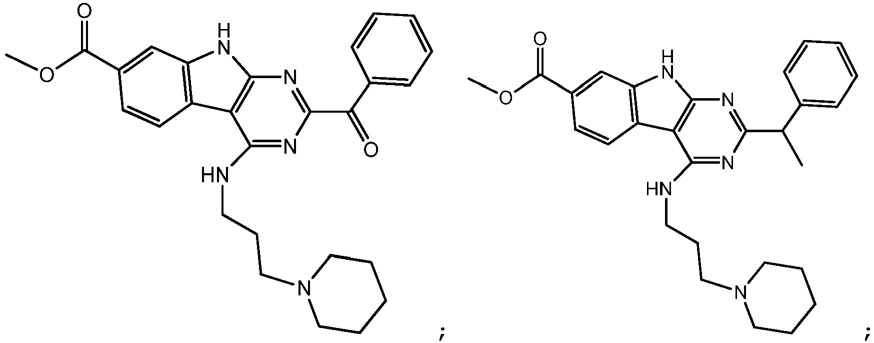 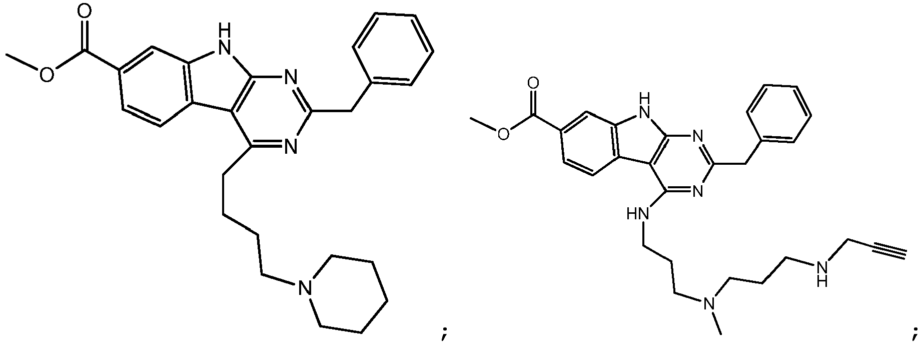 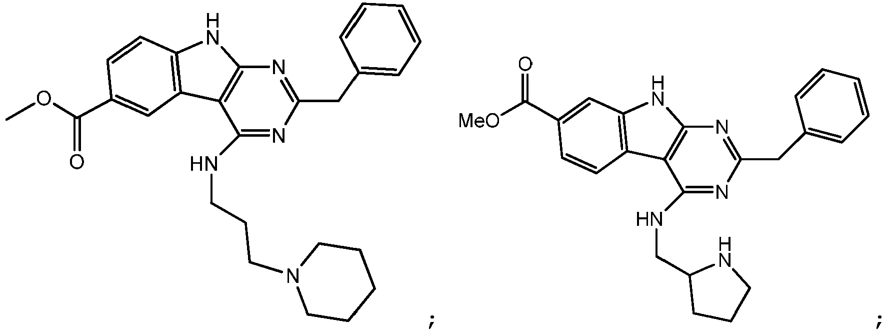 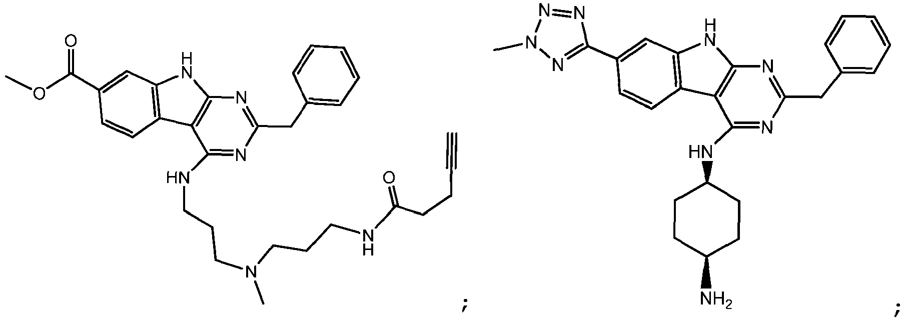 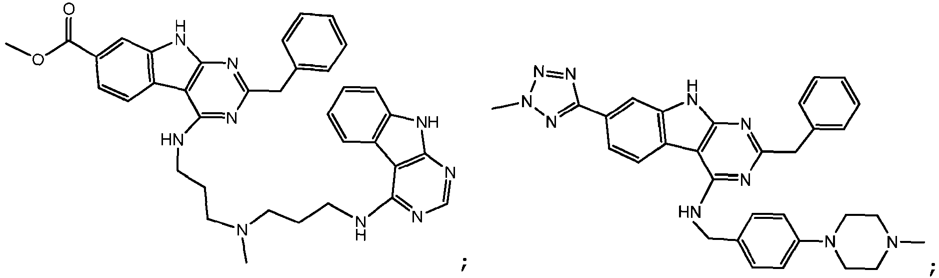 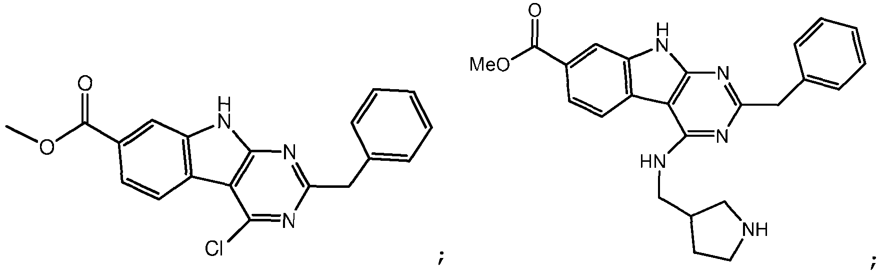 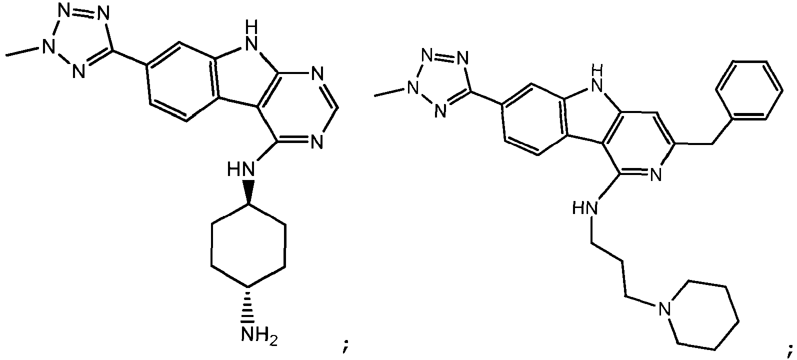 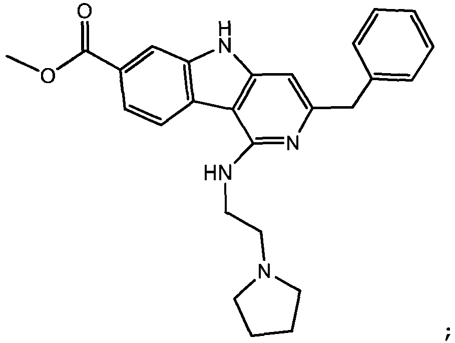 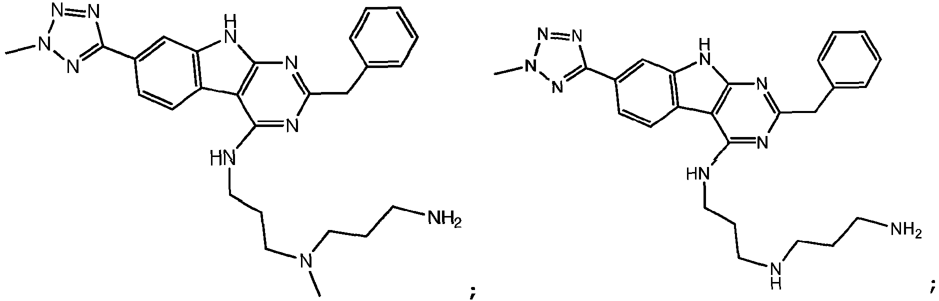 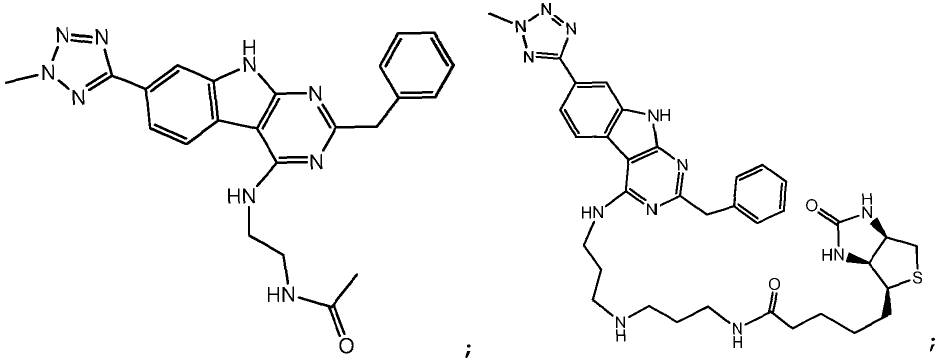 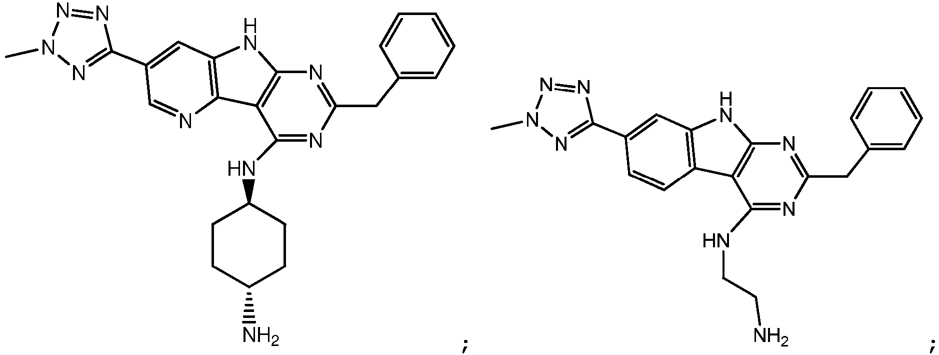 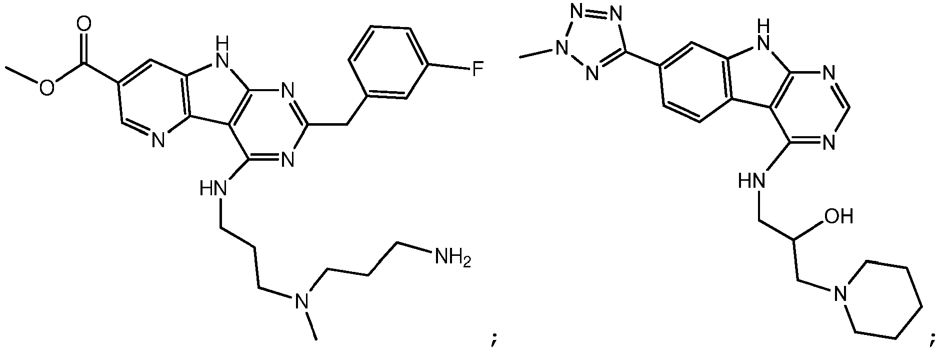 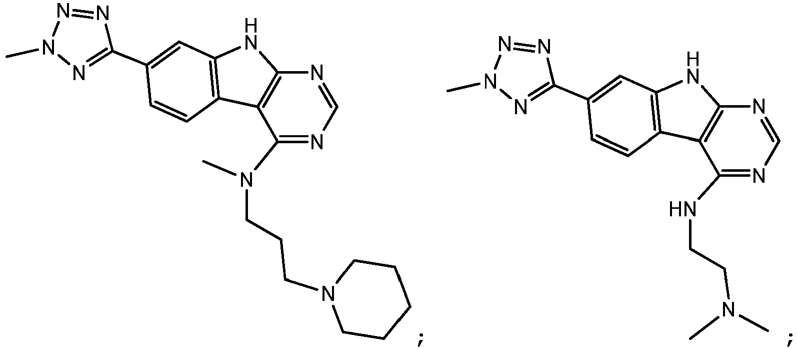 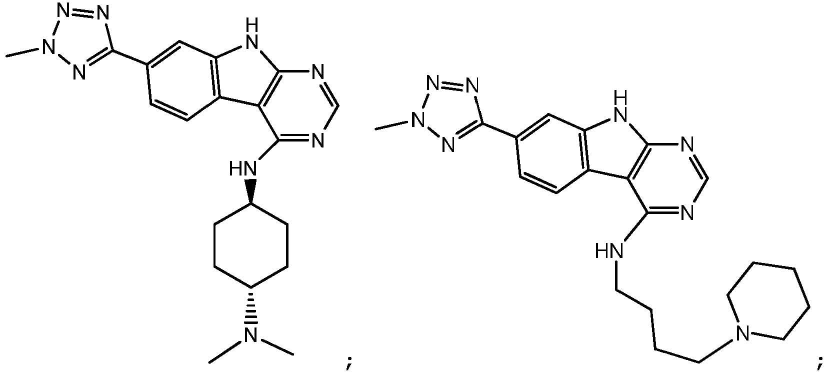 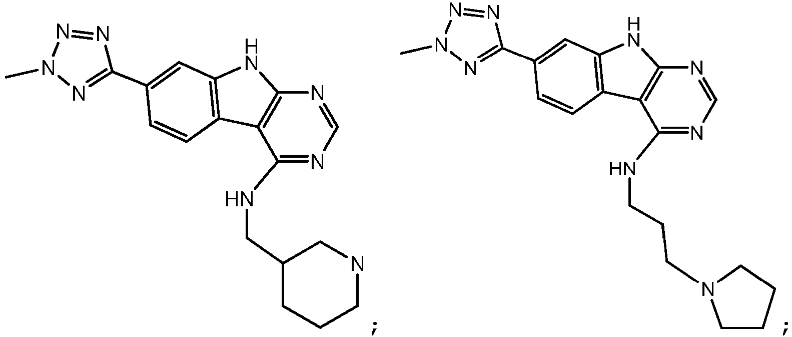 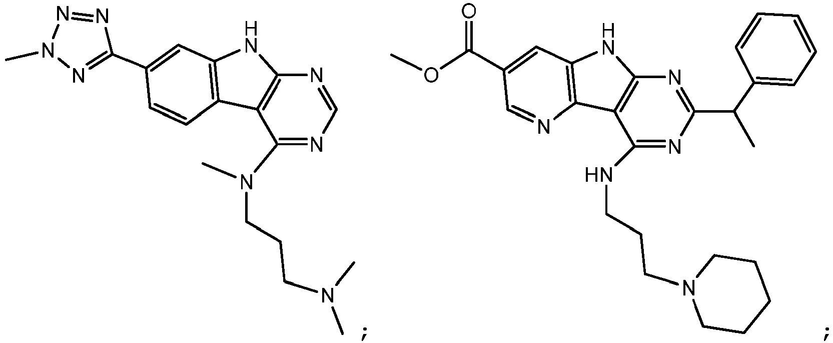 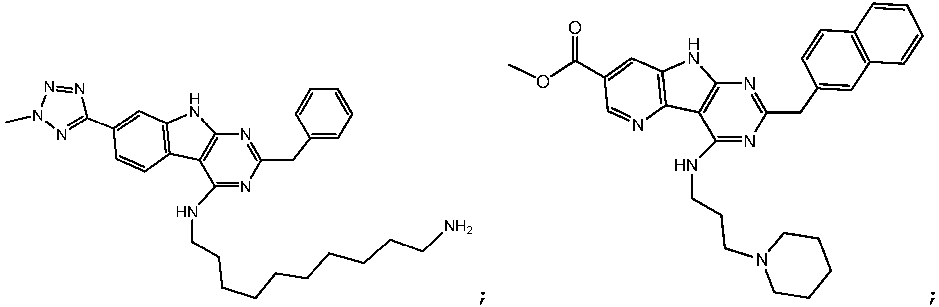 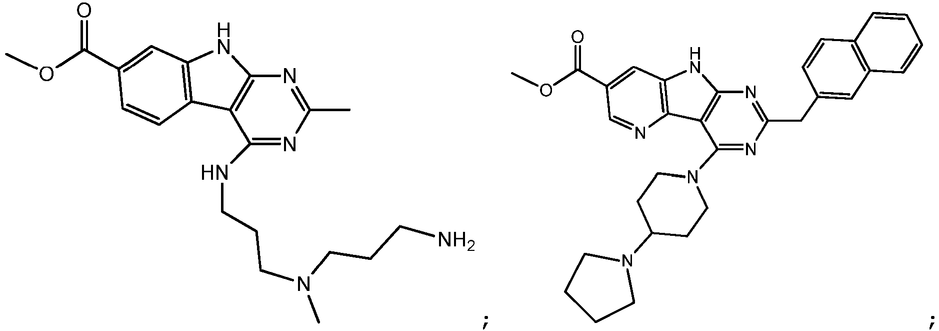 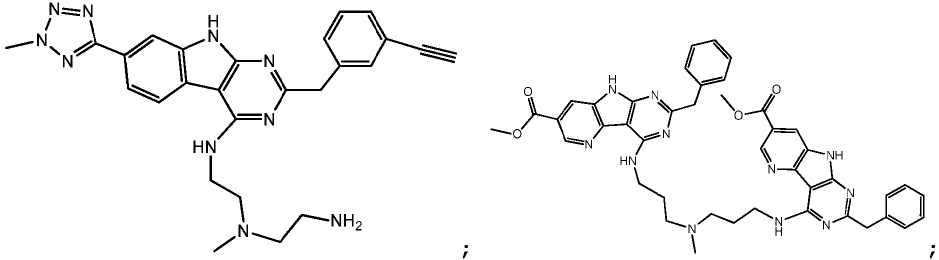 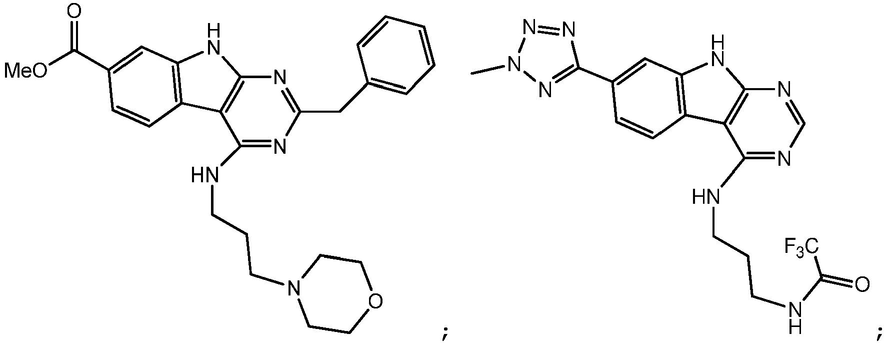 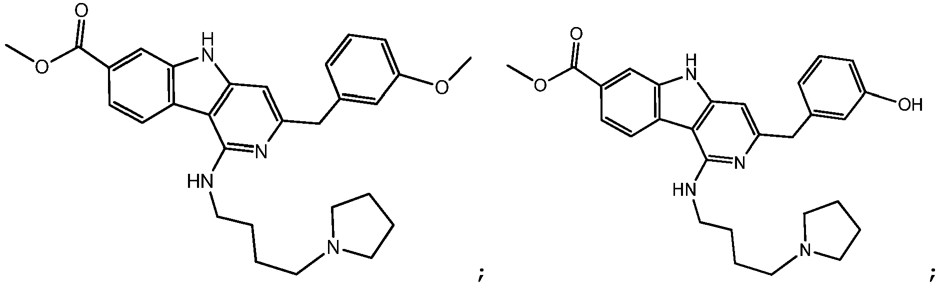 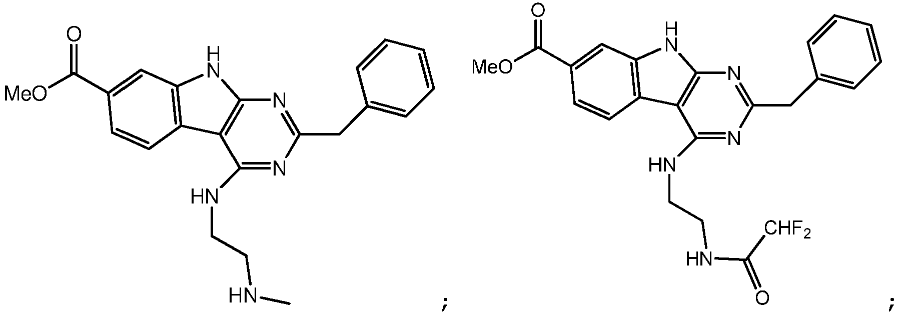 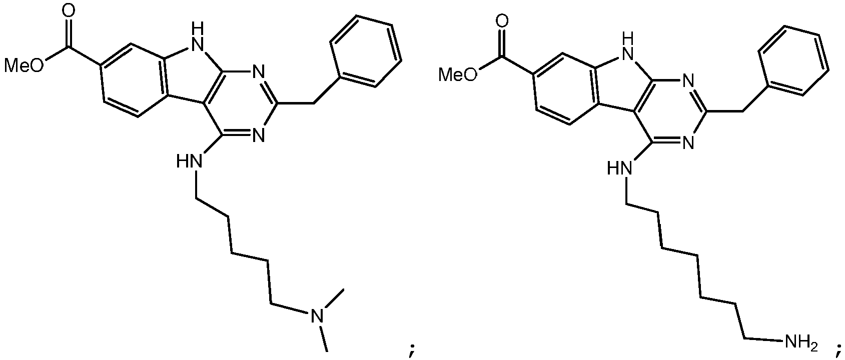 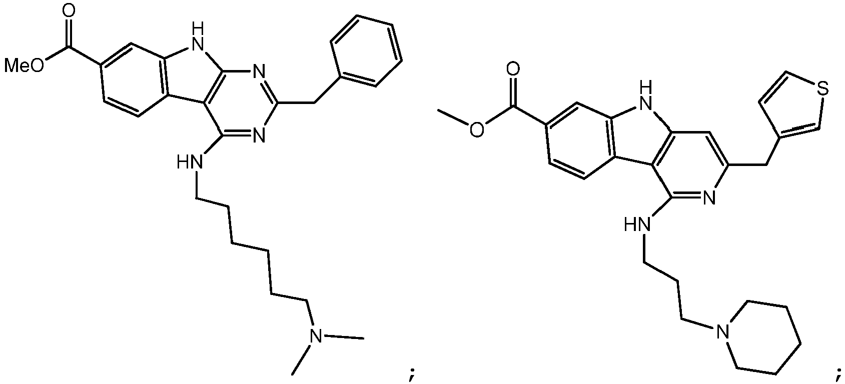 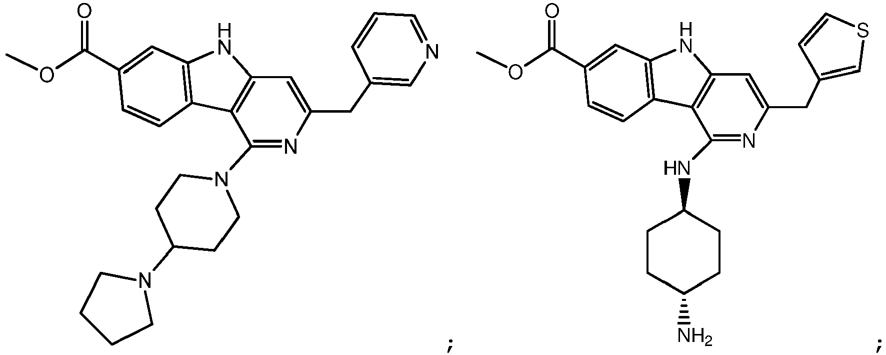 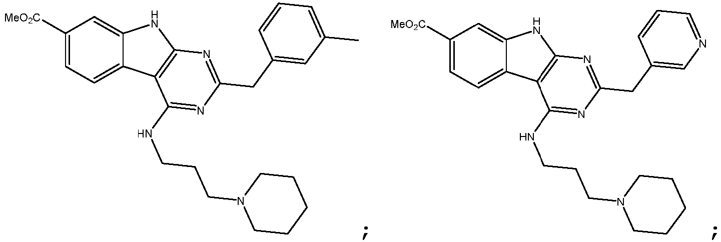 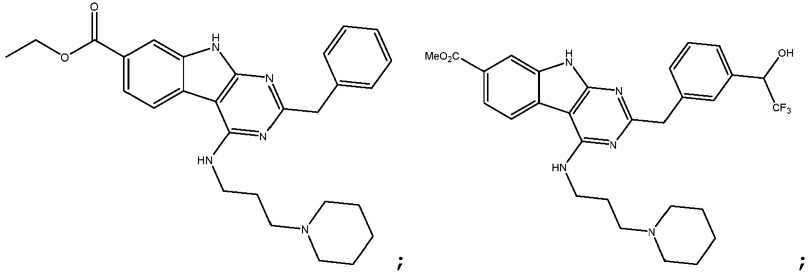 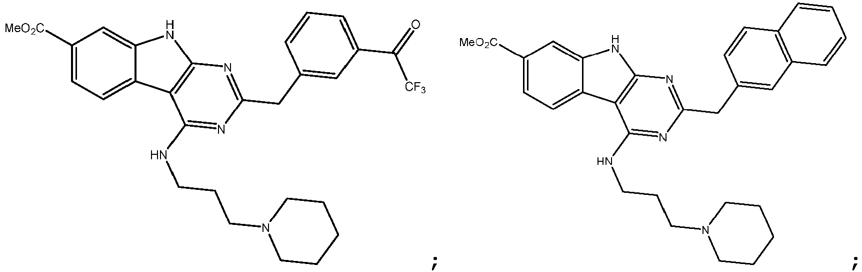 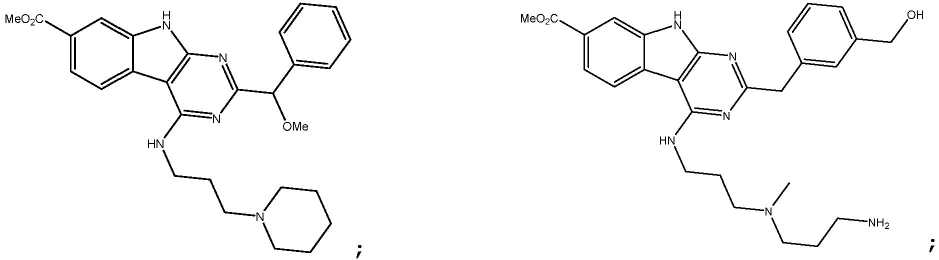 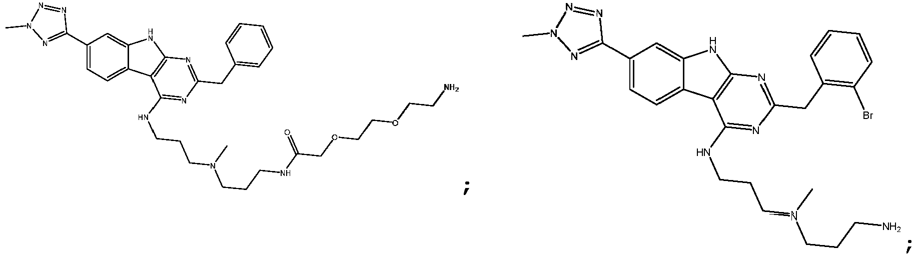 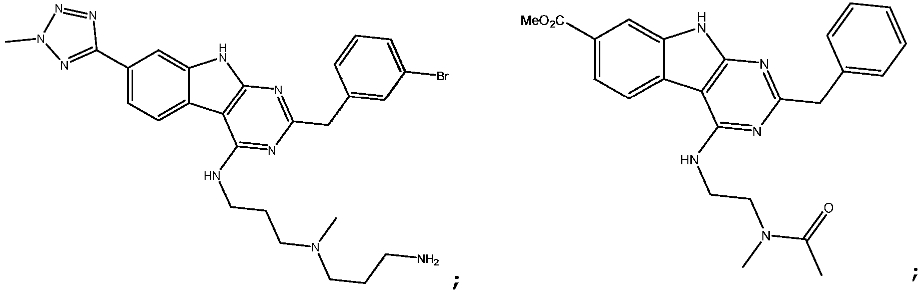 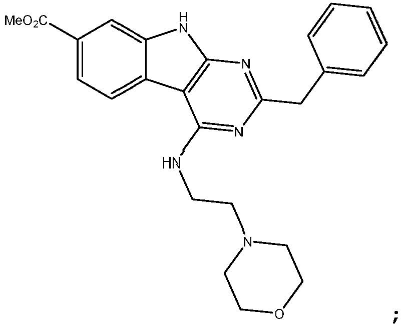 or a salt thereof, for use in the treatment of cancer in a patient in need thereof.

12. A method of inhibiting proliferation of cancerous cells *ex vivo* comprising administering the compound as defined in any one of claims 1-5, 9-11 in to the medium containing the cells in proliferation, wherein the compound has an antineoplastic effect on the proliferation of the cancerous cells.

## Patentansprüche

1. Verbindung mit der Formel I zur Verwendung bei der Behandlung von Krebs bei einem Patienten, wobei die mindestens eine Verbindung mit der Formel I: 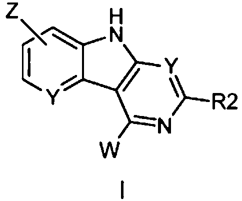
oder ein Salz davon ist,
wobei:
jedes Y unabhängig ausgewählt ist aus N und CH;
Z jeweils unabhängig ausgewählt ist aus:
-
CN,
-
C(O)OR1,
-
C(O)N(R1)R3,
-
C(O)R1,
oder
- Heteroaryl, das wahlweise mit einem oder mehreren RA- oder R4-Substituenten substituiert ist,
wobei, wenn (R1) und R3 an ein Stickstoffatom gebunden sind, sie sich wahlweise zusammen mit dem Stickstoffatom verbinden, um einen 3- bis 7-gliedrigen Ring zu bilden, der wahlweise ein oder mehrere andere Heteroatome umfasst, das bzw. die ausgewählt ist bzw. sind aus N, O und S, wobei der Ring wahlweise mit einem oder mehreren von RA oder R4 substituiert ist;
W ist:
-
CN,
-
N(R1)R3,
-
C(O)OR1,
-
C(O)N(R1)R3,
-
NR1C(O)R1,
-
NR1C(O)OR1,
-
OC(O)N(R1)R3,
-
OC(O)R1,
-
C(O)R1),
-
NR1C(O)N(R1)R3,
-
NR1S(O)₂R1,
- Benzyl, das wahlweise mit 1, 2 oder 3 RA- oder R1-Substituenten substituiert ist,
-
X-L-(X-L)n - N(R1)R3
- X-L-(X-L)n - Heteroaryl, das wahlweise mit einem oder mehreren RA- oder R4-Substituenten substituiert ist, der bzw. die an eine oder beide von den L- und Heteroaryl-Gruppen gebunden ist bzw. sind,
- X-L-(X-L)n - Heterocyclyl, das wahlweise mit einem oder mehreren RA- oder R4-Substituenten substituiert ist, der bzw. die an eine oder beide von den L- und Heterocyclyl-Gruppen gebunden ist bzw. sind,
- X-L-(X-L)n- Aryl, das wahlweise mit einem oder mehreren RA- oder R4-Substituenten substituiert ist,
-
X-L-(X-L)ₙ-NR1RA,
oder
-
(N(R1)-L)ₙ N⁺R1R3R5 R6;
wobei n eine ganze Zahl gleich entweder 0, 1, 2, 3, 4 oder 5 ist,
und wobei, wenn R1 und R3 an ein Stickstoffatom gebunden sind, sie sich wahlweise zusammen mit dem Stickstoffatom verbinden, um einen 3- bis 7-gliedrigen Ring zu bilden, der wahlweise ein oder mehrere andere Heteroatome umfasst, das bzw. die ausgewählt ist bzw. sind aus N, O und S, wobei der Ring wahlweise mit einem oder mehreren RA oder R4 substituiert ist;
jedes X unabhängig ausgewählt ist aus CH₂, O, S und NR1;
jedes L unabhängig ist:
-
C₁₋₆-Alkylen,
-
C₂₋₆-Alkenylen,
-
C₂₋₆-Alkynylen,
- C₃₋₇-Cycloalkylen, das wahlweise ein oder mehrere andere Heteroatome umfasst, das bzw. die ausgewählt ist bzw. sind aus N, O und S, oder
- C₃₋₇-Cycloalkenylen, das wahlweise ein oder mehrere andere Heteroatome umfasst, das bzw. die ausgewählt ist bzw. sind aus N, O und S,
wobei die Alkylen-, die Akenylen-, die Alkynylen-, die Cycloalkylen- und die Cycloalkenylen-Gruppen wahlweise jeweils unabhängig substituiert sind mit einem oder zwei R4- oder RA-Substituenten;
R1 unabhängig ist:
-
H,
-
C₁₋₆-Alkyl,
-
C₂₋₆-Alkenyl,
-
C₂₋₆-Alkynyl,
-
C₃₋₇-Cylcloalkyl,
-
C₃₋₇-Cycloalkenyl,
-
perfluoriertes C₁₋₅-Alkyl,
-
Heterocyclyl,
-
Aryl,
-
Heteroaryl,
oder
-
Benzyl,
wobei die Alkyl-, die Alkenyl-, die Alkynyl-, die Cycloalkenyl-, die perfluorierte Alkyl-, die Heterocyclyl-, die Aryl-, die Heteroaryl- und die Benzyl-Gruppen jeweils unabhängig mit 1, 2, oder 3 RA- oder Rd-Substituenten substituiert sind;
R2 ist:
-
H,
- C₁₋₆-Alkyl, wahlweise substituiert mit einem oder mehreren RA-Substituenten,
-
C(O)R4,
- L-Heteroaryl, wahlweise substituiert mit einem oder mehreren RA- oder R4-Substituenten,
- L-Heterocyclyl, wahlweise substituiert mit einem oder mehreren RA oder R4, oder
- L-Aryl, wahlweise substituiert mit einem oder mehreren RA- oder R4-Substituenten,
R3 unabhängig ist:
-
H,
-
C₁₋₆-Alkyl,
-
C₂₋₆-Alkenyl,
-
C₂₋₆-Alkynyl,
-
C₃₋₇-Cylcloalkyl,
-
C₃₋₇-Cycloalkenyl,
-
perfluoriertes C₁₋₅-Alkyl,
-
Heterocyclyl,
-
Aryl,
-
Heteroaryl,
oder
-
Benzyl,
wobei die Alkyl-, die Alkenyl-, die Alkynyl-, die Cycloalkyl-, die Cycloalkenyl-, die perfluorierte Alkyl-, die Heterocyclyl-, die Aryl-, die Heteroaryl- und die Benzyl-Gruppen jeweils unabhängig mit 1, 2, oder 3 RA- oder Rd-Substituenten substituiert sind;
R4 jeweils unabhängig ist:
-
H,
-
C₁₋₆-Alkyl,
-
C₁₋₆-Haloalkyl
-
C₂₋₆-Alkenyl,
-
C₂₋₆-Alkynyl,
-
C₃₋₇-Cylcloalkyl,
-
C₃₋₇-Cycloalkenyl,
-
perfluoriertes C₁₋₅-Alkyl,
-
Heterocyclyl,
-
Aryl,
-
Heteroaryl,
oder
-
Benzyl,
wobei die Alkyl-, die Alkenyl-, die Alkynyl-, die Cycloalkyl-, die Cycloalkenyl-, die perfluorierte Alkyl-, die Heterocyclyl-, die Aryl-, die Heteroaryl- und die Benzyl-Gruppen jeweils unabhängig mit 1, 2, oder 3 RA- oder Rd-Substituenten substituiert sind;
R5 jeweils unabhängig ist:
-
C₁₋₆-Alkyl,
- C₁₋₆-Alkylen-C₂₋₆-Alkenyl, das wahlweise ein oder mehrere andere Heteroatome umfasst, das bzw. die ausgewählt ist bzw. sind aus N, O und S,
- C₁₋₆-Alkylen-C₂₋₆-Alkynyl, das wahlweise ein oder mehrere andere Heteroatome umfasst, das bzw. die ausgewählt ist bzw. sind aus N, O und S,
- L-Aryl, das wahlweise einen oder mehrere RA- oder R4-Substituenten umfasst,
- L-Heteroaryl, das wahlweise einen oder mehrere RA- oder R4-Substituenten umfasst,
-
C₁₋₆-Alkylen-C(O)O-,
-
C₁₋₆-Alkylen-C(O)OR1,
-
C₁₋₆-Alkylen-CN,
- C₁₋₆-Alkylen-C(O)NR1R3, wobei R1 und R3 sich wahlweise zusammen mit dem Stickstoffatom verbinden, um einen 3- bis 7-gliedrigen Ring zu bilden, der wahlweise ein oder mehrere andere Heteroatome umfasst, das bzw. die ausgewählt ist bzw. sind aus N, O und S; oder
-
C₁₋₆-Alkylen-OH;
R6 ist:
-
Halogen
-
OC(O)CF₃
oder
-
OC(O)R1;
RA jeweils unabhängig ist:
-
Halogen,
-
CF₃,
-
OR1,
-
L-OR1,
-
OCF₃,
-
SR1,
-
CN,
-
NO₂,
-
NR1R3,
-
L-NR1R1,
-
C(O)OR1,
-
S(O)₂R4,
-
C(O)N(R1)R3,
-
NR1C(O)R1,
-
NR1C(O)OR1,
-
OC(O)N(R1)R3,
-
OC(O)R1,
-
C(O)R4,
-
NHC(O)N(R1)R3,
-
NR1C(0)N(R1)R3,
oder
-
N₃;
und
Rd jeweils unabhängig ist:
-
H,
-
C₁₋₆-Alkyl,
-
C₂₋₆-Alkenyl,
-
C₂₋₆-Alkynyl,
-
C₃₋₇-Cylcloalkyl,
-
C₃₋₇-Cycloalkenyl,
-
perfluoriertes C₁₋₅-Alkyl,
-
Benzyl
oder
-
Heterocyclyl.

2. Verbindung zur Verwendung nach Anspruch 1, wobei die Verbindung mit der Formel I 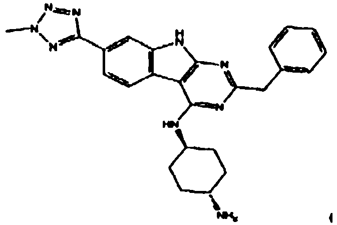 oder ein pharmazeutisch annehmbares Salz davon ist.

3. Verbindung zur Verwendung nach Anspruch 1, wobei die Verbindung mit der Formel I ein Hydrobromidsalz ist von

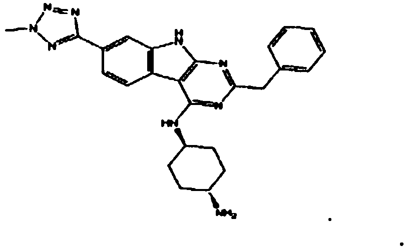

4. Verbindung zur Verwendung nach Anspruch 1, wobei die Verbindung mit der Formel I 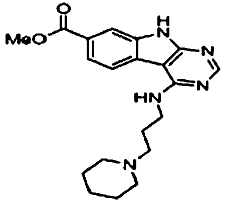 oder ein pharmazeutisch annehmbares Salz davon ist.

5. Verbindung zur Verwendung nach Anspruch 1, wobei die Verbindung mit der Formel I 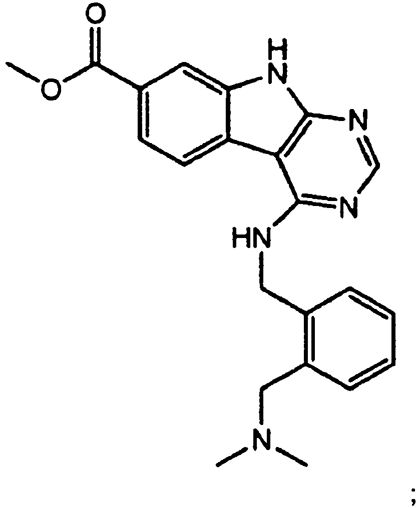 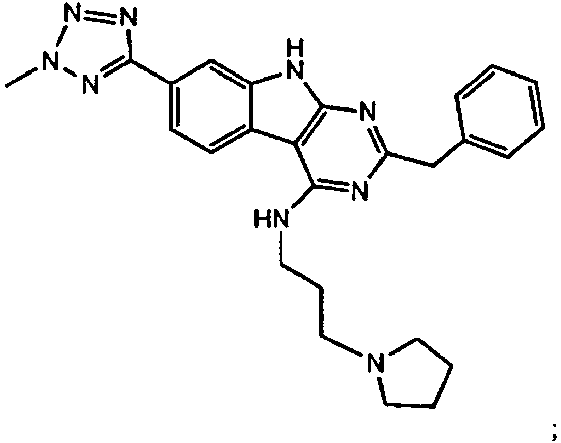 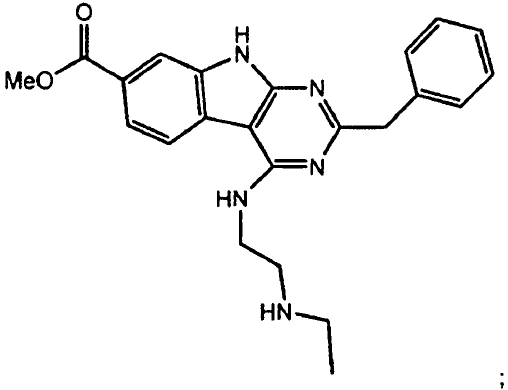 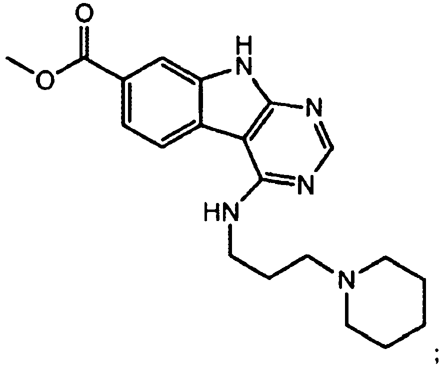 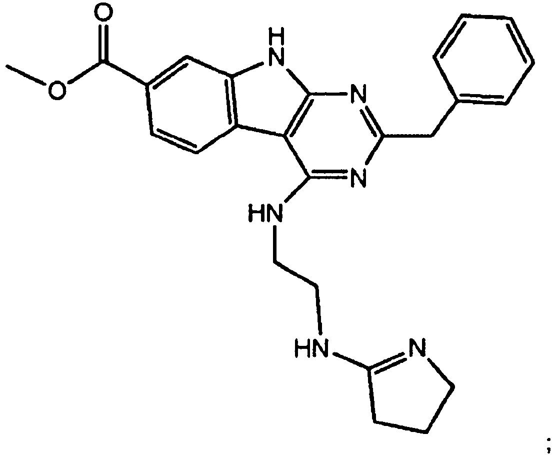 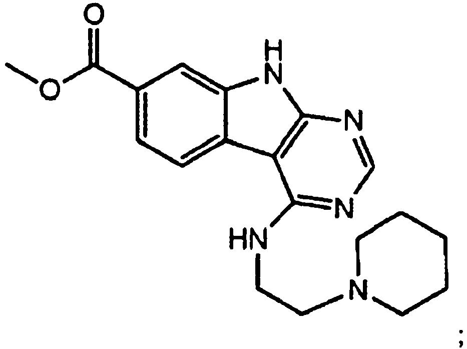 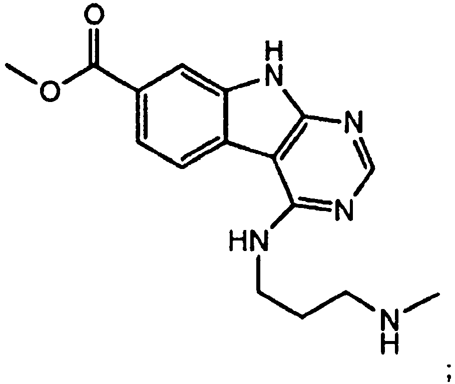 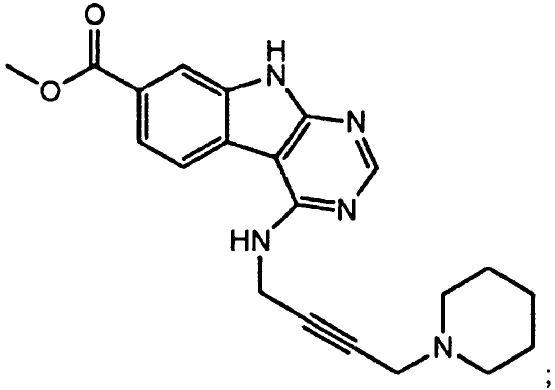 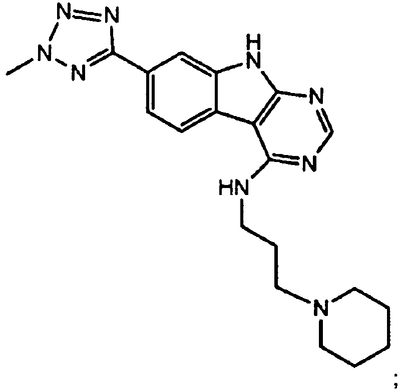 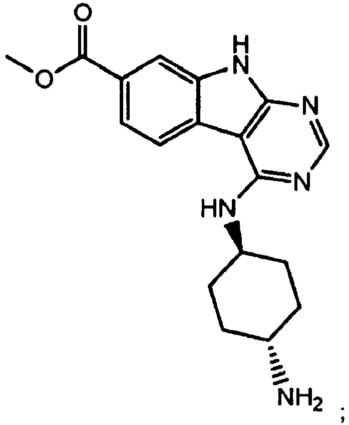 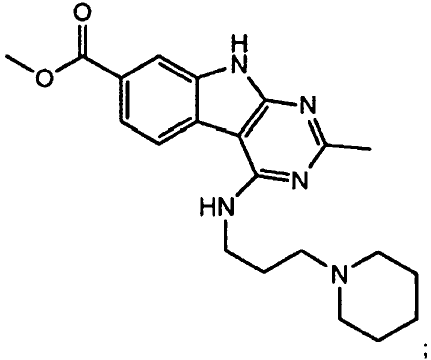 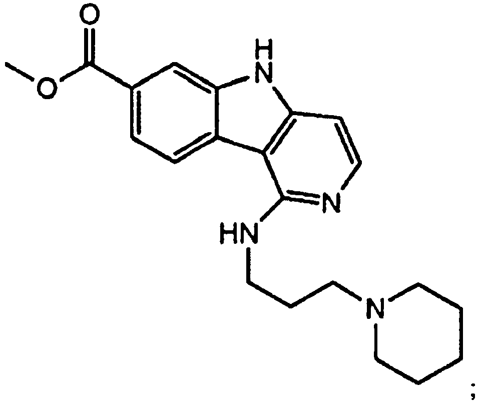 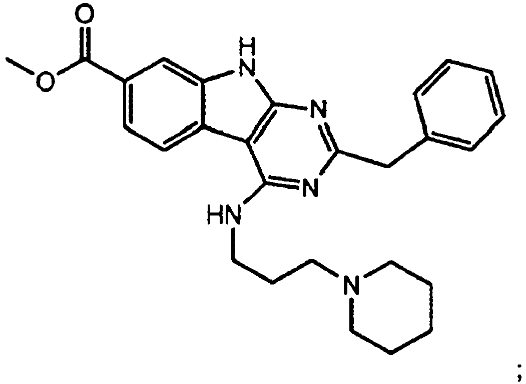 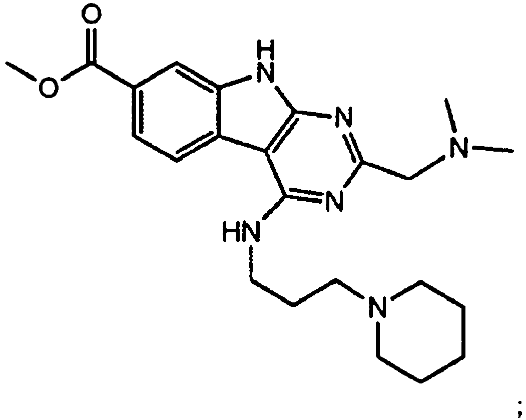 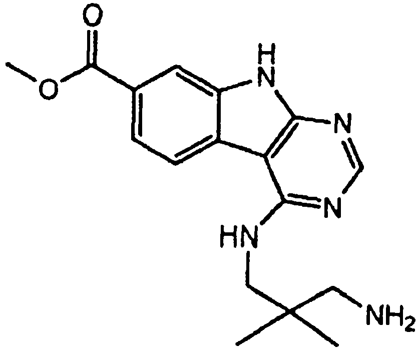 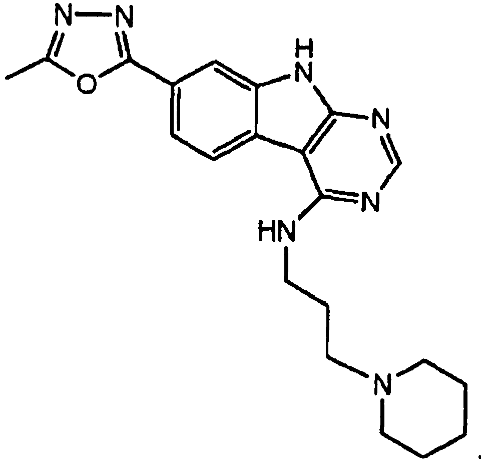 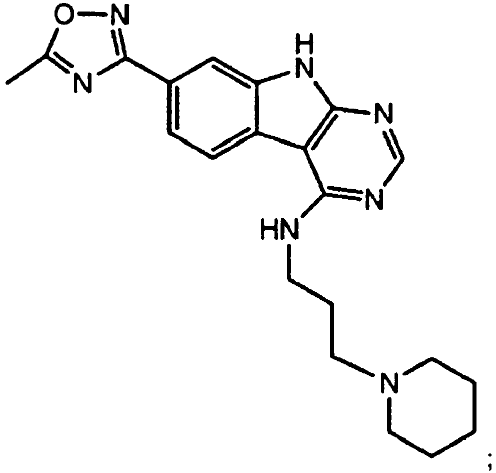 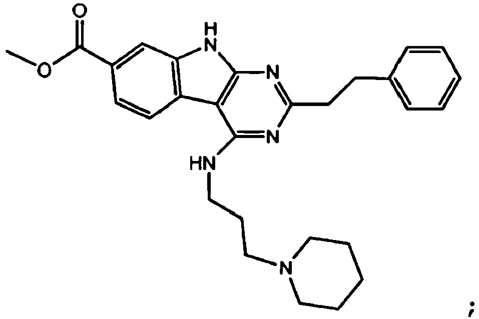 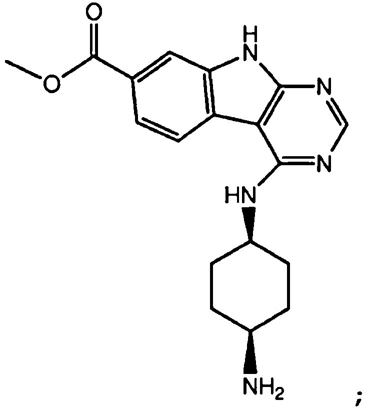 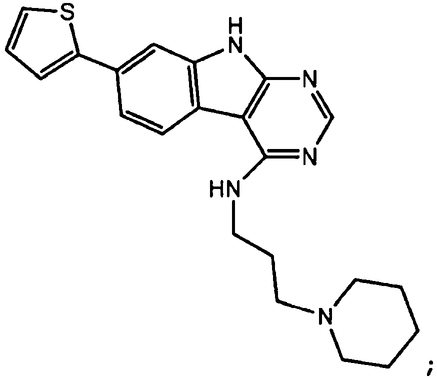 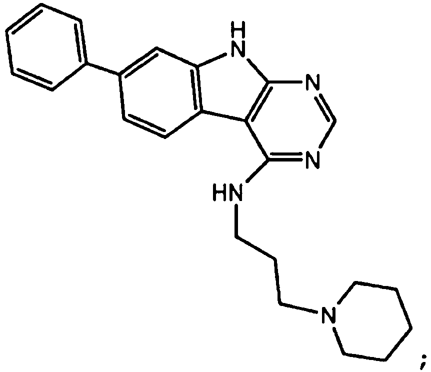 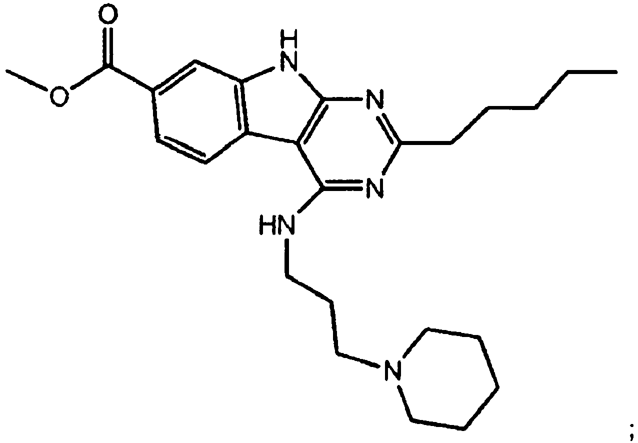 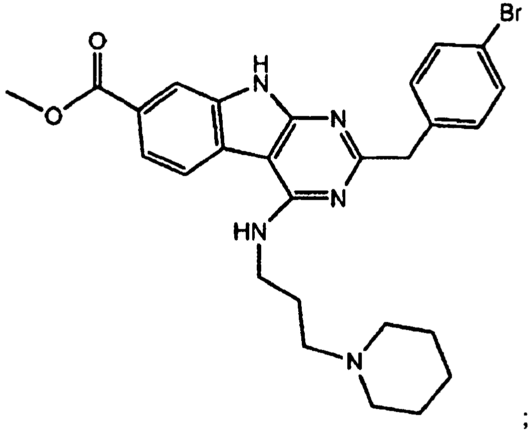 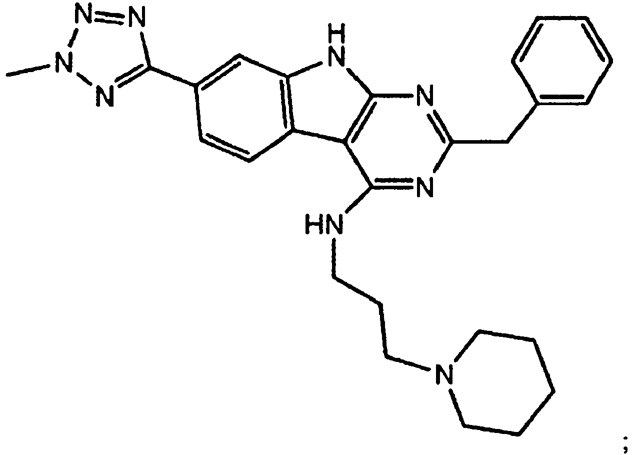 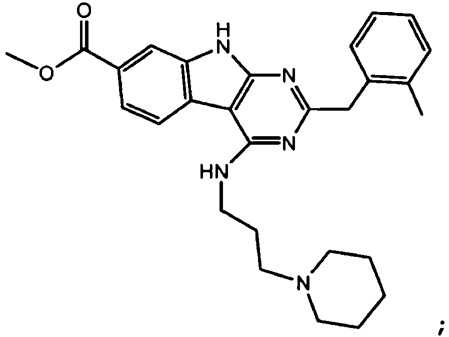 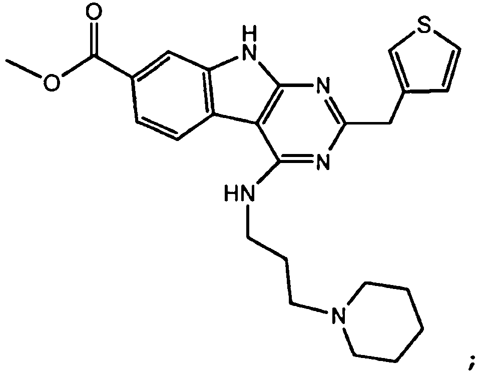 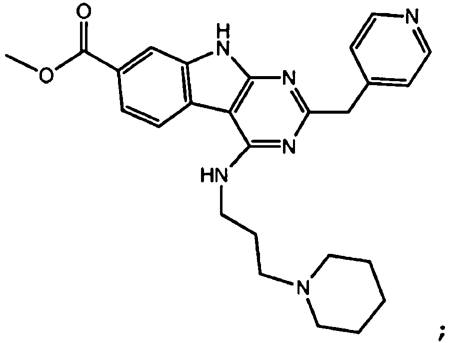 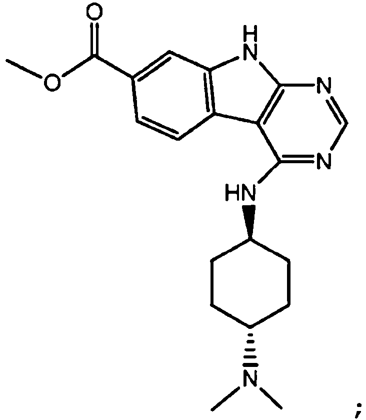 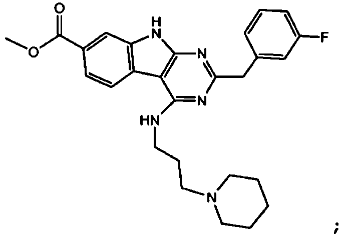 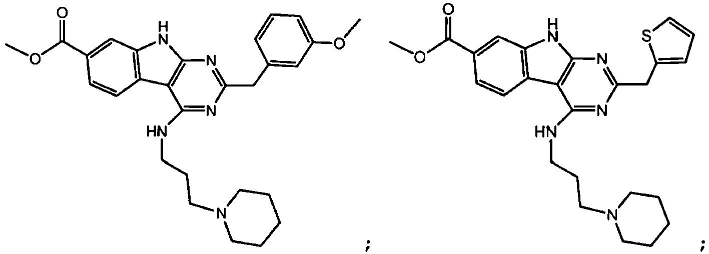 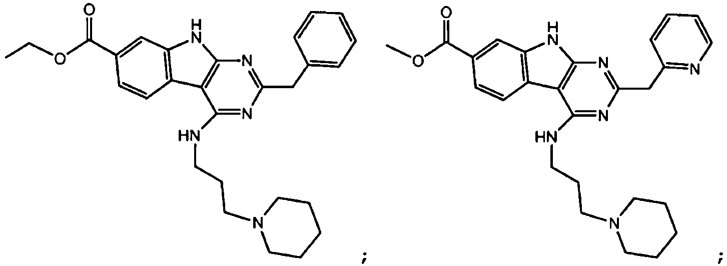 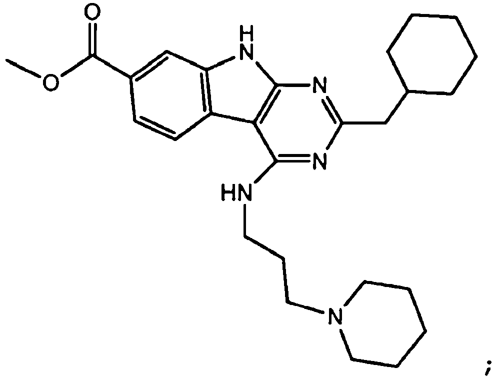 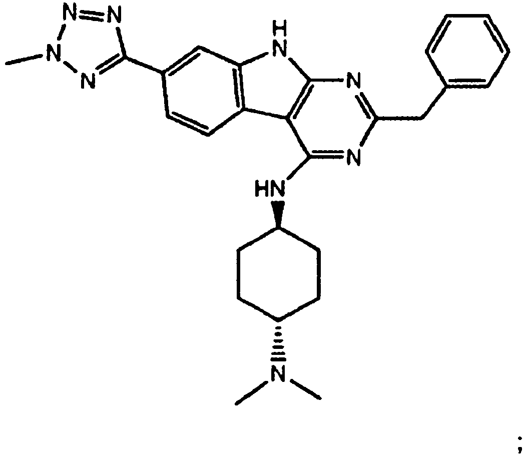 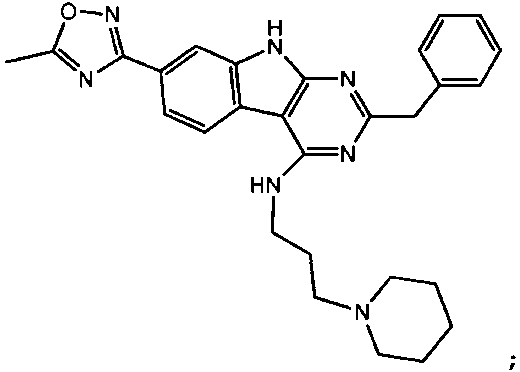 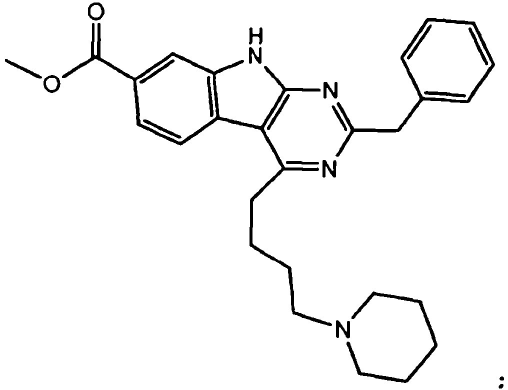 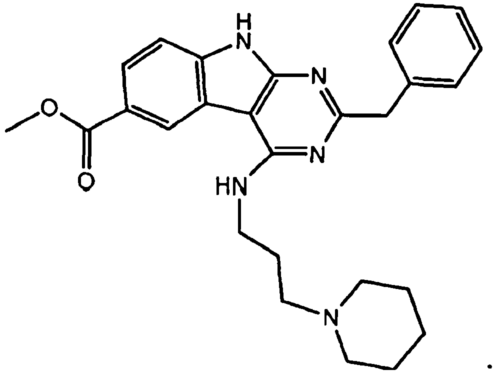 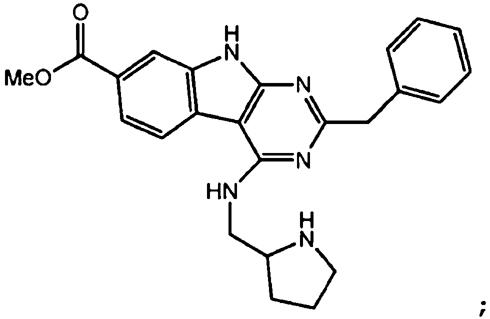 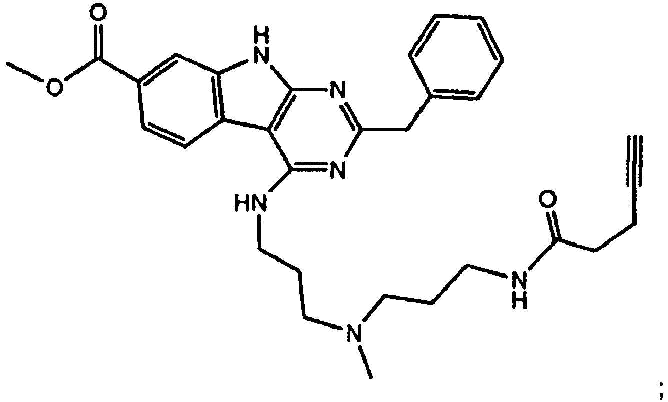 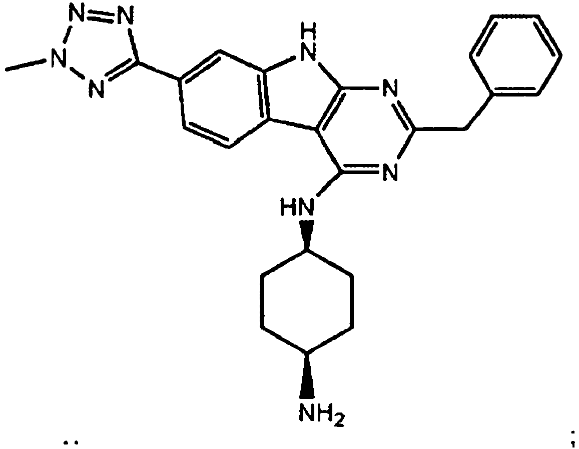 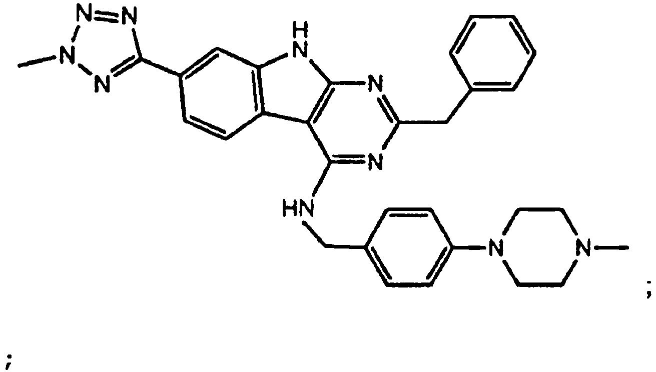 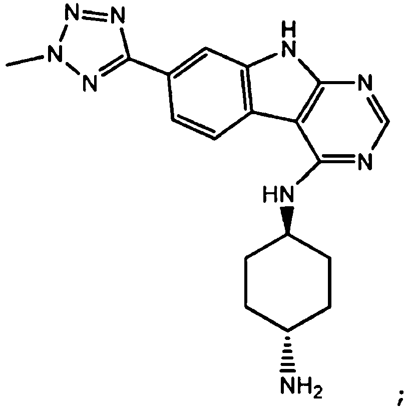 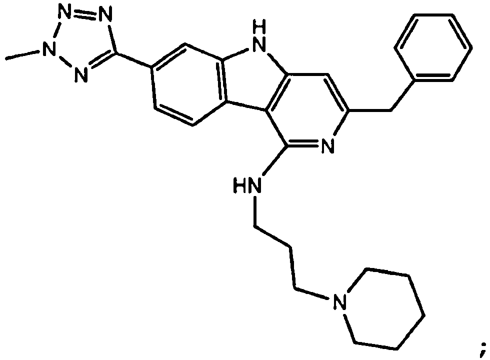 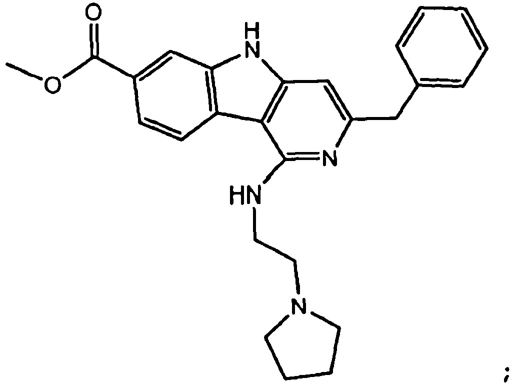 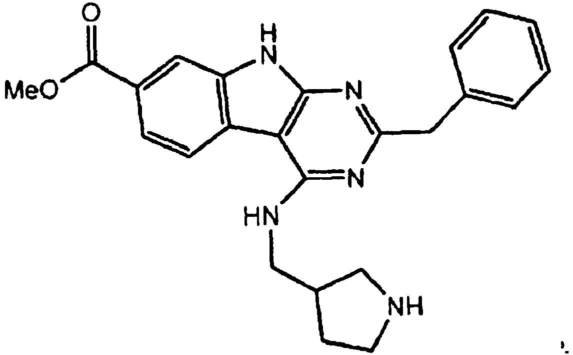 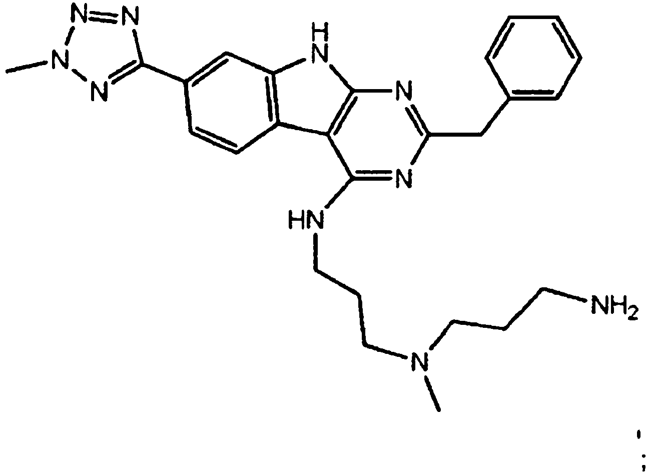 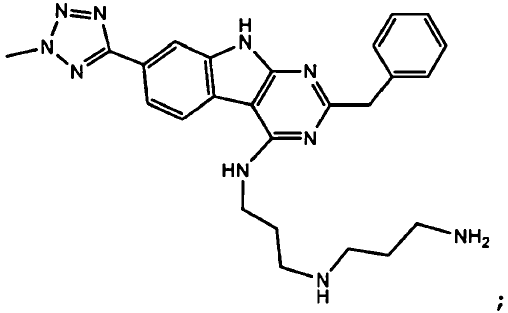 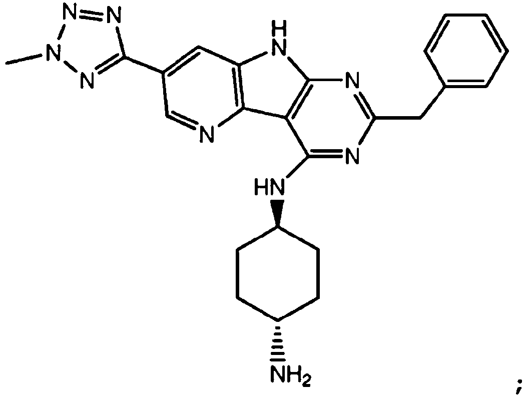 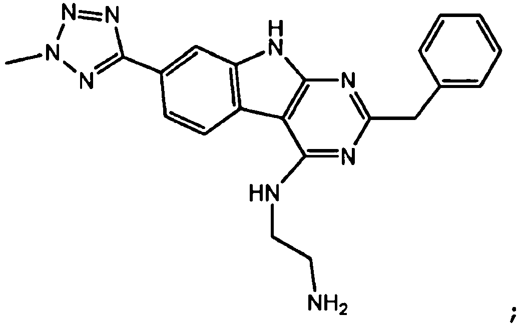 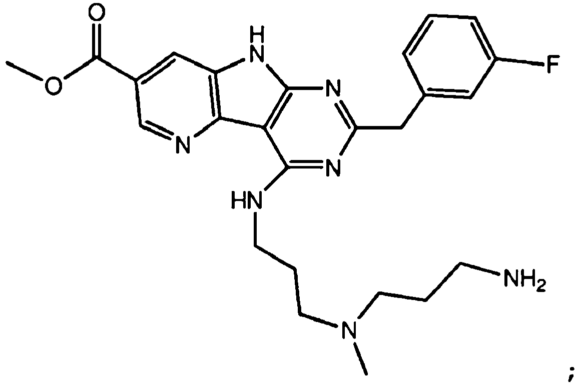 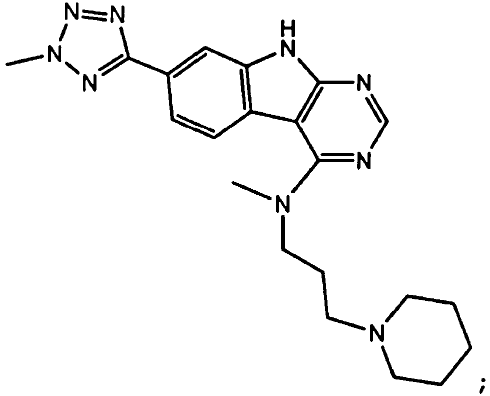 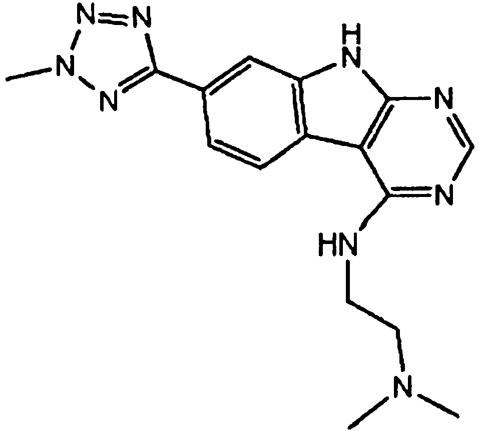 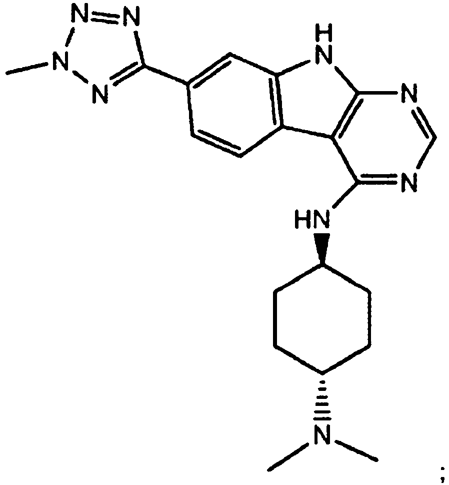 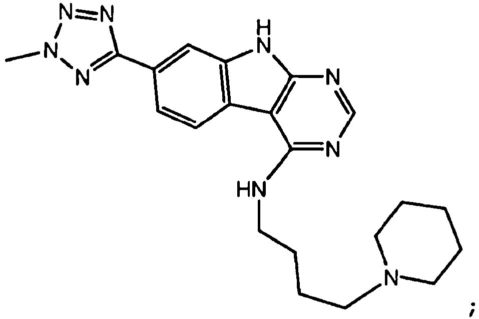 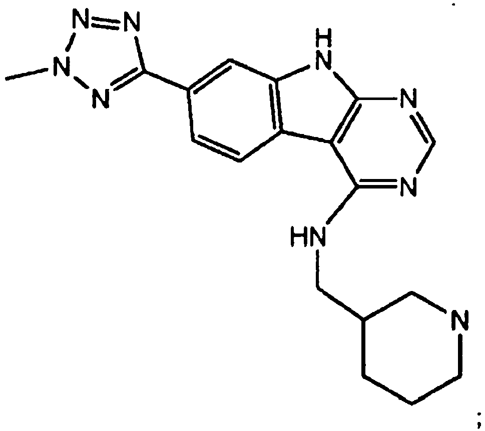 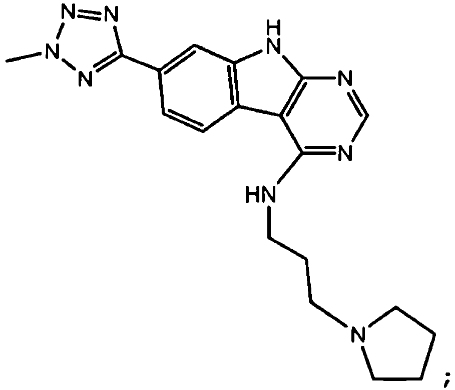 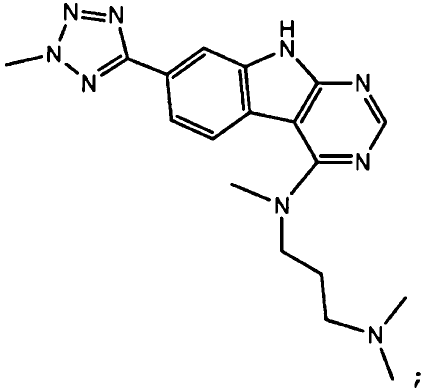 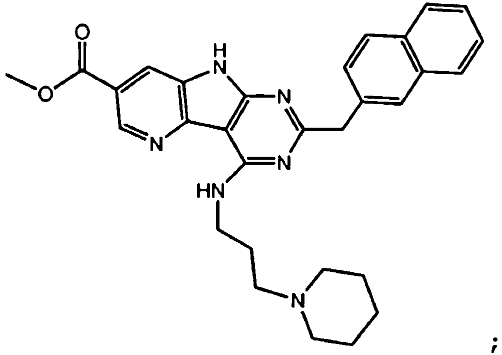 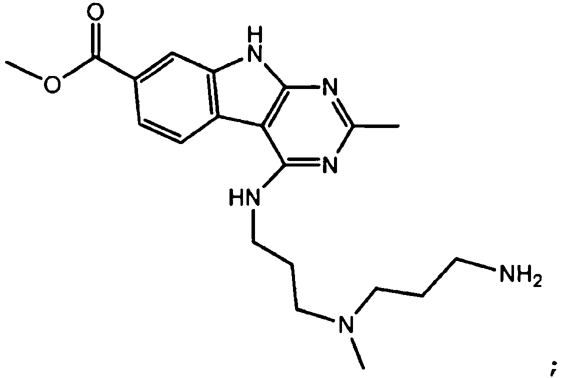 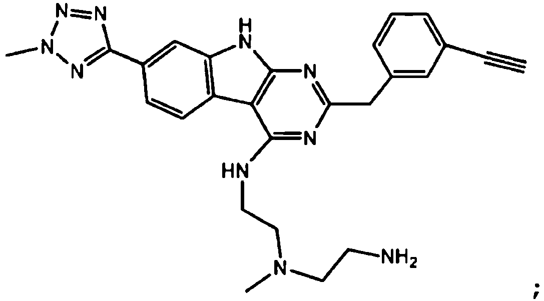 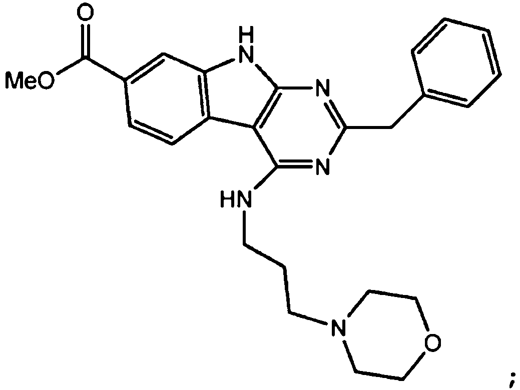 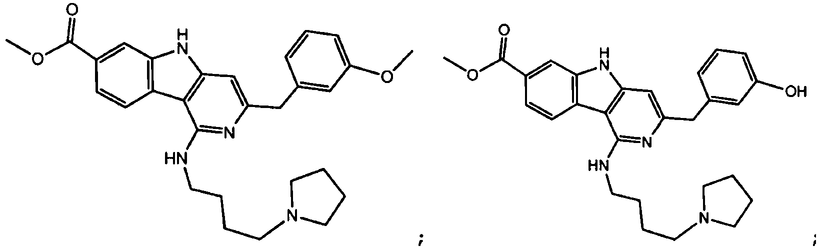 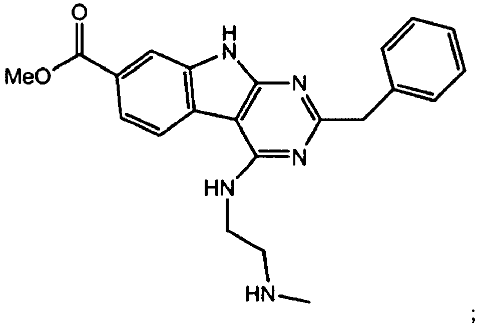 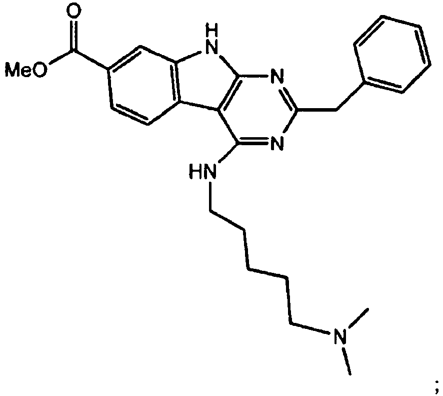 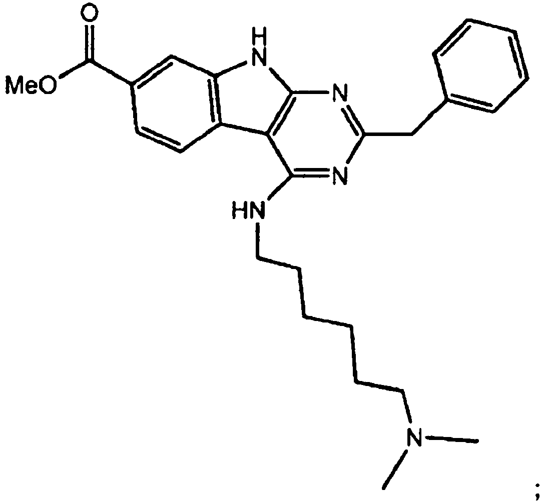 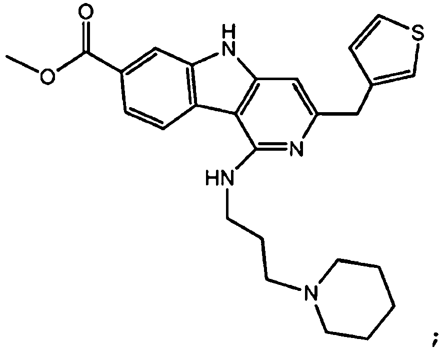 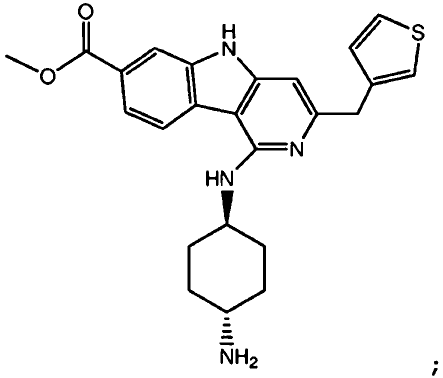 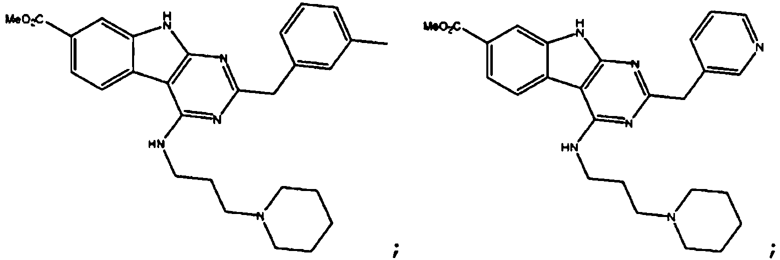 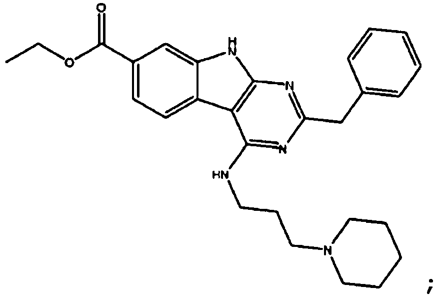 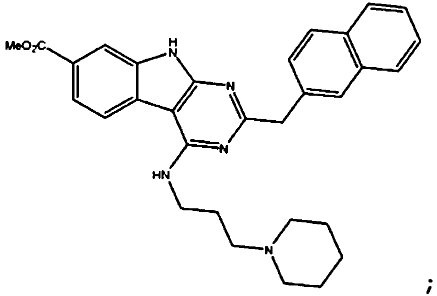 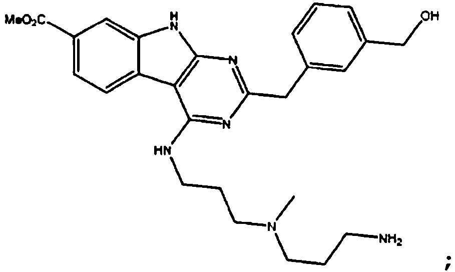 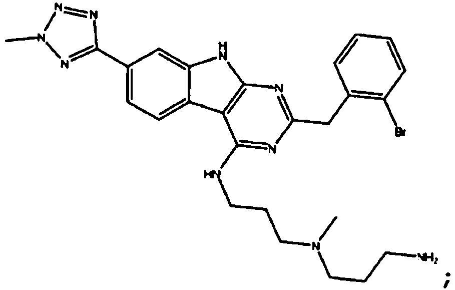 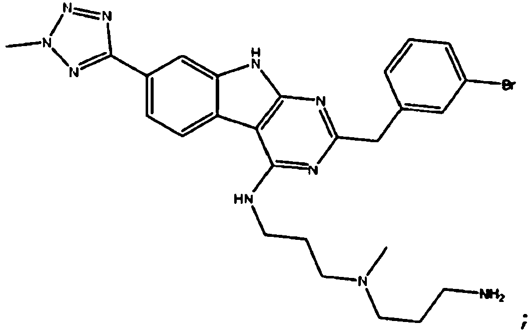 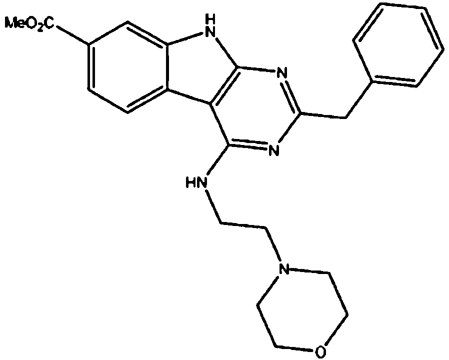 oder ein Salz davon ist.

6. Verbindung zur Verwendung nach einem der Ansprüche 1 bis 5, wobei der Patient ein Mensch oder ein Tier ist.

7. Verbindung zur Verwendung nach Anspruch 6, wobei das Tier eine Maus ist.

8. Verbindung zur Verwendung nach einem der Ansprüche 1 bis 7, wobei die Verbindung für eine orale, intramuskuläre, intravenöse oder subkutane Verabreichung formuliert ist.

9. Verbindung zur Verwendung nach einem der Ansprüche 1 bis 8, wobei die Verbindung mindestens eines von LSD1, RCOR1, HDAC2 und CoREST abbaut.

10. Verbindung zur Verwendung nach einem der Ansprüche 1 bis 9, wobei der Krebs auf einer K27-Mutation, EZH2-Mutation oder PRC2-Mutation gründet.

11. Verbindung, die ausgewählt ist aus 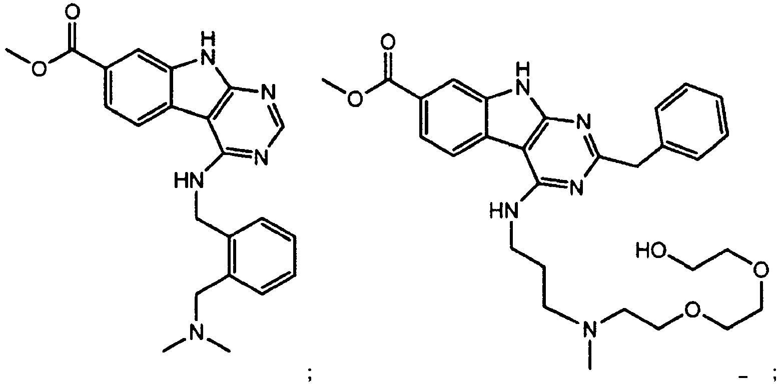 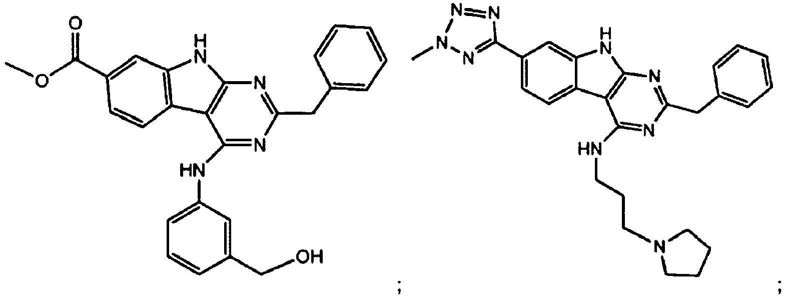 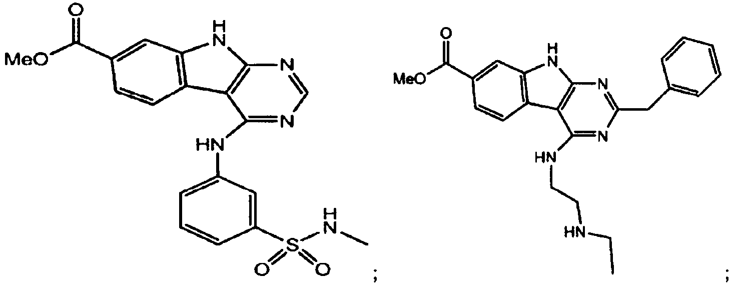 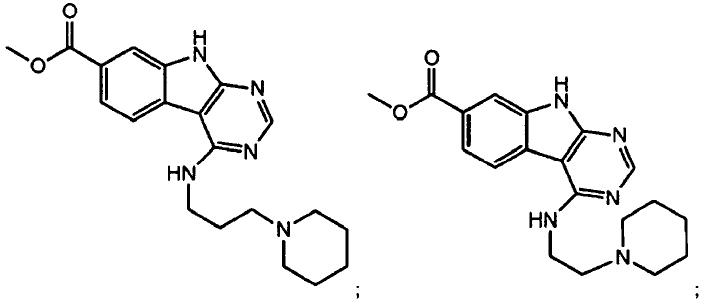 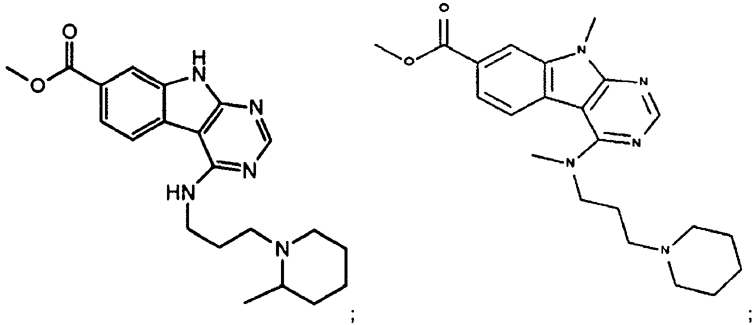 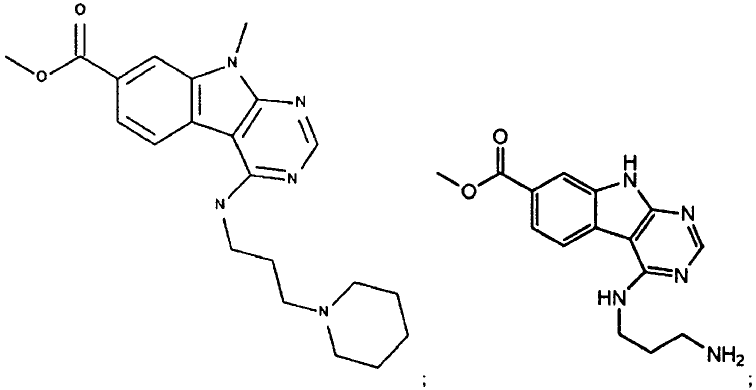 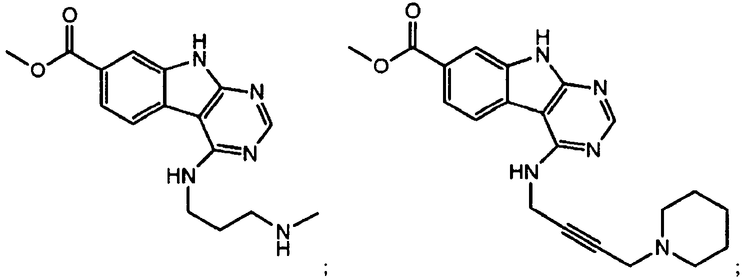 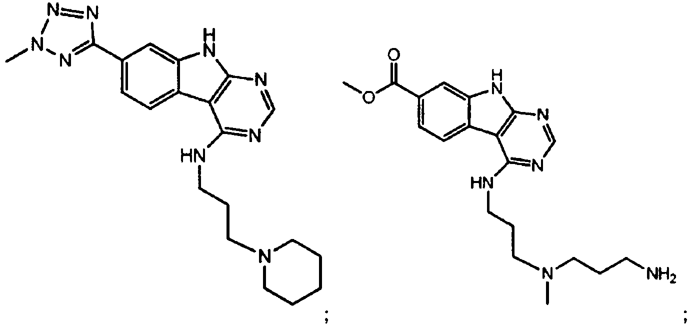 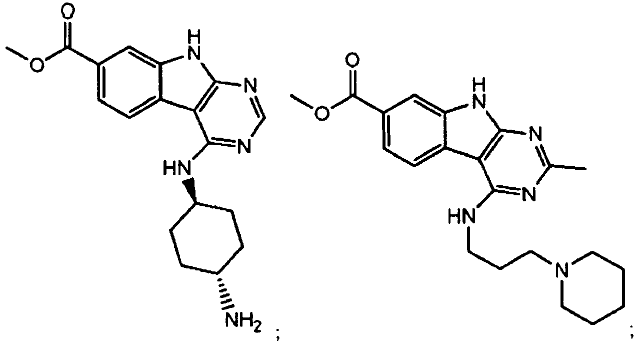 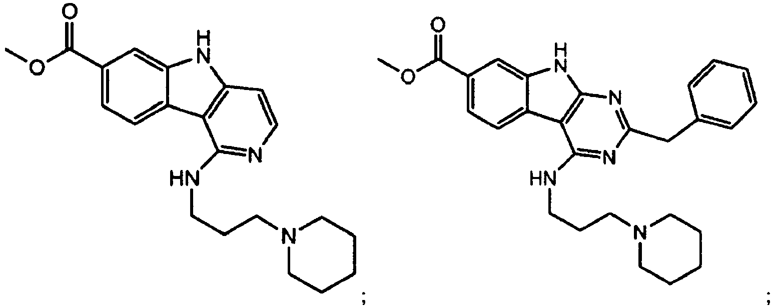 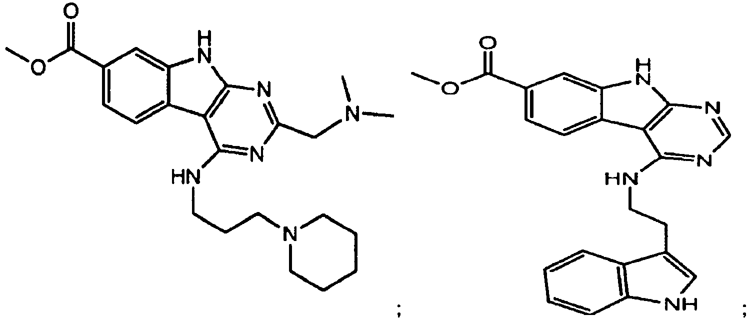 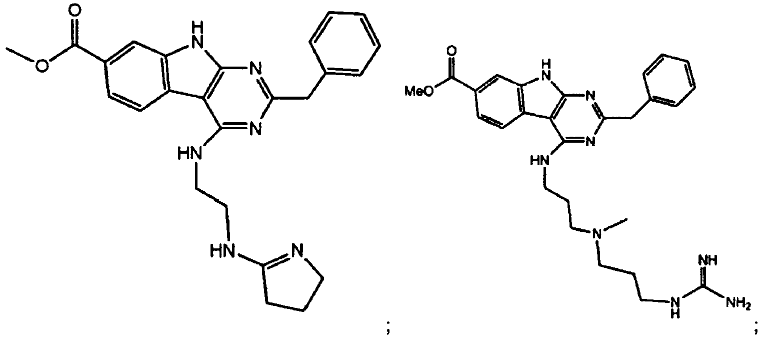 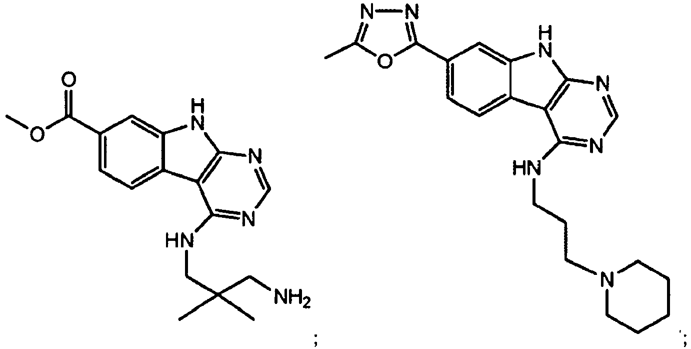 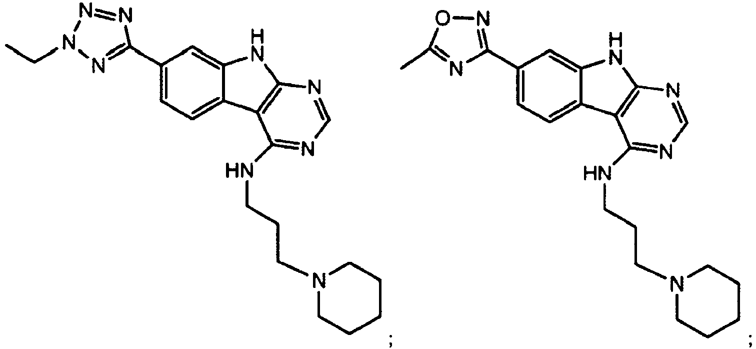 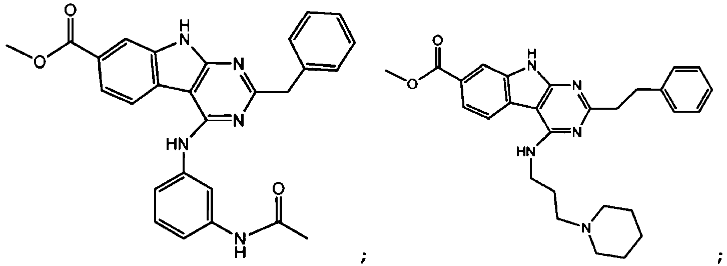 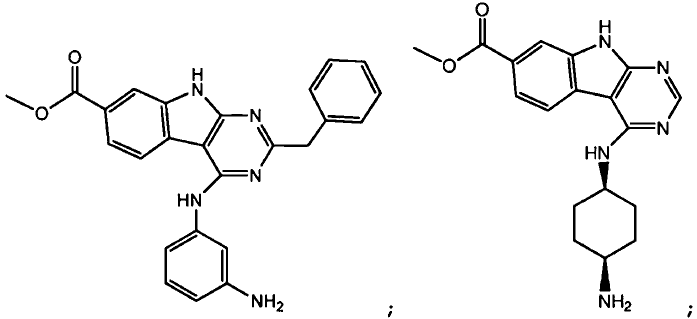 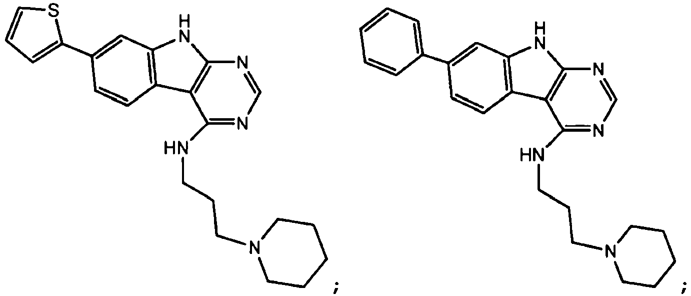 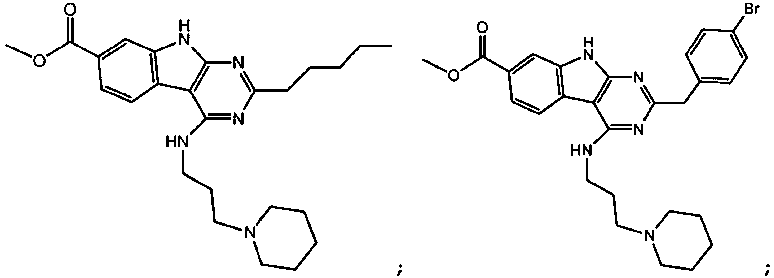 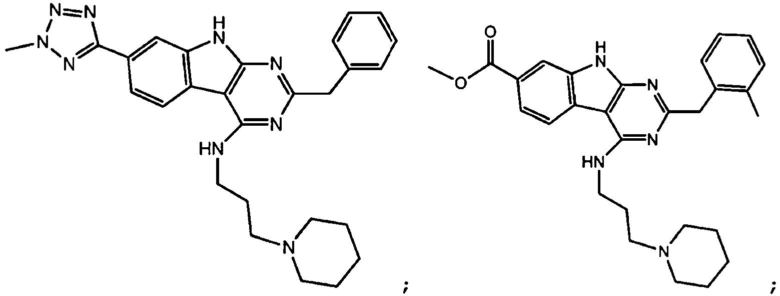 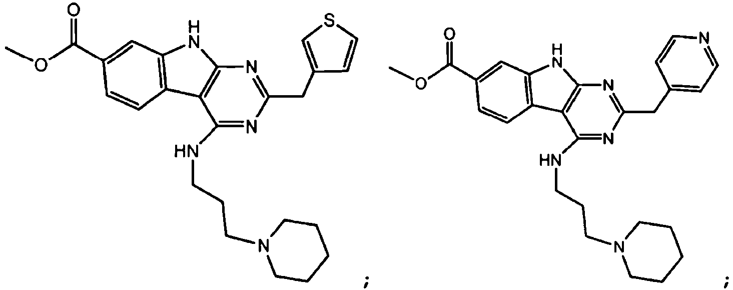 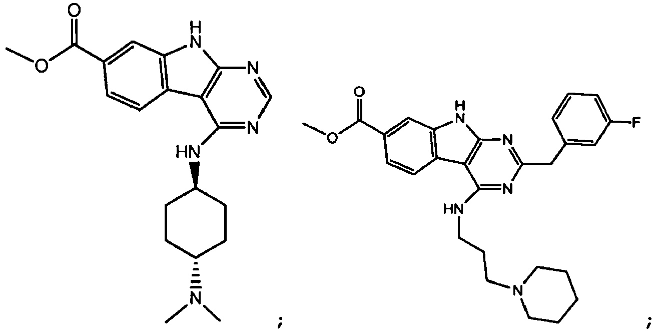 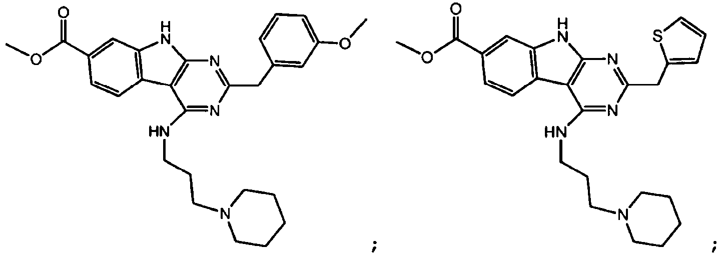 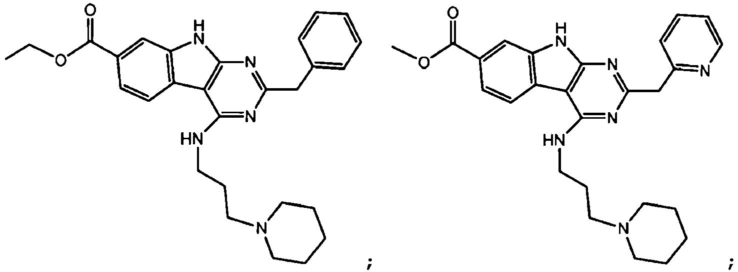 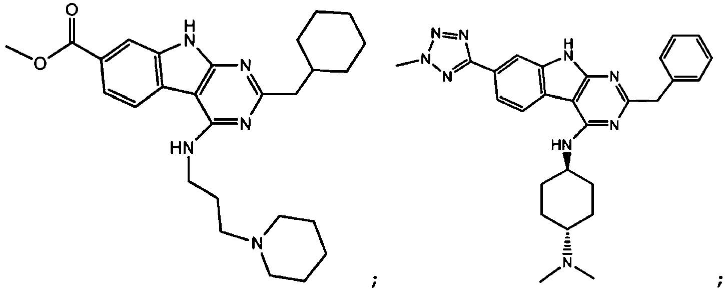 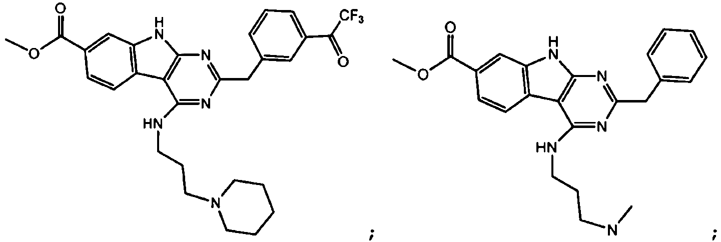 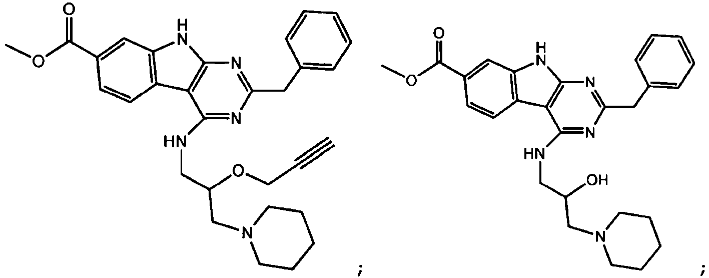 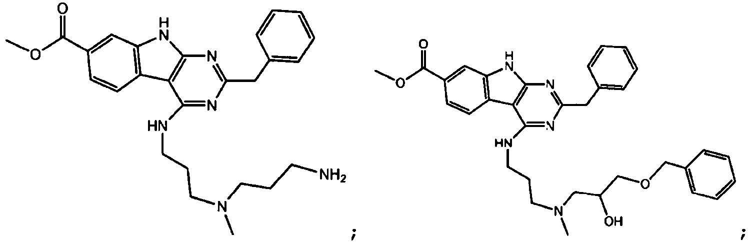 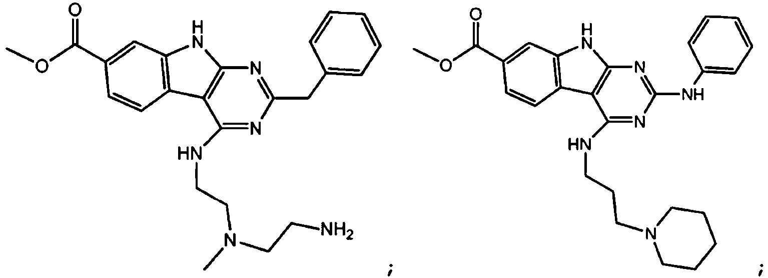 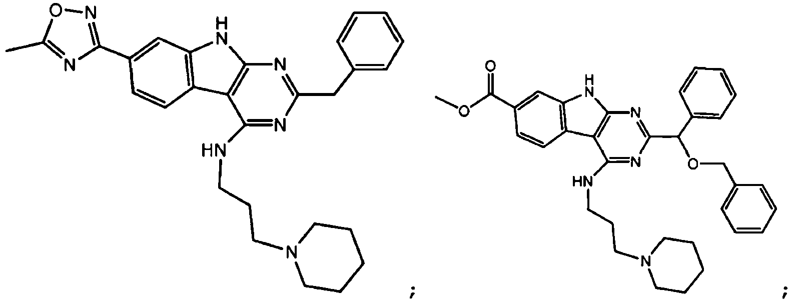 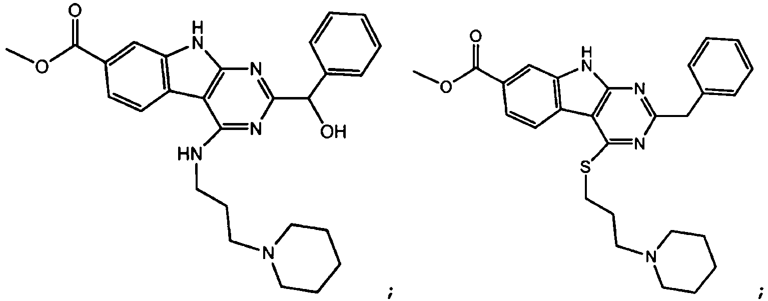 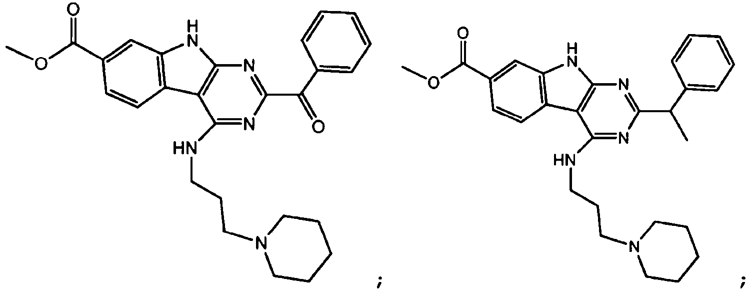 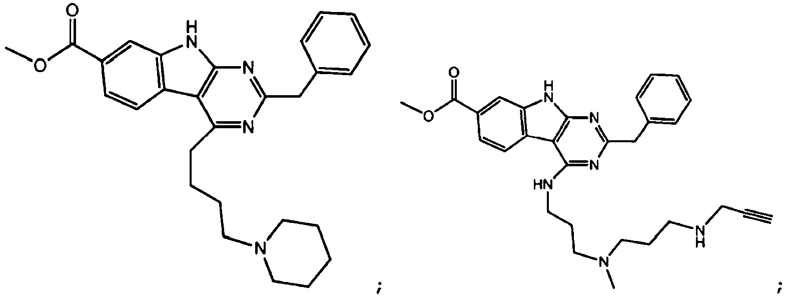 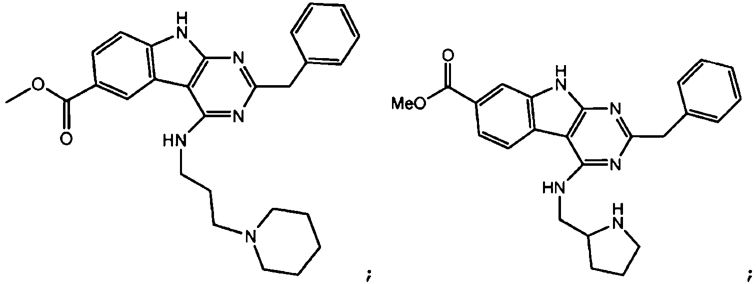 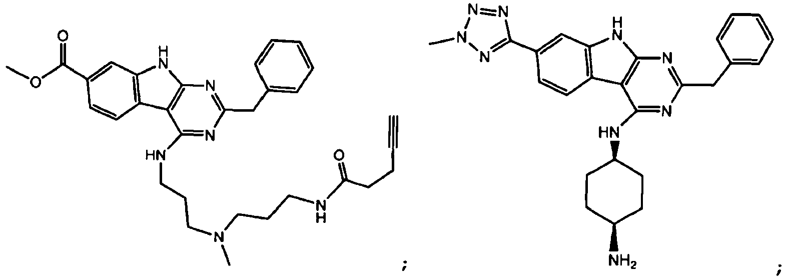 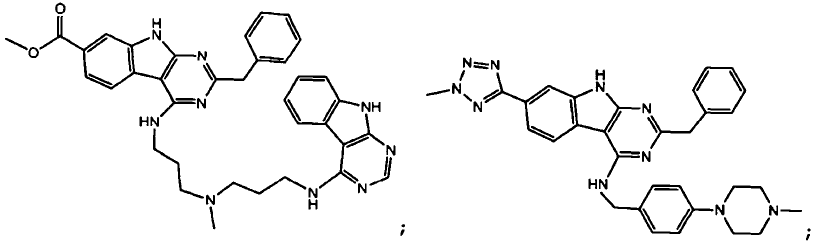 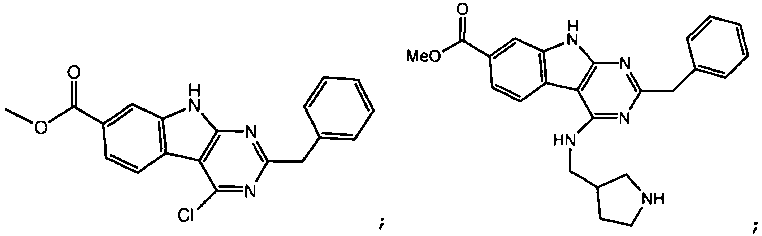 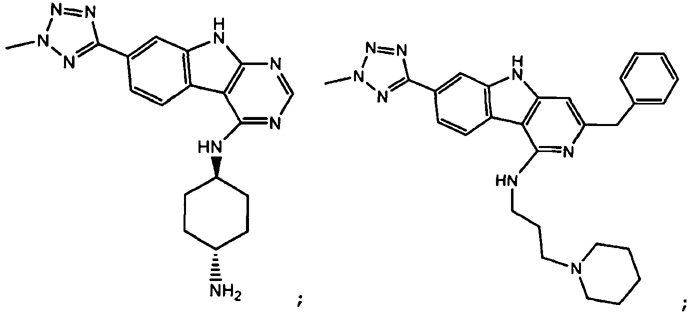 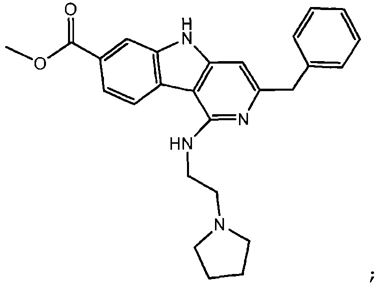 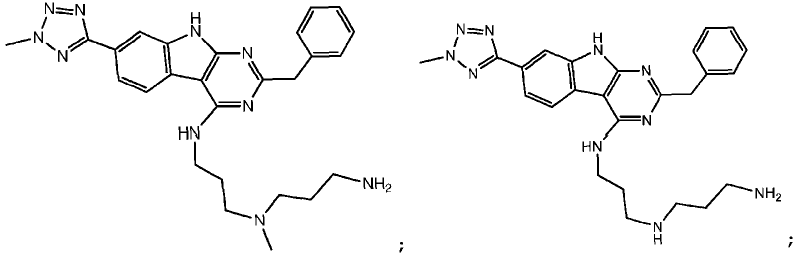 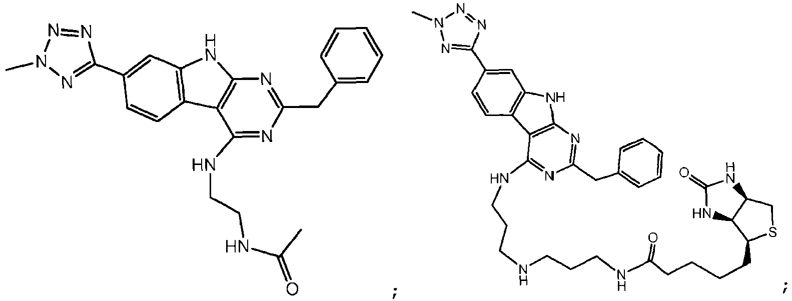 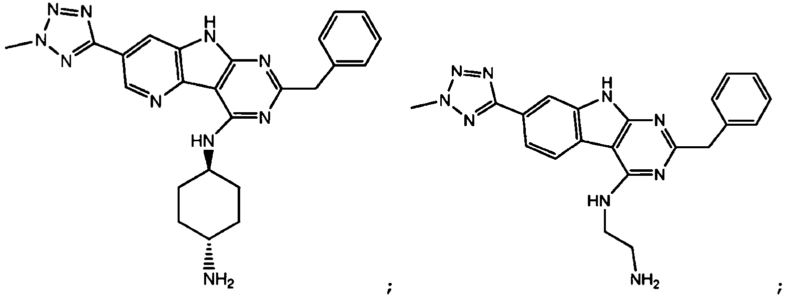 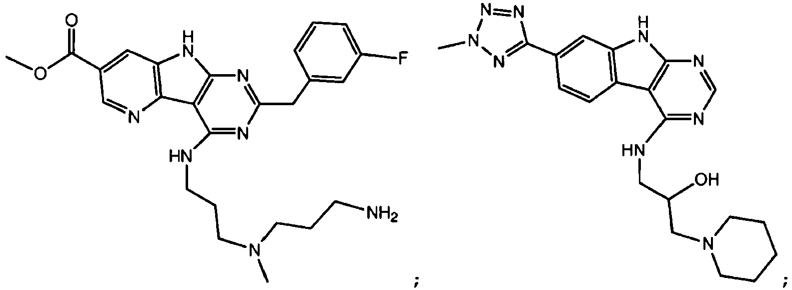 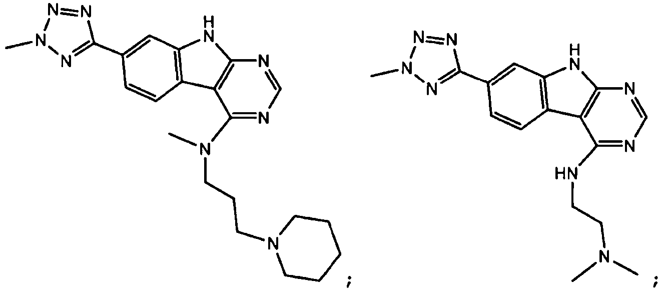 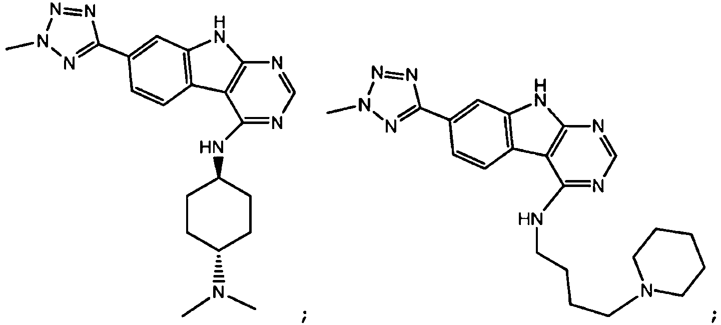 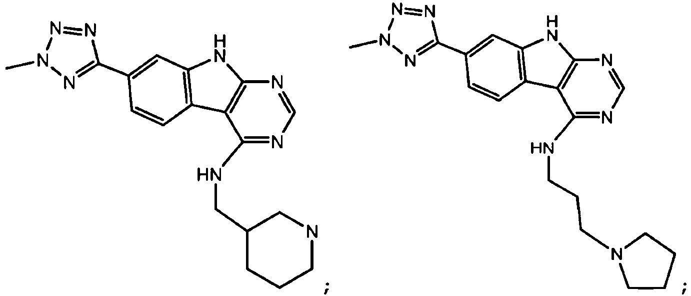 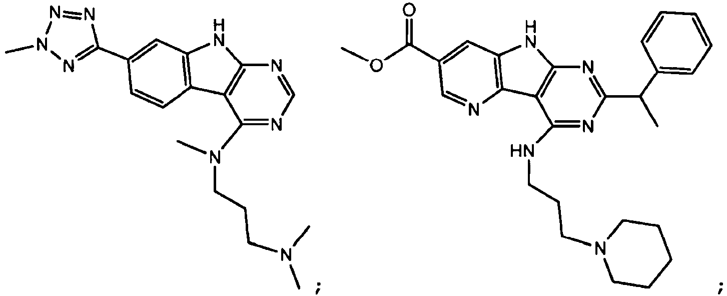 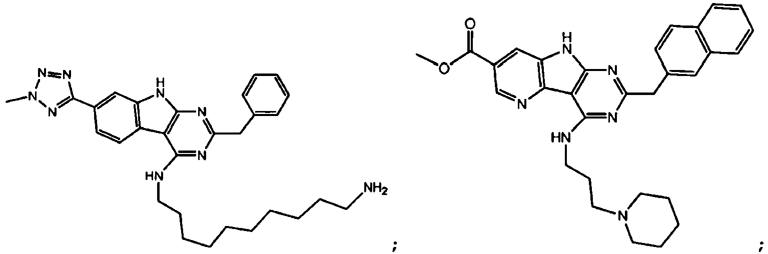 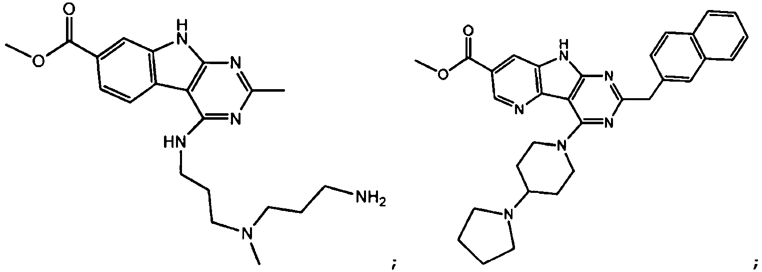 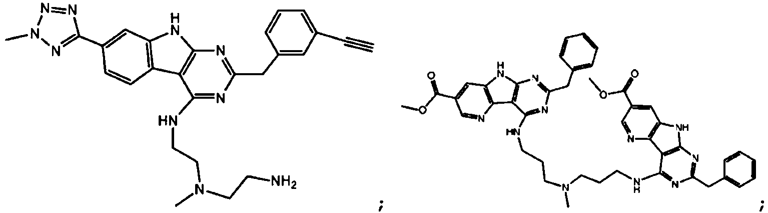 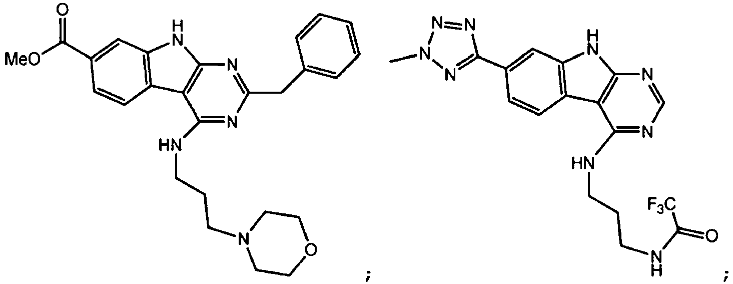 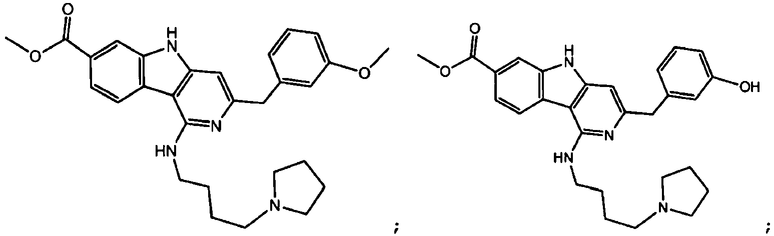 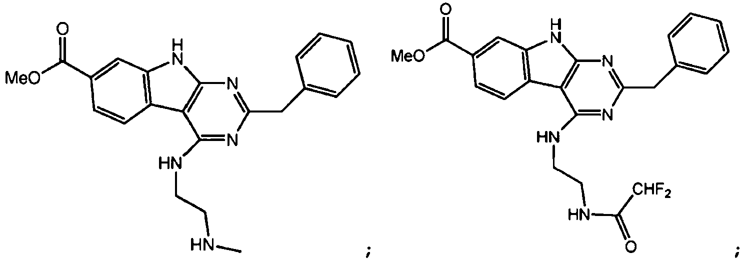 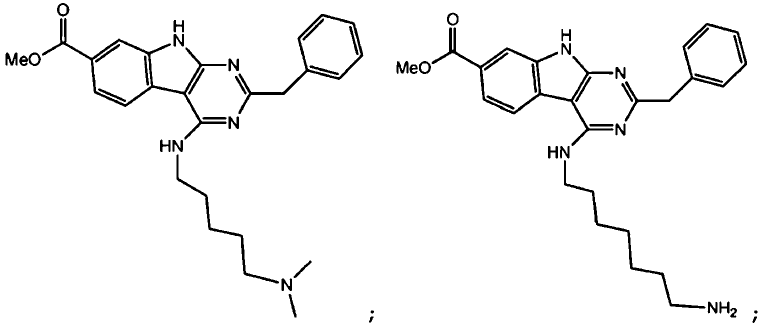 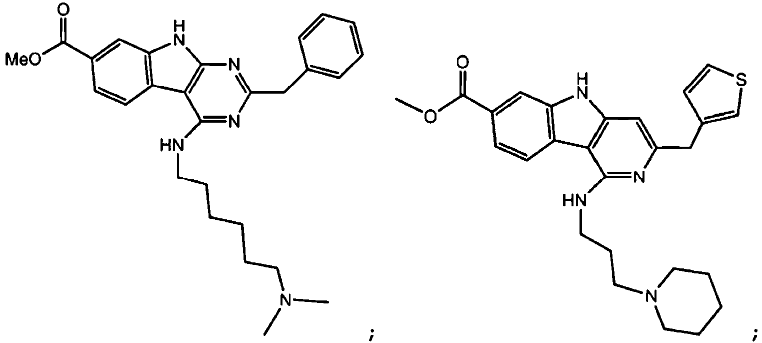 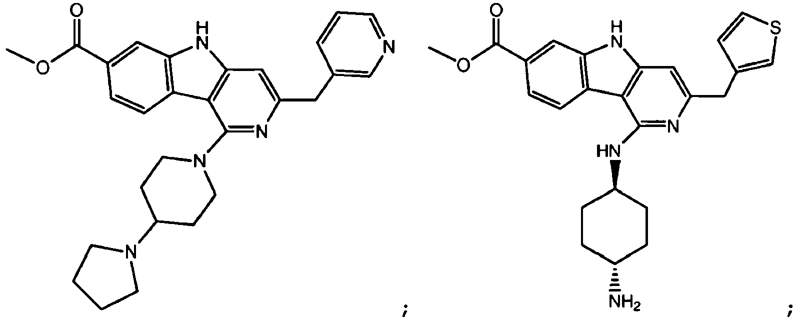 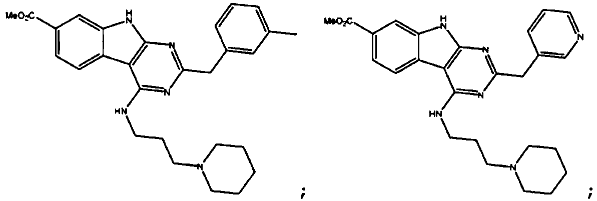 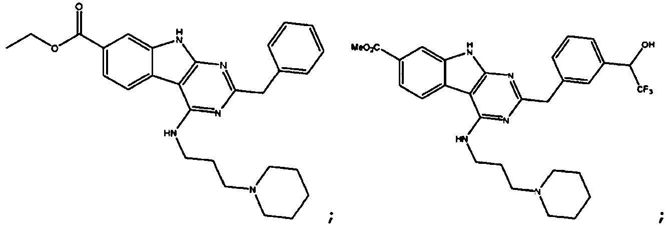 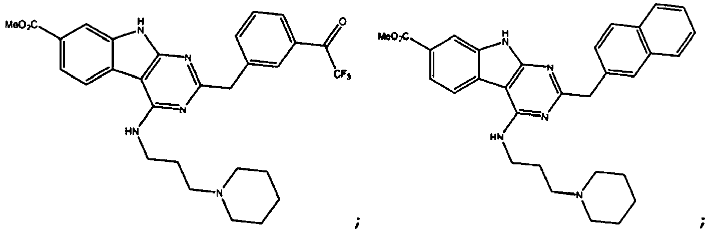 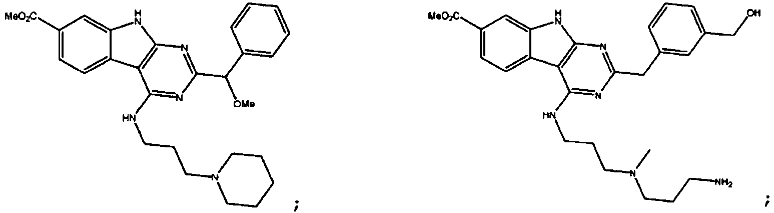 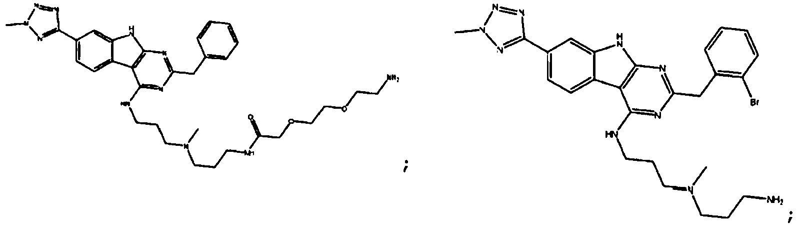 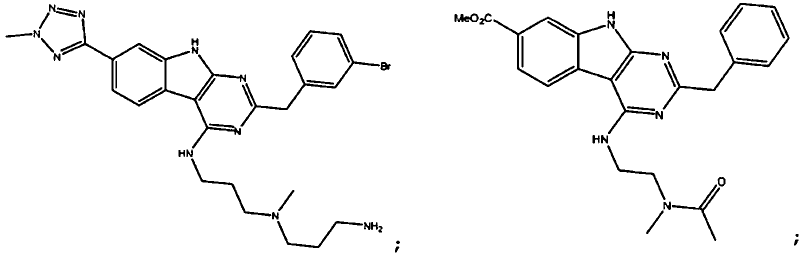 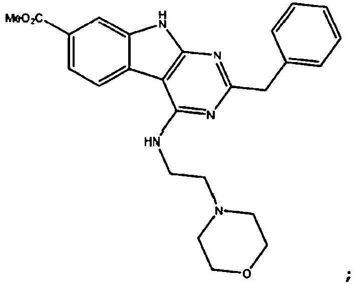 oder einem Salz davon zur Verwendung bei der Behandlung von Krebs bei einem Patienten, der diese benötigt.

12. Verfahren zum Hemmen der Vermehrung von Krebszellen *ex vivo,* welches das Verabreichen der Verbindung nach einem der Ansprüche 1 bis 5 und 9 bis 11 in dem Medium umfasst, das die sich vermehrenden Zellen enthält, wobei die Verbindung eine antineoplastische Wirkung auf die Vermehrung der Krebszellen aufweist.

## Revendications

1. Composé de formule I pour son utilisation dans le traitement du cancer chez un patient, dans lequel ledit au moins un composé de formule I est : 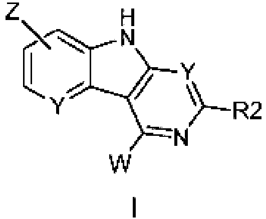
ou un sel de celui-ci,
dans laquelle :
chaque Y est indépendamment choisi parmi N et CH ;
Z est à chaque fois indépendamment choisi parmi :
-
CN,
-
C(O)OR1,
-
C(O)N(R1)R3,
-
C(O)R1,
ou
- hétéroaryle éventuellement substitué par un ou plusieurs substituants RA ou R4,
dans lequel, lorsque (R1) et R3 sont liés à un atome d'azote, ils se lient éventuellement ensemble avec l'atome d'azote pour former un cycle à 3 à 7 chaînons comprenant éventuellement un ou plusieurs autres hétéroatomes choisis parmi N, O et S, ledit cycle étant éventuellement substitué par un ou plusieurs RA ou R4 ;
W est :
-
CN,
-
N(R1)R3,
-
C(O)OR1,
-
C(O)N(R1)R3,
-
NR1C(O)R1,
-
NR1C(O)OR1,
-
OC(O)N(R1)R3,
-
OC(O)R1,
-
C(O)R1),
-
NR1C(O)N(R1)R3,
-
NR1S(O)₂R1,
- benzyle éventuellement substitué par 1, 2 ou 3 substituants RA ou R1,
-
X-L-(X-L)n - N(R1)R3
- X-L-(X-L)n - hétéroaryle éventuellement substitué par un ou plusieurs substituants RA ou R4 liés à l'un ou aux deux groupes L et hétéroaryle,
- X-L-(X-L)n - hétérocyclyle éventuellement substitué par un ou plusieurs substituants RA ou R4 liés à l'un ou aux deux groupes L et hétérocyclyle,
- X-L-(X-L)n- aryle éventuellement substitué par un ou plusieurs substituants RA ou R4,
-
X-L-(X-L)ₙ -NR1RA,
ou
- (N(R1)-L)ₙ -N⁺R1R3R5 R6 ;
où n est un nombre entier égal à 0, 1, 2, 3, 4 ou 5,
et dans lequel, lorsque R1 et R3 sont liés à un atome d'azote, ils se lient éventuellement ensemble avec l'atome d'azote pour former un cycle à 3 à 7 chaînons comprenant éventuellement un ou plusieurs autres hétéroatomes choisis parmi N, O et S, ledit cycle étant éventuellement substitué par un ou plusieurs RA ou R4 ;
chaque X est indépendamment choisi parmi CH₂, O, S et NR1 ;
chaque L est indépendamment :
-
C₁₋₆ alkylène,
-
C₂₋₆ alcénylène,
-
C₂₋₆ alcynylène,
- C₃₋₇ cycloalkylène, qui comprend éventuellement un ou plusieurs autres hétéroatomes choisis parmi N, O et S, ou
- C₃₋₇ cycloalcénylène, qui comprend éventuellement un ou plusieurs autres hétéroatomes choisis parmi N, O et S,
les groupes alkylène, alcénylène, alcynylène, cycloalkylène et cycloalcénylène étant éventuellement substitués indépendamment par un ou deux substituants R4 ou RA ;
R1 est indépendamment :
-
H,
-
C₁₋₆ alkyle,
-
C₂₋₆ alcényle,
-
C₂₋₆ alcynyle,
-
C₃₋₇ cycloalkyle,
-
C₃₋₇ cycloalcényle,
-
C₁₋₅ alkyle perfluoré,
-
hétérocyclyle,
-
aryle,
-
hétéroaryle,
ou
-
benzyle,
les groupes alkyle, alcényle, alcynyle, cycloalcényle, alkyle perfluoré, hétérocyclyle, aryle, hétéroaryle et benzyle étant chacun indépendamment éventuellement substitués par 1, 2 ou 3 substituants RA ou Rd ;
R2 est :
-
H,
- C₁₋₆ -alkyle, éventuellement substitué par un ou plusieurs substituants RA,
-
C(O)R4,
- L-hétéroaryle, éventuellement substitué par un ou plusieurs substituants RA ou R4,
- L-hétérocyclyle, éventuellement substitué par un ou plusieurs RA ou R4, ou
- L-aryle, éventuellement substitué par un ou plusieurs substituants RA ou R4,
R3 est indépendamment :
-
H,
-
C₁₋₆ alkyle,
-
C₂₋₆ alcényle,
-
C₂₋₆ alcynyle,
-
C₃₋₇ cycloalkyle,
-
C₃₋₇ cycloalcényle,
-
C₁₋₅ alkyle perfluoré,
-
hétérocyclyle,
-
aryle,
-
hétéroaryle,
ou
-
benzyle,
les groupes alkyle, alcényle, alcynyle, cycloalkyle, cycloalcényle, alkyle perfluoré, hétérocyclyle, aryle, hétéroaryle et benzyle étant chacun indépendamment éventuellement substitués par 1, 2 ou 3 substituants RA ou Rd ;
R4 est indépendamment :
-
H,
-
C₁₋₆ alkyle,
-
C₁₋₆ haloalkyle
-
C₂₋₆ alcényle,
-
C₂₋₆ alcynyle,
-
C₃₋₇ cycloalkyle,
-
C₃₋₇ cycloalcényle,
-
C₁₋₅ alkyle perfluoré,
-
hétérocyclyle,
-
aryle,
-
hétéroaryle,
ou
-
benzyle,
où les groupes alkyle, alcényle, alcynyle, cycloalkyle, cycloalcényle, alkyle perfluoré, hétérocyclyle, aryle, hétéroaryle et benzyle sont chacun indépendamment éventuellement substitués par 1, 2 ou 3 substituants RA ou Rd ;
R5 est indépendamment :
-
C₁₋₆ alkyle,
- C₁₋₄ alkylène-C₂₋₆ alcényle, qui comprend éventuellement un ou plusieurs autres hétéroatomes choisis parmi N, O et S,
- C₁₋₆ alkylène-C₂₋₆ alcynyle, qui comprend éventuellement un ou plusieurs autres hétéroatomes choisis parmi N, O et S,
- L-aryle comprenant éventuellement un ou plusieurs substituants RA ou R4,
- L-hétéroaryle, qui comprend éventuellement un ou plusieurs substituants RA ou R4,
-
C₁₋₆ alkylène-C(O)O-,
-
C₁₋₆ alkylène-C(O)OR1,
-
C₁₋₆ alkylène-CN,
- C₁₋₆ alkylène-C(O)NR1R3, où R1 et R3 se lient éventuellement ensemble à l'atome d'azote pour former un cycle à 3 à 7 chaînons comprenant éventuellement un ou plusieurs autres hétéroatomes choisis parmi N, O et S ; ou
-
C₁₋₆ alkylène-OH ;
R6 est :
-
halogène
-
OC(O)CF₃
ou
-
OC(O)R1 ;
chaque RA est indépendamment :
-
halogène,
-
CF₃,
-
OR1,
-
L-OR1,
-
OCF₃,
-
SR1,
-
CN,
-
NO₂,
-
NR1R3,
-
L-NR1R1,
-
C(O)OR1,
-
S(O)₂R4,
-
C(O)N(R1)R3,
-
NR1C(O)R1,
-
NR1C(O)OR1,
-
OC(O)N(R1)R3,
-
OC(O)R1,
-
C(O)R4,
-
NHC(O)N(R1)R3,
-
NR1C(O)N(R1)R3,
ou
-
N₃ ;
et
chaque Rd est indépendamment :
-
H,
-
C₁₋₆ alkyle,
-
C₂₋₆ alcényle,
-
C₂₋₆ alcynyle,
-
C₃₋₇ cycloalkyle,
-
C₃₋₇ cycloalcényle,
-
C₁₋₅ alkyle perfluoré,
-
benzyle
ou
-
hétérocyclyle.

2. Composé pour son utilisation selon la revendication 1, dans lequel le composé de formule I 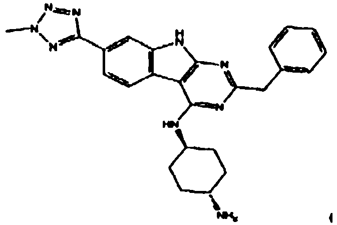 ou un sel pharmaceutiquement acceptable de celui-ci.

3. Composé pour son utilisation selon la revendication 1, dans lequel le composé de formule I est un sel bromhydrate de

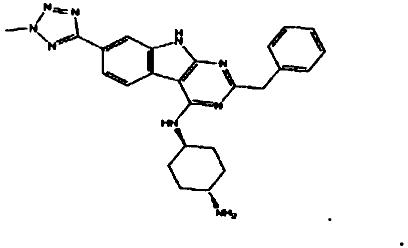

4. Composé pour son utilisation selon la revendication 1, dans lequel le composé de formule I 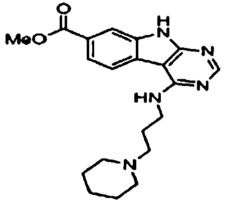 ou un sel pharmaceutiquement acceptable de celui-ci.

5. Composé pour son utilisation selon la revendication 1, dans lequel le composé répond à la formule I 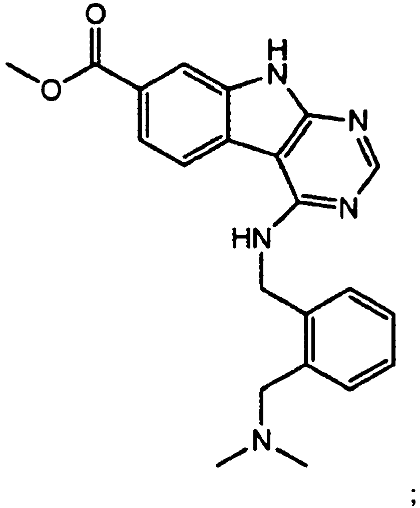 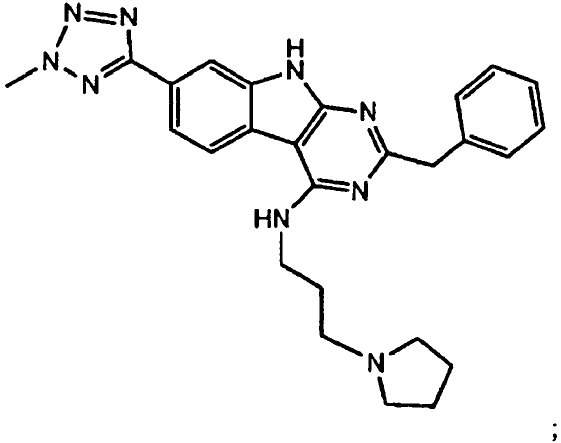 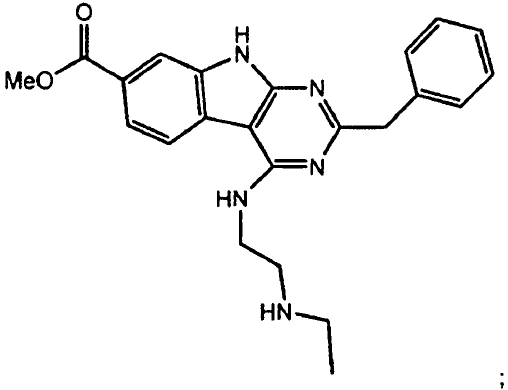 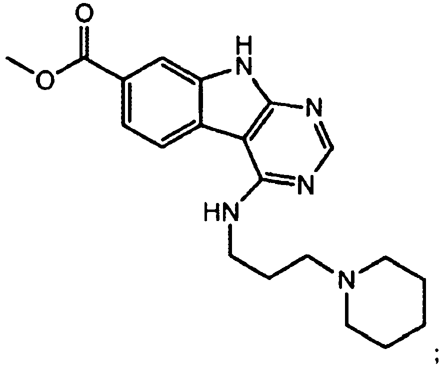 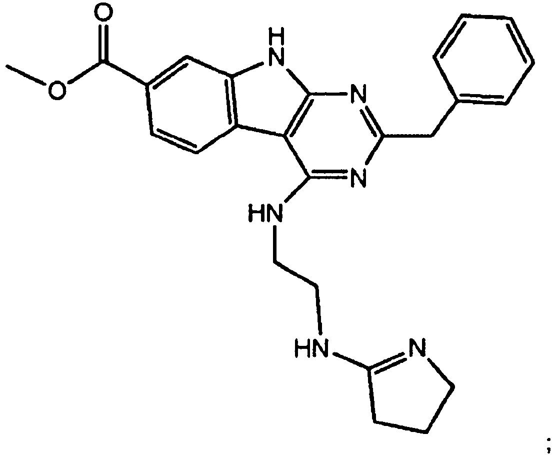 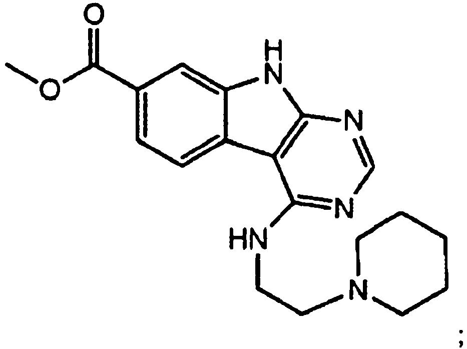 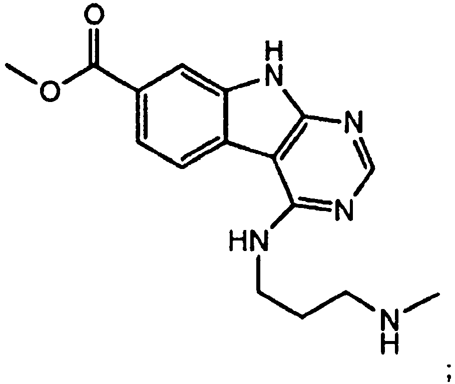 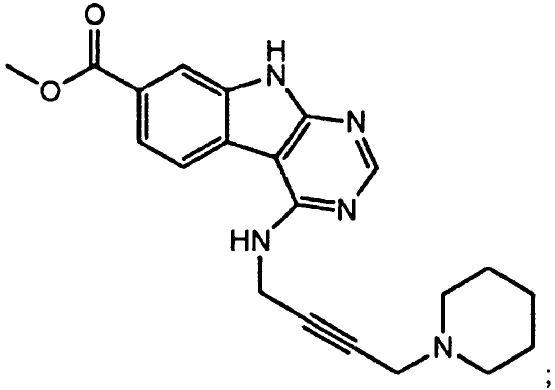 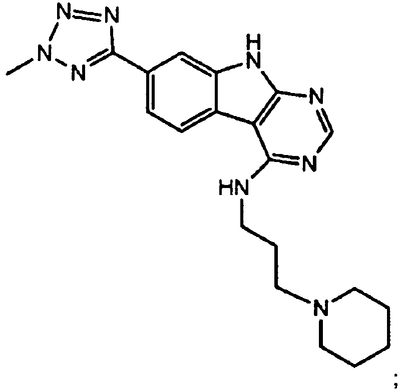 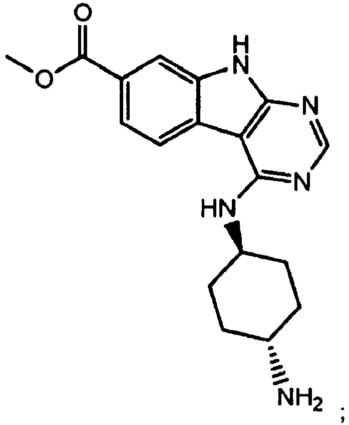 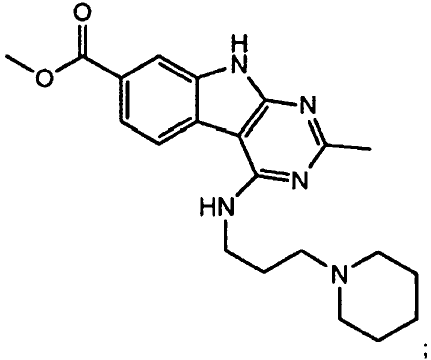 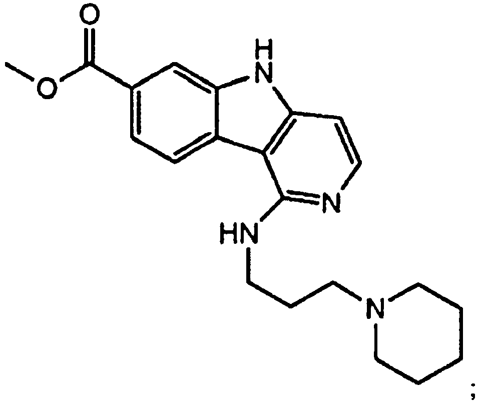 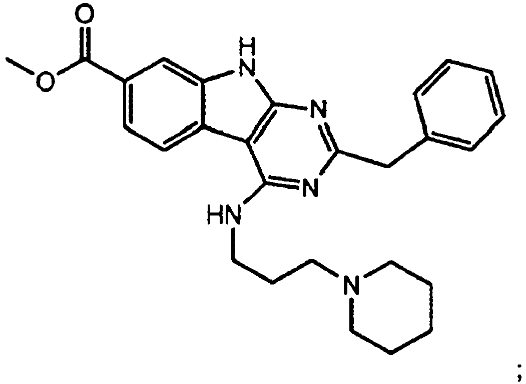 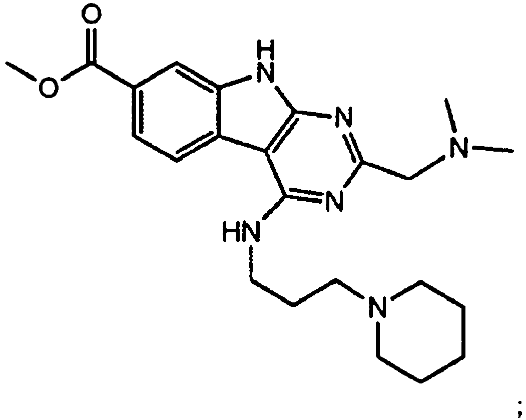 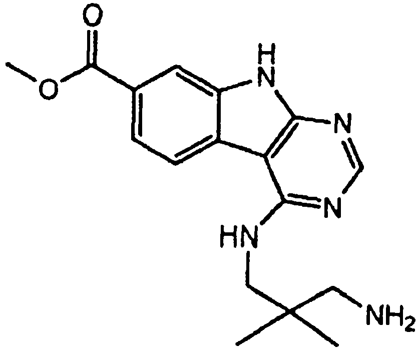 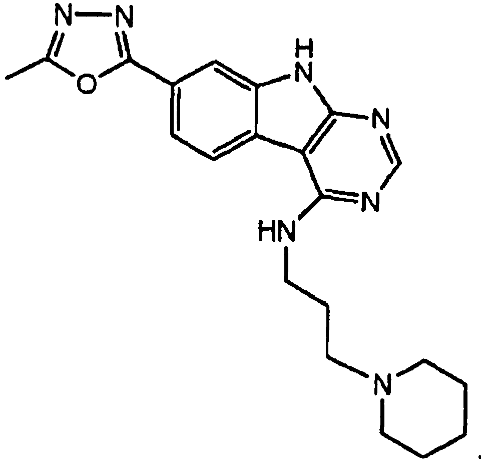 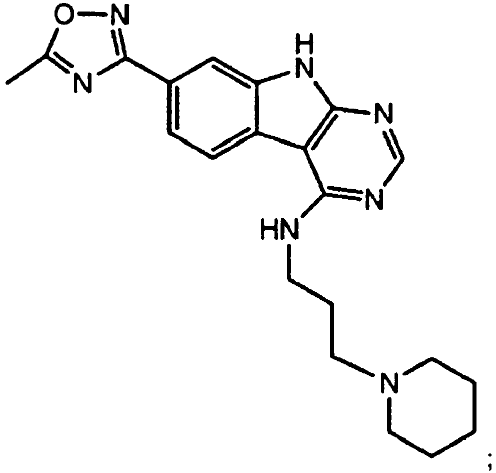 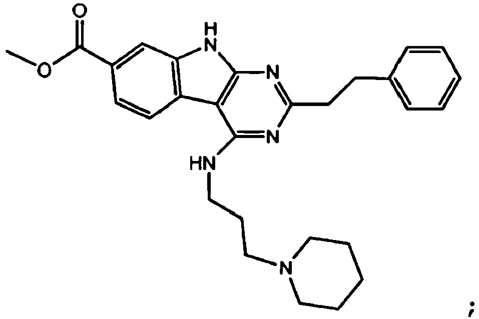 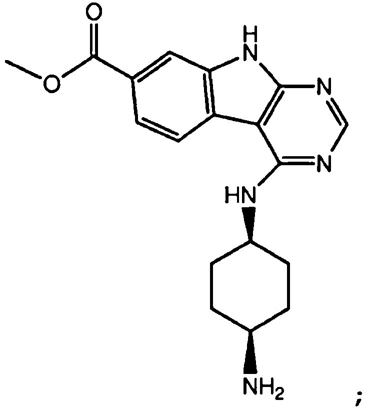 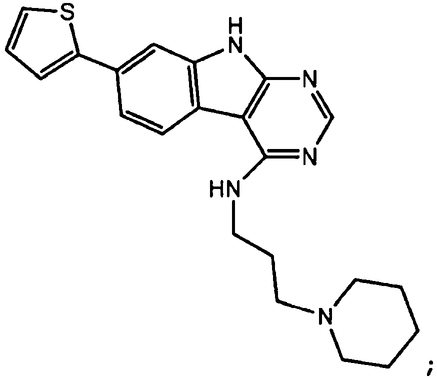 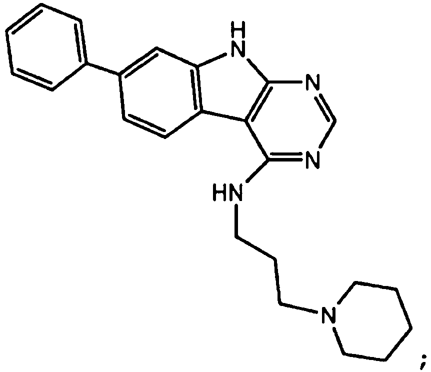 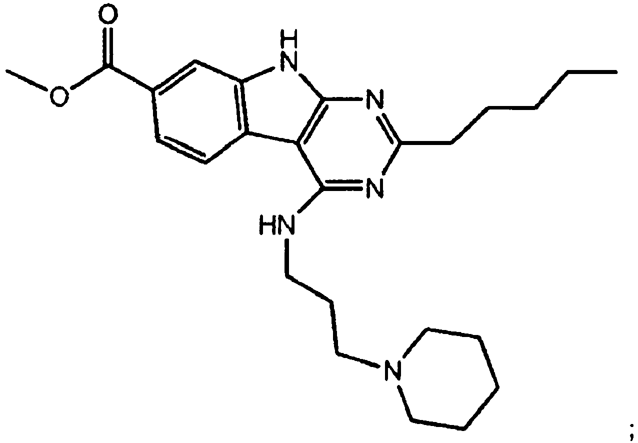 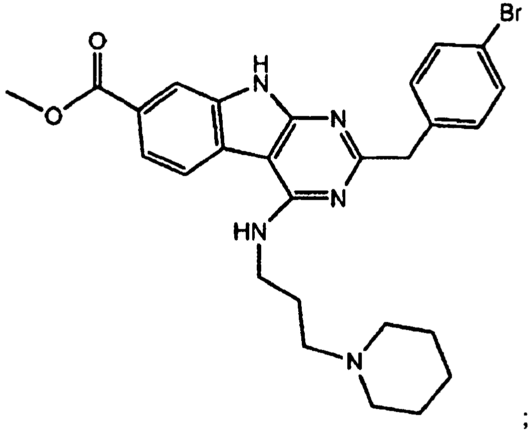 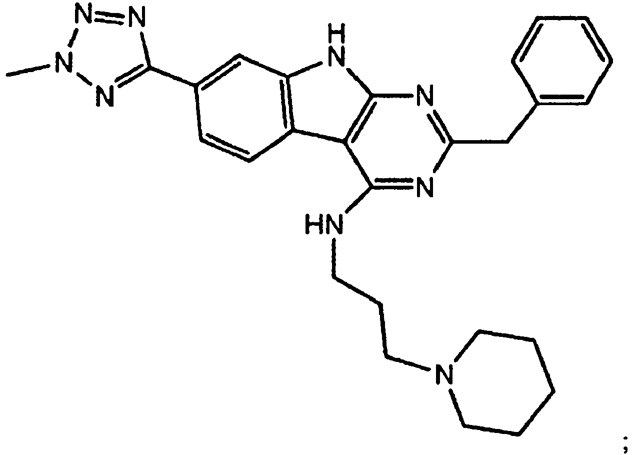 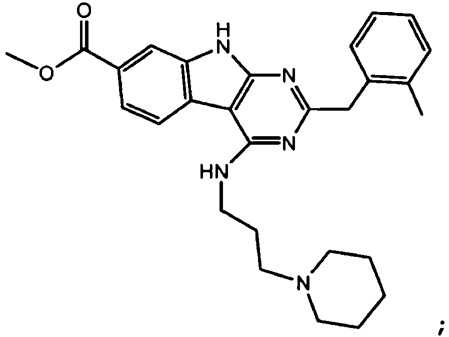 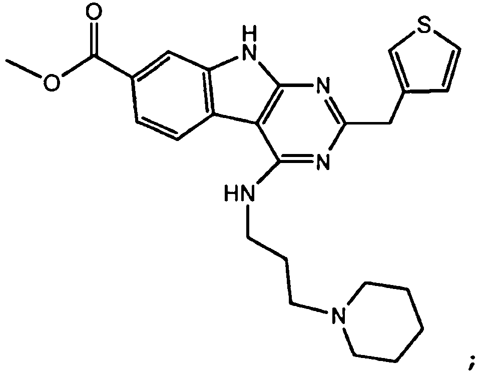 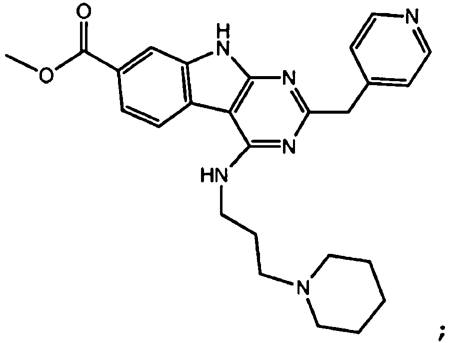 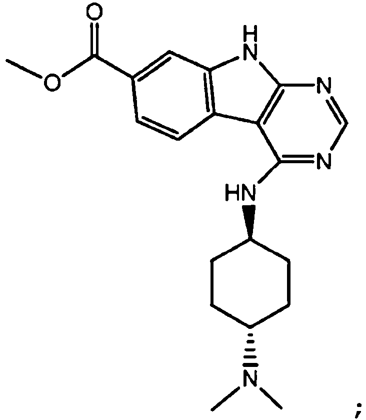 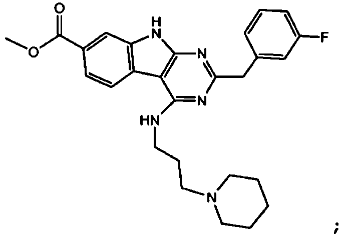 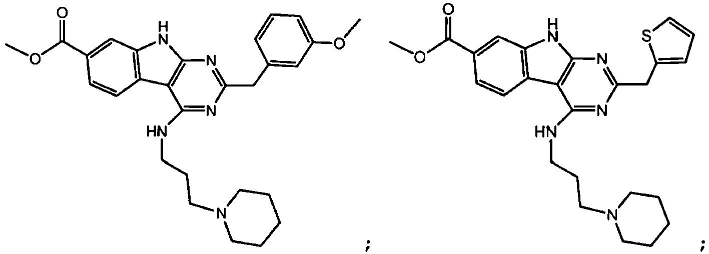 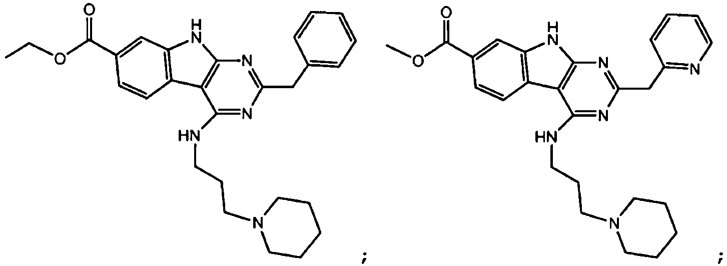 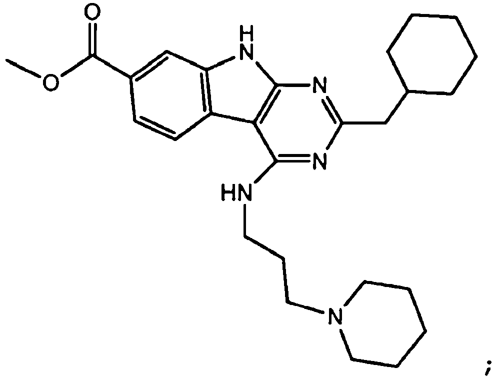 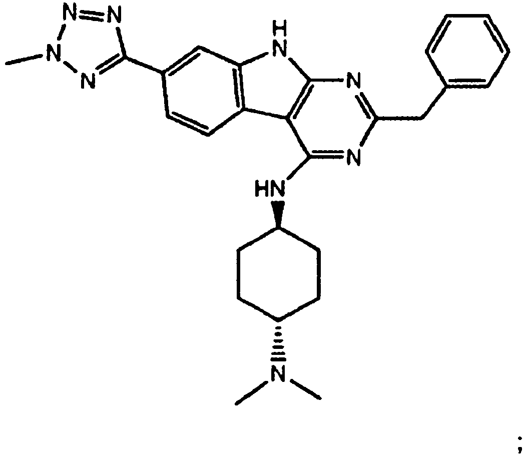 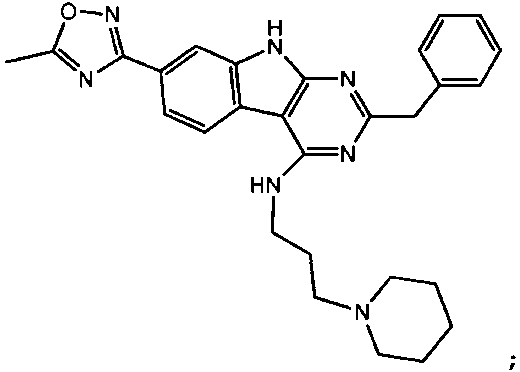 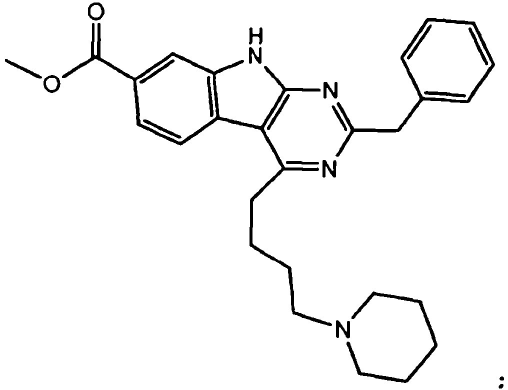 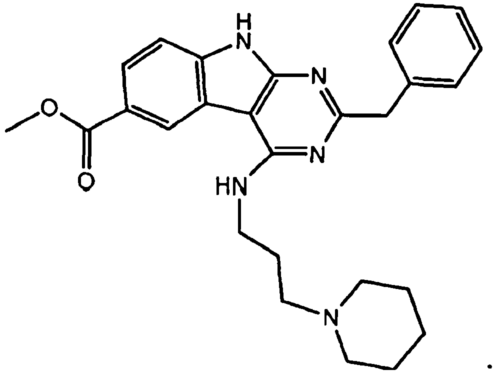 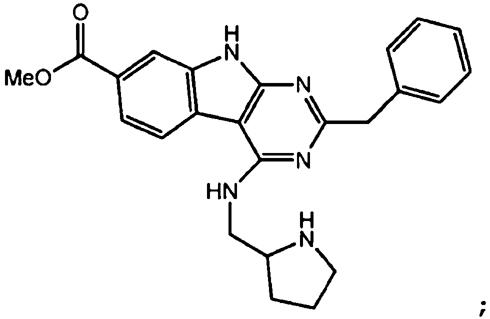 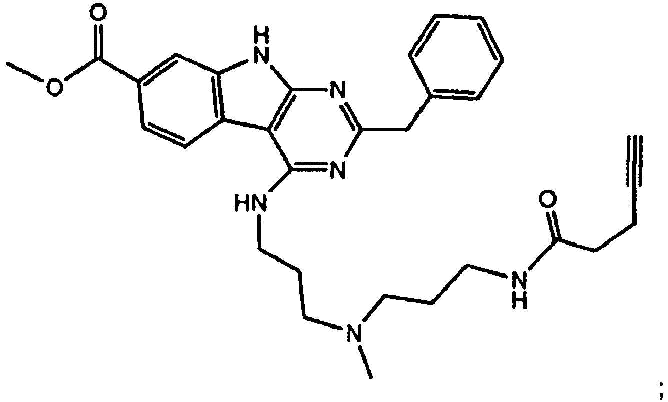 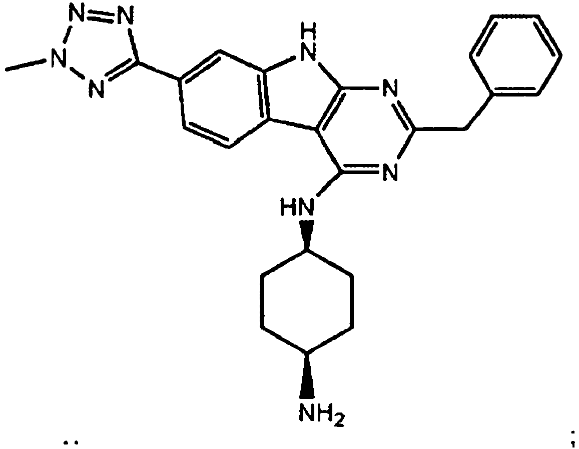 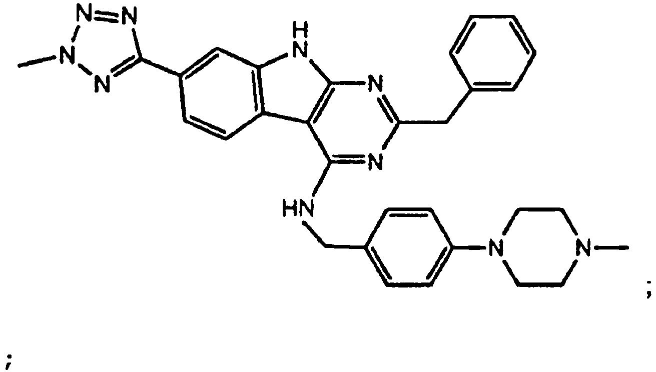 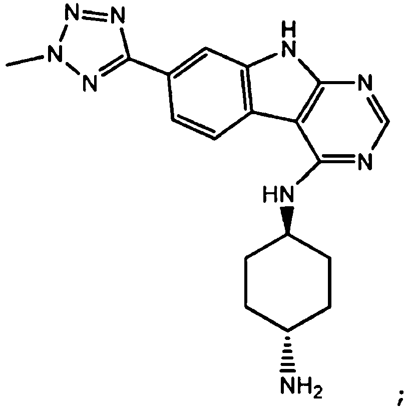 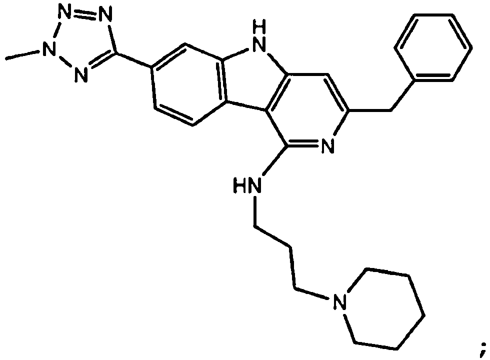 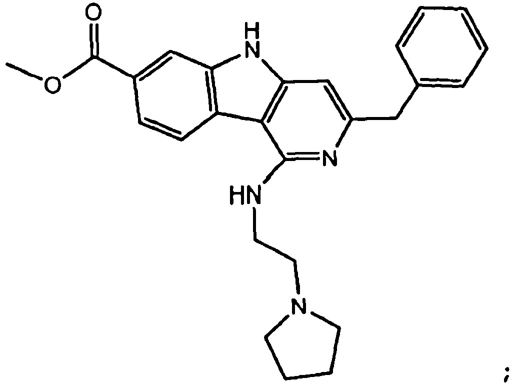 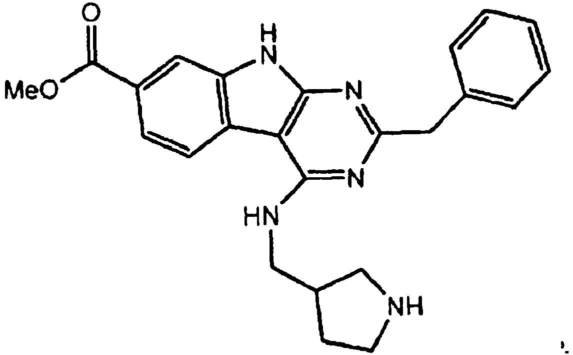 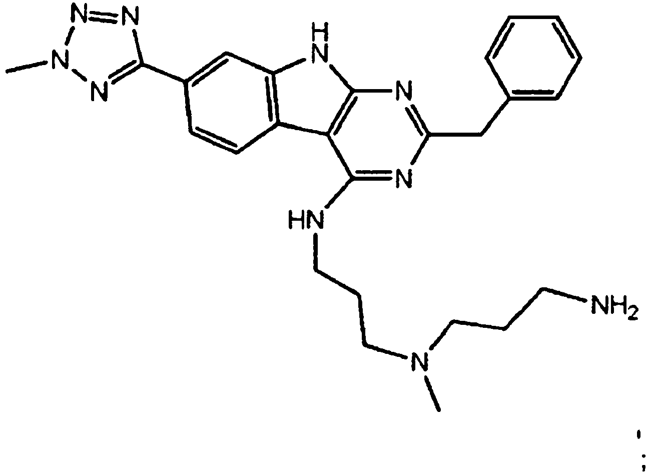 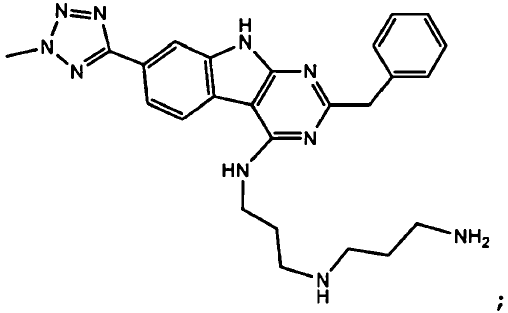 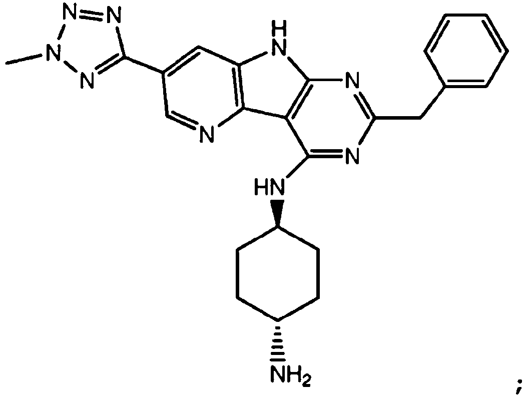 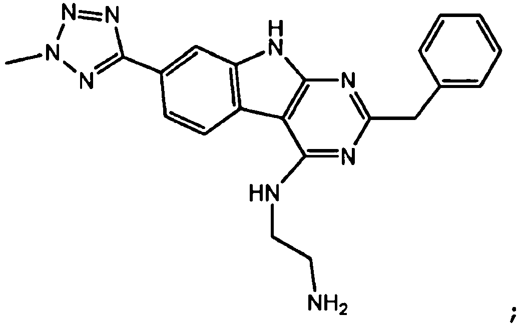 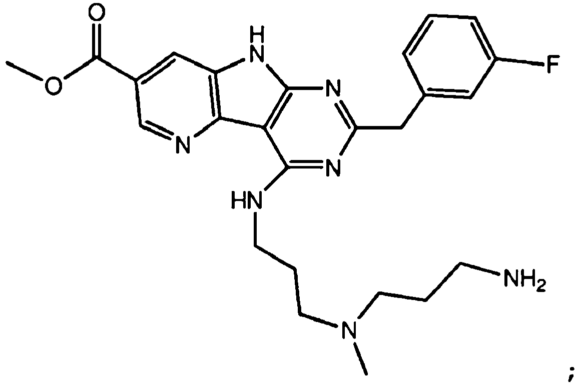 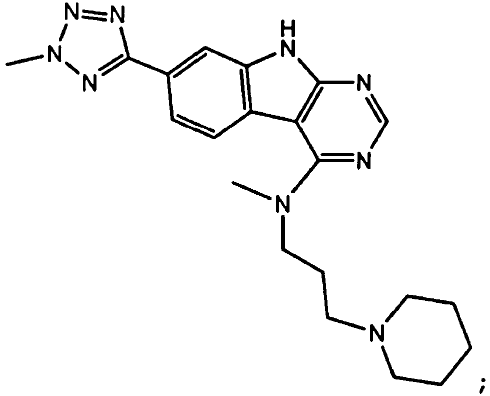 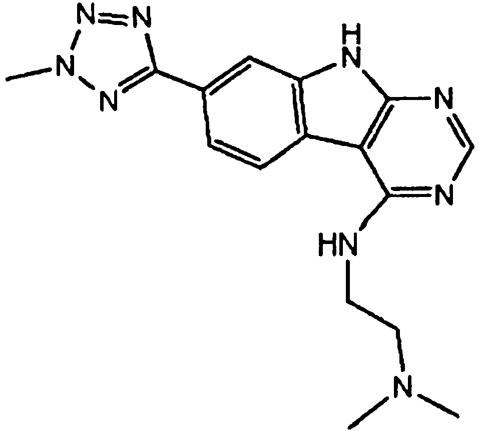 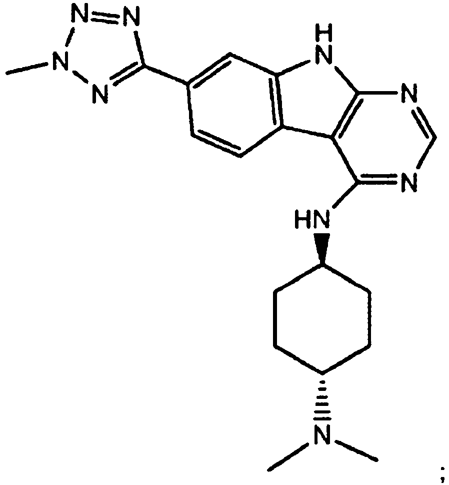 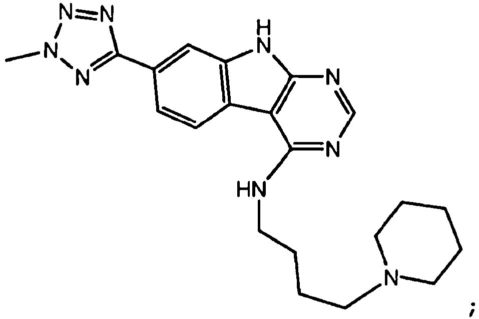 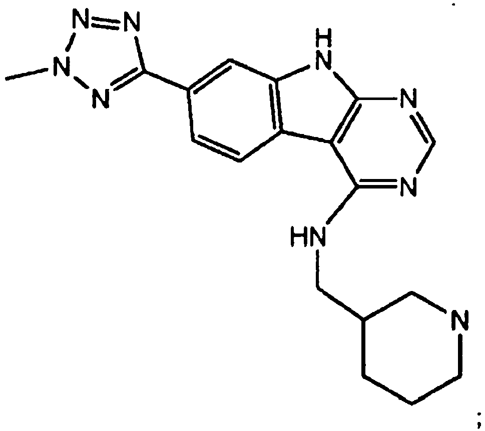 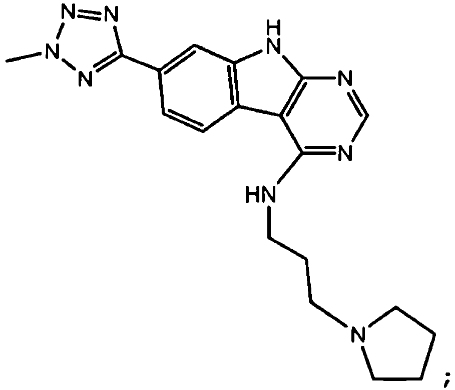 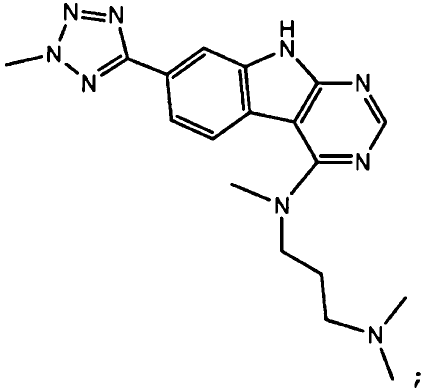 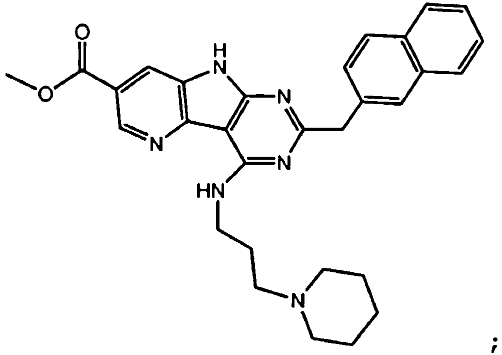 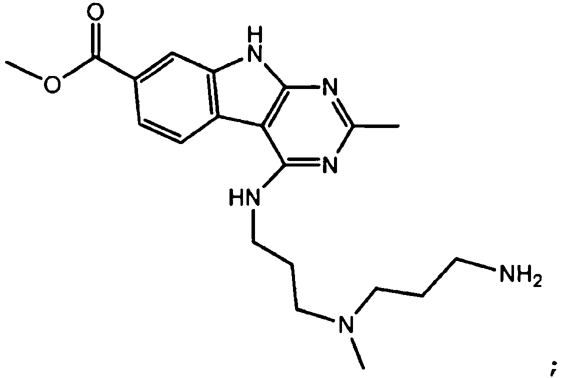 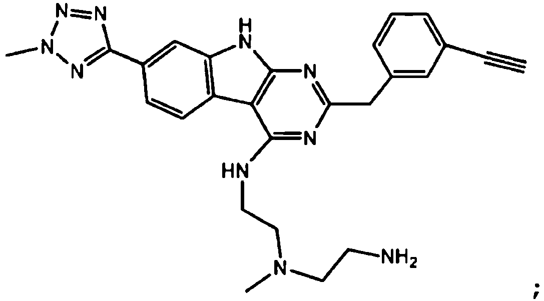 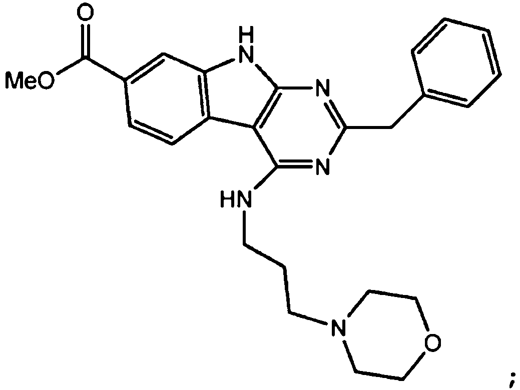 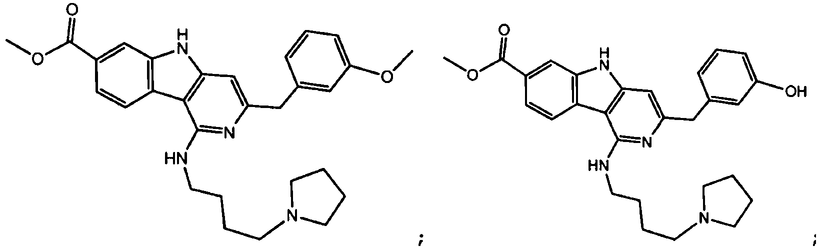 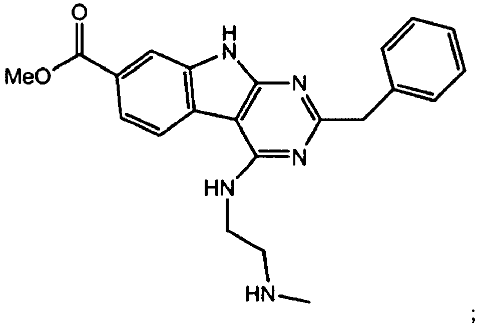 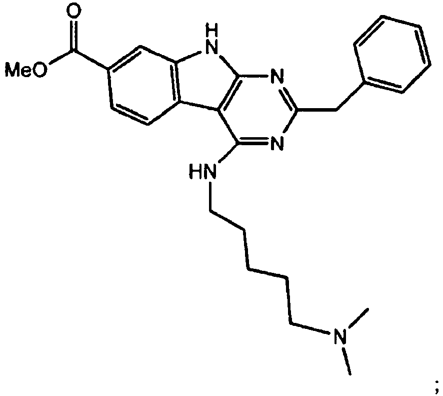 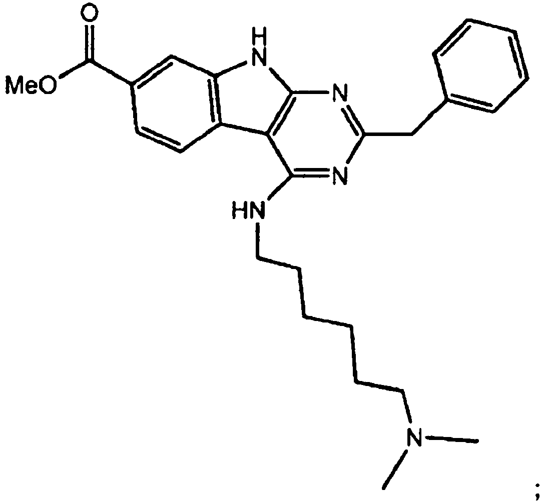 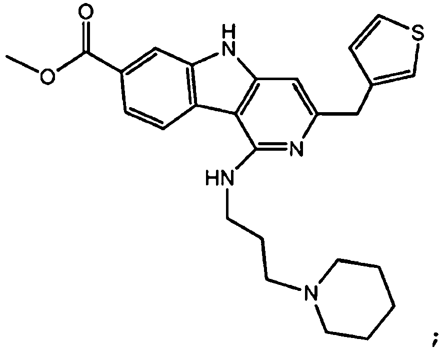 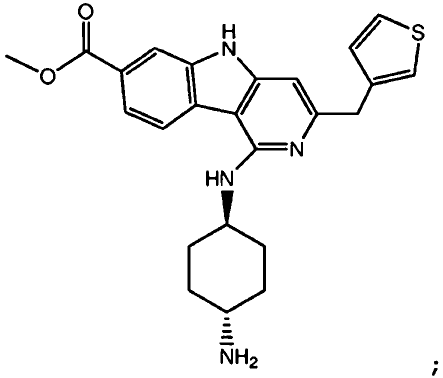 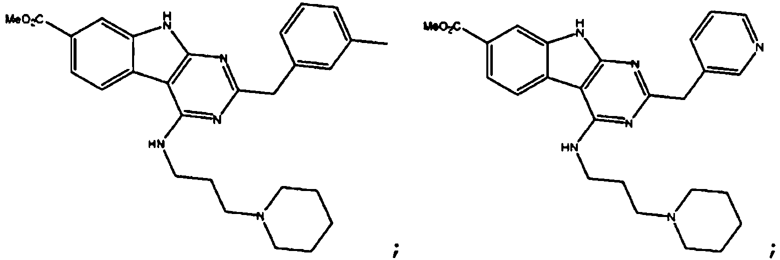 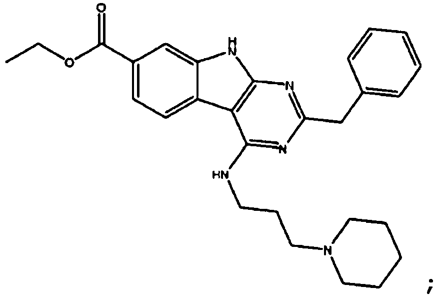 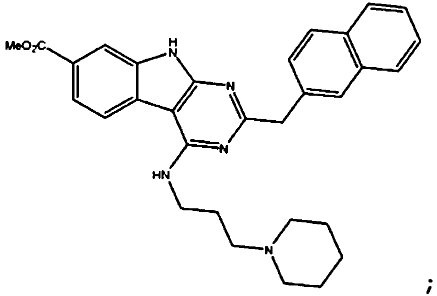 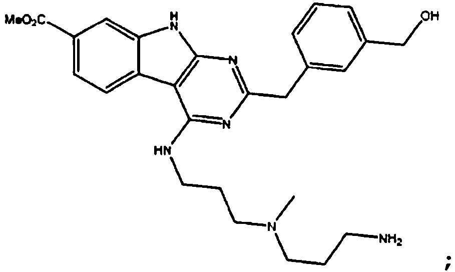 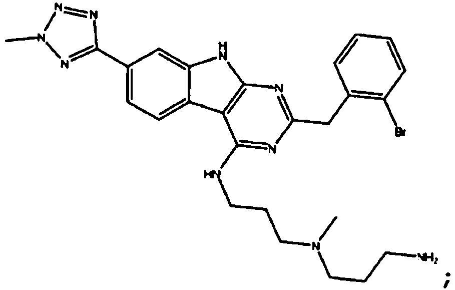 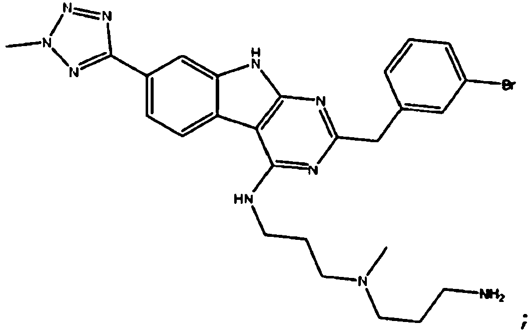 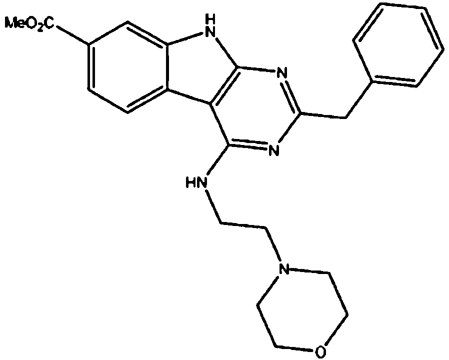 ou un sel de celui-ci.

6. Composé pour son utilisation selon l'une quelconque des revendications 1 à 5, dans lequel ledit patient est un être humain ou un animal.

7. Composé pour son utilisation selon la revendication 6, dans lequel ledit animal est une souris.

8. Composé pour son utilisation selon l'une quelconque des revendications 1 à 7, dans lequel le composé est formulé pour une administration orale, intramusculaire, intraveineuse ou sous-cutanée.

9. Composé pour son utilisation selon l'une quelconque des revendications 1 à 8, dans lequel le composé dégrade au moins l'un parmi LSD1, RCOR1, HDAC2 et CoREST.

10. Composé pour son utilisation selon l'une quelconque des revendications 1 à 9, dans lequel le cancer est basé sur une mutation K27, une mutation EZH2 ou une mutation PRC2.

11. Composé choisi parmi 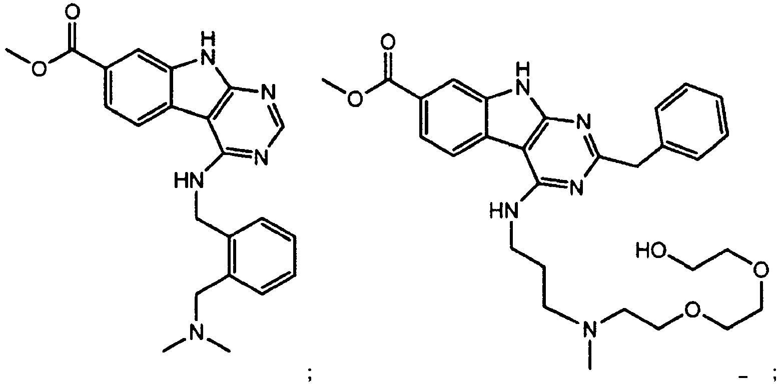 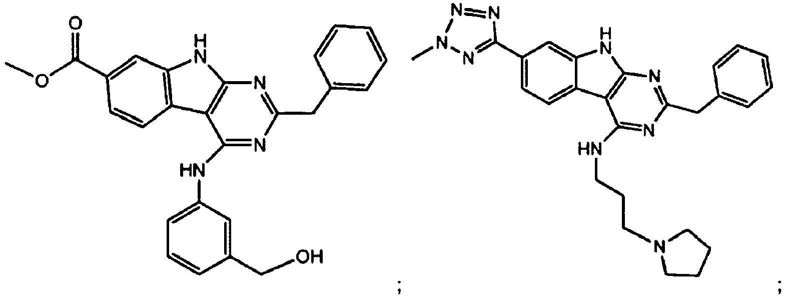 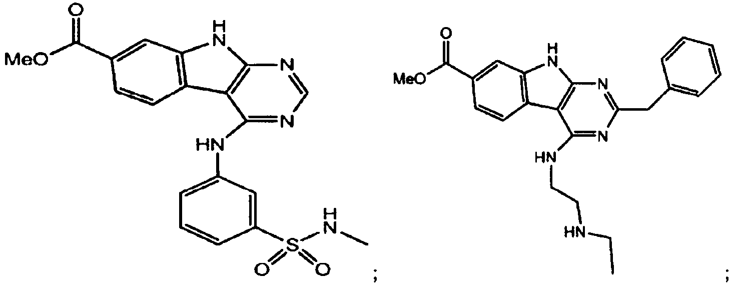 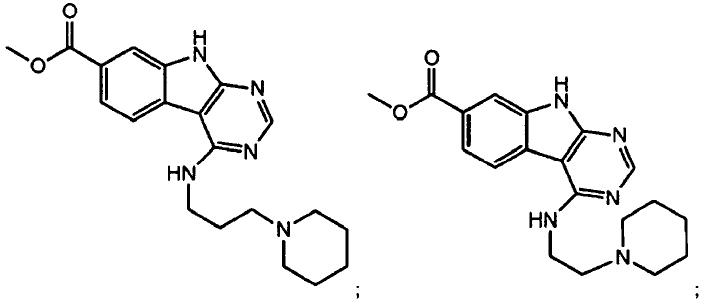 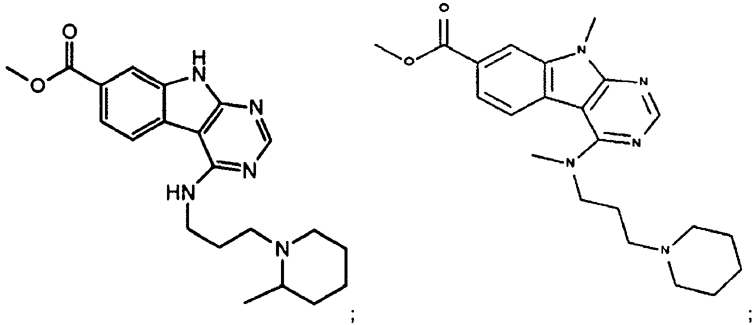 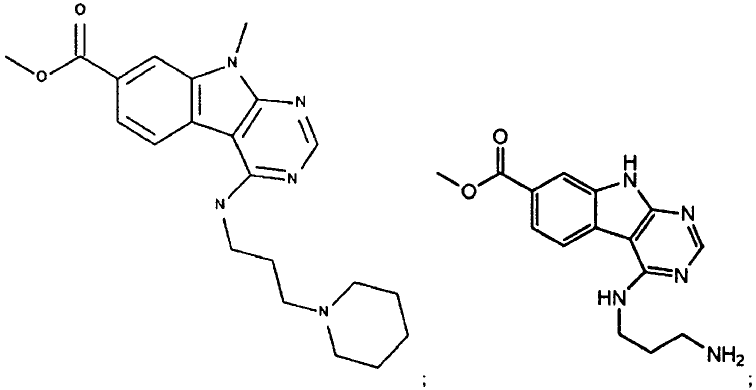 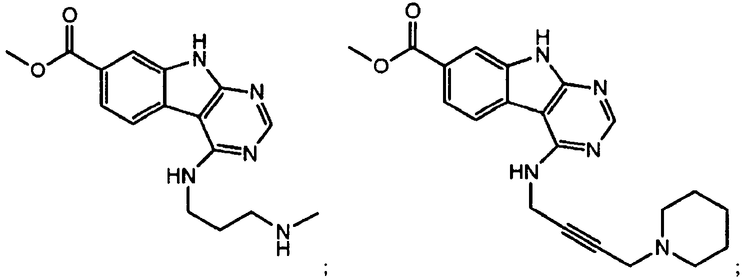 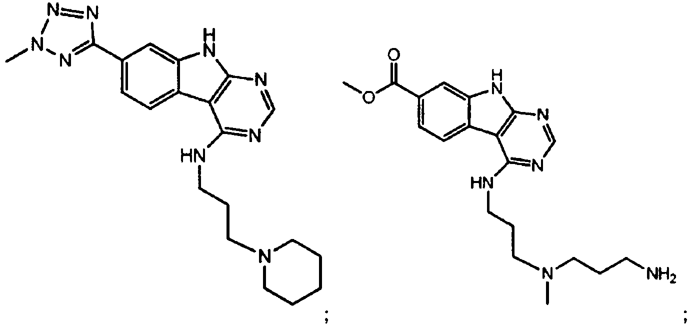 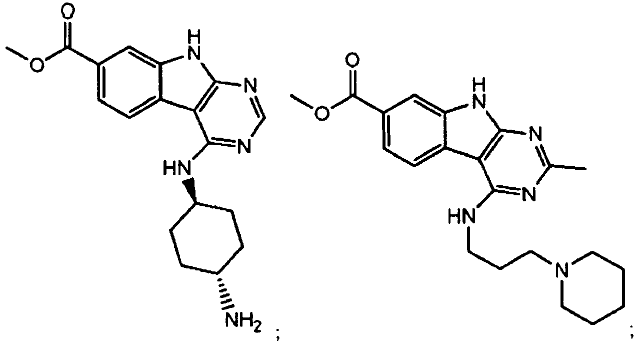 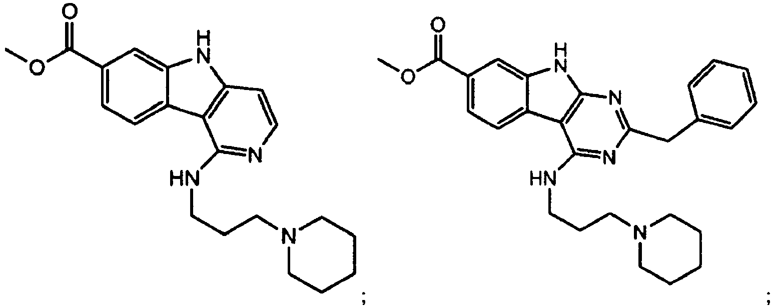 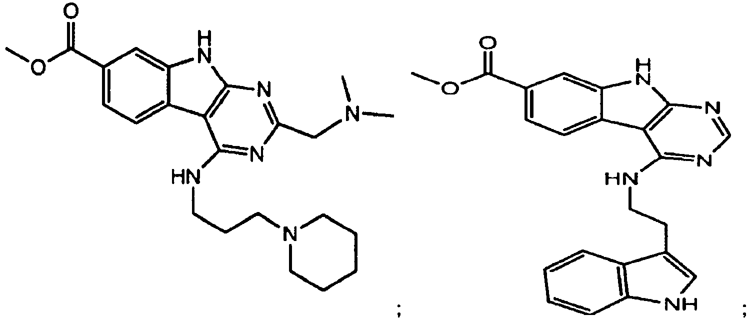 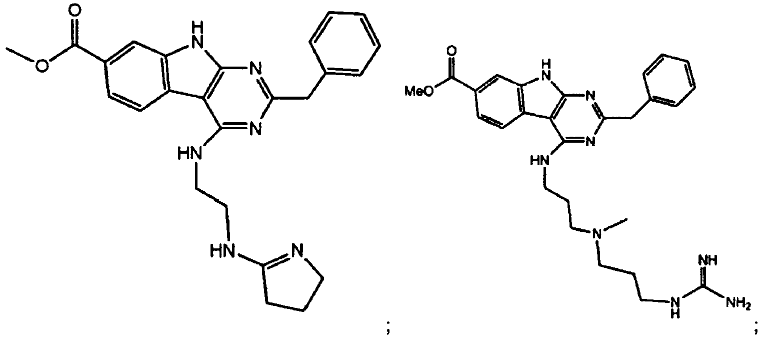 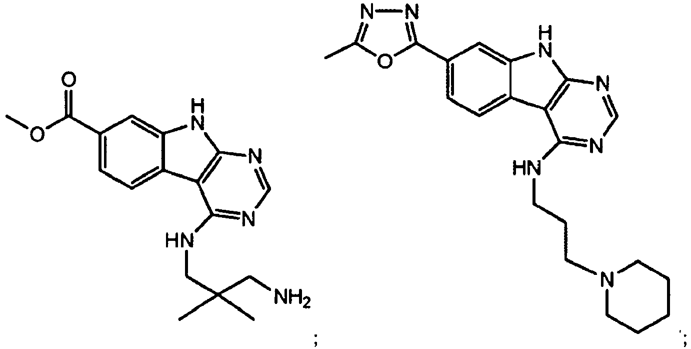 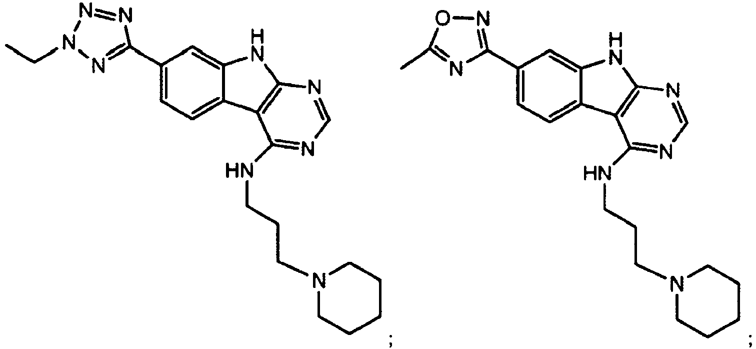 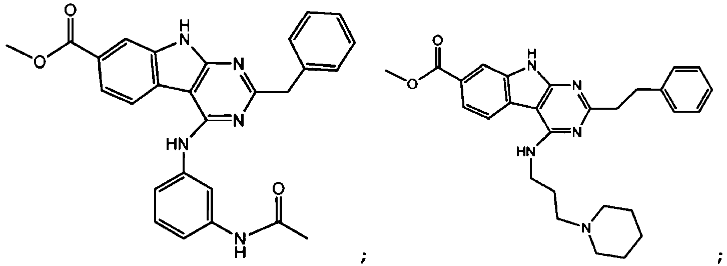 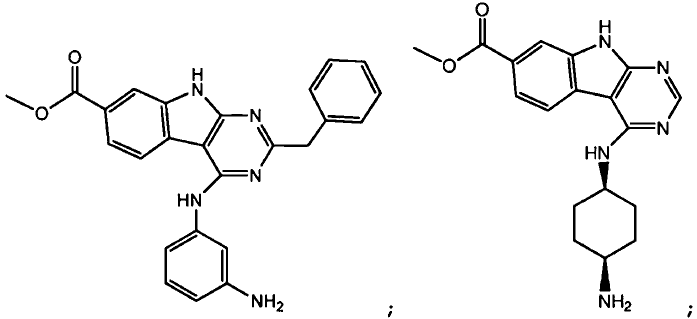 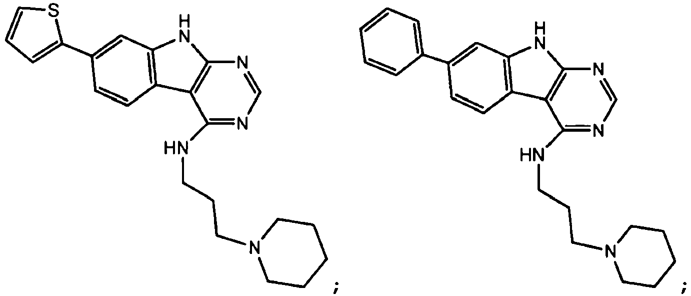 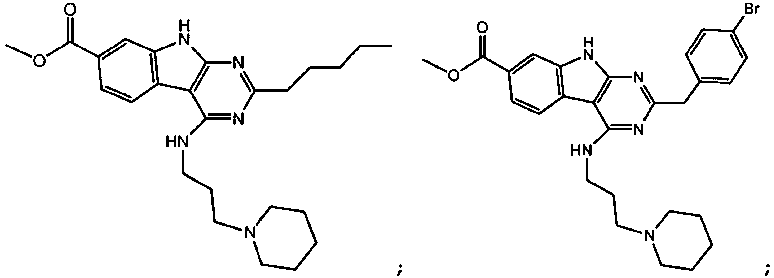 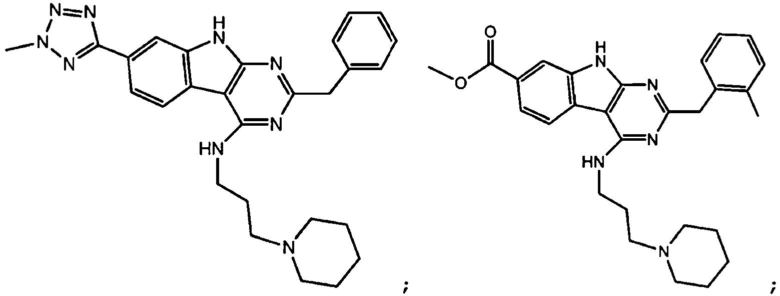 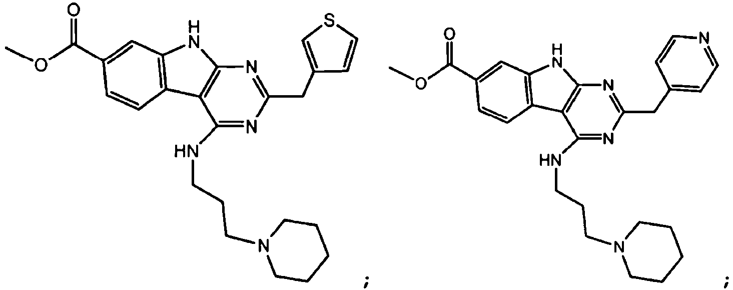 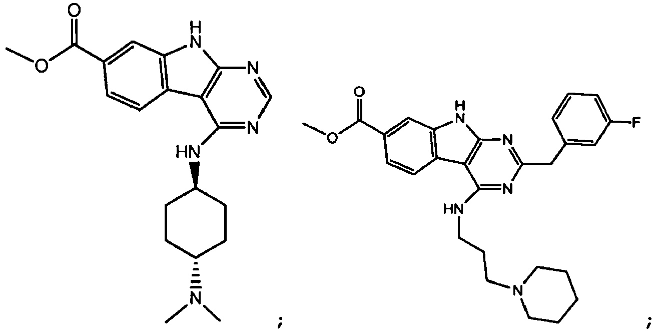 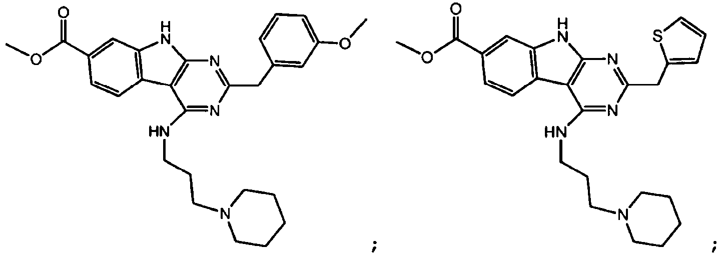 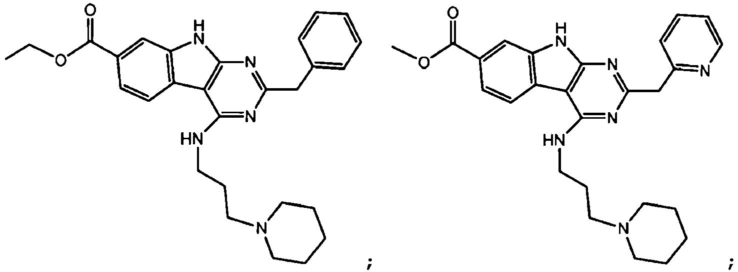 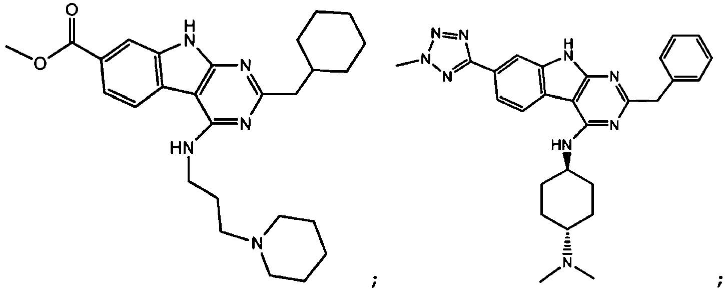 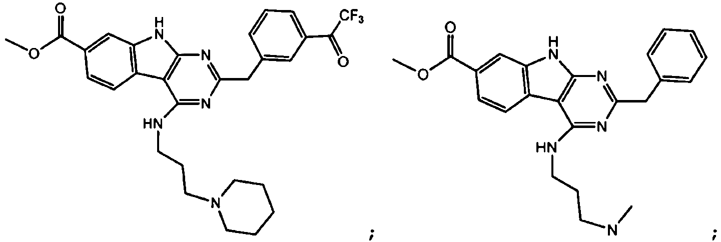 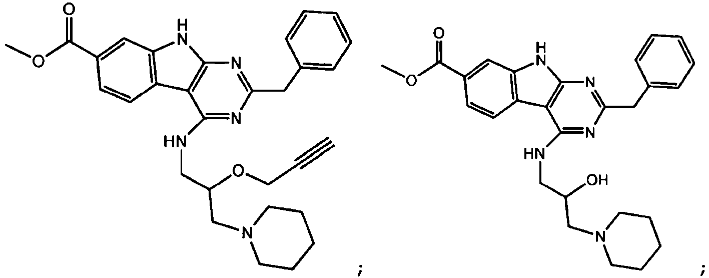 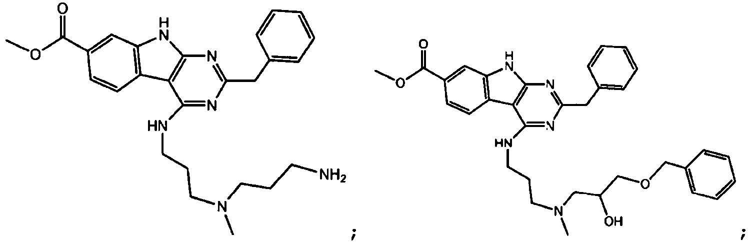 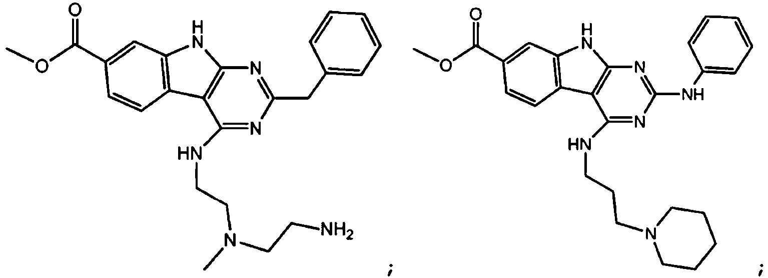 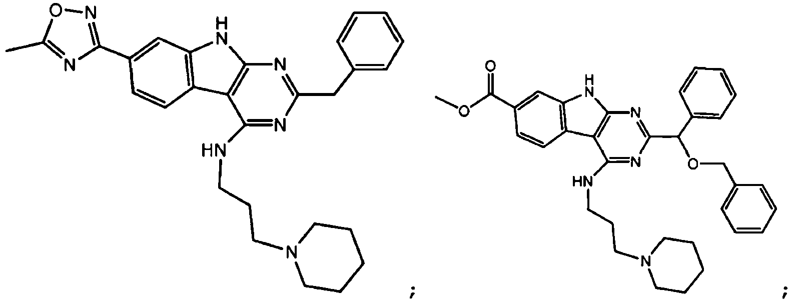 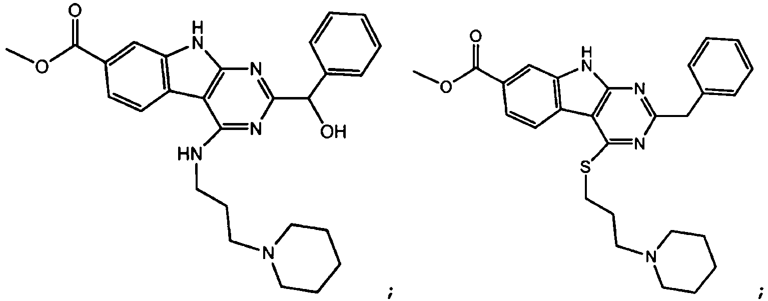 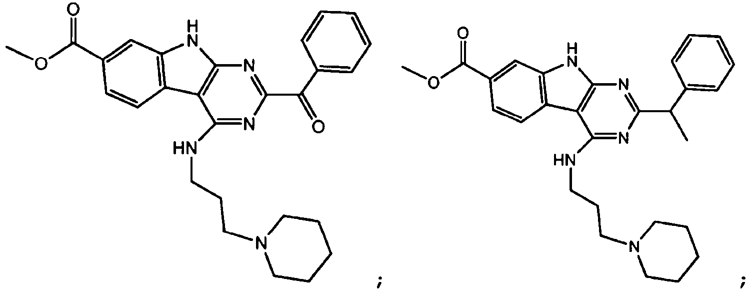 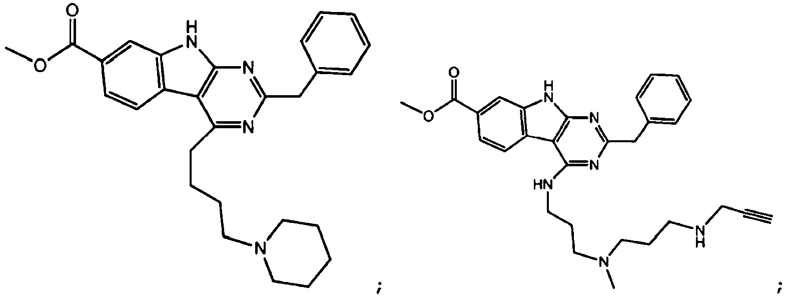 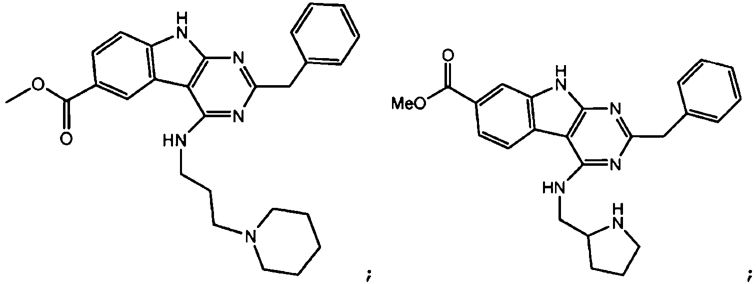 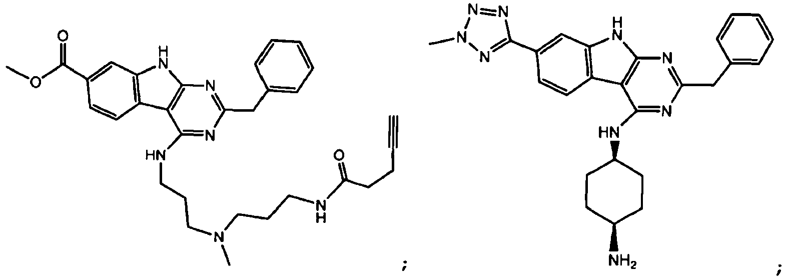 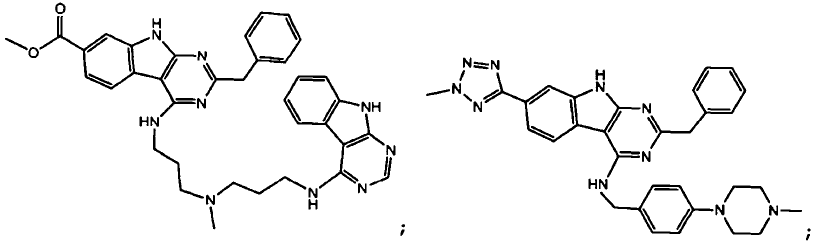 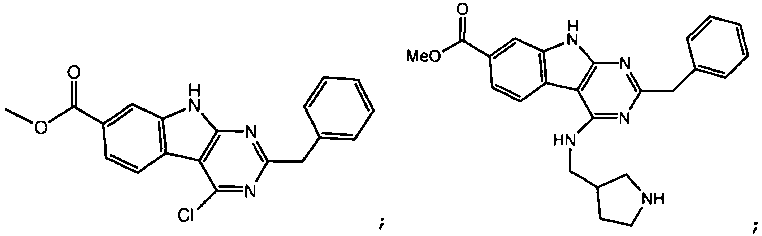 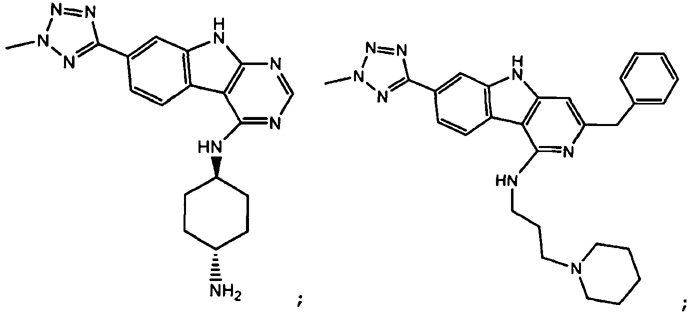 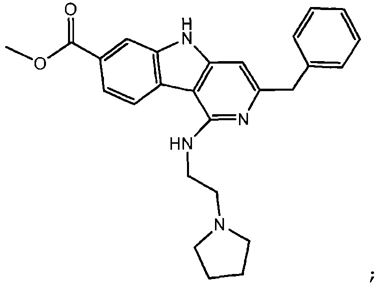 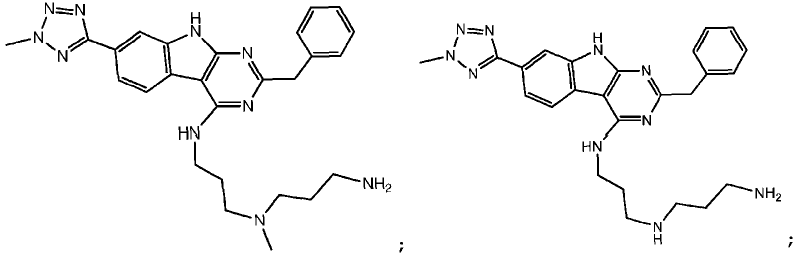 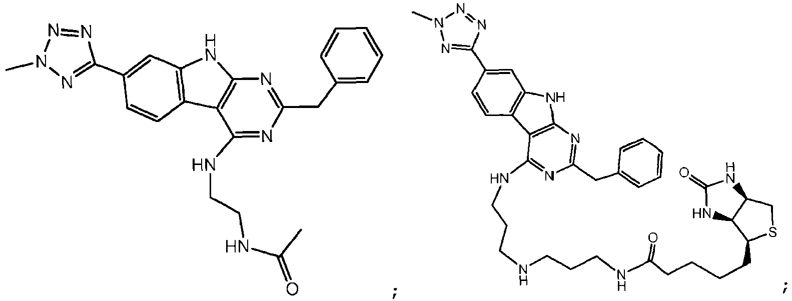 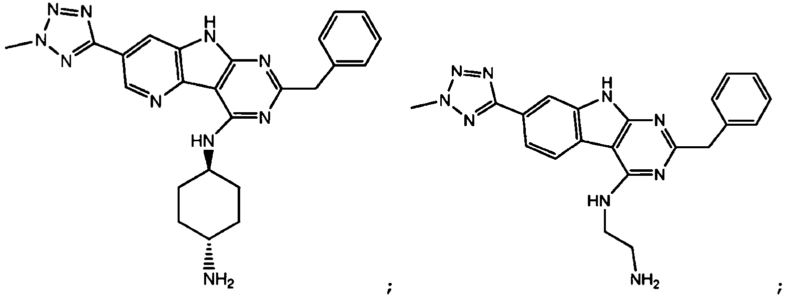 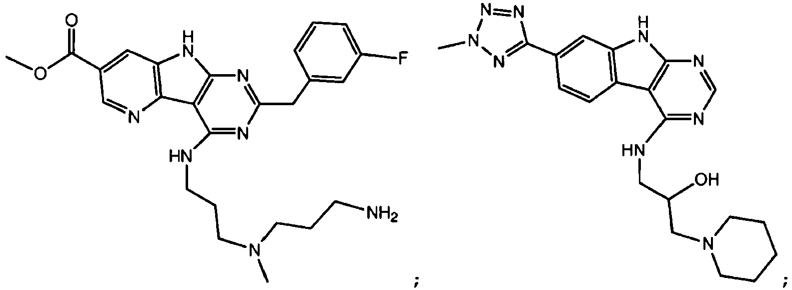 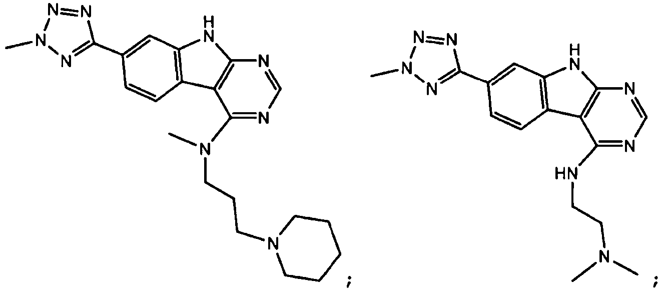 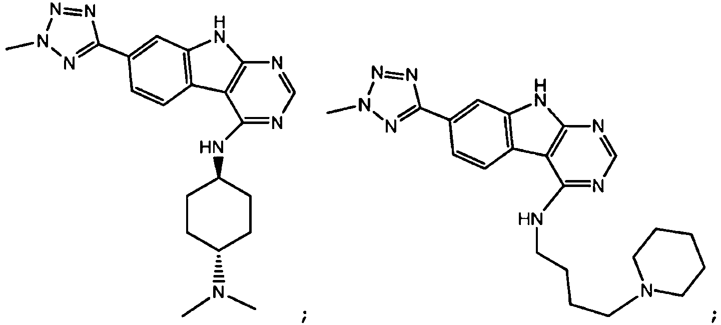 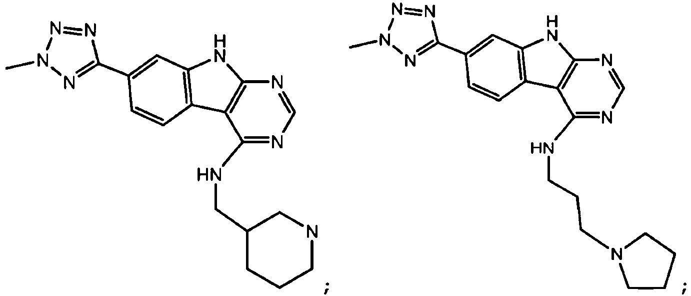 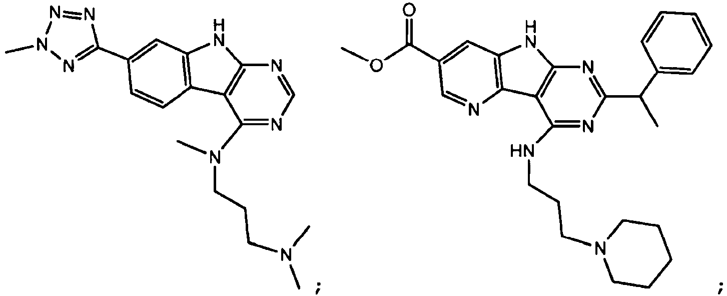 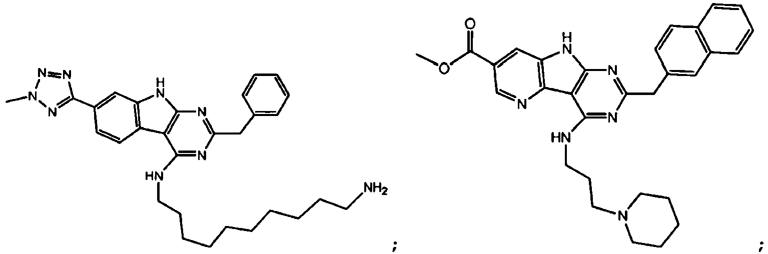 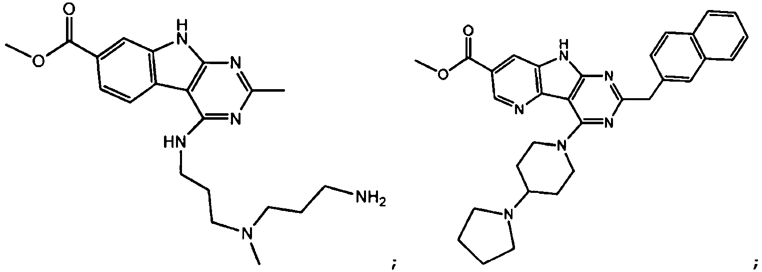 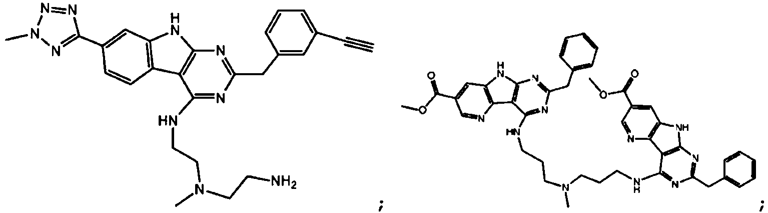 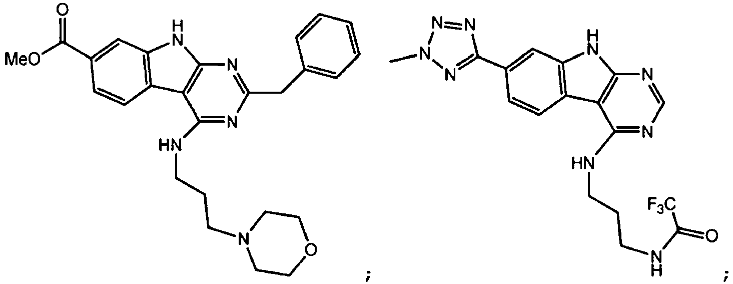 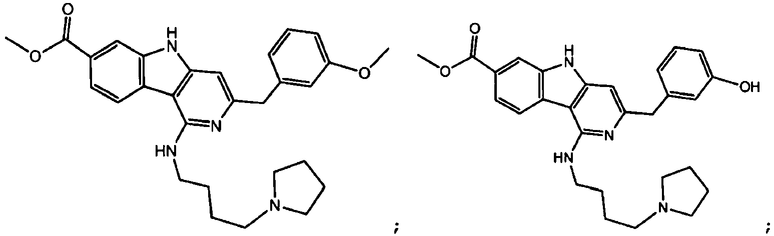 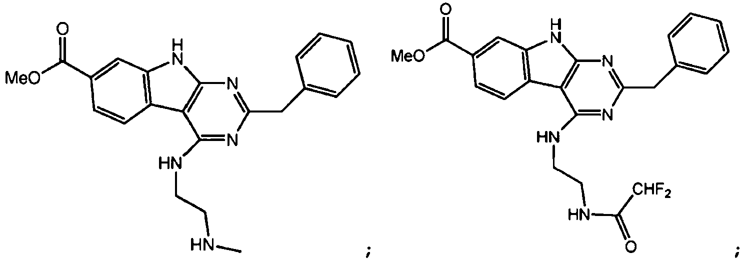 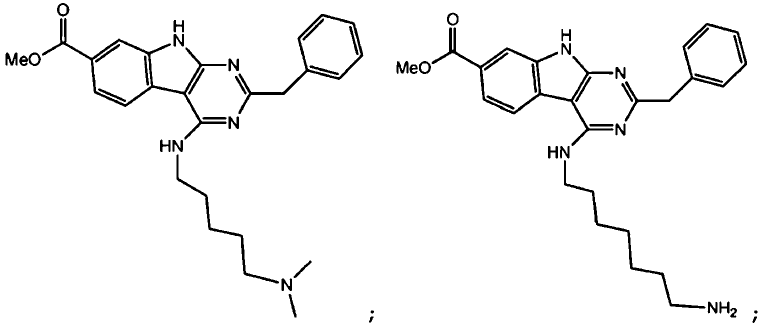 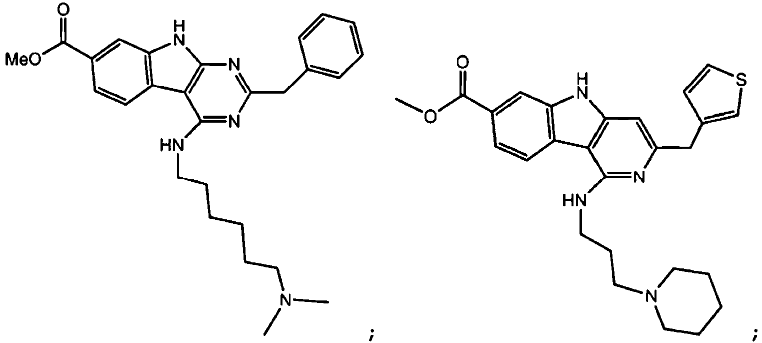 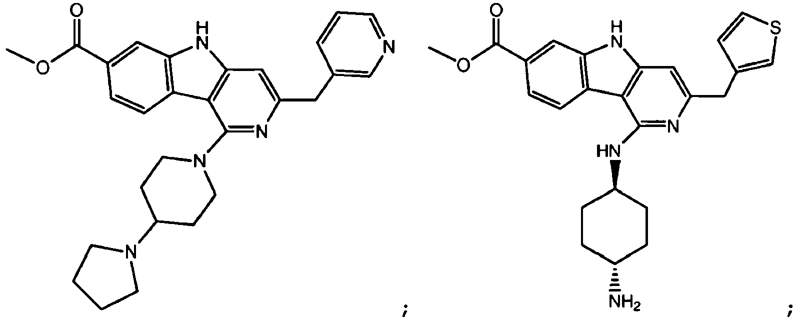 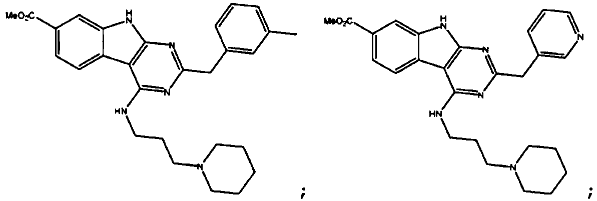 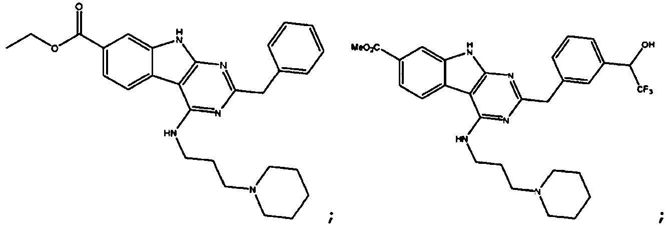 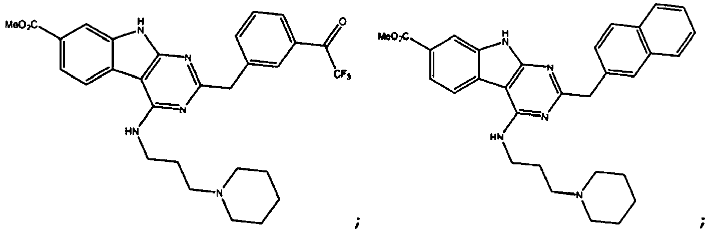 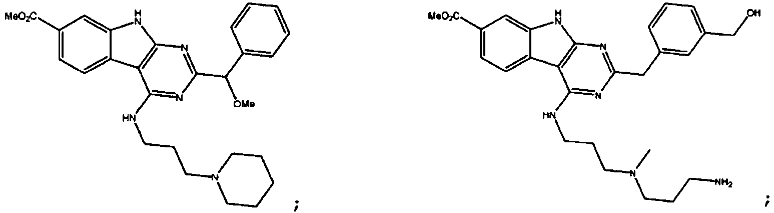 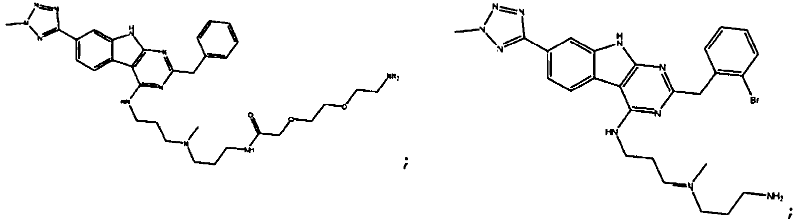 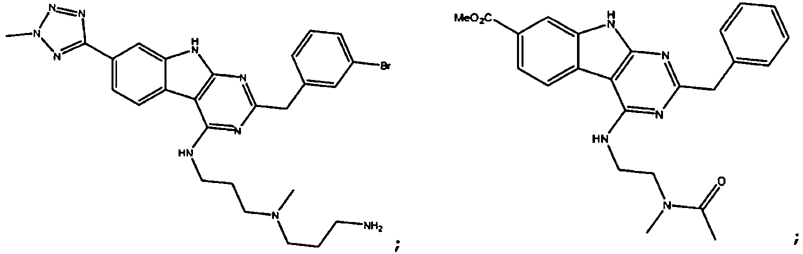 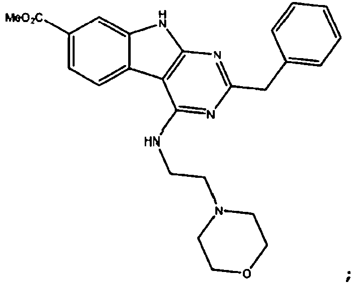 ou un sel de celui-ci, pour son utilisation dans le traitement du cancer chez un patient qui en a besoin.

12. Procédé pour inhiber la prolifération de cellules cancéreuses *ex vivo,* qui comprend l'administration du composé selon l'une quelconque des revendications 1 à 5 et 9 à 11 dans le milieu contenant les cellules en prolifération, dans lequel le composé a un effet antinéoplasique sur la prolifération des cellules cancéreuses.
